(19) Europäisches Patentamt
European Patent Office
Office européen des brevets

(11) **EP 4 541 280 A1**

(12) **EUROPEAN PATENT APPLICATION**
published in accordance with Art. 153(4) EPC


(43) Date of publication:
**23.04.2025 Bulletin 2025/17**

(21) Application number: **22946905.1**

(22) Date of filing: **17.06.2022**

(51) International Patent Classification (IPC):
***A61B 5/346*** *(2021.01)*

(52) Cooperative Patent Classification (CPC):
**A61B 5/346**

(86) International application number:
**PCT/JP2022/024370**

(87) International publication number:
**WO 2023/243090 (21.12.2023 Gazette 2023/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**



(71) Applicant: **NIPPON TELEGRAPH AND TELEPHONE CORPORATION**
**Chiyoda-ku, Tokyo 100-8116 (JP)**

(72) Inventor: **TSUKADA Shingo**
**Musashino-shi, Tokyo 180-8585 (JP)**

(74) Representative: **Brevalex**
**Tour Trinity**
**1 B Place de la Défense**
**92400 Courbevoie (FR)**


(54) **SIGNAL COMPOSITING DEVICE, SIGNAL COMPOSITING METHOD, AND PROGRAM**


(57) A signal synthesis device includes a waveform synthesis unit that obtains a synthesized waveform of an R wave represented by a function obtained by subtracting, from a function obtained by multiplying a function specified by a parameter value of a parameter specifying a first unimodal distribution by a parameter value of a first weight parameter, a function obtained by multiplying a function specified by a parameter value of a parameter specifying a second unimodal distribution by a parameter value of a second weight parameter, and adding a parameter value of a first level parameter, obtains a synthesized waveform of a T wave obtained by inverting the time axis of a waveform represented by a function obtained by subtracting, from a function obtained by multiplying a function specified by a parameter value of a parameter specifying a third unimodal distribution by a parameter value of a third weight parameter, a function obtained by multiplying a function specified by a parameter value of a parameter specifying a fourth unimodal distribution by a parameter value of a fourth weight parameter, and adding a parameter value of a second level parameter, and connects the synthesized waveform of the R wave and the synthesized waveform of the T wave together.


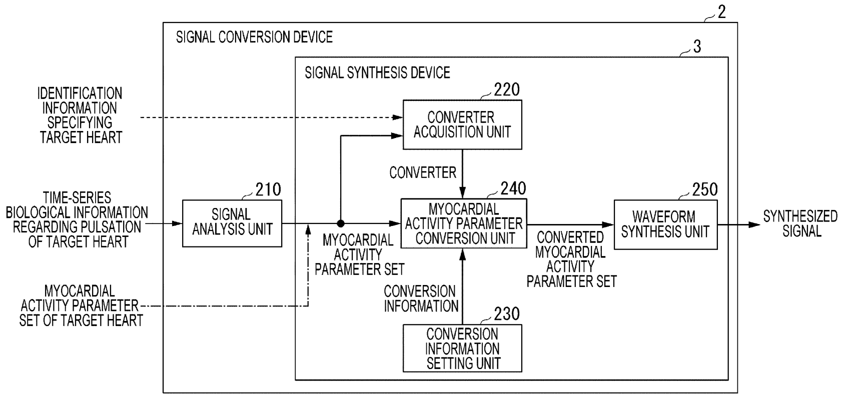


FIG. 60

EP 4 541 280 A1

## Description

Technical Field

The present invention relates to a signal synthesis apparatus, a signal synthesis method and a program.

Background Art

An electrocardiogram is useful information for grasping a state of a heart, and for example, if the electrocardiogram is used, whether a target is in a state in which there is a high possibility that heart failure occurs can be determined (Non Patent Document 1).

Citation List

Non Patent Document

Non Patent Document 1: Hiroshi Tanaka, "On the Inverse Solution of Electrocardiology", Medical Electronics and Biological Engineering, 1985, Vol. 23, No. 3, p. 147-158

Summary of Invention

Technical Problem

However, a waveform of an electrocardiogram acquired from a living body is not necessarily sufficient for grasping the state of the heart in some cases. For example, even if waveforms of electrocardiograms are similar to each other, manners of the onset of a disease related to the heart may be different from each other. As described above, it may be difficult to grasp the state of the heart depending on the disease only by looking at the waveform itself of the electrocardiogram acquired from the living body. For example, in a case of heart failure, for suppression of it, the onset can be suppressed by observing the state of the heart by using the waveform of the electrocardiogram in daily life. In order to further increase accuracy of suppressing the onset, it is conceivable to acquire other information by using another technique such as collecting blood, but it is not realistic to do that in daily life. For that reason, depending on the disease, the state of the heart must be grasped substantially on the basis of only the waveform of the electrocardiogram in some cases.

Furthermore, such circumstances are not limited to a case of grasping the state of the heart on the basis of the waveform of the electrocardiogram. Such circumstances are also common in a case of grasping the state of the heart on the basis of time-series biological information on one channel regarding pulsation of the heart acquired by a sensor in contact with a body surface, a sensor close to the body surface, a sensor inserted into a body, a sensor embedded in the body, or the like. Note that the time-series biological information regarding the pulsation of the heart is, for example, a waveform indicating a change in cardiac potential, a waveform indicating a change in pressure of the heart, a waveform indicating a change in blood flow rate, and a waveform indicating a change in heart sound. Note that the waveform of the electrocardiogram is also an example of the time-series biological information regarding the pulsation of the heart.

In view of the above circumstances, an object of the present invention is to provide a technique for obtaining useful information for grasping a state of a heart from time-series biological information on one channel regarding pulsation of the heart.

Solution to Problem

An aspect of the present invention is a signal synthesis device including a waveform synthesis unit that: sets a waveform in a time section of an R wave included in a waveform for one cycle indicating a cardiac cycle of a heart as a first target time waveform; sets a waveform in a time section of a T wave included in the waveform as a second target time waveform; sets a waveform obtained by inverting a time axis of the second target time waveform as a second target inverse time waveform; sets a set as a myocardial activity parameter set, the set including a value of a parameter specifying a first unimodal distribution or a value of a parameter specifying a first cumulative distribution function, a value of a parameter specifying a second unimodal distribution or a value of a parameter specifying a second cumulative distribution function, a value of a first weight parameter, a value of a second weight parameter, and a value of a first level parameter when the first target time waveform is approximated by a first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplying the second cumulative distribution function that is a cumulative distribution function of the second unimodal distribution by the value of the second weight parameter, and adding the value of the first level parameter, and a value of a parameter specifying a third unimodal distribution or a value of a parameter specifying a third cumulative distribution function, a value of a parameter specifying a fourth unimodal distribution or a value of a parameter specifying a fourth cumulative distribution function, a value of a third weight parameter, a value of a fourth weight parameter, and a value of a second level parameter when the second target inverse time waveform is approximated by a second approximate inverse time waveform that is a waveform represented by a function obtained by subtracting, from a function obtained by multiplying the third cumulative distribution function that is a cumulative distribution function of the third unimodal distribution by the value of the third weight parameter, a function obtained by multiplying the fourth cumulative distribution function that is a cumulative distribution function of the fourth unimodal distribution by the value of the fourth weight parameter, and adding the value of the second level parameter; sets each of element values included in the myocardial activity parameter set as a myocardial activity parameter value; uses each of myocardial activity parameter values included in the myocardial activity parameter set of a heart to be a target (The heart is referred to as a "target heart".); obtains, as a synthesized waveform in the time section of the R wave, a time waveform represented by a function obtained by subtracting, from a function obtained by multiplying a cumulative distribution function specified by the myocardial activity parameter value of the parameter specifying the first unimodal distribution or the first cumulative distribution function by the myocardial activity parameter value of the first weight parameter, a function obtained by multiplying a cumulative distribution function specified by the myocardial activity parameter value of the parameter specifying the second unimodal distribution or the second cumulative distribution function by the myocardial activity parameter value of the second weight parameter, and adding the myocardial activity parameter value of the first level parameter; obtains, as a synthesized waveform in the time section of the T wave, a waveform obtained by inverting a time axis of a waveform represented by a function obtained by subtracting, from a function obtained by multiplying a cumulative distribution function specified by the myocardial activity parameter value of the parameter specifying the third unimodal distribution or the third cumulative distribution function by the myocardial activity parameter value of the third weight parameter, a function obtained by multiplying a cumulative distribution function specified by the myocardial activity parameter value of the parameter specifying the fourth unimodal distribution or the fourth cumulative distribution function by the myocardial activity parameter value of the fourth weight parameter, and adding the myocardial activity parameter value of the second level parameter; and connects the synthesized waveform in the time section of the R wave and the synthesized waveform in the time section of the T wave together to obtain a synthesized signal for a waveform indicating the cardiac cycle of the target heart.

An aspect of the present invention is a signal synthesis device including a waveform synthesis unit that: sets a waveform in a time section of an R wave included in a waveform for one cycle indicating a cardiac cycle of a heart as a first target time waveform; sets a waveform in a time section of a T wave included in the waveform as a second target time waveform; sets a set as a myocardial activity parameter set, the set including a value of a parameter specifying a first unimodal distribution or a value of a parameter specifying a first cumulative distribution function, a value of a parameter specifying a second unimodal distribution or a value of a parameter specifying a second cumulative distribution function, a value of a first weight parameter, a value of a second weight parameter, and a value of a first level parameter when the first target time waveform is approximated by a first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplying the second cumulative distribution function that is a cumulative distribution function of the second unimodal distribution by the value of the second weight parameter, and adding the value of the first level parameter, and a value of a parameter specifying a third unimodal distribution or a value of a parameter specifying a third cumulative distribution function, a value of a parameter specifying a fourth unimodal distribution or a value of a parameter specifying a fourth cumulative distribution function, a value of a third weight parameter, a value of a fourth weight parameter, and a value of a second level parameter when the second target time waveform is approximated by a second approximate time waveform that is a time waveform represented by a function obtained by subtracting, from a function obtained by subtracting, from 1, a function obtained by multiplying the third cumulative distribution function that is a cumulative distribution function of the third unimodal distribution by the value of the third weight parameter, a function obtained by subtracting, from 1, a function obtained by multiplying the fourth cumulative distribution function that is a cumulative distribution function of the fourth unimodal distribution by the value of the fourth weight parameter, and adding the value of the second level parameter; sets each of element values included in the myocardial activity parameter set as a myocardial activity parameter value; uses each of myocardial activity parameter values included in the myocardial activity parameter set of a heart to be a target (The heart is referred to as a "target heart".); obtains, as a synthesized waveform in the time section of the R wave, a time waveform represented by a function obtained by subtracting, from a function obtained by multiplying a cumulative distribution function specified by the myocardial activity parameter value of the parameter specifying the first unimodal distribution or the first cumulative distribution function by the myocardial activity parameter value of the first weight parameter, a function obtained by multiplying a cumulative distribution function specified by the myocardial activity parameter value of the parameter specifying the second unimodal distribution or the second cumulative distribution function by the myocardial activity parameter value of the second weight parameter, and adding the myocardial activity parameter value of the first level parameter; obtains, as a synthesized waveform in the time section of the T wave, a time waveform represented by a function obtained by subtracting, from a function obtained by subtracting, from 1, a function obtained by multiplying a cumulative distribution function specified by the myocardial activity parameter value of the parameter specifying the third unimodal distribution or the third cumulative distribution function by the myocardial activity parameter value of the third weight parameter, a function obtained by subtracting, from 1, a function obtained by multiplying a cumulative distribution function specified by the myocardial activity parameter value of the parameter specifying the fourth unimodal distribution or the fourth cumulative distribution function by the myocardial activity parameter value of the fourth weight parameter, and adding the myocardial activity parameter value of the second level parameter; and connects the synthesized waveform in the time section of the R wave and the synthesized waveform in the time section of the T wave together to obtain a synthesized signal for a waveform indicating the cardiac cycle of the target heart.

An aspect of the present invention is a signal synthesis method executed by a signal synthesis device, the signal synthesis method including a waveform synthesis step of: setting a waveform in a time section of an R wave included in a waveform for one cycle indicating a cardiac cycle of a heart as a first target time waveform; setting a waveform in a time section of a T wave included in the waveform as a second target time waveform; setting a waveform obtained by inverting a time axis of the second target time waveform as a second target inverse time waveform; setting a set as a myocardial activity parameter set, the set including a value of a parameter specifying a first unimodal distribution or a value of a parameter specifying a first cumulative distribution function, a value of a parameter specifying a second unimodal distribution or a value of a parameter specifying a second cumulative distribution function, a value of a first weight parameter, a value of a second weight parameter, and a value of a first level parameter when the first target time waveform is approximated by a first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplying the second cumulative distribution function that is a cumulative distribution function of the second unimodal distribution by the value of the second weight parameter, and adding the value of the first level parameter, and a value of a parameter specifying a third unimodal distribution or a value of a parameter specifying a third cumulative distribution function, a value of a parameter specifying a fourth unimodal distribution or a value of a parameter specifying a fourth cumulative distribution function, a value of a third weight parameter, a value of a fourth weight parameter, and a value of a second level parameter when the second target inverse time waveform is approximated by a second approximate inverse time waveform that is a waveform represented by a function obtained by subtracting, from a function obtained by multiplying the third cumulative distribution function that is a cumulative distribution function of the third unimodal distribution by the value of the third weight parameter, a function obtained by multiplying the fourth cumulative distribution function that is a cumulative distribution function of the fourth unimodal distribution by the value of the fourth weight parameter, and adding the value of the second level parameter; setting each of element values included in the myocardial activity parameter set as a myocardial activity parameter value; using each of myocardial activity parameter values included in the myocardial activity parameter set of a heart to be a target (The heart is referred to as a "target heart".); obtaining, as a synthesized waveform in the time section of the R wave, a time waveform represented by a function obtained by subtracting, from a function obtained by multiplying a cumulative distribution function specified by the myocardial activity parameter value of the parameter specifying the first unimodal distribution or the first cumulative distribution function by the myocardial activity parameter value of the first weight parameter, a function obtained by multiplying a cumulative distribution function specified by the myocardial activity parameter value of the parameter specifying the second unimodal distribution or the second cumulative distribution function by the myocardial activity parameter value of the second weight parameter, and adding the myocardial activity parameter value of the first level parameter; obtaining, as a synthesized waveform in the time section of the T wave, a waveform obtained by inverting a time axis of a waveform represented by a function obtained by subtracting, from a function obtained by multiplying a cumulative distribution function specified by the myocardial activity parameter value of the parameter specifying the third unimodal distribution or the third cumulative distribution function by the myocardial activity parameter value of the third weight parameter, a function obtained by multiplying a cumulative distribution function specified by the myocardial activity parameter value of the parameter specifying the fourth unimodal distribution or the fourth cumulative distribution function by the myocardial activity parameter value of the fourth weight parameter, and adding the myocardial activity parameter value of the second level parameter; and connecting the synthesized waveform in the time section of the R wave and the synthesized waveform in the time section of the T wave together to obtain a synthesized signal for a waveform indicating the cardiac cycle of the target heart.

An aspect of the present invention is a signal synthesis method executed by a signal synthesis device, the signal synthesis method including a waveform synthesis step of: setting a waveform in a time section of an R wave included in a waveform for one cycle indicating a cardiac cycle of a heart as a first target time waveform; setting a waveform in a time section of a T wave included in the waveform as a second target time waveform; setting a set as a myocardial activity parameter set, the set including a value of a parameter specifying a first unimodal distribution or a value of a parameter specifying a first cumulative distribution function, a value of a parameter specifying a second unimodal distribution or a value of a parameter specifying a second cumulative distribution function, a value of a first weight parameter, a value of a second weight parameter, and a value of a first level parameter when the first target time waveform is approximated by a first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplying the second cumulative distribution function that is a cumulative distribution function of the second unimodal distribution by the value of the second weight parameter, and adding the value of the first level parameter, and a value of a parameter specifying a third unimodal distribution or a value of a parameter specifying a third cumulative distribution function, a value of a parameter specifying a fourth unimodal distribution or a value of a parameter specifying a fourth cumulative distribution function, a value of a third weight parameter, a value of a fourth weight parameter, and a value of a second level parameter when the second target time waveform is approximated by a second approximate time waveform that is a time waveform represented by a function obtained by subtracting, from a function obtained by subtracting, from 1, a function obtained by multiplying the third cumulative distribution function that is a cumulative distribution function of the third unimodal distribution by the value of the third weight parameter, a function obtained by subtracting, from 1, a function obtained by multiplying the fourth cumulative distribution function that is a cumulative distribution function of the fourth unimodal distribution by the value of the fourth weight parameter, and adding the value of the second level parameter; setting each of element values included in the myocardial activity parameter set as a myocardial activity parameter value; using each of myocardial activity parameter values included in the myocardial activity parameter set of a heart to be a target (The heart is referred to as a "target heart".); obtaining, as a synthesized waveform in the time section of the R wave, a time waveform represented by a function obtained by subtracting, from a function obtained by multiplying a cumulative distribution function specified by the myocardial activity parameter value of the parameter specifying the first unimodal distribution or the first cumulative distribution function by the myocardial activity parameter value of the first weight parameter, a function obtained by multiplying a cumulative distribution function specified by the myocardial activity parameter value of the parameter specifying the second unimodal distribution or the second cumulative distribution function by the myocardial activity parameter value of the second weight parameter, and adding the myocardial activity parameter value of the first level parameter; obtaining, as a synthesized waveform in the time section of the T wave, a time waveform represented by a function obtained by subtracting, from a function obtained by subtracting, from 1, a function obtained by multiplying a cumulative distribution function specified by the myocardial activity parameter value of the parameter specifying the third unimodal distribution or the third cumulative distribution function by the myocardial activity parameter value of the third weight parameter, a function obtained by subtracting, from 1, a function obtained by multiplying a cumulative distribution function specified by the myocardial activity parameter value of the parameter specifying the fourth unimodal distribution or the fourth cumulative distribution function by the myocardial activity parameter value of the fourth weight parameter, and adding the myocardial activity parameter value of the second level parameter; and connecting the synthesized waveform in the time section of the R wave and the synthesized waveform in the time section of the T wave together to obtain a synthesized signal for a waveform indicating the cardiac cycle of the target heart.

An aspect of the present invention is a program for causing a computer to function as the signal synthesis device described above.

## Advantageous Effects of Invention

According to the present invention, it is possible to provide a technique for obtaining useful information for grasping a state of a heart from time-series biological information on one channel regarding pulsation of the heart.

## Brief Description of Drawings

[Fig. 1] Fig. 1 is a diagram illustrating an example of a hardware configuration of a signal analysis device 1 of a first embodiment.

[Fig. 2] Fig. 2 is a diagram schematically illustrating a function obtained by multiplying a first cumulative distribution function by weight, a function obtained by multiplying a second cumulative distribution function by weight, and an approximate time waveform that is a weighted difference between the first cumulative distribution function and the second cumulative distribution function, for a first target time waveform.

[Fig. 3] Fig. 3 is a diagram schematically illustrating a function obtained by multiplying a third inverse cumulative distribution function by weight, a function obtained by multiplying a fourth inverse cumulative distribution function by weight, and an approximate time waveform that is a weighted difference between the third inverse cumulative distribution function and the fourth inverse cumulative distribution function, for a second target time waveform.

[Fig. 4] Fig. 4 is a diagram illustrating an example of results of fitting a waveform of an electrocardiogram of a target heart by four cumulative distribution functions in the first embodiment.

[Fig. 5] Fig. 5 is an explanatory diagram describing that a difference between two cumulative distribution functions in the first embodiment can be fitted to substantially the same waveform as a falling waveform of a T wave.

[Fig. 6] Fig. 6 is a diagram schematically illustrating a function obtained by multiplying the first cumulative distribution function by the weight and adding a level value, a function obtained by multiplying the second cumulative distribution function by the weight and adding the level value, and an approximate time waveform obtained by addition of the level value and the weighted difference between the first cumulative distribution function and the second cumulative distribution function, for the first target time waveform.

[Fig. 7] Fig. 7 is a diagram schematically illustrating a function obtained by multiplying the third inverse cumulative distribution function by the weight and adding a level value, a function obtained by multiplying the fourth inverse cumulative distribution function by the weight and adding the level value, and an approximate time waveform obtained by addition of the level value and the weighted difference between the third inverse cumulative distribution function and the fourth inverse cumulative distribution function, for the second target time waveform.

[Fig. 8] Fig. 8 is a diagram schematically illustrating a Δ wave included in a target time waveform.

[Fig. 9] Fig. 9 is a diagram illustrating an example of a functional configuration of a control unit 11 in the first embodiment.

[Fig. 10] Fig. 10 is a flowchart illustrating an example of a flow of processing executed by the signal analysis device 1 in the first embodiment.

[Fig. 11] Fig. 11 is a first diagram illustrating an example of analysis results by the signal analysis device 1 in the first embodiment.

[Fig. 12] Fig. 12 is a second diagram illustrating an example of analysis results by the signal analysis device 1 in the first embodiment.

[Fig. 13] Fig. 13 is a third diagram illustrating an example of analysis results by the signal analysis device 1 in the first embodiment.

[Fig. 14] Fig. 14 is a fourth diagram illustrating an example of analysis results by the signal analysis device 1 in the first embodiment.

[Fig. 15] Fig. 15 is a first explanatory diagram of an example in which an electrocardiogram of ventricular premature contractions is analyzed by the signal analysis device 1 of the first embodiment.

[Fig. 16] Fig. 16 is a second explanatory diagram of the example in which the electrocardiogram of the ventricular premature contractions is analyzed by the signal analysis device 1 of the first embodiment.

[Fig. 17] Fig. 17 is a third explanatory diagram of the example in which the electrocardiogram of the ventricular premature contractions is analyzed by the signal analysis device 1 of the first embodiment.

[Fig. 18] Fig. 18 is a first explanatory diagram in which an electrocardiogram of the target heart in a depolarization period of Brugada syndrome type 1 is analyzed by the signal analysis device 1 in the first embodiment.

[Fig. 19] Fig. 19 is a second explanatory diagram in which the electrocardiogram of the target heart in the depolarization period of Brugada syndrome type 1 is analyzed by the signal analysis device 1 in the first embodiment.

[Fig. 20] Fig. 20 is a third explanatory diagram in which the electrocardiogram of the target heart in the depolarization period of Brugada syndrome type 1 is analyzed by the signal analysis device 1 in the first embodiment.

[Fig. 21] Fig. 21 is a diagram illustrating a first example in which an electrocardiogram is analyzed by the signal analysis device 1 in the first embodiment.

[Fig. 22] Fig. 22 is a diagram illustrating a second example in which an electrocardiogram is analyzed by the signal analysis device 1 in the first embodiment.

[Fig. 23] Fig. 23 is a diagram illustrating a third example in which an electrocardiogram is analyzed by the signal analysis device 1 in the first embodiment.

[Fig. 24] Fig. 24 is a diagram illustrating a fourth example in which an electrocardiogram is analyzed by the signal analysis device 1 in the first embodiment.

[Fig. 25] Fig. 25 is a diagram illustrating a fifth example in which an electrocardiogram is analyzed by the signal analysis device 1 in the first embodiment.

[Fig. 26] Fig. 26 is a diagram illustrating a sixth example in which an electrocardiogram is analyzed by the signal analysis device 1 in the first embodiment.

[Fig. 27] Fig. 27 is a diagram illustrating a seventh example in which an electrocardiogram is analyzed by the signal analysis device 1 in the first embodiment.

[Fig. 28] Fig. 28 is a diagram illustrating an eighth example in which an electrocardiogram is analyzed by the signal analysis device 1 in the first embodiment.

[Fig. 29] Fig. 29 is a diagram illustrating a ninth example in which an electrocardiogram is analyzed by the signal analysis device 1 in the first embodiment.

[Fig. 30] Fig. 30 is a diagram illustrating a tenth example in which an electrocardiogram is analyzed by the signal analysis device 1 in the first embodiment.

[Fig. 31] Fig. 31 is a diagram illustrating an eleventh example in which an electrocardiogram is analyzed by the signal analysis device 1 in the first embodiment.

[Fig. 32] Fig. 32 is a diagram illustrating a twelfth example in which an electrocardiogram is analyzed by the signal analysis device 1 in the first embodiment.

[Fig. 33] Fig. 33 is a diagram illustrating a thirteenth example in which an electrocardiogram is analyzed by the signal analysis device 1 in the first embodiment.

[Fig. 34] Fig. 34 is a diagram illustrating a fourteenth example in which an electrocardiogram is analyzed by the signal analysis device 1 in the first embodiment.

[Fig. 35] Fig. 35 is a diagram illustrating a fifteenth example in which an electrocardiogram is analyzed by the signal analysis device 1 in the first embodiment.

[Fig. 36] Fig. 36 is a diagram illustrating a sixteenth example in which an electrocardiogram is analyzed by the signal analysis device 1 in the first embodiment.

[Fig. 37] Fig. 37 is a diagram illustrating a seventeenth example in which an electrocardiogram is analyzed by the signal analysis device 1 in the first embodiment.

[Fig. 38] Fig. 38 is a diagram illustrating an eighteenth example in which an electrocardiogram is analyzed by the signal analysis device 1 in the first embodiment.

[Fig. 39] Fig. 39 is a diagram illustrating a nineteenth example in which an electrocardiogram is analyzed by the signal analysis device 1 in the first embodiment.

[Fig. 40] Fig. 40 is a diagram illustrating a twentieth example in which an electrocardiogram is analyzed by the signal analysis device 1 in the first embodiment.

[Fig. 41] Fig. 41 is a diagram illustrating a twenty-first example in which an electrocardiogram is analyzed by the signal analysis device 1 in the first embodiment.

[Fig. 42] Fig. 42 is a diagram illustrating a twenty-second example in which an electrocardiogram is analyzed by the signal analysis device 1 in the first embodiment.

[Fig. 43] Fig. 43 is a diagram illustrating a twenty-third example in which an electrocardiogram is analyzed by the signal analysis device 1 in the first embodiment.

[Fig. 44] Fig. 44 is a diagram illustrating a twenty-fourth example in which an electrocardiogram is analyzed by the signal analysis device 1 in the first embodiment.

[Fig. 45] Fig. 45 is a diagram illustrating a twenty-fifth example in which an electrocardiogram is analyzed by the signal analysis device 1 in the first embodiment.

[Fig. 46] Fig. 46 is a diagram illustrating a twenty-sixth example in which an electrocardiogram is analyzed by the signal analysis device 1 in the first embodiment.

[Fig. 47] Fig. 47 is a diagram illustrating a twenty-seventh example in which an electrocardiogram is analyzed by the signal analysis device 1 in the first embodiment.

[Fig. 48] Fig. 48 is a diagram illustrating a twenty-eighth example in which an electrocardiogram is analyzed by the signal analysis device 1 in the first embodiment.

[Fig. 49] Fig. 49 is a diagram illustrating a twenty-ninth example in which an electrocardiogram is analyzed by the signal analysis device 1 in the first embodiment.

[Fig. 50] Fig. 50 is a diagram illustrating a thirtieth example in which an electrocardiogram is analyzed by the signal analysis device 1 in the first embodiment.

[Fig. 51] Fig. 51 is a diagram illustrating a thirty-first example in which an electrocardiogram is analyzed by the signal analysis device 1 in the first embodiment.

[Fig. 52] Fig. 52 is a diagram illustrating a thirty-second example in which an electrocardiogram is analyzed by the signal analysis device 1 in the first embodiment.

[Fig. 53] Fig. 53 is a diagram illustrating a thirty-third example in which an electrocardiogram is analyzed by the signal analysis device 1 in the first embodiment.

[Fig. 54] Fig. 54 is a diagram illustrating a thirty-fourth example in which an electrocardiogram is analyzed by the signal analysis device 1 in the first embodiment.

[Fig. 55] Fig. 55 is a diagram illustrating a thirty-fifth example in which an electrocardiogram is analyzed by the signal analysis device 1 in the first embodiment.

[Fig. 56] Fig. 56 is a diagram illustrating a thirty-sixth example in which an electrocardiogram is analyzed by the signal analysis device 1 in the first embodiment.

[Fig. 57] Fig. 57 is a diagram illustrating a thirty-seventh example in which an electrocardiogram is analyzed by the signal analysis device 1 in the first embodiment.

[Fig. 58] Fig. 58 is a diagram illustrating a thirty-eighth example in which an electrocardiogram is analyzed by the signal analysis device 1 in the first embodiment.

[Fig. 59] Fig. 59 is a diagram illustrating a thirty-ninth example in which an electrocardiogram is analyzed by the signal analysis device 1 in the first embodiment.

[Fig. 60] Fig. 60 is a diagram illustrating an example of functional configurations of a signal conversion device 2 and a signal synthesis device 3 in a second embodiment and Modifications 1 and 5 thereof.

[Fig. 61] Fig. 61 is a flowchart illustrating an example of a flow of processing executed by the signal conversion device 2 and the signal synthesis device 3 in the second embodiment and Modifications 1 and 5 thereof.

[Fig. 62] Fig. 62 is a diagram illustrating an example of functional configurations of the signal conversion device 2 and the signal synthesis device 3 in Modification 2, Modification 3, and Modification 5 of the second embodiment.

[Fig. 63] Fig. 63 is a flowchart illustrating an example of a flow of processing executed by the signal conversion device 2 and the signal synthesis device 3 in Modification 2, Modification 3, and Modification 5 of the second embodiment.

[Fig. 64] Fig. 64 is a diagram illustrating an example of functional configurations of the signal conversion device 2 and the signal synthesis device 3 in Modification 4 of the second embodiment.

[Fig. 65] Fig. 65 is a flowchart illustrating an example of a flow of processing executed by the signal conversion device 2 and the signal synthesis device 3 in Modification 4 of the second embodiment.

[Fig. 66] Fig. 66 is a diagram illustrating an example of functional configurations of the signal conversion device 2 and the signal synthesis device 3 in Modification 6 of the second embodiment.

[Fig. 67] Fig. 67 is a flowchart illustrating an example of a flow of processing executed by the signal conversion device 2 and the signal synthesis device 3 in Modification 6 of the second embodiment.

## Description of Embodiments

### <First Embodiment>

Fig. 1 is a diagram illustrating an example of a hardware configuration of a signal analysis device 1 of a first embodiment. Hereinafter, for simplicity of description, the signal analysis device 1 will be described with an example of a case where analysis is performed on the basis of a waveform of one channel of an electrocardiogram. However, the signal analysis device 1 can perform similar analysis on the basis of not only a waveform of an electrocardiogram but also time-series biological information regarding pulsation of a heart. Note that the time-series biological information regarding the pulsation of the heart is, for example, a waveform indicating a change in cardiac potential, a waveform indicating a change in pressure of the heart, a waveform indicating a change in blood flow rate, and a waveform indicating a change in heart sound. For that reason, the signal analysis device 1 may use not only a waveform of an electric signal acquired from a body surface but also any waveform as long as the waveform indicates a cardiac cycle acquired from any point regardless of whether the point is on the body surface or in the body, by using a sensor in contact with the body surface, a sensor close to the body surface, a sensor inserted into the body, a sensor embedded in the body, or the like.

That is, the signal analysis device 1 may use the waveform indicating the change in pressure of the heart as the time-series biological information regarding the pulsation of the heart instead of the waveform of the electrocardiogram. Furthermore, the signal analysis device 1 may use the waveform indicating the change in blood flow rate as the time-series biological information regarding the pulsation of the heart instead of the waveform of the electrocardiogram. Furthermore, the signal analysis device 1 may use the waveform indicating the change in heart sound as the time-series biological information regarding the pulsation of the heart instead of the waveform of the electrocardiogram. Note that the waveform of the electrocardiogram is also an example of the time-series biological information regarding the pulsation of the heart. Note that the time-series biological information regarding the pulsation of the heart may be time-series biological information regarding cyclical pulsation of the heart.

The signal analysis device 1 acquires a waveform of an electrocardiogram of a heart that is an analysis target (hereinafter, referred to as a "target heart"). On the basis of the acquired waveform of the electrocardiogram, the signal analysis device 1 acquires a parameter indicating activity of a myocardium of the target heart (hereinafter referred to as a "myocardial activity parameter") including at least one of a parameter indicating activity of an outer layer of the myocardium (hereinafter, referred to as a "myocardial outer layer") of the target heart (hereinafter, the parameter is referred to as a "myocardial outer layer parameter") or a parameter indicating activity of an inner layer of the myocardium (hereinafter, referred to as a "myocardial inner layer") of the target heart (hereinafter, the parameter is referred to as a "myocardial inner layer parameter").

Here, a relationship between activity of a myocardium and a waveform of an electrocardiogram will be described. In the medical field, a model for describing a relationship between movement of a myocardium and an electrocardiogram called an electromotive force dipole model (Reference Document 1) is known. According to the electromotive force dipole model, a myocardium is modeled using two layers of a myocardial outer layer and a myocardial inner layer.

Reference Document 1: Yoshifumi Tanaka, "Understanding electrocardiogram waveforms from their origins, Interpreting myocardial action potentials", Gakken Medical Shujunsha Co., Ltd. (2012)

In the electromotive force dipole model, the myocardial outer layer and the myocardial inner layer are modeled as different electromotive force generation sources. According to the electromotive force dipole model, a synthesized wave of an epicardial myocardial action potential and an endocardial myocardial action potential substantially corresponds to a temporal change in potential of a body surface observed on the body surface. A graph representing the temporal change in potential of the body surface is the waveform of the electrocardiogram. The epicardial myocardial action potential is a result of directly measuring a change in electromotive force generated by pulsation of the myocardial outer layer by inserting a catheter electrode. The endocardial myocardial action potential is a result of directly measuring a change in electromotive force generated by pulsation of the myocardial inner layer by inserting the catheter electrode. The above is a schematic description of the electromotive force dipole model.

Meanwhile, the myocardial outer layer in the electromotive force dipole model is a population of cells. For that reason, pulsation timings of cells in the myocardial outer layer in one-time pulsation of the myocardial outer layer are not necessarily the same in all the cells, and there is a possibility that there is a distribution in the pulsation timings. The same applies to the myocardial inner layer. That is, pulsation timings of cells in the myocardial inner layer in one-time pulsation of the myocardial inner layer are not necessarily the same in all the cells, and there is a possibility that there is a distribution in the pulsation timings. However, such a possibility that there is a distribution in pulsation timings of cells is not assumed in the electromotive force dipole model.

Furthermore, there is also a distribution in distances between each cell and an electrode on the body surface, and configurations of body tissue between each cell and the electrode on the body surface are not the same. For that reason, pieces of conversion efficiency at which excitation of a cell in the myocardial outer layer is reflected in the waveform of the electrocardiogram are not necessarily the same in all the cells, and there is a possibility that there is a distribution in pieces of conversion efficiency at which pulsation is reflected in the waveform of the electrocardiogram. Similarly, pieces of conversion efficiency at which excitation of a cell in the myocardial inner layer is reflected in the waveform of the electrocardiogram are not necessarily the same in all the cells, and there is a possibility that there is a distribution in pieces of conversion efficiency at which pulsation is reflected in the waveform of the electrocardiogram. However, such a possibility that there is a distribution in pieces of conversion efficiency at which pulsation of a cell is reflected in the waveform of the electrocardiogram is not assumed in the electromotive force dipole model.

In the signal analysis device 1, in consideration of the possibility that there is the distribution in the pulsation timings of cells and the possibility that there is the distribution in the pieces of conversion efficiency at which the pulsation of the cell is reflected in the waveform of the electrocardiogram, analysis is performed in which it is assumed that each of a distribution of timings at which the start of pulsation of each cell of the myocardial outer layer appears in the waveform of the electrocardiogram, a distribution of timings at which the start of pulsation of each cell of the myocardial inner layer appears in the waveform of the electrocardiogram, a distribution of timings at which the end of the pulsation of each cell of the myocardial outer layer appears in the waveform of the electrocardiogram, and a distribution of timings at which the end of the pulsation of each cell of the myocardial inner layer appears in the waveform of the electrocardiogram is a Gaussian distribution. That is, in the signal analysis device 1, analysis is performed in which it is assumed that the start of activity of the myocardial inner layer by all cells of the myocardial inner layer is included in the waveform of the electrocardiogram as a cumulative Gaussian distribution, the start of activity of the myocardial outer layer by all cells of the myocardial outer layer is included in the waveform of the electrocardiogram as a cumulative Gaussian distribution, the end of the activity of the myocardial inner layer by all the cells of the myocardial inner layer is included in the waveform of the electrocardiogram as a cumulative Gaussian distribution, and the end of the activity of the myocardial outer layer by all the cells of the myocardial outer layer is included in the waveform of the electrocardiogram as a cumulative Gaussian distribution.

Note that, although a cumulative Gaussian distribution function is preferably used in the signal analysis device 1, a sigmoid function, a Gompertz function, a logistic function, or the like may be used instead of the cumulative Gaussian distribution function. That is, the signal analysis device 1 may use, instead of the cumulative Gaussian distribution function, a cumulative distribution function of a unimodal distribution, that is, a cumulative distribution function corresponding to a distribution in which a value monotonically increases until time at which the value is a maximum value and monotonically decreases after the time at which the value is the maximum value. However, the cumulative distribution function used by the signal analysis device 1 needs to be a cumulative distribution function having a shape that can be specified by a parameter representing a shape of the cumulative distribution function or a parameter representing a shape of a unimodal distribution that is a cumulative source of the cumulative distribution function. Hereinafter, the parameter representing the shape of the cumulative distribution function (that is, a parameter specifying the cumulative distribution function) is referred to as a shape parameter of the cumulative distribution function, and the parameter representing the shape of the unimodal distribution (that is, a parameter specifying the unimodal distribution) is referred to as a shape parameter of the unimodal distribution. However, as a matter of course, the shape parameter of the cumulative distribution function and the shape parameter of the unimodal distribution are substantially the same. For example, in a case where the cumulative distribution function used by the signal analysis device 1 is a cumulative Gaussian distribution function, a standard deviation (or variance) and an average value of a Gaussian distribution that is a cumulative source of the cumulative Gaussian distribution function are shape parameters of the unimodal distribution and also shape parameters of the cumulative distribution function.

The signal analysis device 1 sets a waveform in a time section of any of an R wave and a T wave included in a waveform for one cycle of an acquired electrocardiogram of the target heart as a target time waveform, and acquires, as parameters representing characteristics of the target time waveform, that is, acquires, as myocardial activity parameters, a parameter specifying a first unimodal distribution or a parameter specifying a first cumulative distribution function, and a parameter specifying a second unimodal distribution or a parameter specifying a second cumulative distribution function when the target time waveform is approximated by a time waveform represented by a difference or a weighted difference between the first cumulative distribution function that is a cumulative distribution function of the first unimodal distribution and the second cumulative distribution function that is a cumulative distribution function of the second unimodal distribution (hereinafter, the time waveform is referred to as an "approximate time waveform"). Hereinafter, approximating the target time waveform represented by the approximate time waveform, that is, specifying the approximate time waveform is referred to as "fitting", and the first cumulative distribution function and the second cumulative distribution function included in the approximate time waveform are referred to as "fitting results". Note that, in a case where approximation is performed by the weighted difference, the signal analysis device 1 may also acquire, as parameters representing the characteristics of the target time waveform (that is, myocardial activity parameters), weight given to the first cumulative distribution function and weight given to the second cumulative distribution function, or may also acquire, as a parameter representing the characteristics of the target time waveform (that is, a myocardial activity parameter), a ratio between the weight given to the first cumulative distribution function and the weight given to the second cumulative distribution function.

In a case where the target time waveform is approximated by an approximate time waveform represented by a difference between the first cumulative distribution function and the second cumulative distribution function, for example, the signal analysis device 1 uses each of combinations ($M \times N$) of parameters specifying cumulative distribution functions for a respective plurality of (M) candidates for the first cumulative distribution function and parameters specifying cumulative distribution functions for a respective plurality of (N) candidates for the second cumulative distribution function, to generate a time waveform (hereinafter referred to as a "candidate time waveform") represented by the difference between the first cumulative distribution function and the second cumulative distribution function, specifies, as the approximate time waveform, a candidate time waveform closest to the target time waveform among generated $M \times N$ candidate time waveforms, and acquires, as parameters representing the characteristics of the target time waveform, a parameter specifying a candidate for the first cumulative distribution function and a parameter specifying a candidate for the second cumulative distribution function used for generation of the specified approximate time waveform. Processing of specifying the candidate time waveform closest to the target time waveform as the approximate time waveform only needs to be performed by, for example, processing of specifying the candidate time waveform having a smallest square error between the candidate time waveform and the target time waveform.

Alternatively, for example, the signal analysis device 1 repeats obtaining a candidate time waveform that is a time waveform represented by a difference between the candidate for the first cumulative distribution function and the candidate for the second cumulative distribution function and that approximates the target time waveform, and updating at least any of parameters specifying respective cumulative distribution functions in a direction in which the square error between the candidate time waveform and the target time waveform decreases, until the square error is less than or equal to a predetermined standard, or a predetermined number of times, to specify a finally obtained candidate time waveform as the approximate time waveform, and acquires, as parameters representing the characteristics of the target time waveform, a parameter specifying a candidate for the first cumulative distribution function and a parameter specifying a candidate for the second cumulative distribution function used for generation of the specified approximate time waveform.

In a case where the target time waveform is approximated by an approximate time waveform represented by a weighted difference between the first cumulative distribution function and the second cumulative distribution function, for example, the signal analysis device 1 uses each of combinations ($K \times L \times M \times N$) of parameters specifying cumulative distribution functions for a respective plurality of (M) candidates for the first cumulative distribution function, parameters specifying cumulative distribution functions for a respective plurality of (N) candidates for the second cumulative distribution function, a plurality of (K) candidates for the weight given to the first cumulative distribution function, and a plurality of (L) candidates for the weight given to the second cumulative distribution function, to generate a candidate time waveform that is a time waveform represented by the weighted difference between the first cumulative distribution function and the second cumulative distribution function, specifies, as the approximate time waveform, a candidate time waveform closest to the target time waveform among generated $K \times L \times M \times N$ candidate time waveforms, and acquires, as parameters representing the characteristics of the target time waveform, a parameter specifying a candidate for the first cumulative distribution function, a parameter specifying a candidate for the second cumulative distribution function, weight given to the first cumulative distribution function, and weight given to the second cumulative distribution function used for generation of the specified approximate time waveform.

Alternatively, for example, the signal analysis device 1 repeats obtaining a candidate time waveform that is a time waveform represented by a weighted difference between the candidate for the first cumulative distribution function and the candidate for the second cumulative distribution function and that approximates the target time waveform, and updating at least any of parameters specifying respective cumulative distribution functions and pieces of weight given to respective cumulative distribution functions in a direction in which the square error between the candidate time waveform and the target time waveform decreases, until the square error is less than or equal to a predetermined standard, or a predetermined number of times, to specify a finally obtained candidate time waveform as the approximate time waveform, and acquires, as parameters representing the characteristics of the target time waveform, a parameter specifying a candidate for the first cumulative distribution function, a parameter specifying a candidate for the second cumulative distribution function, weight given to the first cumulative distribution function, and weight given to the second cumulative distribution function used for generation of the specified approximate time waveform.

Hereinafter, processing of acquiring the parameters representing the characteristics of the target time waveform included in the waveform for one cycle of the acquired electrocardiogram of the target heart is referred to as myocardial activity information parameter acquisition processing.

When information representing time is x, in a case where Gaussian distributions are used as the first unimodal distribution and the second unimodal distribution, the first unimodal distribution is represented by following Formula (1), a first cumulative distribution function $f_1(x)$ is represented by Formula (2), the second unimodal distribution is represented by Formula (3), and a second cumulative distribution function $f_2(x)$ is represented by Formula (4).

[Math. 1]

$$\frac{1}{\sqrt{2\pi\sigma_1{}^2}} exp\left(-\frac{(x-\mu_1)^2}{2\sigma_1{}^2}\right) \quad \cdots (1)$$

[Math. 2]

$$f_1(x) = \frac{1}{2}\left(1 + erf\left(\frac{x-\mu_1}{\sqrt{2\sigma_1{}^2}}\right)\right) \quad \cdots (2)$$

[Math. 3]

$$\frac{1}{\sqrt{2\pi\sigma_2{}^2}} exp\left(-\frac{(x-\mu_2)^2}{2\sigma_2{}^2}\right) \quad \cdots (3)$$

[Math. 4]

$$f_2(x) = \frac{1}{2}\left(1 + erf\left(\frac{x-\mu_2}{\sqrt{2\sigma_2{}^2}}\right)\right) \quad \cdots (4)$$

Formula (1) is a Gaussian distribution (normal distribution) having an average of $\mu_1$ and a standard deviation of $\sigma_1$ (variance of $\sigma_1{}^2$). Formula (3) is a Gaussian distribution (normal distribution) having an average of $\mu_2$ and a standard deviation of $\sigma_2$ (variance of $\sigma_2{}^2$). Formula (2) is a cumulative distribution function of Formula (1). Formula (4) is a cumulative distribution function of Formula (3). A function "erf" is a sigmoid function (error function). The unit of the information x representing time is arbitrary, and for example, it is sufficient to use, as the information x representing time, a sample number or relative time with a waveform of one cycle of an electrocardiogram as the start edge.

The difference between the first cumulative distribution function and the second cumulative distribution function is expressed by, for example, following Formula (5). The function expressed by following Formula (5) is a function obtained by subtracting the second cumulative distribution function from the first cumulative distribution function.

[Math. 5]

$$f_1(x) - f_2(x) = \frac{1}{2}\left(1 + erf\left(\frac{x - \mu_1}{\sqrt{2\sigma_1{}^2}}\right)\right) - \frac{1}{2}\left(1 + erf\left(\frac{x - \mu_2}{\sqrt{2\sigma_2{}^2}}\right)\right) \quad \cdots (5)$$

That is, when the target time waveform is approximated by the approximate time waveform that is the difference between the first cumulative distribution function and the second cumulative distribution function, the average $\mu_1$ and the standard deviation $\sigma_1$ that are parameters specifying the first unimodal distribution or parameters specifying the first cumulative distribution function, and the average $\mu_2$ and the standard deviation $\sigma_2$ that are parameters specifying the second unimodal distribution or the second cumulative distribution function are acquired as parameters representing the characteristics of the target time waveform. Note that instead of acquiring a standard deviation as a parameter, a variance may be acquired as a parameter. The same applies to the following description of acquiring a standard deviation as a parameter.

The weighted difference between the first cumulative distribution function and the second cumulative distribution function is expressed by, for example, following Formula (6) with weight of the first cumulative distribution function as $k_1$ and weight of the second cumulative distribution function as $k_2$. The function expressed by following Formula (6) is a function obtained by subtracting, from a function obtained by multiplying the first cumulative distribution function by the weight $k_1$, a function obtained by multiplying the second cumulative distribution function by the weight $k_2$.

[Math. 6]

$$k_1 f_1(x) - k_2 f_2(x) = k_1 \frac{1}{2}\left(1 + erf\left(\frac{x - \mu_1}{\sqrt{2\sigma_1{}^2}}\right)\right) - k_2 \frac{1}{2}\left(1 + erf\left(\frac{x - \mu_2}{\sqrt{2\sigma_2{}^2}}\right)\right) \quad \cdots (6)$$

That is, when the target time waveform is approximated by the approximate time waveform that is the weighted difference between the first cumulative distribution function and the second cumulative distribution function, the average $\mu_1$ and the standard deviation $\sigma_1$ that are the parameters specifying the first unimodal distribution or the parameters specifying the first cumulative distribution function, and the average $\mu_2$ and the standard deviation $\sigma_2$ that are the parameters specifying the second unimodal distribution or the parameters specifying the second cumulative distribution function are at least acquired as parameters representing the characteristics of the target time waveform. Note that the weight $k_1$ of the first cumulative distribution function and the weight $k_2$ of the second cumulative distribution function or a ratio between the weight $k_1$ of the first cumulative distribution function and the weight $k_2$ of the second cumulative distribution function ($k_1/k_2$ or $k_2/k_1$) may also be acquired as parameters representing the characteristics of the target time waveform.

In consideration that the function of Formula (6) is the function obtained by subtracting, from the function obtained by multiplying the first cumulative distribution function by the weight $k_1$, the function obtained by multiplying the second cumulative distribution function by the weight $k_2$, both the weight $k_1$ and the weight $k_2$ are positive values. However, in a case where the target heart is in a peculiar state, a possibility cannot be denied that at least one of the weight $k_1$ or the weight $k_2$ obtained by fitting is not a positive value. Thus, the signal analysis device 1 may perform fitting such that both the weight $k_1$ and the weight $k_2$ have positive values, but it is not essential to perform the fitting such that both the weight $k_1$ and the weight $k_2$ have positive values.

Note that, the signal analysis device 1 preferably sets the R wave as the first target time waveform and the T wave as the second target time waveform among the R wave and the T wave included in the waveform for one cycle of the acquired electrocardiogram of the target heart, and acquires the parameters representing the characteristics of the target time waveform described above for each of the first target time waveform and the second target time waveform.

For example, in a case where the first target time waveform (that is, the R wave) is approximated by the difference between the first cumulative distribution function and the second cumulative distribution function, the first target time waveform is approximated by an approximate time waveform of Formula (9) that is a function obtained by subtracting, from a first cumulative distribution function $f_a(x)$ expressed by Formula (7), a second cumulative distribution function $f_b(x)$ expressed by Formula (8), and an average $\mu_a$ and a standard deviation $\sigma_a$ that are parameters specifying the first cumulative distribution function, and an average $\mu_b$ and a standard deviation $\sigma_b$ that are parameters specifying the second cumulative distribution function are acquired as parameters representing the characteristics of the first target time waveform (that is, the R wave).

[Math. 7]

$$f_a(x) = \frac{1}{2}\left(1 + erf\left(\frac{x-\mu_a}{\sqrt{2\sigma_a{}^2}}\right)\right) \quad \cdots (7)$$

[Math. 8]

$$f_b(x) = \frac{1}{2}\left(1 + erf\left(\frac{x-\mu_b}{\sqrt{2\sigma_b{}^2}}\right)\right) \quad \cdots (8)$$

[Math. 9]

$$f_a(x) - f_b(x) = \frac{1}{2}\left(1 + erf\left(\frac{x-\mu_a}{\sqrt{2\sigma_a{}^2}}\right)\right) - \frac{1}{2}\left(1 + erf\left(\frac{x-\mu_b}{\sqrt{2\sigma_b{}^2}}\right)\right) \quad \cdots (9)$$

For example, in a case where the first target time waveform (that is, the R wave) is approximated by the weighted difference between the first cumulative distribution function and the second cumulative distribution function, the first target time waveform is approximated by an approximate time waveform of Formula (10) that is a function obtained by subtracting, from a function obtained by multiplying the first cumulative distribution function $f_a(x)$ expressed by Formula (7) by weight $k_a$, a function obtained by multiplying the second cumulative distribution function $f_b(x)$ expressed by Formula (8) by weight $k_b$, and the average $\mu_a$ and the standard deviation $\sigma_a$ that are the parameters specifying the first cumulative distribution function, and the average $\mu_b$ and the standard deviation $\sigma_b$ that are the parameters specifying the second cumulative distribution function are at least acquired as parameters representing the characteristics of the first target time waveform (that is, the R wave). Note that the weight $k_a$ of the first cumulative distribution function and the weight $k_b$ of the second cumulative distribution function or a ratio between the weight $k_a$ of the first cumulative distribution function and the weight $k_b$ of the second cumulative distribution function ($k_a/k_b$ or $k_b/k_a$) may also be acquired as parameters representing the characteristics of the first target time waveform (that is, the R wave).

[Math. 10]

$$k_a f_a(x) - k_b f_b(x) = k_a \frac{1}{2}\left(1 + erf\left(\frac{x-\mu_a}{\sqrt{2\sigma_a{}^2}}\right)\right) - k_b \frac{1}{2}\left(1 + erf\left(\frac{x-\mu_b}{\sqrt{2\sigma_b{}^2}}\right)\right) \quad \cdots (10)$$

In consideration that the function of Formula (10) is the function obtained by subtracting, from the function obtained by multiplying the first cumulative distribution function by the weight $k_a$, the function obtained by multiplying the second cumulative distribution function by the weight $k_b$, both the weight $k_a$ and the weight $k_b$ are positive values. However, in a case where the target heart is in a peculiar state, a possibility cannot be denied that at least one of the weight $k_a$ or the weight $k_b$ obtained by fitting is not a positive value. Thus, the signal analysis device 1 may perform fitting such that both the weight $k_a$ and the weight $k_b$ have positive values, but it is not essential to perform the fitting such that both the weight $k_a$ and the weight $k_b$ have positive values.

Since the R wave corresponds to a fact that excitation of all cells of the myocardium starts sequentially in accordance with a Gaussian distribution, as for the R wave, the target time waveform in the forward direction of time only needs to be approximated by an approximate time waveform of a difference or a weighted difference between two cumulative Gaussian distributions as described above. On the other hand, in consideration that the T wave corresponds to a fact that the excitation of all the cells of the myocardium sequentially fades in accordance with a Gaussian distribution, the T wave can be interpreted as a phenomenon in a reverse direction to the R wave in the time axis. That is, as for the T wave, a waveform obtained by inverting the time axis of the target time waveform only needs to be approximated by a difference or a weighted difference of cumulative Gaussian distributions. Hereinafter, this is referred to as a first method. Furthermore, in consideration that the T wave corresponds to a fact that all the cells of the myocardium fade out a state of excitation in accordance with the Gaussian distribution, it can also be said that, as for the T wave, the target time waveform in the forward direction of time only needs to be approximated by a difference or a weighted difference between two functions (functions each obtained by subtracting a cumulative Gaussian distribution from 1). Hereinafter, this is referred to as a second method. Specific examples of the first method and the second method will be described below, but in order to avoid confusion between cumulative distribution functions for the R wave described above and cumulative distribution functions for the T wave described below, in the following, the description will be given by referring to the first cumulative distribution function described above as a third cumulative distribution function, and the second cumulative distribution function described above as a fourth cumulative distribution function, as for the T wave.

In a case where the second target time waveform (that is, the T wave) is approximated by a difference between the third cumulative distribution function and the fourth cumulative distribution function by using the first method, information representing time in the reverse direction is set as **x'** and a waveform obtained by inverting the time axis of the second target time waveform is referred to as a second target inverse time waveform, the second target inverse time waveform is approximated by an approximate inverse time waveform of Formula (13) that is a function obtained by subtracting, from a third cumulative distribution function $f_e(x')$ expressed by Formula (11), a fourth cumulative distribution function $f_g(x')$ expressed by Formula (12), and an average $\mu_e$ and a standard deviation $\sigma_e$ that are parameters specifying the third cumulative distribution function, and an average $\mu_g$ and a standard deviation $\sigma_g$ that are parameters specifying the fourth cumulative distribution function are acquired as parameters representing the characteristics of the second target time waveform (that is, the T wave).

[Math. 11]

$$f_e(x') = \frac{1}{2}\left(1 + erf\left(\frac{x' - \mu_e}{\sqrt{2\sigma_e{}^2}}\right)\right) \quad \cdots (11)$$

[Math. 12]

$$f_g(x') = \frac{1}{2}\left(1 + erf\left(\frac{x' - \mu_g}{\sqrt{2\sigma_g{}^2}}\right)\right) \quad \cdots (12)$$

[Math. 13]

$$f_e(x') - f_g(x') = \frac{1}{2}\left(1 + erf\left(\frac{x' - \mu_e}{\sqrt{2\sigma_e{}^2}}\right)\right) - \frac{1}{2}\left(1 + erf\left(\frac{x' - \mu_g}{\sqrt{2\sigma_g{}^2}}\right)\right) \quad \cdots (13)$$

For example, in a case where the second target time waveform (that is, the T wave) is approximated by a weighted difference between the third cumulative distribution function and the fourth cumulative distribution function by using the first method, the second target inverse time waveform is approximated by an approximate inverse time waveform of Formula (14) that is a function obtained by subtracting, from a function obtained by multiplying the third cumulative distribution function $f_e(x')$ expressed by Formula (11) by weight $k_e$, a function obtained by multiplying the fourth cumulative distribution function $f_g(x')$ expressed by Formula (12) by weight $k_g$, and the average $\mu_e$ and the standard deviation $\sigma_e$ that are the parameters specifying the third cumulative distribution function, and the average $\mu_g$ and the standard deviation $\sigma_g$ that are the parameters specifying the fourth cumulative distribution function are at least acquired as parameters representing the characteristics of the second target time waveform (that is, the T wave). Note that the weight $k_e$ of the third cumulative distribution function and the weight $k_g$ of the fourth cumulative distribution function or a ratio between the weight $k_e$ of the third cumulative distribution function and the weight $k_g$ of the fourth cumulative distribution function ($k_e/k_g$ or $k_g/k_e$) may also be acquired as parameters representing the characteristics of the second target time waveform (that is, the T wave).

[Math. 14]

$$k_e f_e(x') - k_g f_g(x') = k_e\frac{1}{2}\left(1 + erf\left(\frac{x' - \mu_e}{\sqrt{2\sigma_e{}^2}}\right)\right) - k_g\frac{1}{2}\left(1 + erf\left(\frac{x' - \mu_g}{\sqrt{2\sigma_g{}^2}}\right)\right) \quad \cdots (14)$$

In consideration that the function of Formula (14) is the function obtained by subtracting, from the function obtained by multiplying the third cumulative distribution function by the weight $k_e$, the function obtained by multiplying the fourth cumulative distribution function by the weight $k_g$, both the weight $k_e$ and the weight $k_g$ are positive values. However, in a case where the target heart is in a peculiar state, a possibility cannot be denied that at least one of the weight $k_e$ or the weight $k_g$ obtained by fitting is not a positive value. Thus, the signal analysis device 1 may perform fitting such that both the weight $k_e$ and the weight $k_g$ have positive values, but it is not essential to perform the fitting such that both the weight $k_e$ and the weight $k_g$ have positive values.

For example, in a case where the second target time waveform (that is, the T wave) is approximated by a difference between a function $f'_c(x)$ (hereinafter, referred to as a "third inverse cumulative distribution function") obtained by subtracting, from 1, a third cumulative distribution function $f_c(x)$ expressed by Formula (15), and a function $f'_d(x)$ (hereinafter, the function is referred to as a "fourth inverse cumulative distribution function") obtained by subtracting, from 1, a fourth cumulative distribution function $f_d(x)$ expressed by Formula (16), by using the second method, the second target time waveform is approximated by an approximate time waveform of Formula (17) that is a function obtained by subtracting, from the third inverse cumulative distribution function $f'_c(x)$, the fourth inverse cumulative distribution function $f'_d(x)$, and an average $\mu_c$ and a standard deviation $\sigma_c$ that are parameters specifying the third cumulative distribution function, and an average $\mu_d$ and a standard deviation $\sigma_d$ that are parameters specifying the fourth cumulative distribution function are acquired as parameters representing the characteristics of the second target time waveform (that is, the T wave).
[Math. 15]

$$f_c(x) = \frac{1}{2}\left(1 + erf\left(\frac{x - \mu_c}{\sqrt{2\sigma_c{}^2}}\right)\right) \quad \cdots (15)$$

[Math. 16]

$$f_d(x) = \frac{1}{2}\left(1 + erf\left(\frac{x - \mu_d}{\sqrt{2\sigma_d{}^2}}\right)\right) \quad \cdots (16)$$

[Math. 17]

$$f'_c(x) - f'_d(x) = \left(1 - f_c(x)\right) - \left(1 - f_d(x)\right)$$
$$= f_d(x) - f_c(x) = \frac{1}{2}\left(1 + erf\left(\frac{x - \mu_d}{\sqrt{2\sigma_d{}^2}}\right)\right) - \frac{1}{2}\left(1 + erf\left(\frac{x - \mu_c}{\sqrt{2\sigma_c{}^2}}\right)\right) \quad \cdots (17)$$

For example, in a case where the second target time waveform (that is, the T wave) is approximated by a weighted difference between the third inverse cumulative distribution function and the fourth inverse cumulative distribution function by using the second method, the second target time waveform is approximated by an approximate time waveform of Formula (18) that is a function obtained by subtracting, from a function obtained by multiplying the third inverse cumulative distribution function $f'_c(x)$ by weight $k_c$, a function obtained by multiplying the fourth inverse cumulative distribution function $f'_d(x)$ by weight $k_d$, and the average $\mu_c$ and the standard deviation $\sigma_c$ that are the parameters specifying the third cumulative distribution function, and the average $\mu_d$ and the standard deviation $\sigma_d$ that are the parameters specifying the fourth cumulative distribution function are acquired as parameters representing the characteristics of the second target time waveform (that is, the T wave). Note that the weight $k_c$ of the third inverse cumulative distribution function and the weight $k_d$ of the fourth inverse cumulative distribution function or a ratio between the weight $k_c$ of the third inverse cumulative distribution function and the weight $k_d$ of the fourth inverse cumulative distribution function ($k_c/k_d$ or $k_d/k_c$) may also be acquired as parameters representing the characteristics of the second target time waveform (that is, the T wave).
[Math. 18]

$$k_c f'_c(x) - k_d f'_d(x)$$
$$= k_c\left(1 - f_c(x)\right) - k_d\left(1 - f_d(x)\right) = k_d f_d(x) - k_c f_c(x) + k_c - k_d$$
$$= k_d \frac{1}{2}\left(1 + erf\left(\frac{x - \mu_d}{\sqrt{2\sigma_d{}^2}}\right)\right) - k_c \frac{1}{2}\left(1 + erf\left(\frac{x - \mu_c}{\sqrt{2\sigma_c{}^2}}\right)\right) + k_c - k_d \quad \cdots (18)$$

In consideration that the function of Formula (18) is the function obtained by subtracting, from the function obtained by multiplying the third inverse cumulative distribution function by the weight $k_c$, the function obtained by multiplying the fourth inverse cumulative distribution function by the weight $k_d$, both the weight $k_c$ and the weight $k_d$ are positive values. However, in a case where the target heart is in a peculiar state, a possibility cannot be denied that at least one of the weight $k_c$ or the weight $k_d$ obtained by fitting is not a positive value. Thus, the signal analysis device 1 may perform fitting such that both the weight $k_c$ and the weight $k_d$ have positive values, but it is not essential to perform the fitting such that both the weight $k_c$ and the weight $k_d$ have positive values.

Note that the approximate time waveform of Formula (17) is a function obtained by subtracting, from the fourth cumulative distribution function $f_d(x)$, the third cumulative distribution function $f_c(x)$, and thus, is a difference between the third cumulative distribution function $f_c(x)$ and the fourth cumulative distribution function $f_d(x)$. Furthermore, the approximate time waveform of Formula (18) is obtained by adding a constant term to a weighted difference between the third cumulative distribution function $f_c(x)$ and the fourth cumulative distribution function $f_d(x)$, and a shape of a curved portion is the same as the weighted difference between the third cumulative distribution function $f_c(x)$ and the fourth cumulative distribution function $f_d(x)$. Furthermore, as described above, the T wave can be interpreted as a phenomenon in the reverse direction to the R wave in the time axis, and as for the T wave, the waveform obtained by inverting the time axis of the target time waveform can be approximated by a difference or a weighted difference between a third cumulative distribution function $f_e(x)$ and a fourth cumulative distribution function $f_g(x)$. For these reasons, in the following description of the T wave, a cumulative distribution function and an inverse cumulative distribution function are not described together, and only the cumulative distribution function may be simply used.

Fig. 2 is a diagram schematically illustrating a function $k_af_a(x)$ obtained by multiplying the first cumulative distribution function $f_a(x)$ by the weight $k_a$, a function $k_bf_b(x)$ obtained by multiplying the second cumulative distribution function $f_b(x)$ by the weight $k_b$, and an approximate time waveform $k_af_a(x) - k_bf_b(x)$ that is the weighted difference between the first cumulative distribution function and the second cumulative distribution function, for the first target time waveform (that is, the R wave). A one-dot chain line is the function $k_af_a(x)$ obtained by multiplying the first cumulative distribution function $f_a(x)$ by the weight $k_a$, a two-dot chain line is the function $k_bf_b(x)$ obtained by multiplying the second cumulative distribution function $f_b(x)$ by the weight $k_b$, and a broken line is the approximate time waveform $k_af_a(x) - k_bf_b(x)$. The approximate time waveform $k_af_a(x) - k_bf_b(x)$ is a waveform approximating the first target time waveform (that is, the R wave).

Fig. 3 is a diagram schematically illustrating a function $k_cf'_c(x)$ obtained by multiplying the third inverse cumulative distribution function $f'_c(x) = 1 - f_c(x)$ by the weight $k_c$, a function $k_df'_d(x)$ obtained by multiplying the fourth inverse cumulative distribution function $f'_d(x) = 1 - f_d(x)$ by the weight $k_d$, and an approximate time waveform $k_cf'_c(x) - k_df'_d(x)$ that is the weighted difference between the third inverse cumulative distribution function and the fourth inverse cumulative distribution function, for the second target time waveform (that is, the T wave). A one-dot chain line is the function $k_cf'_c(x)$ obtained by multiplying the third inverse cumulative distribution function $f'_c(x) = 1 - f_c(x)$ by the weight $k_c$, a two-dot chain line is the function $k_df'_d(x)$ obtained by multiplying the fourth inverse cumulative distribution function $f'_d(x) = 1 - f_d(x)$ by the weight $k_d$, and a broken line is the approximate time waveform $k_cf'_c(x) - k_df'_d(x)$. The approximate time waveform $k_cf'_c(x) - k_df'_d(x)$ is a waveform approximating the second target time waveform (that is, the T wave).

Fig. 4 is a diagram schematically illustrating results of fitting each of the first target time waveform and the second target time waveform represented by a difference between two cumulative distribution functions, with the R wave included in the waveform for one cycle of the electrocardiogram of the target heart in the first embodiment as the first target time waveform and the T wave as the second target time waveform. In Fig. 4, the horizontal axis represents time and the vertical axis represents a potential. Units of both the horizontal axis and the vertical axis are all arbitrary units.

Specifically, Fig. 4 illustrates an example in which the first target time waveform (that is, the R wave) is fitted by the difference between the first cumulative distribution function and the second cumulative distribution function, and the second target time waveform (that is, the T wave) is fitted by the difference between the third cumulative distribution function and the fourth cumulative distribution function. A domain of the first cumulative distribution function and a domain of the second cumulative distribution function are the same, and are from time T1 to time T3, which is the time section of the first target time waveform (that is, the R wave). A domain of the third cumulative distribution function and a domain of the fourth cumulative distribution function are the same, and are from time T4 to time T6.

A "first fitting result" and a "second fitting result" in Fig. 4 are results of fitting to the R wave. A "third fitting result" and a "fourth fitting result" in Fig. 4 are results of fitting to the T wave.

In Fig. 4, the "first fitting result" indicates the first cumulative distribution function among the results of fitting to the first target time waveform (that is, the R wave) of the electrocardiogram. In Fig. 4, the "second fitting result" indicates the second cumulative distribution function among the results of fitting to the first target time waveform (that is, the R wave) of the electrocardiogram. In Fig. 4, the "third fitting result" indicates the third cumulative distribution function among the results of fitting to the second target time waveform (that is, the T wave) of the electrocardiogram. In Fig. 4, the "fourth fitting result" indicates the fourth cumulative distribution function among the results of fitting to the second target time waveform (that is, the T wave) of the electrocardiogram. In Fig. 4, "potential of body surface" represents a waveform of the electrocardiogram to be fitted.

Note that the signal analysis device 1 does not perform fitting for a period from the time T3 to the time T4 that does not belong to the time section of the first target time waveform (that is, the R wave) or the time section of the second target time waveform (that is, the T wave). Note that, for the period in which fitting is not performed in the signal analysis device 1, in Fig. 4, expression is performed by a line connecting the first fitting result at the time T3 to the third fitting result at the time T4, and a line connecting the second fitting result at the time T3 to the fourth fitting result at the time T4. That is, in a case where the signal analysis device 1 displays the fitting results, as illustrated in Fig. 4, the signal analysis device 1 only needs to display lines respectively connecting the first fitting result at the time T3 and the third fitting result at the time T4 together and the second fitting result at the time T3 and the fourth fitting result at the time T4 together by predetermined functions such as constant functions or linear functions.

Note that in a case where the signal analysis device 1 displays the fitting results, a value of the weight may be corrected so that the first fitting result at the time T3 and the third fitting result at the time T4 can be displayed using the same value. That is, although the actual first fitting result at the time T3 is $k_a f_a(T3)$ and the actual third fitting result at the time T4 is $k_c f'_c(T4)$, $\alpha_1$ that satisfies $k_a f_a(T3) = \alpha_1 k_c f'_c(T4)$ may be obtained and the fitting results may be displayed using $\alpha_1 k_c$ instead of the weight $k_c$, or the fitting results may be displayed using $k_a/\alpha_1$ instead of the weight $k_a$. Similarly, in a case where the signal analysis device 1 displays the fitting results, a value of the weight may be corrected so that the second fitting result at the time T3 and the fourth fitting result at the time T4 can be displayed using the same value. That is, although the second fitting result at the time T3 is $k_b f_b(T3)$ and the fourth fitting result at the time T4 is $k_d f'_d(T4)$, $\alpha_2$ that satisfies $k_b f_b(T3) = \alpha_2 k_d f'_d(T4)$ is obtained and the fitting results may be displayed using $\alpha_2 k_d$ instead of the weight $k_d$, or the fitting results may be displayed using $k_b/\alpha_2$ instead of the weight $k_b$.

Note that fitting to the first target time waveform and fitting to the second target time waveform may not be executed individually. That is, fitting to the first target time waveform and the second target time waveform may be collectively executed as fitting to both the first target time waveform and the second target time waveform. For example, in a case where the fitting to the first target time waveform and the second target time waveform is collectively performed, the signal analysis device 1 preferably executes fitting in consideration of reducing a difference between the first fitting result at the time T3 and the third fitting result at the time T4 and reducing a difference between the second fitting result at the time T3 and the fourth fitting result at the time T4.

Fig. 5 is an explanatory diagram describing that it is possible to visualize typical characteristics of the T wave by approximating the T wave by a function obtained by subtracting the fourth inverse cumulative distribution function from the third inverse cumulative distribution function, and displaying the third inverse cumulative distribution function and the fourth inverse cumulative distribution function or displaying the parameters specifying the respective cumulative distribution functions. Fig. 5 illustrates four images of an image G1, an image G2, an image G3, and an image G4. Each of the images G1 to G4 illustrates a graph in which the horizontal axis represents time and the vertical axis represents a potential. Units of the horizontal axis and the vertical axis of each of the images G1 to G4 in Fig. 5 are all arbitrary units.

A "first function" in Fig. 5 is an example of the third inverse cumulative distribution function. A "second function" in Fig. 5 is an example of the fourth inverse cumulative distribution function. A "third function" in Fig. 5 represents a function obtained by subtracting the "second function" from the "first function", that is, a function obtained by subtracting the fourth inverse cumulative distribution function from the third inverse cumulative distribution function. The "third function" in Fig. 5 has substantially the same shape as a shape of the T wave of a normal heart.

A "fourth function" in Fig. 5 is an example of the third inverse cumulative distribution function. A "fifth function" in Fig. 5 is an example of the fourth inverse cumulative distribution function. A "sixth function" in Fig. 5 represents a function obtained by subtracting the "fifth function" from the "fourth function", that is, a function obtained by subtracting the fourth inverse cumulative distribution function from the third inverse cumulative distribution function. The "sixth function" in Fig. 5 has substantially the same shape as a shape of decrease in height of the T wave of one of three typical abnormal patterns of the T wave. An interval between a falling portion of the third inverse cumulative distribution function and a falling portion of the fourth inverse cumulative distribution function in the image G2 in Fig. 5 is narrower than an interval between a falling portion of the third inverse cumulative distribution function and a falling portion of the fourth inverse cumulative distribution function in the image G1 of the normal heart, whereby it is visualized that delay of the activity of the myocardial outer layer from the activity of the myocardial inner layer is small in the decrease in height of the T wave.

A "seventh function" in Fig. 5 is an example of the third inverse cumulative distribution function. An "eighth function" in Fig. 5 is an example of the fourth inverse cumulative distribution function. A "ninth function" in Fig. 5 represents a function obtained by subtracting the "eighth function" from the "seventh function", that is, a function obtained by subtracting the fourth inverse cumulative distribution function from the third inverse cumulative distribution function. The "ninth function" in Fig. 5 has substantially the same shape as a shape of increase in height of the T wave of one of the three typical abnormal patterns of the T wave. An interval between a falling portion of the third inverse cumulative distribution function and a falling portion of the fourth inverse cumulative distribution function in the image G3 in Fig. 5 is wider than the interval between the falling portion of the third inverse cumulative distribution function and the falling portion of the fourth inverse cumulative distribution function in the image G1 of the normal heart, whereby it is visualized that delay of activity of the myocardial outer layer from activity of the myocardial inner layer is large in the increase in height of the T wave.

A "tenth function" in Fig. 5 is an example of the third inverse cumulative distribution function. An "eleventh function" in Fig. 5 is an example of the fourth inverse cumulative distribution function. A "twelfth function" in Fig. 5 represents a function obtained by subtracting the "eleventh function" from the "tenth function", that is, a function obtained by subtracting the fourth inverse cumulative distribution function from the third inverse cumulative distribution function. The "twelfth function" in Fig. 5 has substantially the same shape as a shape of a negative T wave of one of the three typical abnormal patterns of the T wave. The order of a falling portion of the third inverse cumulative distribution function and a falling portion of the fourth inverse cumulative distribution function in the image G4 in Fig. 5 is opposite to the order of the falling portion of the third inverse cumulative distribution function and the falling portion of the fourth inverse cumulative distribution function in the image G1 of the normal heart, whereby it is visualized that the myocardial outer layer ends activity earlier than the myocardial inner layer in the negative T wave.

The function obtained by subtracting the fourth inverse cumulative distribution function from the third inverse cumulative distribution function can express waves having different widths in the vertical axis direction and in the horizontal axis direction, such as the "third function", the "sixth function", and the "ninth function". Furthermore, the function obtained by subtracting the fourth inverse cumulative distribution function from the third inverse cumulative distribution function can express a negative wave such as the "twelfth function". That is, the T wave is approximated by the function obtained by subtracting the fourth inverse cumulative distribution function from the third inverse cumulative distribution function, or the T wave is approximated by a function obtained by inverting the time axis and subtracting the fourth cumulative distribution function from the third cumulative distribution function, whereby it is possible to express activity of each of the myocardial inner layer and the myocardial outer layer included in the T wave and a relationship between the activity of the myocardial inner layer and the activity of the myocardial outer layer. The same applies to a case where the R wave is approximated by the function obtained by subtracting the second cumulative distribution function from the first cumulative distribution function.

As described above, the signal analysis device 1 performs fitting for a waveform of the R wave or the T wave of the electrocardiogram of the target heart by a difference or a weighted difference between two cumulative distribution functions. Then, the signal analysis device 1 acquires a parameter specifying the approximate time waveform specified by fitting as the parameter indicating the activity of the myocardium.

[Approximation by Addition of Value and Difference or Weighted Difference between Two Cumulative Distribution Functions]

The signal analysis device 1 may set, as the target time waveform, the waveform in the time section of any of the R wave or the T wave included in the waveform for one cycle of the acquired electrocardiogram of the target heart, and set, as the approximate time waveform, a time waveform represented by addition of a value (hereinafter, the value is referred to as a "level value") and the difference or the weighted difference between the first cumulative distribution function that is a cumulative distribution function of the first unimodal distribution and the second cumulative distribution function that is a cumulative distribution function of the second unimodal distribution. In this case, in addition to the parameter specifying the first unimodal distribution or the parameter specifying the first cumulative distribution function and the parameter specifying the second unimodal distribution or the parameter specifying the second cumulative distribution function when the target time waveform is approximated by the approximate time waveform, the level value is also acquired as a parameter representing the characteristics of the target time waveform. Of course, in a case where approximation is performed by the weighted difference, the weight given to the first cumulative distribution function and the weight given to the second cumulative distribution function may also be acquired as parameters representing the characteristics of the target time waveform, or the ratio between the weight given to the first cumulative distribution function and the weight given to the second cumulative distribution function may also be acquired as the parameter representing the characteristics of the target time waveform.

In a case where the weighted difference is used, for example, the signal analysis device 1 uses each of combinations ($J \times K \times L \times M \times N$) of parameters specifying cumulative distribution functions for a respective plurality of (M) candidates for the first cumulative distribution function, parameters specifying cumulative distribution functions for a respective plurality of (N) candidates for the second cumulative distribution function, a plurality of (K) candidates for the weight given to the first cumulative distribution function, a plurality of (L) candidates for the weight given to the second cumulative distribution function, and a plurality of (J) candidates for the level value, to generate a candidate time waveform that is a time waveform represented by addition of the level value and the weighted difference between the first cumulative distribution function and the second cumulative distribution function, specifies, as the approximate time waveform, a candidate time waveform closest to the target time waveform among generated $J \times K \times L \times M \times N$ candidate time waveforms, and acquires, as parameters representing the characteristics of the target time waveform, a parameter specifying a candidate for the first cumulative distribution function, a parameter specifying a candidate for the second cumulative distribution function, weight given to the first cumulative distribution function, weight given to the second cumulative distribution function, and a level value used for generation of the specified approximate time waveform.

Alternatively, for example, the signal analysis device 1 repeats obtaining a candidate time waveform that is a time waveform represented by addition of a level value and a weighted difference between the candidate for the first cumulative distribution function and the candidate for the second cumulative distribution function and that approximates the target time waveform, and updating at least any of parameters specifying respective cumulative distribution functions, pieces of weight given to respective cumulative distribution functions, and the level value in a direction in which the square error between the candidate time waveform and the target time waveform decreases, until the square error is less than or equal to a predetermined standard, or a predetermined number of times, to specify a finally obtained candidate time waveform as the approximate time waveform, and acquires, as parameters representing the characteristics of the target time waveform, a parameter specifying a candidate for the first cumulative distribution function, a parameter specifying a candidate for the second cumulative distribution function, weight given to the first cumulative distribution function, weight given to the second cumulative distribution function, and a level value used for generation of the specified approximate time waveform.

Note that the level value may be determined before fitting. In this case, the signal analysis device 1 first acquires a potential at the start edge (corresponding to the time T1 in Fig. 4) of the target time waveform as the level value in a case where the target time waveform is the R wave, and acquires a potential at the end edge (corresponding to the time T6 in Fig. 4) of the target time waveform as the level value in a case where the target time waveform is the T wave. Then, the signal analysis device 1 uses each of combinations ($K \times L \times M \times N$) of parameters specifying cumulative distribution functions for a respective plurality of (M) candidates for the first cumulative distribution function, parameters specifying cumulative distribution functions for a respective plurality of (N) candidates for the second cumulative distribution function, a plurality of (K) candidates for the weight given to the first cumulative distribution function, and a plurality of (L) candidates for the weight given to the second cumulative distribution function, to generate a candidate time waveform that is a time waveform represented by addition of the level value and the weighted difference between the first cumulative distribution function and the second cumulative distribution function, specifies, as the approximate time waveform, a candidate time waveform closest to the target time waveform among generated $K \times L \times M \times N$ candidate time waveforms, and acquires, as parameters representing the characteristics of the target time waveform, a parameter specifying a candidate for the first cumulative distribution function, a parameter specifying a candidate for the second cumulative distribution function, weight given to the first cumulative distribution function, and weight given to the second cumulative distribution function used for generation of the specified approximate time waveform, and the level value determined in the first processing.

Alternatively, for example, the signal analysis device 1 first acquires a potential at the start edge (corresponding to the time T1 in Fig. 4) of the target time waveform as the level value in a case where the target time waveform is the R wave, and acquires a potential at the end edge (corresponding to the time T6 in Fig. 4) of the target time waveform as the level value in a case where the target time waveform is the T wave. Then, the signal analysis device 1 repeats obtaining a candidate time waveform that is a time waveform represented by addition of a level value and a weighted difference between the candidate for the first cumulative distribution function and the candidate for the second cumulative distribution function and that approximates the target time waveform, and updating at least any of parameters specifying respective cumulative distribution functions and pieces of weight given to respective cumulative distribution functions in a direction in which the square error between the candidate time waveform and the target time waveform decreases, until the square error is less than or equal to a predetermined standard, or a predetermined number of times, to specify a finally obtained candidate time waveform as the approximate time waveform, and acquires, as parameters representing the characteristics of the target time waveform, a parameter specifying a candidate for the first cumulative distribution function, a parameter specifying a candidate for the second cumulative distribution function, weight given to the first cumulative distribution function, and weight given to the second cumulative distribution function used for generation of the specified approximate time waveform, and the level value determined in the first processing.

When the level value is $\beta$, addition of the level value and the weighted difference between the first cumulative distribution function and the second cumulative distribution function is expressed by, for example, following Formula (19). The function expressed by following Formula (19) is a function obtained by subtracting, from a function obtained by multiplying the first cumulative distribution function by the weight $k_1$, a function obtained by multiplying the second cumulative distribution function by the weight $k_2$, and adding the level value $\beta$.

[Math. 19]

$$k_1 f_1(x) - k_2 f_2(x) + \beta = k_1 \frac{1}{2}\left(1 + erf\left(\frac{x-\mu_1}{\sqrt{2\sigma_1^2}}\right)\right) - k_2 \frac{1}{2}\left(1 + erf\left(\frac{x-\mu_2}{\sqrt{2\sigma_2^2}}\right)\right) + \beta \quad \cdots (19)$$

When the target time waveform is approximated by an approximate time waveform of Formula (19) obtained by adding the level value to the weighted difference between the first cumulative distribution function and the second cumulative distribution function, the average $\mu_1$ and the standard deviation $\sigma_1$ that are the parameters specifying the first unimodal distribution or the parameters specifying the first cumulative distribution function, the average $\mu_2$ and the standard deviation $\sigma_2$ that are the parameters specifying the second unimodal distribution or the parameters specifying the second cumulative distribution function, and the level value $\beta$ are at least acquired as parameters representing the characteristics of the target time waveform. Note that the weight $k_1$ of the first cumulative distribution function and the weight $k_2$ of the second cumulative distribution function or a ratio between the weight $k_1$ of the first cumulative distribution function and the weight $k_2$ of the second cumulative distribution function ($k_1/k_2$ or $k_2/k_1$) may also be acquired as parameters representing the characteristics of the target time waveform.

In consideration that the function of Formula (19) is the function obtained by subtracting, from the function obtained by multiplying the first cumulative distribution function by the weight $k_1$, the function obtained by multiplying the second cumulative distribution function by the weight $k_2$, and adding the level value $\beta$, both the weight $k_1$ and the weight $k_2$ are positive values. However, in a case where the target heart is in a peculiar state, a possibility cannot be denied that at least one of the weight $k_1$ or the weight $k_2$ obtained by fitting is not a positive value. Thus, the signal analysis device 1 may perform fitting such that both the weight $k_1$ and the weight $k_2$ have positive values, but it is not essential to perform the fitting such that both the weight $k_1$ and the weight $k_2$ have positive values.

The signal analysis device 1 preferably sets the R wave as the first target time waveform and the T wave as the second target time waveform among the R wave and the T wave included in the waveform for one cycle of the acquired electrocardiogram of the target heart, and acquires the parameters representing the characteristics of the target time waveform described above for each of the first target time waveform and the second target time waveform.

For example, in a case where the first target time waveform (that is, the R wave) is approximated by the function obtained by adding the level value to the weighted difference between the first cumulative distribution function and the second cumulative distribution function, a potential at the start edge of the first target time waveform is set as a level value $\beta_R$, the first target time waveform is approximated by an approximate time waveform of Formula (20) that is a function obtained by subtracting, from the function obtained by multiplying the first cumulative distribution function $f_a(x)$ expressed by Formula (7) by the weight $k_a$, the function obtained by multiplying the second cumulative distribution function $f_b(x)$ expressed by Formula (8) by the weight $k_b$, and adding the level value $\beta_R$, and the average $\mu_a$ and the standard deviation $\sigma_a$ that are the parameters specifying the first cumulative distribution function, the average $\mu_b$ and the standard deviation $\sigma_b$ that are the parameters specifying the second cumulative distribution function, and the level value $\beta_R$ are at least acquired as parameters representing the characteristics of the first target time waveform (that is, the R wave). Note that the weight $k_a$ of the first cumulative distribution function and the weight $k_b$ of the second cumulative distribution function or a ratio between the weight $k_a$ of the first cumulative distribution function and the weight $k_b$ of the second cumulative distribution function ($k_a/k_b$ or $k_b/k_a$) may also be acquired as parameters representing the characteristics of the first target time waveform (that is, the R wave).

[Math. 20]

$$k_a f_a(x) - k_b f_b(x) + \beta_R = k_a \frac{1}{2}\left(1 + erf\left(\frac{x-\mu_a}{\sqrt{2\sigma_a^2}}\right)\right) - k_b \frac{1}{2}\left(1 + erf\left(\frac{x-\mu_b}{\sqrt{2\sigma_b^2}}\right)\right) + \beta_R \quad \cdots (20)$$

In consideration that the function of Formula (20) is the function obtained by subtracting, from the function obtained by multiplying the first cumulative distribution function by the weight $k_a$, the function obtained by multiplying the second cumulative distribution function by the weight $k_b$, and adding the level value $\beta_R$, both the weight $k_a$ and the weight $k_b$ are positive values. However, in a case where the target heart is in a peculiar state, a possibility cannot be denied that at least one of the weight $k_a$ or the weight $k_b$ obtained by fitting is not a positive value. Thus, the signal analysis device 1 may perform fitting such that both the weight $k_a$ and the weight $k_b$ have positive values, but it is not essential to perform the fitting such that both the weight $k_a$ and the weight $k_b$ have positive values.

For example, in a case where the second target inverse time waveform that is the waveform obtained by inverting the time axis of the second target time waveform (that is, the T wave) is approximated by a function obtained by adding the level value to the weighted difference between the third cumulative distribution function and the fourth cumulative distribution function, a potential at the end edge of the second target time waveform is set as a level value $\beta_T$, the second target inverse time waveform is approximated by an approximate inverse time waveform of Formula (21) that is a function obtained by subtracting, from the function obtained by multiplying the third cumulative distribution function $f_e(x')$ expressed by Formula (11) by the weight $k_e$, the function obtained by multiplying the fourth cumulative distribution function $f_g(x')$ expressed by Formula (12) by the weight $k_g$, and adding the level value $\beta_T$, and the average $\mu_e$ and the standard deviation $\sigma_e$ that are the parameters specifying the third cumulative distribution function, the average $\mu_g$ and the standard deviation $\sigma_g$ that are the parameters specifying the fourth cumulative distribution function, and the level value $\beta_T$ are at least acquired as parameters representing the characteristics of the second target time waveform (that is, the T wave). Note that the weight $k_e$ of the third cumulative distribution function and the weight $k_g$ of the fourth cumulative distribution function or the ratio between the weight $k_e$ of the third cumulative distribution function and the weight $k_g$ of the fourth cumulative distribution function ($k_e/k_g$ or $k_g/k_e$) may also be acquired as parameters representing the characteristics of the second target time waveform (that is, the T wave).

[Math. 21]

$$k_e f_e(x') - k_g f_g(x') + \beta_T$$

$$= k_e \frac{1}{2}\left(1 + erf\left(\frac{x' - \mu_e}{\sqrt{2\sigma_e{}^2}}\right)\right) - k_g \frac{1}{2}\left(1 + erf\left(\frac{x' - \mu_g}{\sqrt{2\sigma_g{}^2}}\right)\right) + \beta_T \quad \cdots (21)$$

In consideration that the function of Formula (21) is the function obtained by subtracting, from the function obtained by multiplying the third cumulative distribution function by the weight $k_e$, the function obtained by multiplying the fourth cumulative distribution function by the weight $k_g$, and adding the level value $\beta_T$, both the weight $k_e$ and the weight $k_g$ are positive values. However, in a case where the target heart is in a peculiar state, a possibility cannot be denied that at least one of the weight $k_e$ or the weight $k_g$ obtained by fitting is not a positive value. Thus, the signal analysis device 1 may perform fitting such that both the weight $k_e$ and the weight $k_g$ have positive values, but it is not essential to perform the fitting such that both the weight $k_e$ and the weight $k_g$ have positive values.

For example, in a case where the second target time waveform (that is, the T wave) is approximated by a function obtained by adding the level value to a weighted difference between a function obtained by subtracting the third cumulative distribution function from 1 (that is, the third inverse cumulative distribution function) and a function obtained by subtracting the fourth cumulative distribution function from 1 (that is, the fourth inverse cumulative distribution function), the potential at the end edge of the second target time waveform is set as the level value $\beta_T$, the second target time waveform is approximated by an approximate time waveform of Formula (22) that is a function obtained by subtracting, from the function obtained by multiplying the third inverse cumulative distribution function $f'_c(x)$ by the weight $k_c$, the function obtained by multiplying the fourth inverse cumulative distribution function $f'_d(x)$ by the weight $k_d$, and adding the level value $\beta_T$, and the average $\mu_c$ and the standard deviation $\sigma_c$ that are the parameters specifying the third cumulative distribution function, the average $\mu_d$ and the standard deviation $\sigma_d$ that are the parameters specifying the fourth cumulative distribution function, and the level value $\beta_T$ are acquired as parameters representing the characteristics of the second target time waveform (that is, the T wave). Note that the weight $k_c$ of the third inverse cumulative distribution function and the weight $k_d$ of the fourth inverse cumulative distribution function or a ratio between the weight $k_c$ of the third inverse cumulative distribution function and the weight $k_d$ of the fourth inverse cumulative distribution function ($k_c/k_d$ or $k_d/k_c$) may also be acquired as parameters representing the characteristics of the second target time waveform (that is, the T wave).
[Math. 22]

$$k_c f'_c(x) - k_d f'_d(x) + \beta_T = k_c\left(1 - f_c(x)\right) - k_d\left(1 - f_d(x)\right) + \beta_T$$
$$= k_d f_d(x) - k_c f_c(x) + k_c - k_d + \beta_T$$
$$= k_d \frac{1}{2}\left(1 + erf\left(\frac{x - \mu_d}{\sqrt{2\sigma_d^2}}\right)\right) - k_c \frac{1}{2}\left(1 + erf\left(\frac{x - \mu_c}{\sqrt{2\sigma_c^2}}\right)\right) + k_c - k_d + \beta_T$$

・・・(22)

In consideration that the function of Formula (22) is the function obtained by subtracting, from the function obtained by multiplying the third inverse cumulative distribution function by the weight $k_c$, the function obtained by multiplying the fourth inverse cumulative distribution function by the weight $k_d$, and adding the level value $\beta_T$, both the weight $k_c$ and the weight $k_d$ are positive values. However, in a case where the target heart is in a peculiar state, a possibility cannot be denied that at least one of the weight $k_c$ or the weight $k_d$ obtained by fitting is not a positive value. Thus, the signal analysis device 1 may perform fitting such that both the weight $k_c$ and the weight $k_d$ have positive values, but it is not essential to perform the fitting such that both the weight $k_c$ and the weight $k_d$ have positive values.

Fig. 6 is a diagram schematically illustrating a function $k_af_a(x) + \beta_R$ obtained by multiplying the first cumulative distribution function $f_a(x)$ by the weight $k_a$ and adding the level value $\beta_R$, a function $k_bf_b(x) + \beta_R$ obtained by multiplying the second cumulative distribution function $f_b(x)$ by the weight $k_b$ and adding the level value $\beta_R$, and an approximate time waveform $k_af_a(x) - k_bf_b(x) + \beta_R$ obtained by addition of the level value $\beta_R$ and the weighted difference between the first cumulative distribution function and the second cumulative distribution function, for the first target time waveform (that is, the R wave). A one-dot chain line is the function $k_af_a(x) + \beta_R$ obtained by multiplying the first cumulative distribution function $f_a(x)$ by the weight $k_a$ and adding the level value $\beta_R$, a two-dot chain line is the function $k_bf_b(x) + \beta_R$ obtained by multiplying the second cumulative distribution function $f_b(x)$ by the weight $k_b$ and adding the level value $\beta_R$, and a broken line is the approximate time waveform $k_af_a(x) - k_bf_b(x) + \beta_R$. The approximate time waveform $k_af_a(x) - k_bf_b(x) + \beta_R$ is a waveform approximating the first target time waveform (that is, the R wave).

Fig. 7 is a diagram schematically illustrating a function $k_cf'_c(x) + \beta_T$ obtained by multiplying the third inverse cumulative distribution function $f'_c(x) = 1 - f_c(x)$ by the weight $k_c$ and adding the level value $\beta_T$, a function $k_df'_d(x) + \beta_T$ obtained by multiplying the fourth inverse cumulative distribution function $f'_d(x) = 1 - f_d(x)$ by the weight $k_d$ and adding the level value $\beta_T$, and an approximate time waveform $k_cf'_c(x) - k_df'_d(x) + \beta_T$ obtained by addition of the level value $\beta_T$ and the weighted difference between the third inverse cumulative distribution function and the fourth inverse cumulative distribution function, for the second target time waveform (that is, the T wave). A one-dot chain line is the function $k_cf'_c(x) + \beta_T$ obtained by multiplying the third inverse cumulative distribution function $f'_c(x) = 1 - f_c(x)$ by the weight $k_c$ and adding the level value $\beta_T$, a two-dot chain line is the function $k_df'_d(x) + \beta_T$ obtained by multiplying the fourth inverse cumulative distribution function $f'_d(x) = 1 - f_d(x)$ by the weight $k_d$ and adding the level value $\beta_T$, and a broken line is the approximate time waveform $k_cf'_c(x) - k_df'_d(x) + \beta_T$. The approximate time waveform $k_cf'_c(x) - k_df'_d(x) + \beta_T$ is a waveform approximating the second target time waveform (that is, the T wave).

Note that the level value $\beta_R$ that is the potential at the start edge of the R wave (to be exact, QRS wave) is a value representing the magnitude of a DC component at the start edge of the R wave, and in a case where there is an abnormality in a coronary artery, the level value $\beta_R$ may fall to the negative side. Furthermore, the level value $\beta_T$ that is the potential at the end edge of the T wave is a value representing the magnitude of a DC component at the end edge of the T wave, and in a case where there is an abnormality in repolarization of the myocardium, the level value $\beta_T$ may rise to the positive side.

[Approximation of Difference between Target Time Waveform and Approximate Time Waveform]

In a case where the R wave or the T wave in a particular state is used as the target time waveform, a portion (hereinafter, referred to as a "residual portion") may be left in which the target time waveform cannot be approximated by the above-described approximate time waveform. For example, in a case where early repolarization or conduction disturbance (such as accessory pathway) occurs in the target heart, a Δ wave as indicated using a broken line in Fig. 8 may be included in the target time waveform (R wave). This Δ wave portion is left as the residual portion as the target time waveform cannot be approximated by the above-described approximate time waveform. In consideration that the residual portion is also a time waveform caused by certain activity of the heart, the signal analysis device 1 preferably performs analysis assuming that the residual portion is a cumulative Gaussian distribution, a function obtained by multiplying a cumulative Gaussian distribution by weight, a difference between cumulative Gaussian distributions, or a weighted difference between cumulative Gaussian distributions.

That is, as for a residual time waveform that is a difference between the target time waveform and the approximate time waveform, when the residual time waveform is approximated by a cumulative distribution function (for convenience, referred to as a "fifth cumulative distribution function") of a certain unimodal distribution (for convenience, referred to as a "fifth unimodal distribution") or a function obtained by multiplying the fifth cumulative distribution function by weight, the signal analysis device 1 may also acquire, as a parameter representing the characteristics of the target time waveform, a parameter specifying the fifth unimodal distribution or a parameter specifying the fifth cumulative distribution function.

Alternatively, as for the residual time waveform that is the difference between the target time waveform and the approximate time waveform, when the residual time waveform is approximated by a time waveform (hereinafter, referred to as an "approximate residual time waveform") represented by a difference or a weighted difference between the cumulative distribution function (for convenience, referred to as the "fifth cumulative distribution function") of the certain unimodal distribution (for convenience, referred to as the "fifth unimodal distribution") and a cumulative distribution function (for convenience, referred to as a "sixth cumulative distribution function") of a unimodal distribution (for convenience, referred to as a "sixth unimodal distribution") different from the fifth unimodal distribution, the signal analysis device 1 may also acquire, as parameters representing the characteristics of the target time waveform, the parameter specifying the fifth unimodal distribution or the parameter specifying the fifth cumulative distribution function, and a parameter specifying the sixth unimodal distribution or a parameter specifying the sixth cumulative distribution function.

More specifically, in a case where the residual time waveform is approximated by the fifth cumulative distribution function that is a cumulative distribution function of the fifth unimodal distribution expressed by Formula (23), as for the residual time waveform that is the difference between the target time waveform and the approximate time waveform, the signal analysis device 1 approximates the residual time waveform by an approximate time waveform $f_5(x)$ of the fifth cumulative distribution function expressed by Formula (24), and acquires, as parameters representing the characteristics of the target time waveform, an average $\mu_5$ and a standard deviation $\sigma_5$ that are parameters specifying the fifth cumulative distribution function, in addition to the above-described parameters representing the characteristics of the target time waveform.

[Math. 23]

$$\frac{1}{\sqrt{2\pi\sigma_5{}^2}} exp\left(-\frac{(x-\mu_5)^2}{2\sigma_5{}^2}\right) \quad \cdots (23)$$

[Math. 24]

$$f_5(x) = \frac{1}{2}\left(1 + erf\left(\frac{x-\mu_5}{\sqrt{2\sigma_5{}^2}}\right)\right) \quad \cdots (24)$$

In a case where the residual time waveform is approximated by the function obtained by multiplying the fifth cumulative distribution function by the weight, as for the residual time waveform that is the difference between the target time waveform and the approximate time waveform, the signal analysis device 1 may approximate the residual time waveform by an approximate time waveform $k_5f_5(x)$ of a function obtained by multiplying the fifth cumulative distribution function $f_5(x)$ expressed by Formula (24) by weight $k_5$, and acquire, as parameters representing the characteristics of the target time waveform, the average $\mu_5$ and the standard deviation $\sigma_5$ that are the parameters specifying the fifth cumulative distribution function, in addition to the above-described parameters representing the characteristics of the target time waveform. The signal analysis device 1 may further acquire the weight $k_5$ as a parameter representing the characteristics of the target time waveform.

In a case where the residual time waveform is approximated by the difference between the fifth cumulative distribution function and the sixth cumulative distribution function that is a cumulative distribution function of the sixth unimodal distribution expressed by Formula (25), as for the residual time waveform that is the difference between the target time waveform and the approximate time waveform, for example, the signal analysis device 1 approximates the residual time waveform by an approximate time waveform $f_5(x) - f_6(x)$ that is a waveform obtained by subtracting the sixth cumulative distribution function expressed by Formula (26) from the fifth cumulative distribution function expressed by Formula (24), and also acquires, as parameters representing the characteristics of the target time waveform, the average $\mu_5$ and the standard deviation $\sigma_5$ that are the parameters specifying the fifth cumulative distribution function and an average $\mu_6$ and a standard deviation $\sigma_6$ that are parameters specifying the sixth cumulative distribution function, in addition to the above-described parameters representing the characteristics of the target time waveform.

[Math. 25]

$$\frac{1}{\sqrt{2\pi\sigma_6{}^2}} exp\left(-\frac{(x-\mu_6)^2}{2\sigma_6{}^2}\right) \quad \cdots (25)$$

[Math. 26]

$$f_6(x) = \frac{1}{2}\left(1 + erf\left(\frac{x-\mu_6}{\sqrt{2\sigma_6{}^2}}\right)\right) \quad \cdots (26)$$

In a case where the residual time waveform is approximated by the weighted difference between the fifth cumulative distribution function and the sixth cumulative distribution function, as for the residual time waveform that is the difference between the target time waveform and the approximate time waveform, the signal analysis device 1 approximates the residual time waveform by an approximate time waveform $k_5f_5(x)$ - $k_6f_6(x)$ that is a waveform obtained by subtracting a function $k_6f_6(x)$ obtained by multiplying the sixth cumulative distribution function $f_6(x)$ by weight $k_6$ from a function $k_5f_5(x)$ obtained by multiplying the fifth cumulative distribution function $f_5(x)$ by the weight $k_5$, and also acquires, as parameters representing the characteristics of the target time waveform, the average $\mu_5$ and the standard deviation $\sigma_5$ that are the parameters specifying the fifth cumulative distribution function and the average $\mu_6$ and the standard deviation $\sigma_6$ that are the parameters specifying the sixth cumulative distribution function, in addition to the above-described parameters representing the characteristics of the target time waveform. The signal analysis device 1 may further acquire the weight $k_5$ and the weight $k_6$ or a ratio of the weight $k_5$ and the weight $k_6$ ($k_5/k_6$ or $k_6/k_5$) as parameters representing the characteristics of the target time waveform.

In consideration that the residual time waveform is approximated by the approximate time waveform obtained by multiplying the sixth cumulative distribution function by the weight $k_6$ from the function obtained by multiplying the fifth cumulative distribution function by the weight $k_5$, both the weight $k_5$ and the weight $k_6$ are positive values. However, in a case where the target heart is in a peculiar state, a possibility cannot be denied that at least one of the weight $k_5$ or the weight $k_6$ obtained by fitting is not a positive value. Thus, the signal analysis device 1 may perform fitting such that both the weight $k_5$ and the weight $k_6$ have positive values, but it is not essential to perform the fitting such that both the weight $k_5$ and the weight $k_6$ have positive values.

The description returns to Fig. 1. The signal analysis device 1 includes a control unit 11 including a processor 91 such as a central processing unit (CPU) and a memory 92 connected together by a bus, and executes a program. The signal analysis device 1 functions as a device including the control unit 11, an input unit 12, a communication unit 13, a storage unit 14, and an output unit 15 by executing the program.

More specifically, the processor 91 reads the program stored in the storage unit 14, and stores the read program in the memory 92. The processor 91 executes the program stored in the memory 92, whereby the signal analysis device 1 functions as the device including the control unit 11, the input unit 12, the communication unit 13, the storage unit 14, and the output unit 15.

The control unit 11 controls operation of various functional units included in the signal analysis device 1. The control unit 11 executes, for example, the myocardial activity information parameter acquisition processing. The control unit 11 controls operation of the output unit 15, for example, and causes the output unit 15 to output an acquisition result of the myocardial activity information parameter acquisition processing. The control unit 11 records, in the storage unit 14, various types of information generated by execution of the myocardial activity information parameter acquisition processing, for example.

The input unit 12 includes an input device such as a mouse, a keyboard, or a touchscreen. The input unit 12 may be configured as an interface that connects these input devices to the signal analysis device 1. The input unit 12 receives input of various types of information to the signal analysis device 1.

For example, information indicating a shape of a distribution represented by each cumulative distribution function (hereinafter, the information is referred to as "distribution shape designation information") is input to the input unit 12 for a plurality of candidates for each cumulative distribution function used for fitting.

Note that the distribution shape designation information may be stored in the storage unit 14 in advance. In such a case, the distribution shape designation information stored in the storage unit 14 does not need to be input from the input unit 12. Hereinafter, the signal analysis device 1 will be described by taking, as an example, a case where the storage unit 14 stores the distribution shape designation information in advance for simplicity of description.

The communication unit 13 includes a communication interface for connecting the signal analysis device 1 to an external device. The communication unit 13 communicates with the external device in a wired or wireless manner. The external device is, for example, a device of a transmission source of a waveform of an electrocardiogram of a target heart. The device of the transmission source of the waveform of the electrocardiogram of the target heart is, for example, an electrocardiogram measurement device. For example, in a case where the external device is the electrocardiogram measurement device, the communication unit 13 acquires the waveform of the electrocardiogram from the electrocardiogram measurement device by communication. Note that the waveform of the electrocardiogram may be input to the input unit 12.

The storage unit 14 includes a non-transitory computer-readable storage medium device such as a magnetic hard disk device or a semiconductor storage device. The storage unit 14 stores various types of information regarding the signal analysis device 1. The storage unit 14 stores, for example, information input via the input unit 12 or the communication unit 13. The storage unit 14 stores, for example, an electrocardiogram input via the input unit 12 or the communication unit 13. The storage unit 14 stores, for example, various types of information generated by execution of the myocardial activity information parameter acquisition processing.

The output unit 15 outputs various types of information. The output unit 15 includes, for example, a display device such as a cathode ray tube (CRT) display, a liquid crystal display, or an organic electro-luminescence (EL) display. The output unit 15 may be configured as an interface that connects these display devices to the signal analysis device 1. The output unit 15 outputs, for example, information input to the input unit 12. The output unit 15 may display, for example, an electrocardiogram input to the input unit 12 or the communication unit 13. The output unit 15 may display, for example, an execution result of the myocardial activity information parameter acquisition processing.

Fig. 9 is a diagram illustrating an example of a functional configuration of the control unit 11 in the first embodiment. The control unit 11 includes an electrocardiogram acquisition unit 110, a fitting information acquisition unit 120, an analysis unit 130, and a recording unit 140.

The electrocardiogram acquisition unit 110 acquires a waveform for one cycle from the waveform of the electrocardiogram of the target heart input to the input unit 12 or the communication unit 13, and outputs the waveform to the analysis unit 130. The waveform of the electrocardiogram of the target heart is a waveform in which waveforms of a plurality of beats (a plurality of cycles) is arranged in time series. Even in a case where an abnormality occurs in the target heart, not all waveforms of beats included in the waveform of the electrocardiogram are particular waveforms, and only waveforms of any small number of beats included in the waveform of the electrocardiogram are often characteristic waveforms. In the myocardial activity information parameter acquisition processing, this characteristic waveform is preferably targeted. Thus, the electrocardiogram acquisition unit 110 acquires a waveform for one cycle that is a characteristic waveform from the waveform of the electrocardiogram of the target heart. For example, the electrocardiogram acquisition unit 110 only needs to acquire the waveform for one cycle that is the characteristic waveform from the waveform of the electrocardiogram of the target heart by using a known technique for determining similarity and peculiarity. Furthermore, for example, the electrocardiogram acquisition unit 110 may cause the output unit 15 to display the waveform of the electrocardiogram, cause the input unit 12 to receive designation of a waveform for one cycle by a user such as a doctor, and acquire a waveform for one cycle corresponding to the designation received by the input unit 12 from the waveform of the electrocardiogram.

The electrocardiogram acquisition unit 110 outputs the waveform for one cycle as digital time-series data sampled at a predetermined sampling frequency. The predetermined sampling frequency is a sampling frequency of a signal used in processing in the fitting information acquisition unit 120, and is, for example, 250 Hz. In a case where the input waveform of the electrocardiogram is sampled at the predetermined sampling frequency, the electrocardiogram acquisition unit 110 only needs to cut out digital time-series data of a waveform for one cycle from digital time-series data of the input waveform of the electrocardiogram and output the digital time-series data. In a case where the input waveform of the electrocardiogram is sampled at a sampling frequency different from the predetermined sampling frequency, the electrocardiogram acquisition unit 110 only needs to cut out digital time-series data of a waveform for one cycle from digital time-series data of the input waveform of the electrocardiogram, convert the digital time-series data to the predetermined frequency, and then output the digital time-series data.

Moreover, the electrocardiogram acquisition unit 110 specifies a time section of the R wave and a time section of the T wave included in the waveform of one cycle of the electrocardiogram, acquires information specifying the time section of the R wave and information specifying the time section of the T wave, and outputs the information to the analysis unit 130. For example, the electrocardiogram acquisition unit 110 only needs to specify the start edge of the R wave, the end edge of the R wave, the start edge of the T wave, and the end edge of the T wave by a known technique, and acquire sample numbers corresponding to the specified start edge of the R wave, end edge of the R wave, start edge of the T wave, and end edge of the T wave, relative times from the start edges of the waveforms, and the like as the information specifying the time section of the R wave and the information specifying the time section of the T wave. Note that the R wave in the present specification refers to a QRS wave, to be exact. Although it is true that there are various interpretations as to which point of a waveform the start edge of the R wave (that is, start edge of the QRS wave) is, the electrocardiogram acquisition unit 110 only needs to set a point specified by any known technique as the start edge of the R wave.

Note that, in the waveform of the electrocardiogram of the target heart, a characteristic waveform may appear that changes as time passes. Thus, the electrocardiogram acquisition unit 110 may acquire, from the waveform of the electrocardiogram of the target heart, waveforms for a plurality of cycles as a target waveform on which the myocardial activity information parameter acquisition processing is performed. That is, the electrocardiogram acquisition unit 110 may acquire a time-series waveform (trend graph) in a predetermined long-term from the waveform of the electrocardiogram of the target heart, and output, for a waveform of each cycle included in the acquired waveform, the waveform, the information specifying the time section of the R wave included in the waveform, and the information specifying the time section of the T wave included in the waveform to the analysis unit 130.

The fitting information acquisition unit 120 acquires the distribution shape designation information. In a case where the distribution shape designation information is stored in the storage unit 14, the fitting information acquisition unit 120 reads the distribution shape designation information from the storage unit 14.

The analysis unit 130 includes a fitting unit 131 and a myocardial activity information parameter acquisition unit 132.

Using candidates for cumulative distribution functions indicated by the distribution shape designation information, the fitting unit 131 performs fitting on the target time waveform that is the waveform in the time section of at least one of the R wave or the T wave included in the waveform for one cycle of the electrocardiogram acquired by the electrocardiogram acquisition unit 110.

The myocardial activity information parameter acquisition unit 132 acquires parameters representing characteristics of the target time waveform on the basis of results of fitting by the fitting unit 131. The myocardial activity information parameter acquisition unit 132 acquires, as parameters representing the characteristics of the target time waveform, for example, the parameter specifying the first unimodal distribution or the parameter specifying the first cumulative distribution function, and the parameter specifying the second unimodal distribution or the parameter specifying the second cumulative distribution function when the target time waveform is approximated by the approximate time waveform that is the time waveform represented by the difference or the weighted difference between the first cumulative distribution function that is a cumulative distribution function of the first unimodal distribution and the second cumulative distribution function that is a cumulative distribution function of the second unimodal distribution. The parameters representing the characteristics of the target time waveform acquired by the myocardial activity information parameter acquisition unit 132 are examples of myocardial activity parameters.

As described above, the analysis unit 130 acquires the myocardial activity parameters on the basis of the target time waveform that is the waveform in the time section of at least one of the R wave or the T wave included in the waveform for one cycle of the electrocardiogram of the target heart and distribution candidate information.

The storage unit 14 records various types of information generated by processing executed by the control unit 11 in the storage unit 14.

Fig. 10 is a flowchart illustrating an example of a flow of processing executed by the signal analysis device 1 in the first embodiment. The electrocardiogram acquisition unit 110 acquires the waveform for one cycle of the electrocardiogram of the target heart, the information specifying the time section of the R wave, and the information specifying the time section of the T wave via the input unit 12 or the communication unit 13 (step S101). Next, the fitting information acquisition unit 120 acquires the distribution shape designation information (step S102). Next, using the candidates for the cumulative distribution functions indicated by the distribution shape designation information, the fitting unit 131 performs fitting on the target time waveform that is the waveform in the time section of at least one of the R wave or the T wave included in the waveform for one cycle of the electrocardiogram acquired in step S101 (step S103). Next, the myocardial activity information parameter acquisition unit 132 acquires the myocardial activity parameters on the basis of the fitting results (step S104). The acquired myocardial activity parameters are output to the output unit 15 (step S105).

In step S105, a graph of the fitting results for each target time waveform may be displayed. Furthermore, the processing in step S102 only needs to be executed before the processing in step S103 is executed, and may be executed before execution of step S101. The processing in steps S103 and S104 is an example of processing executed by the analysis unit 130.

Fig. 11 is a first diagram illustrating an example of analysis results by the signal analysis device 1 in the first embodiment. More specifically, Fig. 11 is an example of analysis results by the signal analysis device 1 for the waveform of the electrocardiogram of the target heart in which the motion is normal. In Fig. 11, the horizontal axis represents time and the vertical axis represents a potential. A unit of the vertical axis is an arbitrary unit.

Fig. 11 illustrates an example in which results of performing first fitting and second fitting on the R wave in depolarization of the electrocardiogram of the target heart in which the motion is normal, and results of performing fourth fitting and third fitting on the T wave in a repolarization phase of the electrocardiogram of the target heart in which the motion is normal, are displayed using $k_a/\alpha_1$ instead of the weight $k_a$ so that the first fitting result at the time T3 and the third fitting result at the time T4 are the same value, and using $k_b/\alpha_2$ instead of the weight $k_b$ so that the second fitting result at the time T3 and the fourth fitting result at the time T4 are the same value. Hereinafter, a result obtained by connecting the first fitting result having changed weight and the third fitting result together via a straight line is referred to as a myocardial inner layer activity approximation function, and a result obtained by connecting the second fitting result having changed weight and the fourth fitting result together via a straight line is referred to as a myocardial outer layer activity approximation function.

Fig. 11 corresponds to a fact that, in depolarization in a normal heart, activity (that is, activity of ion channels) of the myocardial inner layer starts earlier and proceeds rapidly than that of the myocardial outer layer, activity of the myocardial outer layer starts slightly later than a timing at which activity of ion channels of the myocardial inner layer starts, and a difference between the timing at which the activity of ion channels of the myocardial inner layer starts and a timing at which the activity of the myocardial outer layer starts results in a positive and sharp R wave. That is, an average value and a standard deviation of the myocardial inner layer activity approximation function of a portion of the first fitting result, in the myocardial inner layer activity approximation function, and an average value and a standard deviation of a portion of the second fitting result, in the myocardial outer layer activity approximation function, are parameters representing timing and progress of activity of ion channels in depolarization of the normal heart.

Furthermore, Fig. 11 corresponds to a fact that, in a repolarization phase of the normal heart, inactivation of activity of ion channels in the myocardial outer layer starts earlier than that in the myocardial inner layer, inactivation of the myocardial inner layer starts later than inactivation of the myocardial outer layer, both the inactivation of the myocardial outer layer and the inactivation of the myocardial inner layer proceed slowly, and a difference between the inactivation of the myocardial inner layer and the inactivation of the myocardial outer layer results in a positive and gentle T wave. That is, an average value and a standard deviation of a portion of the fourth fitting result, in the myocardial outer layer activity approximation function, and an average value and a standard deviation of a portion of the third fitting result, in the myocardial inner layer activity approximation function, are parameters representing timing and progress of inactivation of activity of ion channels in the repolarization phase of the normal heart. As described above, the myocardial inner layer activity approximation function and the myocardial outer layer activity approximation function obtained by the first to fourth fitting correspond to timing and progress of collective activation of ion channels in the depolarization and collective inactivation of ion channels in the repolarization phase, and average values and standard deviations of the fitting results of the functions are parameters representing activity of the myocardium.

Shapes of the myocardial inner layer activity approximation function and the myocardial outer layer activity approximation function in Fig. 11 substantially correspond to results of measurement of electromotive force of the myocardium directly measured by inserting a catheter electrode into the myocardium of the target heart in which the motion is normal. This indicates that, with the signal analysis device 1, information representing the activity of the myocardium can be acquired only from the electrocardiogram without inserting the catheter electrode.

Fig. 12 is a second diagram illustrating an example of analysis results by the signal analysis device 1 in the first embodiment. More specifically, Fig. 12 is an example of analysis results by the signal analysis device 1 for the waveform of the electrocardiogram of the target heart in which the motion is normal.

Fig. 12 illustrates three results of a graph G5, a graph G6, and a result G7. In Fig. 12, "inner layer side cumulative distribution function" indicates a fitting result of a function representing a collective channel activity timing distribution of ion channels existing in the myocardial inner layer. In Fig. 12, "outer layer side cumulative distribution function" indicates a fitting result of a function representing a collective channel activity timing distribution of ion channels existing in the myocardial outer layer. In Fig. 12, "potential of body surface" is a function representing a temporal change in potential of the body surface and is the waveform of the electrocardiogram. In Fig. 12, the horizontal axis represents time and the vertical axis represents a potential. Units of both the horizontal axis and the vertical axis are all arbitrary units. Note that the length of time represented by an interval of one scale on each of horizontal axes in Figs. 12 to 14 is the same length. Furthermore, in each of vertical axes in Figs. 12 to 14, 1 represents the maximum value of a cumulative Gaussian distribution.

The graph G5 represents an entire waveform of the electrocardiogram generated in one-time pulsation. The graph G6 illustrates an enlarged view of a T wave region that is a part of the graph G5. The T wave region is a region indicated as a region A1 in Fig. 12. The result G7 indicates statistics of two Gaussian distributions of a Gaussian distribution that is a cumulative source of the inner layer side cumulative distribution function and a Gaussian distribution that is a cumulative source of the outer layer side cumulative distribution function. Values of the result G7 represent the statistics of the two Gaussian distributions. Specifically, the statistics of the two Gaussian distributions are average values and standard deviations of Gaussian distributions that are cumulative sources of the inner layer side cumulative distribution function and the outer layer side cumulative distribution function.

Fig. 13 is a third diagram illustrating an example of analysis results by the signal analysis device 1 in the first embodiment. More specifically, Fig. 13 is an example of analysis results by the signal analysis device 1 for the waveform of the electrocardiogram of the target heart in which the motion is long T type 3.

Fig. 13 illustrates three results of a graph G8, a graph G9, and a result G10. In Fig. 13, "inner layer side cumulative distribution function" indicates a fitting result of a function representing a collective channel activity timing distribution of channels existing in the myocardial inner layer. In Fig. 13, "outer layer side cumulative distribution function" indicates a fitting result of a function representing a collective channel activity timing distribution of channels existing in the myocardial outer layer. In Fig. 13, "potential of body surface" is a function representing a temporal change in potential of the body surface and is the waveform of the electrocardiogram. In Fig. 13, the horizontal axis represents time and the vertical axis represents a potential. Units of both the horizontal axis and the vertical axis are all arbitrary units.

The graph G8 represents an entire waveform of the electrocardiogram generated in one-time pulsation. The graph G9 illustrates an enlarged view of a T wave region that is a part of the graph G8. The T wave region is a region indicated as a region A2 in Fig. 13. The result G10 indicates statistics of two Gaussian distributions of a Gaussian distribution that is a cumulative source of the inner layer side cumulative distribution function and a Gaussian distribution that is a cumulative source of the outer layer side cumulative distribution function. Values of the result G10 represent the statistics of the two Gaussian distributions, that is, average values and standard deviations of Gaussian distributions that are cumulative sources of the inner layer side cumulative distribution function and the outer layer side cumulative distribution function.

Shapes of the inner layer side cumulative distribution function and the outer layer side cumulative distribution function in Fig. 13 substantially correspond to results obtained by directly measuring a change in electromotive force generated by pulsation of the myocardial outer layer of the target heart in which the motion is long T type 3 by inserting a catheter electrode. This indicates that, with the signal analysis device 1, information representing the activity of the myocardium can be acquired only from the electrocardiogram without inserting the catheter electrode.

Fig. 14 is a fourth diagram illustrating an example of analysis results by the signal analysis device 1 in the first embodiment. More specifically, Fig. 14 is an example of analysis results by the signal analysis device 1 for the waveform of the electrocardiogram of the target heart in which the motion is long QT type 1.

Fig. 14 illustrates three results of a graph G11, a graph G12, and a result G13. In Fig. 14, "inner layer side cumulative distribution function" indicates a fitting result of a function representing a collective channel activity timing distribution of channels existing in the myocardial inner layer. In Fig. 14, "outer layer side cumulative distribution function" indicates a fitting result of a function representing a collective channel activity timing distribution of channels existing in the myocardial outer layer. In Fig. 14, "potential of body surface" is a function representing a temporal change in potential of the body surface and is the waveform of the electrocardiogram. In Fig. 14, the horizontal axis represents time and the vertical axis represents a potential. Units of both the horizontal axis and the vertical axis are all arbitrary units.

The graph G11 represents an entire waveform of the electrocardiogram generated in one-time pulsation. The graph G12 illustrates an enlarged view of a T wave region that is a part of the graph G11. The T wave region is a region indicated as a region A3 in Fig. 13. The result G13 indicates statistics of two Gaussian distributions of a Gaussian distribution that is a cumulative source of the inner layer side cumulative distribution function and a Gaussian distribution that is a cumulative source of the outer layer side cumulative distribution function. Values of the result G13 represent the statistics of the two Gaussian distributions. Specifically, the statistics of the two Gaussian distributions are average values and standard deviations of Gaussian distributions that are cumulative sources of the inner layer side cumulative distribution function and the outer layer side cumulative distribution function.

Shapes of the inner layer side cumulative distribution function and the outer layer side cumulative distribution function in Fig. 14 substantially correspond to results obtained by directly measuring a change in electromotive force generated by pulsation of the myocardial outer layer of the target heart in which the motion is long QT type 3 by inserting a catheter electrode. This indicates that, with the signal analysis device 1, information representing the activity of the myocardium can be acquired only from the electrocardiogram without inserting the catheter electrode.

Note that Fig. 14 is also an example of a result of estimation of channel current characteristics related to sudden death by the signal analysis device 1.

With reference to Figs. 15 to 17, it will be described that information representing activity of a myocardium can be acquired only from an electrocardiogram by the signal analysis device 1 also for an electrocardiogram of ventricular premature contractions. In Figs. 15 to 17, the horizontal axis represents time (second) and the vertical axis represents a potential (mV).

Fig. 15 is a first explanatory diagram for describing an example in which the electrocardiogram of the ventricular premature contractions is analyzed by the signal analysis device 1 of the first embodiment. Fig. 16 is a second explanatory diagram of the example in which the electrocardiogram of the ventricular premature contractions is analyzed by the signal analysis device 1 of the first embodiment. Fig. 17 is a third explanatory diagram of the example in which the electrocardiogram of the ventricular premature contractions is analyzed by the signal analysis device 1 of the first embodiment.

More specifically, Fig. 15 illustrates a cardiac potential of the body surface. That is, Fig 15 illustrates normal one-time heartbeat and two consecutive ventricular premature contractions recorded in the electrocardiogram. More specifically, Fig. 16 illustrates a myocardial inner layer activity approximation function including an inner layer side cumulative distribution function in depolarization and an inner layer side cumulative distribution function of a repolarization phase, of a ventricular premature contraction analyzed by the signal analysis device 1, and a myocardial outer layer activity approximation function including an outer layer side cumulative distribution function in the depolarization and an outer layer side cumulative distribution function in the repolarization phase, of the ventricular premature contraction analyzed by the signal analysis device 1. More specifically, Fig. 17 illustrates an example of a waveform of a ventricular premature contraction of an actually measured electrocardiogram.

Fig. 16 illustrates that the inner layer side cumulative distribution function in the depolarization precedes the outer layer side cumulative distribution function, and a standard deviation of each of the inner layer side cumulative distribution function and the outer layer side cumulative distribution function is larger than that of a normal heartbeat, and a spread of excitation is gentle. This analysis result corresponds to characteristics of a waveform of the R wave having a wide skirt.

Fig. 16 corresponds to a fact that the inner layer side cumulative distribution function starts inactivation earlier than the outer layer side cumulative distribution function in the repolarization phase, and illustrates the order of inactivation on the inner layer side and the outer layer side as a magnitude relationship between average values of two cumulative distribution functions in the repolarization phase. A function obtained by subtracting the outer layer side cumulative distribution function from the inner layer side cumulative distribution function in Fig. 16 corresponds to characteristics of a large negative T wave in the repolarization phase, and as illustrated in Fig. 17, a waveform obtained by subtracting the outer layer side cumulative distribution function from the inner layer side cumulative distribution function substantially corresponds to a waveform of the ventricular premature contraction of the actually measured electrocardiogram.

Note that results of Figs. 15 to 17 indicate that analysis by the signal analysis device 1 corresponds to an example in which a giant wave or a negative potential is generated due to modified conduction of excitation of the myocardium, early repolarization, or delay of repolarization. Note that, in Figs. 15 to 17, an average $\mu$ of the inner layer side cumulative distribution function in the depolarization phase is -1, and a standard deviation $\sigma$ is 0.32. Furthermore, in Figs. 15 to 17, an average $\mu$ of the outer layer side cumulative distribution function in the depolarization is -0.8, and a standard deviation $\sigma$ is 0.21. Furthermore, in Figs. 15 to 17, an average $\mu$ of the inner layer side cumulative distribution function in the repolarization phase is 1, and a standard deviation $\sigma$ is 1. Furthermore, in Figs. 15 to 17, an average $\mu$ of the outer layer side cumulative distribution function in the repolarization phase is 2.99, and a standard deviation $\sigma$ is 0.7.

With reference to Figs. 18 to 20, it will be described that information representing activity of a myocardium can be acquired from only an electrocardiogram by the signal analysis device 1 also for an electrocardiogram of a target heart in a depolarization period of Brugada syndrome type 1. In Figs. 18 to 20, the vertical axis represents a potential in millivolts.

Fig. 18 is a first explanatory diagram for describing an example in which an electrocardiogram of a target heart in a depolarization period of Brugada syndrome type 1 is analyzed by the signal analysis device 1 in the first embodiment. Fig. 19 is a second explanatory diagram for the example in which the electrocardiogram of the target heart in the depolarization period of Brugada syndrome type 1 is analyzed by the signal analysis device 1 in the first embodiment. Fig. 20 is a third explanatory diagram for the example in which the electrocardiogram of the target heart in the depolarization period of Brugada syndrome type 1 is analyzed by the signal analysis device 1 in the first embodiment.

More specifically, Fig. 18 illustrates the electrocardiogram of chest second lead of Brugada syndrome. In Fig. 18, an inner frame W1 indicates a depolarization phase, and an inner frame W2 indicates a repolarization phase. The same applies to Fig. 19. That is, also in Fig. 19, the inner frame W1 represents the depolarization phase, and the inner frame W2 represents the repolarization phase.

More specifically, Fig. 19 illustrates the inner layer side cumulative distribution function and the outer layer side cumulative distribution function in the depolarization and the repolarization phase analyzed by the signal analysis device 1. In the example illustrated in Fig. 19, the repolarization phase of the inner layer side cumulative distribution function starts following the depolarization phase, indicating characteristics of early repolarization. On the other hand, in the example of Fig. 19, as for a potential amplitude of the outer layer side cumulative distribution function, a difference is observed between the depolarization phase and the repolarization phase. Furthermore, Fig. 19 illustrates a gap and anisotropy between the depolarization phase and the repolarization phase of the outer layer side cumulative distribution function. As described above, the signal analysis device 1 can express the early repolarization and the anisotropy between the depolarization and the repolarization that are characteristics of the waveform indicated by the electrocardiogram of the target heart of Brugada syndrome by averages and standard deviations of the depolarization phase and the repolarization phase of the inner layer side cumulative distribution function and the outer layer side cumulative distribution function, and a ratio of weight given to the outer layer side cumulative distribution function to weight given to the inner layer side cumulative distribution function (inner/outer layer ratio).

Fig. 20 is a comparison between an analysis result and an actual measurement value. More specifically, Fig. 20 illustrates a difference between the inner layer side cumulative distribution function and the outer layer side cumulative distribution function in the depolarization phase and the repolarization phase in Fig. 19. Fig. 20 also illustrates the actual measurement value of the electrocardiogram. Except for the tail end, the analysis result and the actual measurement value substantially correspond to each other. The tail end means a potential at a later time.

Note that, in Figs. 18 to 20, an average $\mu$ of the inner layer side cumulative distribution function in the depolarization phase is 15, and a standard deviation σ is 0.15. Furthermore, in Figs. 18 to 20, an average μ of the outer layer side cumulative distribution function in the depolarization phase is 14, and a standard deviation σ is 0.25. In Figs. 18 to 20, an inner/outer layer ratio of the depolarization phase is 0.45. Furthermore, in Figs. 18 to 20, an average μ of the inner layer side cumulative distribution function in the repolarization phase is 25, and a standard deviation σ is 0.25. Furthermore, in Figs. 18 to 20, an average μ of the outer layer side cumulative distribution function in the repolarization phase is 20, and a standard deviation σ is 0.5.

Figs. 21 to 59 illustrate results of analysis performed by the signal analysis device 1 using a public library of electrocardiogram data <https://physionet.org/about/database/>. Figs. 21 to 59 illustrate an inner layer side cumulative distribution function, an outer layer side cumulative distribution function, and a fitting result obtained as a result of performing analysis on a cardiac potential by the signal analysis device 1.

Each of Figs. 21 to 59 is a diagram illustrating an example in which an electrocardiogram is analyzed by the signal analysis device 1 in the first embodiment. Points to be determined illustrated in each diagram represent a Q point, an R point, an S point, a Tstart point, and a Tend point of a cardiac potential in order from the left of the diagram. The points to be determined were determined by an inflection point detection and peak detection algorithm. Each diagram of Figs. 21 to 59 illustrates the inner layer side cumulative distribution function and the outer layer side cumulative distribution function in each section obtained for the section of the depolarization phase (QRS wave) and the section of the repolarization phase (T wave). Figs. 21 to 59 illustrate that, for various QRS waves and T waves, the signal analysis device 1 can express substantially the same shape by adjusting averages and standard deviations of the inner layer side cumulative distribution function and the outer layer side cumulative distribution function. Lower diagrams of Figs. 21 to 59 illustrate original waveforms of electrocardiograms and fitting results. Note that each result in Figs. 21 to 59 is a result of sampling at 300 Hz. For that reason, in the horizontal axis in each diagram of Figs. 21 to 59, the origin represents 0 seconds and a value 1 represents 3.33 milliseconds.

The signal analysis device 1 formed as described above sets a waveform in the time section of the R wave or the T wave included in a waveform for one cycle of the cardiac cycle of the target heart as a target time waveform, and acquires, as parameters representing the characteristics of the target time waveform, a parameter specifying the first unimodal distribution or a parameter specifying the first cumulative distribution function, and a parameter specifying the second unimodal distribution or a parameter specifying the second cumulative distribution function when the target time waveform is approximated by an approximate time waveform that is a time waveform represented by a difference or a weighted difference between the first cumulative distribution function that is a cumulative distribution function of the first unimodal distribution and the second cumulative distribution function that is a cumulative distribution function of the second unimodal distribution. The first cumulative distribution function is information indicating activity of the myocardial inner layer of the target heart, and the second cumulative distribution function is information indicating activity of the myocardial outer layer of the target heart. Thus, a parameter representing a shape of the first cumulative distribution function is a parameter indicating the activity of the myocardial inner layer of the target heart (myocardial inner layer parameter), and a parameter representing a shape of the second cumulative distribution function is a parameter indicating the activity of the myocardial outer layer of the target heart (myocardial outer layer parameter). Information clearly indicating characteristics of the activity of the myocardial inner layer of the target heart and information clearly indicating characteristics of the activity of the myocardial outer layer of the target heart, such as these parameters, cannot be obtained by conventional analysis of the waveform of the electrocardiogram. Therefore, with the signal analysis device 1, useful information for grasping the state of the heart can be obtained from the waveform of the electrocardiogram.

[Acquisition of Only Some Parameters]

In a case where only the characteristics of the activity of the myocardial inner layer of the target heart are desired to be grasped, only the myocardial inner layer parameter may be acquired by the signal analysis device 1, and in a case where only the characteristics of the activity of the myocardial outer layer of the target heart are desired to be grasped, only the myocardial outer layer parameter may be acquired by the signal analysis device 1. Furthermore, only some parameters of the parameters representing the shape of the cumulative distribution function may be acquired by the signal analysis device 1 as the myocardial inner layer parameter or the myocardial outer layer parameter.

For example, the myocardial activity information parameter acquisition unit 132 of the analysis unit 130 of the signal analysis device 1 acquires, as a myocardial activity parameter that is a parameter indicating the activity of the myocardium of the target heart, at least one of, at least some of parameters specifying the first unimodal distribution, at least some of parameters specifying the first cumulative distribution function, at least some of parameters specifying the second unimodal distribution, or at least some of parameters specifying the second cumulative distribution function when the first target time waveform that is the waveform in the time section of the R wave of the target heart is approximated by a first approximate time waveform that is a time waveform represented by a difference or a weighted difference between the first cumulative distribution function that is a cumulative distribution function of the first unimodal distribution and the second cumulative distribution function that is a cumulative distribution function of the second unimodal distribution, or by a first approximate time waveform that is a time waveform represented by addition of a level value and the difference or the weighted difference between the first cumulative distribution function that is a cumulative distribution function of the first unimodal distribution and the second cumulative distribution function that is a cumulative distribution function of the second unimodal distribution.

For example, in a case where both the first unimodal distribution and the second unimodal distribution are Gaussian distributions, the myocardial activity information parameter acquisition unit 132 only needs to acquire, as a myocardial activity parameter, at least one of an average value of the first unimodal distribution, a standard deviation or variance of the first unimodal distribution, an average value of the second unimodal distribution, or a standard deviation or variance of the second unimodal distribution. Note that an average value of a Gaussian distribution is time at which the frequency value is the maximum value in a unimodal distribution, and is time at which the slope of a cumulative distribution function of the unimodal distribution is the maximum. Thus, for example, regardless of whether the first unimodal distribution and the second unimodal distribution are Gaussian distributions, the myocardial activity information parameter acquisition unit 132 only needs to acquire, as a myocardial activity parameter, at least one of time corresponding to the maximum value of the first unimodal distribution, time corresponding to the maximum slope of the first cumulative distribution function, time corresponding to the maximum value of the second unimodal distribution, or time corresponding to the maximum slope of the second cumulative distribution function.

For example, the myocardial activity information parameter acquisition unit 132 of the analysis unit 130 of the signal analysis device 1 acquires, as a myocardial activity parameter that is a parameter indicating the activity of the myocardium of the target heart, at least one of, at least some of parameters specifying the third unimodal distribution, at least some of parameters specifying the third cumulative distribution function, at least some of parameters specifying the fourth unimodal distribution, or at least some of parameters specifying the fourth cumulative distribution function when the second target inverse time waveform that is a waveform obtained by inverting the time axis of the second target time waveform that is a waveform in the time section of the T wave of the target heart is approximated by a second approximate inverse time waveform that is a waveform represented by a difference or a weighted difference between the third cumulative distribution function that is a cumulative distribution function of the third unimodal distribution and the fourth cumulative distribution function that is a cumulative distribution function of the fourth unimodal distribution, or by a second approximate inverse time waveform that is a waveform represented by addition of a level value and the difference or the weighted difference between the third cumulative distribution function that is a cumulative distribution function of the third unimodal distribution and the fourth cumulative distribution function that is a cumulative distribution function of the fourth unimodal distribution.

For example, in a case where both the third unimodal distribution and the fourth unimodal distribution are Gaussian distributions, the myocardial activity information parameter acquisition unit 132 only needs to acquire, as a myocardial activity parameter, at least one of an average value of the third unimodal distribution, a standard deviation or variance of the third unimodal distribution, an average value of the fourth unimodal distribution, or a standard deviation or variance of the fourth unimodal distribution. Furthermore, for example, regardless of whether the third unimodal distribution and the fourth unimodal distribution are Gaussian distributions, the myocardial activity information parameter acquisition unit 132 only needs to acquire, as a myocardial activity parameter, at least one of time corresponding to the maximum value of the distribution of the third unimodal distribution, time corresponding to the maximum slope of the third cumulative distribution function, time corresponding to the maximum value of the fourth unimodal distribution, or time corresponding to the maximum slope of the fourth cumulative distribution function. Note that, in a case where time is acquired as a myocardial activity parameter, the myocardial activity information parameter acquisition unit 132 acquires time (value of x in the above-described examples) instead of information representing time in the reverse direction (value of x' in the above-described examples) even in a case where approximation is performed for the waveform obtained by inverting the time axis.

For example, the myocardial activity information parameter acquisition unit 132 of the analysis unit 130 of the signal analysis device 1 sets a cumulative distribution function of the third unimodal distribution as the third cumulative distribution function, sets a cumulative distribution function of the fourth unimodal distribution as the fourth cumulative distribution function, sets a function obtained by subtracting the third cumulative distribution function from 1 as the third inverse cumulative distribution function, and sets a function obtained by subtracting the fourth cumulative distribution function from 1 as the fourth inverse cumulative distribution function, and acquires, as a myocardial activity parameter that is a parameter indicating the activity of the myocardium of the target heart, at least one of, at least some of parameters specifying the third unimodal distribution, at least some of parameters specifying the third cumulative distribution function, at least some of parameters specifying the fourth unimodal distribution, or at least some of parameters specifying the fourth cumulative distribution function when the second target time waveform that is a waveform in the time section of the T wave of the target heart is approximated by a second approximate time waveform that is a waveform obtained by a difference or a weighted difference between the third inverse cumulative distribution function and the fourth inverse cumulative distribution function, or by a second approximate time waveform that is a waveform represented by addition of a level value and the difference or the weighted difference between the third inverse cumulative distribution function and the fourth inverse cumulative distribution function.

For example, in a case where both the third unimodal distribution and the fourth unimodal distribution are Gaussian distributions, the myocardial activity information parameter acquisition unit 132 only needs to acquire, as a myocardial activity parameter, at least one of an average value of the third unimodal distribution, a standard deviation or variance of the third unimodal distribution, an average value of the fourth unimodal distribution, or a standard deviation or variance of the fourth unimodal distribution. Furthermore, for example, regardless of whether the third unimodal distribution and the fourth unimodal distribution are Gaussian distributions, the myocardial activity information parameter acquisition unit 132 only needs to acquire, as a myocardial activity parameter, at least one of the time corresponding to the maximum value of the third unimodal distribution, the time corresponding to the maximum slope of the third cumulative distribution function, the time corresponding to the maximum value of the fourth unimodal distribution, or the time corresponding to the maximum slope of the fourth cumulative distribution function.

Similarly, for example, in a case where the myocardial activity information parameter acquisition unit 132 of the analysis unit 130 of the signal analysis device 1 further approximates a residual time waveform that is a time waveform of a difference between the first target time waveform and the first approximate time waveform, or a residual time waveform that is a time waveform of a difference between the second target time waveform and the second approximate time waveform, or a residual time waveform that is a time waveform of a difference between the second target time waveform and a second approximate time waveform that is a waveform obtained by inverting the time axis of a second approximate inverse time waveform, by an approximate residual time waveform that is a time waveform represented by the fifth cumulative distribution function that is a cumulative distribution function of the fifth unimodal distribution or multiplication of the fifth cumulative distribution function by weight, at least one of, at least some of parameters specifying the fifth unimodal distribution, or at least some of parameters specifying the fifth cumulative distribution function is also acquired as a myocardial activity parameter that is a parameter indicating the activity of the myocardium of the target heart.

For example, in a case where the fifth unimodal distribution is a Gaussian distribution, the myocardial activity information parameter acquisition unit 132 only needs to acquire, as a myocardial activity parameter, at least one of an average value of the fifth unimodal distribution, or a standard deviation or variance of the fifth unimodal distribution. Furthermore, for example, regardless of whether the fifth unimodal distribution is a Gaussian distribution, the myocardial activity information parameter acquisition unit 132 only needs to acquire, as a myocardial activity parameter, at least one of time corresponding to the maximum value of the fifth unimodal distribution or time corresponding to the maximum slope of the fifth cumulative distribution function.

Furthermore, for example, in a case where the myocardial activity information parameter acquisition unit 132 of the analysis unit 130 of the signal analysis device 1 further approximates the residual time waveform by an approximate residual time waveform that is a waveform represented by a difference or a weighted difference between the fifth cumulative distribution function that is a cumulative distribution function of the fifth unimodal distribution and the sixth cumulative distribution function that is a cumulative distribution function of the sixth unimodal distribution, at least one of, at least some of the parameters specifying the fifth unimodal distribution, at least some of the parameters specifying the fifth cumulative distribution function, at least some of parameters specifying the sixth unimodal distribution, or at least some of parameters specifying the sixth cumulative distribution function is acquired as a myocardial activity parameter that is a parameter indicating the activity of the myocardium of the target heart.

For example, in a case where both the fifth unimodal distribution and the sixth unimodal distribution are Gaussian distributions, the myocardial activity information parameter acquisition unit 132 only needs to acquire, as a myocardial activity parameter, at least one of the average value of the fifth unimodal distribution, the standard deviation or variance of the fifth unimodal distribution, an average value of the sixth unimodal distribution, or a standard deviation or variance of the sixth unimodal distribution. Furthermore, for example, regardless of whether the fifth unimodal distribution and the sixth unimodal distribution are Gaussian distributions, the myocardial activity information parameter acquisition unit 132 only needs to acquire, as a myocardial activity parameter, at least one of the time corresponding to the maximum value of the fifth unimodal distribution, the time corresponding to the maximum slope of the fifth cumulative distribution function, time corresponding to the maximum value of the sixth unimodal distribution, or time corresponding to the maximum slope of the sixth cumulative distribution function.

[Acquisition of Myocardial Activity Parameter by Arithmetic Operation of Parameters Obtained by Fitting]

Characteristics of the activity of the myocardium of the target heart may clearly appear not only in the parameters obtained by the fitting described above but also in a value obtained by an arithmetic operation of the parameters obtained by the fitting. Thus, the value obtained by the arithmetic operation of the parameters obtained by the fitting may be acquired by the signal analysis device 1 as a myocardial activity parameter. The parameter obtained by the fitting is at least one of a parameter specifying a unimodal distribution or a parameter specifying a cumulative distribution function, weight, or a level value. The parameter specifying the unimodal distribution or the parameter specifying the cumulative distribution function is at least one of an average or a standard deviation (or variance) in a case where the unimodal distribution is a Gaussian distribution. Regardless of whether the unimodal distribution is a Gaussian distribution, time corresponding to the maximum value of the unimodal distribution is an example of the parameter specifying the unimodal distribution, and time corresponding to the maximum slope of the cumulative distribution function of the unimodal distribution is an example of the parameter specifying the cumulative distribution function.

For example, the myocardial activity information parameter acquisition unit 132 of the analysis unit 130 of the signal analysis device 1 may acquire a parameter indicating the activity of the myocardium of the target heart (parameter different from each parameter described above) by an arithmetic operation of the myocardial activity parameter obtained by the fitting described above for the waveform in the time section of the R wave included in the waveform for one cycle indicating the cardiac cycle of the target heart (that is, the parameter indicating the activity of the myocardium of the target heart in the time section of the R wave) and the myocardial activity parameter obtained by the fitting described above for the waveform in the time section of the T wave included in the waveform for the one cycle (that is, the parameter indicating the activity of the myocardium of the target heart in the time section of the T wave).

Furthermore, for example, the myocardial activity information parameter acquisition unit 132 of the analysis unit 130 of the signal analysis device 1 may acquire a parameter indicating the activity of the myocardium of the target heart (parameter different from each parameter described above) by an arithmetic operation of the parameter indicating the activity of the myocardial inner layer of the target heart obtained by the fitting described above for the waveform in the time section of the R wave included in the waveform for one cycle indicating the cardiac cycle of the target heart and the parameter indicating the activity of the myocardial outer layer of the target heart obtained by the fitting.

Furthermore, for example, the myocardial activity information parameter acquisition unit 132 of the analysis unit 130 of the signal analysis device 1 may acquire a parameter indicating the activity of the myocardium of the target heart (parameter different from each parameter described above) by an arithmetic operation of the parameter indicating the activity of the myocardial inner layer of the target heart obtained by the fitting described above for the waveform in the time section of the T wave included in the waveform for one cycle indicating the cardiac cycle of the target heart and the parameter indicating the activity of the myocardial outer layer of the target heart obtained by the fitting.

Hereinafter, examples will be described in which, in a case where all of the first to fourth unimodal distributions are Gaussian distributions, values obtained by an arithmetic operation of averages obtained by fitting are used as myocardial activity parameters. In a case where the first to fourth unimodal distributions are not Gaussian distributions, "average" in the following examples only needs to be read as "time at which the value is maximum in the unimodal distribution", that is, "time corresponding to the maximum value of the unimodal distribution", "time at which the slope is maximum in the cumulative distribution function", that is, "time corresponding to the maximum slope of the cumulative distribution function", or the like.

(1) Parameter Representing Time from Depolarization to Switching to Repolarization

It is known that sudden death may occur due to arrhythmia in a case where time from depolarization of the inner layer or the outer layer of the myocardium to switching to repolarization of the inner layer or the outer layer of the myocardium is extremely short or extremely long. That is, shortening or prolongation of the time from depolarization of the myocardium to switching to repolarization may indicate that some pathological state may have occurred in the myocardium. Thus, the signal analysis device 1 preferably acquires the time from depolarization of the inner layer or the outer layer of the myocardium to switching to repolarization of the inner layer or the outer layer of the myocardium as a myocardial activity parameter, and specifically, preferably acquires at least one of the following four parameters (1A) to (1D) as a myocardial activity parameter.

(1A) Parameter Representing Time from Depolarization of Inner Layer of Myocardium to Switching to Repolarization of Inner Layer of Myocardium

The myocardial activity information parameter acquisition unit 132 of the analysis unit 130 of the signal analysis device 1 may acquire, as a myocardial activity parameter, a difference between the average of the first unimodal distribution obtained by the fitting described above for the first target time waveform that is the waveform in the time section of the R wave included in the waveform for one cycle indicating the cardiac cycle of the target heart and the average of the third unimodal distribution obtained by the fitting described above for the second target time waveform that is the waveform in the time section of the T wave included in the waveform for the one cycle. For example, when the average of the first unimodal distribution is $\mu_a$ and the average of the third unimodal distribution is $\mu_c$ as in the example described above, the myocardial activity information parameter acquisition unit 132 may acquire $|\mu_a - \mu_c|$ as a myocardial activity parameter. Note that, in consideration that $\mu_c$ is temporally later than $\mu_a$ the myocardial activity information parameter acquisition unit 132 may acquire $\mu_c - \mu_a$ as a myocardial activity parameter. This myocardial activity parameter is a parameter representing time from depolarization of the inner layer of the myocardium to switching to repolarization of the inner layer of the myocardium.

(1B) Parameter Representing Time from Depolarization of Outer Layer of Myocardium to Switching to Repolarization of Outer Layer of Myocardium

The myocardial activity information parameter acquisition unit 132 of the analysis unit 130 of the signal analysis device 1 may acquire, as a myocardial activity parameter, a difference between the average of the second unimodal distribution obtained by the fitting described above for the first target time waveform that is the waveform in the time section of the R wave included in the waveform for one cycle indicating the cardiac cycle of the target heart and the average of the fourth unimodal distribution obtained by the fitting described above for the second target time waveform that is the waveform in the time section of the T wave included in the waveform for the one cycle. For example, when the average of the second unimodal distribution is $\mu_3$ and the average of the fourth unimodal distribution is $\mu_d$ as in the example described above, the myocardial activity information parameter acquisition unit 132 may acquire $|\mu_b - \mu_d|$ as a myocardial activity parameter. Note that, in consideration that $\mu_d$ is temporally later than $\mu_b$, the myocardial activity information parameter acquisition unit 132 may acquire $\mu_d - \mu_b$ as a myocardial activity parameter. This myocardial activity parameter is a parameter representing time from depolarization of the outer layer of the myocardium to switching to repolarization of the outer layer of the myocardium.

(1C) Parameter Representing Time from Depolarization of Outer Layer of Myocardium to Switching to Repolarization of Inner Layer of Myocardium

The myocardial activity information parameter acquisition unit 132 of the analysis unit 130 of the signal analysis device 1 may acquire, as a myocardial activity parameter, a difference between the average of the second unimodal distribution obtained by the fitting described above for the first target time waveform that is the waveform in the time section of the R wave included in the waveform for one cycle indicating the cardiac cycle of the target heart and the average of the third unimodal distribution obtained by the fitting described above for the second target time waveform that is the waveform in the time section of the T wave included in the waveform for the one cycle. For example, when the average of the second unimodal distribution is $\mu_b$ and the average of the third unimodal distribution is $\mu_c$ as in the example described above, the myocardial activity information parameter acquisition unit 132 may acquire $|\mu_b - \mu_c|$ as a myocardial activity parameter. Note that, in consideration that $\mu_c$ is temporally later than $\mu_b$, the myocardial activity information parameter acquisition unit 132 may acquire $\mu_c - \mu_b$ as a myocardial activity parameter. This myocardial activity parameter is a parameter representing time from depolarization of the outer layer of the myocardium to switching to repolarization of the inner layer of the myocardium.

(1D) Parameter Representing Time from Depolarization of Inner Layer of Myocardium to Switching to Repolarization of Outer Layer of Myocardium

The myocardial activity information parameter acquisition unit 132 of the analysis unit 130 of the signal analysis device 1 may acquire, as a myocardial activity parameter, a difference between the average of the first unimodal distribution obtained by the fitting described above for the first target time waveform that is the waveform in the time section of the R wave included in the waveform for one cycle indicating the cardiac cycle of the target heart and the average of the fourth unimodal distribution obtained by the fitting described above for the second target time waveform that is the waveform in the time section of the T wave included in the waveform for the one cycle. For example, when the average of the first unimodal distribution is $\mu_a$ and the average of the fourth unimodal distribution is $\mu_d$ as in the example described above, the myocardial activity information parameter acquisition unit 132 may acquire $|\mu_a - \mu_d|$ as a myocardial activity parameter. Note that, in consideration that $\mu_d$ is temporally later than $\mu_a$, the myocardial activity information parameter acquisition unit 132 may acquire $\mu_d - \mu_a$ as a myocardial activity parameter. This myocardial activity parameter is a parameter representing time from depolarization of the inner layer of the myocardium to switching to repolarization of the outer layer of the myocardium.

(2) Parameter Representing Time Difference and Order Between Inner Layer Activity and Outer Layer Activity in Depolarization

In a case where a time difference between activity of the inner layer and activity of the outer layer in depolarization is longer than normal, there is a possibility that a disorder such as delay or block occurs in conduction of excitation of the myocardium, or a place where excitation starts or the order in which excitation is transmitted are different from a normal pattern, and in particular, it is suggested that there is a disorder in a stimulation conduction system of the myocardium, ischemic state of the myocardium, and premature contraction. Thus, the signal analysis device 1 preferably acquires, as a myocardial activity parameter, a parameter representing the time difference and order between the activity of the inner layer and the activity of the outer layer in depolarization. Specifically, the myocardial activity information parameter acquisition unit 132 of the analysis unit 130 of the signal analysis device 1 may acquire, as a myocardial activity parameter, a difference between the average of the first unimodal distribution and the average of the second unimodal distribution obtained by the fitting described above for the first target time waveform that is the waveform in the time section of the R wave included in the waveform for one cycle indicating the cardiac cycle of the target heart. For example, when the average of the first unimodal distribution is $\mu_a$ and the average of the second unimodal distribution is $\mu_b$ as in the example described above, the myocardial activity information parameter acquisition unit 132 may acquire $\mu_a - \mu_b$ or $\mu_b - \mu_a$ as a myocardial activity parameter.

**(3)** Parameter Representing Time Difference and Order Between Inner Layer Activity and Outer Layer Activity in Repolarization

In a case where a time difference between activity of the inner layer and activity of the outer layer in repolarization is prolonged or shortened, some abnormality may have occurred in the myocardium. Thus, the signal analysis device 1 preferably acquires, as a myocardial activity parameter, a parameter representing the time difference and order between the activity of the inner layer and the activity of the outer layer in repolarization. Specifically, the myocardial activity information parameter acquisition unit 132 of the analysis unit 130 of the signal analysis device 1 may acquire, as a myocardial activity parameter, a difference between the average of the third unimodal distribution and the average of the fourth unimodal distribution obtained by the fitting described above for the second target time waveform that is the waveform in the time section of the T wave included in the waveform for one cycle indicating the cardiac cycle of the target heart. For example, when the average of the third unimodal distribution is $\mu_c$ and the average of the fourth unimodal distribution is $\mu_d$ as in the example described above, the myocardial activity information parameter acquisition unit 132 may acquire $\mu_c - \mu_d$ or $\mu_d - \mu_c$ as a myocardial activity parameter.

(Correction of Myocardial Activity Parameter)

Note that a myocardial activity parameter regarding the width in the time direction among the myocardial activity parameters described above acquired by the myocardial activity information parameter acquisition unit 132 is characterized in that influence of fluctuation due to the heart rate and influence of individual differences of age, gender, and the like are recognized, similarly to a parameter regarding the width in the time direction (for example, QT interval) among conventional parameters indicating activity of the heart. For the conventional parameter regarding the width in the time direction, it is known that correction for reducing the influence of fluctuation due to the heart rate and the influence of the individual differences is performed, and a corrected value is used as a parameter for evaluating the activity of the heart. Thus, the myocardial activity parameter acquired by the myocardial activity information parameter acquisition unit 132 may also be corrected for reducing the influence of fluctuation due to the heart rate and the influence of the individual differences, and a corrected value may be used as a parameter for evaluating the activity of the myocardium. For example, for the fluctuation due to the heart rate, the myocardial activity parameter acquired by the fitting described above may be corrected by a linear or nonlinear arithmetic operation on the basis of a time interval between an R spine and an R spine adjacent to each other (Hereinafter, the interval is referred to as an "RR interval"), and a corrected value may be used as a myocardial activity parameter. Furthermore, for the individual differences, a relationship between the RR interval and the myocardial activity parameter acquired by the fitting described above may be obtained from electrocardiogram data of each individual to perform correction, and a corrected value may be used as a myocardial activity parameter. This correction may be performed by another device after the signal analysis device 1 outputs the myocardial activity parameter acquired by the fitting, or may be performed by the signal analysis device 1. In a case where the signal analysis device 1 performs correction of the myocardial activity parameter, for example, the myocardial activity information parameter acquisition unit 132 only needs to perform correction on the myocardial activity parameter acquired by the fitting described above and output a corrected value as a myocardial activity parameter. Note that, in a case where the correction is performed in the signal analysis device 1, the myocardial activity parameter acquired by the fitting described above is an intermediate parameter acquired in the device as a result, but is still a parameter representing the activity of the myocardium as in a case of being output outside the device.

(Modification)

Note that the electrocardiogram is preferably an electrocardiogram of lead close to a cardiac electromotive force vector. The electrocardiogram of lead close to a cardiac electromotive force vector is preferably, for example, an electrocardiogram capable of acquiring three-dimensional information on a cardiac potential such as lead II, lead V4, and lead V5. Since an amount of information increases as the number of channels of the electrocardiogram increases, it is desirable that there is a larger number of channels of the electrocardiogram. That is, the signal analysis device 1 may perform analysis with a waveform of each of the channels of the multi-channel electrocardiogram as a target, and obtain a myocardial activity parameter that is an analysis result for each of the channels.

Note that the signal analysis device 1 may be implemented by using a plurality of information processing devices communicably connected to each other via a network. In this case, the functional units included in the signal analysis device 1 may be implemented in a distributed manner in the plurality of information processing devices.

Note that the electrocardiogram acquisition unit 110 is an example of a biological information acquisition unit.

<Second Embodiment>

In the signal analysis device 1 of the first embodiment, the myocardial activity parameter, which is useful information for grasping the state of the heart, can be obtained from the time-series biological information on one channel regarding the pulsation of the heart. However, when the myocardial activity parameter obtained by the signal analysis device 1 of the first embodiment is presented as it is to the user such as a doctor who is familiar with the waveform of the electrocardiogram, it is not always easy for the user such as a doctor to grasp the state of the heart. Thus, a signal conversion device of a second embodiment enables generation of a corrected waveform that makes it easy for the user such as a doctor to grasp the state of the heart.

Fig. 60 is a diagram illustrating an example of a functional configuration of a signal conversion device 2 of the second embodiment. The signal conversion device 2 of the second embodiment includes a signal analysis unit 210, a converter acquisition unit 220, a conversion information setting unit 230, a myocardial activity parameter conversion unit 240, and a waveform synthesis unit 250. Similarly to the signal analysis device 1 of the first embodiment, any time-series biological information regarding the pulsation of the target heart may be input to the signal conversion device 2 of the second embodiment; however, hereinafter, an example will be described in which the waveform of the electrocardiogram of the target heart is input to the signal conversion device 2 of the second embodiment. The waveform of the electrocardiogram of the target heart input to the signal conversion device 2 is a waveform of the electrocardiogram acquired from the target heart by using a known device or the like, and is a waveform to be processed by the signal conversion device 2, that is, a waveform to be corrected by the signal conversion device 2. The waveform of the electrocardiogram of the target heart input to the signal conversion device 2 is usually a continuous waveform for a plurality of beats (for a plurality of cycles) (that is, a waveform in which waveforms for a plurality of cycles are arranged in time series), but may be a waveform for at least one beat (for one cycle).

In the second and subsequent embodiments, portions similar to those of the signal analysis device 1 of the first embodiment will be described using the same words and symbols as those of the first embodiment, so that descriptions similar to those of the first embodiment will be omitted, redundant descriptions will be avoided, and differences from the first embodiment will be mainly described. Note that, in the second and subsequent embodiments, both information on a type of the myocardial activity parameter and information on a value of the myocardial activity parameter are handled, and thus, in the second and subsequent embodiments, the type and the value are distinguished from each other and described by denoting "value of ∞ parameter" in the case of representing the value, and simply denoting "oo parameter" in the case of representing the type.

The signal conversion device 2 obtains and outputs a synthesized signal from the input waveform of the electrocardiogram of the target heart. The synthesized signal obtained by the signal conversion device 2 is a signal having a waveform obtained by correcting the waveform of the electrocardiogram of the target heart input to the signal conversion device 2. The signal conversion device 2 of the second embodiment performs steps S210, S220, S230A, S240A, and S250 illustrated in Fig. 61.

The synthesized signal output by the signal conversion device 2 only needs be input to, for example, a display device including a screen that displays a waveform, and the display device only needs to present the waveform of the synthesized signal. In a case where the signal conversion device 2 includes a display unit that is a display screen, the signal conversion device 2 may display the waveform of the synthesized signal on the display unit included in the device.

Note that, in a case where a set of values of the myocardial activity parameter of the target heart ("myocardial activity parameter set" to be described later) has already been obtained, instead of inputting the waveform of the electrocardiogram of the target heart, the set of values of the myocardial activity parameter of the target heart may be input as indicated by a onedot chain line in Fig. 60. The set of values of the myocardial activity parameter of the target heart input to the signal conversion device 2 in this case is a set of values of the myocardial activity parameters obtained from a waveform that is to be corrected and indicates the cardiac cycle of the target heart. In this case, the signal conversion device 2 does not have to include the signal analysis unit 210, does not have to perform step S210, and can be said to be a signal synthesis device 3 that obtains and outputs a synthesized signal from a set of values of the myocardial activity parameter. A hardware configuration of the signal conversion device 2 or the signal synthesis device 3 is similar to the hardware configuration of the signal analysis device 1 illustrated in Fig. 1, for example.

[Signal Analysis Unit 210]

The waveform of the electrocardiogram of the target heart input to the signal conversion device 2 is input to the signal analysis unit 210. The signal analysis unit 210 performs processing similar to that of the signal analysis device 1 of the first embodiment from the waveform of each cycle of the electrocardiogram of the target heart, thereby obtaining the value of the myocardial activity parameter of each cycle of the waveform of the electrocardiogram of the target heart (step S210). The value of the myocardial activity parameter obtained by the signal analysis unit 210 is output from the signal analysis unit 210 and input to the converter acquisition unit 220 and the myocardial activity parameter conversion unit 240. Specifically, the signal analysis unit 210 performs first analysis processing or second analysis processing below.

The first analysis processing performed by the signal analysis unit 210 is processing of acquiring, as myocardial activity parameters, for the input waveform of each cycle of the electrocardiogram of the target heart: a value of the parameter specifying the first unimodal distribution or a value of the parameter specifying the first cumulative distribution function, a value of the parameter specifying the second unimodal distribution or a value of the parameter specifying the second cumulative distribution function, a value of a first weight parameter, a value of a second weight parameter, and a value of a first level parameter when the first target time waveform that is a waveform in the time section of the R wave of the cycle is approximated by a first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplexing the second cumulative distribution function that is a cumulative distribution function of the second unimodal distribution by the value of the second weight parameter, and adding the value of the first level parameter; and a value of the parameter specifying the third unimodal distribution or a value of the parameter specifying the third cumulative distribution function, a value of the parameter specifying the fourth unimodal distribution or a value of the parameter specifying the fourth cumulative distribution function, a value of a third weight parameter, a value of a fourth weight parameter, and a value of a second level parameter when the second target inverse time waveform that is a waveform obtained by inverting the time axis of the waveform in the time section of the T wave of the cycle is approximated by a second approximate inverse time waveform that is a waveform represented by a function obtained by subtracting, from a function obtained by multiplying the third cumulative distribution function that is a cumulative distribution function of the third unimodal distribution by the value of the third weight parameter, a function obtained by multiplying the fourth cumulative distribution function that is a cumulative distribution function of the fourth unimodal distribution by the value of the fourth weight parameter, and adding the value of the second level parameter. That is, the first weight parameter is the weight $k_a$ of the first cumulative distribution function described in the first embodiment, the second weight parameter is the weight $k_b$ of the second cumulative distribution function described in the first embodiment, the third weight parameter is the weight $k_e$ of the third cumulative distribution function described in the first embodiment, the fourth weight parameter is the weight $k_g$ of the fourth cumulative distribution function described in the first embodiment, the first level parameter is the level value $\beta_R$ of the R wave described in the first embodiment, and the second level parameter is the level value $\beta_T$ of the T wave described in the first embodiment.

In a case where each unimodal distribution is a Gaussian distribution, the parameter specifying each unimodal distribution or the parameter specifying each cumulative distribution function is an average, and a standard deviation or a variance of each Gaussian distribution. Thus, in a case where each of the first to fourth unimodal distributions is a Gaussian distribution, an example of the value of the parameter specifying the first unimodal distribution or the value of the parameter specifying the first cumulative distribution function obtained by the first analysis processing is the average value $\mu_a$ and the standard deviation value $\sigma_a$ of the first unimodal distribution (that is, a first Gaussian distribution), an example of the value of the parameter specifying the second unimodal distribution or the value of the parameter specifying the second cumulative distribution function obtained by the first analysis processing is the average value $\mu_b$ and the standard deviation value $\sigma_b$ of the second unimodal distribution (that is, a second Gaussian distribution), and an example of the value of the parameter specifying the third unimodal distribution or the value of the parameter specifying the third cumulative distribution function obtained by the first analysis processing is the average value $\mu_e$ and the standard deviation value $\sigma_e$ of the third unimodal distribution (that is, a third Gaussian distribution), and an example of the value of the parameter specifying the fourth unimodal distribution or the value of the parameter specifying the fourth cumulative distribution function obtained by the first analysis processing is the average value $\mu_g$ and the standard deviation value $\sigma_g$ of the fourth unimodal distribution (that is, a fourth Gaussian distribution).

The second analysis processing performed by the signal analysis unit 210 is processing of acquiring, as myocardial activity parameters, for the input waveform of each cycle of the electrocardiogram of the target heart: a value of the parameter specifying the first unimodal distribution or a value of the parameter specifying the first cumulative distribution function, a value of the parameter specifying the second unimodal distribution or a value of the parameter specifying the second cumulative distribution function, a value of the first weight parameter, a value of the second weight parameter, and a value of the first level parameter when the first target time waveform that is a waveform in the time section of the R wave of the cycle is approximated by the first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplexing the second cumulative distribution function that is a cumulative distribution function of the second unimodal distribution by the value of the second weight parameter, and adding the value of the first level parameter; and a value of the parameter specifying the third unimodal distribution or a value of the parameter specifying the third cumulative distribution function, a value of the parameter specifying the fourth unimodal distribution or a value of the parameter specifying the fourth cumulative distribution function, a value of the third weight parameter, a value of the fourth weight parameter, and a value of the second level parameter when the second target time waveform that is a waveform in the time section of the T wave of the cycle is approximated by a second approximate time waveform that is a time waveform represented by a function obtained by subtracting, from a function obtained by multiplying the third inverse cumulative distribution function that is a function obtained by subtracting the third cumulative distribution function that is a cumulative distribution function of the third unimodal distribution from 1 by the value of the third weight parameter, a function obtained by multiplying the fourth inverse cumulative distribution function that is a function obtained by subtracting the fourth cumulative distribution function that is a cumulative distribution function of the fourth unimodal distribution from 1 by the value of the fourth weight parameter, and adding the value of the second level parameter. That is, the first weight parameter is the weight $k_a$ of the first cumulative distribution function described in the first embodiment, the second weight parameter is the weight $k_b$ of the second cumulative distribution function described in the first embodiment, the third weight parameter is the weight $k_e$ of the third cumulative distribution function described in the first embodiment, the fourth weight parameter is the weight $k_d$ of the fourth cumulative distribution function described in the first embodiment, the first level parameter is the level value $\beta_a$ of the R wave described in the first embodiment, and the second level parameter is the level value $\beta_T$ of the T wave described in the first embodiment.

In a case where each of the first to fourth unimodal distributions is a Gaussian distribution, an example of the value of the parameter specifying the first unimodal distribution or the value of the parameter specifying the first cumulative distribution function obtained by the second analysis processing is the average value $\mu_a$ and the standard deviation value $\sigma_a$ of the first unimodal distribution (that is, the first Gaussian distribution), an example of the value of the parameter specifying the second unimodal distribution or the value of the parameter specifying the second cumulative distribution function obtained by the second analysis processing is the average value $\mu_b$ and the standard deviation value $\sigma_b$ of the second unimodal distribution (that is, the second Gaussian distribution), and an example of the value of the parameter specifying the third unimodal distribution or the value of the parameter specifying the third cumulative distribution function obtained by the second analysis processing is the average value $\mu_c$ and the standard deviation value $\sigma_c$ of the third unimodal distribution (that is, the third Gaussian distribution), and an example of the value of the parameter specifying the fourth unimodal distribution or the value of the parameter specifying the fourth cumulative distribution function obtained by the second analysis processing is the average value $\mu_d$ and the standard deviation value $\sigma_d$ of the fourth unimodal distribution (that is, the fourth Gaussian distribution).

Even in a case where the first analysis processing is performed or even in a case where the second analysis processing is performed, the signal analysis unit 210 obtains, for the input waveform of each cycle of the electrocardiogram of the target heart, a set of the value of the parameter specifying the first unimodal distribution or the value of the parameter specifying the first cumulative distribution function, the value of the parameter specifying the second unimodal distribution or the value of the parameter specifying the second cumulative distribution function, the value of the first weight parameter, the value of the second weight parameter, the value of the first level parameter, the value of the parameter specifying the third unimodal distribution or the value of the parameter specifying the third cumulative distribution function, the value of the parameter specifying the fourth unimodal distribution or the value of the parameter specifying the fourth cumulative distribution function, the value of the third weight parameter, the value of the fourth weight parameter, and the value of the second level parameter. Hereinafter, a set of values of the myocardial activity parameter obtained from the waveform of each cycle of the electrocardiogram is referred to as a myocardial activity parameter set. In a case where the first to fourth unimodal distributions are Gaussian distributions, the myocardial activity parameter set is a set of values (element values) of a total of 14 types of myocardial activity parameters of a value of the average of the first unimodal distribution, a value of the standard deviation or variance of the first unimodal distribution, a value of the average of the second unimodal distribution, a value of the standard deviation or variance of the second unimodal distribution, a value of the first weight parameter, a value of the second weight parameter, a value of the first level parameter, a value of the average of the third distribution, a value of the standard deviation or variance of the third unimodal distribution, a value of the average of the fourth distribution, a value of the standard deviation or variance of the fourth unimodal distribution, a value of the third weight parameter, a value of the fourth weight parameter, and a value of the second level parameter. For example, the myocardial activity parameter set in a case where each of the first to fourth unimodal distributions is a Gaussian distribution and the signal analysis unit 210 performs the first analysis processing is ($\mu_a$, $\sigma_a$, $\mu_b$, $\sigma_b$, $k_a$, $k_b$, $\beta_R$, $\mu_e$, $\sigma_e$, $\mu_g$, $\sigma_g$, $k_e$, $k_g$, $\beta_T$), and the myocardial activity parameter set in a case where each of the first to fourth unimodal distributions is a Gaussian distribution and the signal analysis unit 210 performs the second analysis processing is ($\mu_a$, $\sigma_a$, $\mu_b$, $\sigma_b$, $k_a$, $k_b$, $\beta_R$, $\mu_c$, $\sigma_c$, $\mu_d$, $\sigma_d$, $k_c$, $k_d$, $\beta_T$).

Note that, as described in the first embodiment, the unit of the information x indicating the time in each piece of analysis processing is arbitrary. However, in the second and subsequent embodiments, in order to handle the information representing the time in a consistent manner in each processing unit, each processing unit of the signal conversion device 2 only needs to be operated with each of the time at the start edge of the cycle and the time at the end edge of the cycle as a predetermined sample number, or with each of the time at the start edge of the cycle and the time at the end edge of the cycle as a predetermined relative time. For this purpose, for example, the signal conversion device 2 may be configured to receive input of the waveform of the electrocardiogram of the target heart subjected to known adjustment processing such as companding in the time direction so as to have a heart rate of 60 times/min, or the signal analysis unit 210 may perform the adjustment processing described above on the waveform of the electrocardiogram of the target heart input to the signal conversion device 2 and then perform processing of obtaining the value of the myocardial activity parameter, or the signal analysis unit 210 may perform processing of obtaining the value of the myocardial activity parameter on the waveform of the electrocardiogram of the target heart input to the signal conversion device 2 and then perform processing corresponding to the adjustment processing described above on the value of the myocardial activity parameter to output a value of a processed myocardial activity parameter.

[Converter Acquisition Unit 220]

The myocardial activity parameter set of each cycle of the electrocardiogram of the target heart output from the signal analysis unit 210 is input to the converter acquisition unit 220. In a case where the myocardial activity parameter set of each cycle of the electrocardiogram of the target heart is input to the signal conversion device 2, the myocardial activity parameter set of each cycle of the electrocardiogram of the target heart input to the signal conversion device 2 is input to the converter acquisition unit 220. In a storage unit (not illustrated) in the converter acquisition unit 220, a myocardial activity parameter set of a standard electrocardiogram (Hereinafter, the electrocardiogram is referred to as a "standard electrocardiogram".) is stored in advance. The standard electrocardiogram is, for example, an electrocardiogram of a healthy subject in which excitation propagation is in a normal sinus rhythm, and the size, shape, position, orientation of the heart, and the physique are average, and is, for example, the electrocardiogram recorded according to the international standard. The myocardial activity parameter set of the standard electrocardiogram is a myocardial activity parameter set obtained by the processing performed by the signal analysis unit 210 described above from the waveform of one cycle of the standard electrocardiogram. The converter acquisition unit 220 obtains a converter that converts the myocardial activity parameter set of the target heart into bijective so that the myocardial activity parameter set of each cycle of the electrocardiogram of the target heart approaches the myocardial activity parameter set of the standard electrocardiogram (step S220). More specifically, the converter acquisition unit 220 obtains a converter that converts the set of the myocardial activity parameter of the target heart into bijective so that the set of the values of the myocardial activity parameter of each cycle of the electrocardiogram of the target heart approaches the set of the values of the myocardial activity parameter of one cycle of the standard electrocardiogram. The converter obtained by the converter acquisition unit 220 is output from the converter acquisition unit 220 and input to the myocardial activity parameter conversion unit 240.

In a case where the signal analysis unit 210 of the signal conversion device 2 performs the first analysis processing or in a case where the myocardial activity parameter set input to the signal conversion device 2 is obtained by the first analysis processing, the storage unit (not illustrated) in the converter acquisition unit 220 stores in advance, for the waveform of one cycle of the standard electrocardiogram, a myocardial activity parameter set of: a value of the parameter specifying the first unimodal distribution or a value of the parameter specifying the first cumulative distribution function, a value of the parameter specifying the second unimodal distribution or a value of the parameter specifying the second cumulative distribution function, a value of the first weight parameter, a value of the second weight parameter, and a value of the first level parameter when the first target time waveform that is a waveform in the time section of the R wave in the waveform of the one cycle is approximated by the first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplexing the second cumulative distribution function that is a cumulative distribution function of the second unimodal distribution by the value of the second weight parameter, and adding the value of the first level parameter; and a value of the parameter specifying the third unimodal distribution or a value of the parameter specifying the third cumulative distribution function, a value of the parameter specifying the fourth unimodal distribution or a value of the parameter specifying the fourth cumulative distribution function, a value of the third weight parameter, a value of the fourth weight parameter, and a value of the second level parameter when the second target inverse time waveform that is a waveform obtained by inverting the time axis of the waveform in the time section of the T wave in the waveform of the one cycle is approximated by a second approximate inverse time waveform that is a waveform represented by a function obtained by subtracting, from a function obtained by multiplying the third cumulative distribution function that is a cumulative distribution function of the third unimodal distribution by the value of the third weight parameter, a function obtained by multiplying the fourth cumulative distribution function that is a cumulative distribution function of the fourth unimodal distribution by the value of the fourth weight parameter, and adding the value of the second level parameter. It is set that each of the first to fourth unimodal distributions is a Gaussian distribution, the average value of the first unimodal distribution is $\mu_{a0}$, the standard deviation value of the first unimodal distribution is $\sigma_{a0}$, the average value of the second unimodal distribution is $\mu_{b0}$, the standard deviation value of the second unimodal distribution is $\sigma_{b0}$, the average value of the third unimodal distribution is $\mu_{e0}$, the standard deviation value of the third unimodal distribution is $\sigma_{e0}$, the average value of the fourth unimodal distribution is $\mu_{g0}$, and the standard deviation value of the fourth unimodal distribution is $\sigma g_0$, the myocardial activity parameter set of the standard electrocardiogram stored in advance in the storage unit (not illustrated) in the converter acquisition unit 220 is ($\mu_{a0}$, $\sigma_{a0}$, $\mu_{b0}$, $\sigma_{b0}$ $k_{a0}$, $k_{b0}$, $\beta_{R0}$, $\mu_{e0}$, $\sigma_{e0}$, $\mu g_0$, $\sigma_{g0}$, $k_{e0}$, $k_{g0}$, $\beta_{T0}$).

In a case where the signal analysis unit 210 of the signal conversion device 2 performs the second analysis processing or in a case where the myocardial activity parameter set input to the signal conversion device 2 is obtained by the second analysis processing, the storage unit (not illustrated) in the converter acquisition unit 220 stores in advance, for the waveform of one cycle of the standard electrocardiogram, a myocardial activity parameter set of: a value of the parameter specifying the first unimodal distribution or a value of the parameter specifying the first cumulative distribution function, a value of the parameter specifying the second unimodal distribution or a value of the parameter specifying the second cumulative distribution function, a value of the first weight parameter, a value of the second weight parameter, and a value of the first level parameter when the first target time waveform that is a waveform in the time section of the R wave in the waveform of the one cycle is approximated by the first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplexing the second cumulative distribution function that is a cumulative distribution function of the second unimodal distribution by the value of the second weight parameter, and adding the value of the first level parameter; and a value of the parameter specifying the third unimodal distribution or a value of the parameter specifying the third cumulative distribution function, a value of the parameter specifying the fourth unimodal distribution or a value of the parameter specifying the fourth cumulative distribution function, a value of the third weight parameter, a value of the fourth weight parameter, and a value of the second level parameter when the second target time waveform that is a waveform in the time section of the T wave in the waveform of the one cycle is approximated by a second approximate time waveform that is a waveform represented by a function obtained by subtracting, from a function obtained by multiplying the third inverse cumulative distribution function that is a function obtained by subtracting the third cumulative distribution function that is a cumulative distribution function of the third unimodal distribution from 1 by the value of the third weight parameter, a function obtained by multiplying the fourth inverse cumulative distribution function that is a function obtained by subtracting the fourth cumulative distribution function that is a cumulative distribution function of the fourth unimodal distribution from 1 by the value of the fourth weight parameter, and adding the value of the second level parameter. It is set that each of the first to fourth unimodal distributions is a Gaussian distribution, the average value of the first unimodal distribution is $\mu_{a0}$, the standard deviation value of the first unimodal distribution is $\sigma_{a0}$, the average value of the second unimodal distribution is $\mu_{b0}$, the standard deviation value of the second unimodal distribution is $\sigma_{b0}$, the average value of the third unimodal distribution is $\mu_{c0}$, the standard deviation value of the third unimodal distribution is $\sigma_{c0}$, the average value of the fourth unimodal distribution is $\mu_{d0}$, and the standard deviation value of the fourth unimodal distribution is $\sigma_{d0}$, the myocardial activity parameter set of the standard electrocardiogram stored in advance in the storage unit (not illustrated) in the converter acquisition unit 220 is ($\mu_{a0}$, $\sigma_{a0}$, $\mu_{b0}$, $\sigma_{b0}$, $k_{a0}$, $k_{b0}$, $\beta_{R0}$, $\mu_{c0}$, $\sigma_{c0}$, $\mu_{d0}$, $\sigma_{d0}$, $k_{c0}$, $k_{d0}$, $\beta_{T0}$).

The converter acquisition unit 220 generates a converter that converts the myocardial activity parameter set of the target heart into bijective so that the myocardial activity parameter set of the electrocardiogram of the target heart approaches the myocardial activity parameter set of the standard electrocardiogram. For example, the converter acquisition unit 220 generates the converter so as to minimize a sum of errors between each of myocardial activity parameter sets after conversion obtained by converting each of myocardial activity parameter sets of a plurality of cycles of the electrocardiogram of the target heart by the converter and the myocardial activity parameter set of the standard electrocardiogram. The converter acquisition unit 220 may generate one converter from the input myocardial activity parameter set of an entire cycle of the electrocardiogram of the target heart, or may generate one converter for each of the myocardial activity parameter sets of the plurality of cycles included in the input myocardial activity parameter set of the entire cycle of the electrocardiogram of the target heart.

The converter generated by the converter acquisition unit 220 may be a transformation matrix, a function, a neural network, or another converter. In any converter, for example, the converter acquisition unit 220 only needs to generate a converter that converts the myocardial activity parameter set of the target heart into bijective by using a known technique so as to minimize the sum of errors between each of the myocardial activity parameter sets after conversion obtained by converting each of the myocardial activity parameter sets of the plurality of cycles of the electrocardiogram of the target heart by the converter and the myocardial activity parameter set of the standard electrocardiogram.

[Modification of Converter Acquisition Unit 220]

A converter generated from a myocardial activity parameter set obtained from the waveform of the electrocardiogram acquired in the past from the target heart and the myocardial activity parameter set of the standard electrocardiogram may be stored in advance in the storage unit (not illustrated) in the converter acquisition unit 220, and the converter acquisition unit 220 may obtain the converter stored in advance. This mode will be described as a modification of the converter acquisition unit 220. Also in this modification, the converter obtained by the converter acquisition unit 220 is output from the converter acquisition unit 220 and input to the myocardial activity parameter conversion unit 240.

In the case of this modification, it is sufficient that, for example, as indicated by a broken line in Fig. 60, identification information (Hereinafter, it is referred to as a "target heart ID".) specifying the target heart is input to the signal conversion device 2 in addition to the waveform of the electrocardiogram of the target heart, and the target heart ID input to the signal conversion device 2 is input to the converter acquisition unit 220. The target heart ID is designated by the user such as a doctor. For example, it is sufficient that the signal conversion device 2 includes a display unit that is a display screen for displaying the target heart ID as an option, and an input unit that is an input interface such as a keyboard or a mouse for receiving selection by the user, and the display unit displays the target heart ID as the option, the input unit receives the selection by the user of any of target heart IDs, and the target heart ID of which the input unit has received selection is input to the converter acquisition unit 220. Note that, in the case of this modification, the myocardial activity parameter set of each cycle of the electrocardiogram of the target heart output from the signal analysis unit 210 does not have to be input to the converter acquisition unit 220. Furthermore, in this modification, the myocardial activity parameter set of the standard electrocardiogram does not have to be stored in the converter acquisition unit 220.

Specific processing in this modification is the following substeps S220-A1 to S220-A3. However, what the converter acquisition unit 220 performs when the signal conversion device 2 is operated is the following substeps S220-A3, and the following substeps S220-A1 and S220-A2 are performed in advance before using the signal conversion device 2.

Substep S220-A1: For each of a plurality of target hearts, one converter is obtained that converts the myocardial activity parameter set into bijective so that each of the myocardial activity parameter sets of the plurality of cycles of the electrocardiogram acquired in the past approaches the myocardial activity parameter set of the standard electrocardiogram. The converter may be a transformation matrix, a function, a neural network, or another converter, and only needs to be obtained by a known technique as described above.

Substep S220-A2: Each converter obtained in substep S220-A1 is stored in advance in the storage unit (not illustrated) in the converter acquisition unit 220 in association with the target heart ID.

Substep S220-A3: The converter acquisition unit 220 obtains a converter associated with the input target heart ID from the storage unit (not illustrated) in the converter acquisition unit 220.

[Conversion Information Setting Unit 230]

The conversion information setting unit 230 receives designation of one or more types of myocardial activity parameters by the user, and outputs conversion information that is information specifying a designated myocardial activity parameter (Hereinafter, the parameter is referred to as a "designated parameter".), to the myocardial activity parameter conversion unit 240 (step S230A). The designated parameter is designated by the user such as a doctor for the purpose of, for example, finding or confirming an abnormality in the activity of the myocardium of the target heart.

For example, the conversion information setting unit 230 includes a display unit that is a display screen for displaying identification information on a myocardial activity parameter as an option, and an input unit that is an input interface such as a keyboard or a mouse for receiving selection by the user, and the display unit displays the identification information on the myocardial activity parameter as the option, the input unit receives the selection by the user of any one or more types of the myocardial activity parameters as options, and the conversion information is output that is information specifying the one or more types of myocardial activity parameters and of which selection is received by the input unit. The myocardial activity parameters as options are, for example, the 14 types of myocardial activity parameters described above.

The conversion information may be any information as long as the information can specify which one of the options of the myocardial activity parameters the designated parameter is, may be an identification number or an identification number group indicating the type of the designated parameter, may be an identification number or an identification number group representing a type of a myocardial activity parameter that is not a designated parameter (Hereinafter, the parameter is also referred to as a "non-designated parameter".), or may be an information group representing whether each type of myocardial activity parameter as an option is the designated parameter or the non-designated parameter.

[Myocardial Activity Parameter Conversion Unit 240]

The myocardial activity parameter conversion unit 240 inputs the myocardial activity parameter set of each cycle of the electrocardiogram of the target heart output from the signal analysis unit 210, the converter output from the converter acquisition unit 220, and the conversion information output from the conversion information setting unit 230. In a case where the myocardial activity parameter set of each cycle of the electrocardiogram of the target heart is input to the signal conversion device 2, the myocardial activity parameter set of each cycle of the electrocardiogram of the target heart input to the signal conversion device 2 is input to the myocardial activity parameter conversion unit 240 instead of the myocardial activity parameter set of each cycle of the electrocardiogram of the target heart output from the signal analysis unit 210. The myocardial activity parameter conversion unit 240 sets the value of the myocardial activity parameter included in the input myocardial activity parameter set as it is as a value of a converted myocardial activity parameter, for the designated parameter specified by the conversion information, and sets a value obtained by converting the value of the myocardial activity parameter included in the input myocardial activity parameter set by the converter as the value of the converted myocardial activity parameter, for the myocardial activity parameter that is not the designated parameter specified by the conversion information (that is, the non-designated parameter), thereby obtaining a converted myocardial activity parameter set of each cycle of the electrocardiogram of the target heart (step S240A). For example, the myocardial activity parameter conversion unit 240 performs the following substeps S240A-1 and S240A-2, thereby obtaining the converted myocardial activity parameter set of each cycle of the electrocardiogram of the target heart. The converted myocardial activity parameter set of each cycle of the electrocardiogram of the target heart obtained by the myocardial activity parameter conversion unit 240 is input to the waveform synthesis unit 250.

[[Substep S240A-1]]

First, for each cycle of the electrocardiogram of the target heart, the myocardial activity parameter conversion unit 240 converts the myocardial activity parameter set by the converter to obtain a myocardial activity parameter set after conversion (Hereinafter, it is referred to as a "provisional converted myocardial activity parameter set".). Hereinafter, each parameter included in the provisional converted myocardial activity parameter set is referred to as a provisional converted myocardial activity parameter.

[[Substep S240A-2]]

Next, for each cycle of the electrocardiogram of the target heart, the myocardial activity parameter conversion unit 240 sets the value of the myocardial activity parameter included in the input myocardial activity parameter set as the value of the converted myocardial activity parameter, for the designated parameter specified by the conversion information, and sets the value of the provisional converted myocardial activity parameter included in the provisional converted myocardial activity parameter set as the value of the converted myocardial activity parameter, for the myocardial activity parameter that is not the designated parameter specified by the conversion information (that is, the non-designated parameter), thereby obtaining the converted myocardial activity parameter set.

The values of some converted myocardial activity parameters included in the converted myocardial activity parameter set are the values of the myocardial activity parameter included in the myocardial activity parameter set, and the values of the remaining converted myocardial activity parameters included in the converted myocardial activity parameter set are the values of the provisional converted myocardial activity parameter included in the provisional converted myocardial activity parameter set; however, hereinafter, the values of the respective converted myocardial activity parameters are denoted by the following symbols without distinguishing whether the values are the values of the myocardial activity parameter or the values of the provisional converted myocardial activity parameter. Note that the symbols of the average and symbols of the standard deviation below are those in a case where the first to fourth unimodal distributions are Gaussian distributions.

For the values of the respective converted myocardial activity parameters obtained by the myocardial activity parameter conversion unit 240 with respect to the values of the respective myocardial activity parameters obtained from the waveform in the time section of the R wave, the average value of the first unimodal distribution is denoted as $\mu'_a$, the standard deviation value of the first unimodal distribution is denoted as $\sigma'_a$, the average value of the second unimodal distribution is denoted as $\mu'_d$, the standard deviation value of the second unimodal distribution is denoted as $\sigma'_b$, the value of the first weight parameter is denoted as $k'_a$, the value of the second weight parameter is denoted as $k'_b$, and the value of the first level parameter is denoted as $\beta'_R$.

For the values of the respective converted myocardial activity parameters obtained by the myocardial activity parameter conversion unit 240 with respect to the values of the respective myocardial activity parameters obtained in the first analysis processing from the waveform in the time section of the T wave, the average value of the third unimodal distribution is denoted as $\mu'_e$, the standard deviation value of the third unimodal distribution is denoted as $\sigma'_e$, the average

value of the fourth unimodal distribution is denoted as $\mu'_g$, the standard deviation value of the fourth unimodal distribution is denoted as $\sigma'_g$, the value of the third weight parameter is denoted as $k'_e$, the value of the fourth weight parameter is denoted as $k'_g$, and the value of the second level parameter is denoted as $\beta'_T$.

For the values of the respective converted myocardial activity parameters obtained by the myocardial activity parameter conversion unit 240 with respect to the values of the respective myocardial activity parameters obtained in the second analysis processing from the waveform in the time section of the T wave, the average value of the third unimodal distribution is denoted by $\mu'_c$, the standard deviation value of the third unimodal distribution is denoted by $\sigma'_c$, the average value of the fourth unimodal distribution is denoted by $\mu'_d$, the standard deviation value of the fourth unimodal distribution is denoted by $\sigma'_d$, the value of the third weight parameter is denoted by $k'_c$, the value of the fourth weight parameter is denoted by $k'_d$, and the value of the second level parameter is denoted by $\beta'_T$.

[Waveform Synthesis Unit 250]

The converted myocardial activity parameter set of each cycle of the electrocardiogram of the target heart obtained by the myocardial activity parameter conversion unit 240 is input to the waveform synthesis unit 250. The waveform synthesis unit 250 performs first generation processing or second generation processing below for each cycle of the electrocardiogram of the target heart to obtain a synthesized signal of each cycle from the converted myocardial activity parameter set (step S250). Note that the waveform synthesis unit 250 may further connect the synthesized signals of a plurality of cycles together in order of cycles to obtain a synthesized signal for a plurality of cycles.

The first generation processing (step S250A) performed by the waveform synthesis unit 250 for each cycle is performed in a case where the signal analysis unit 210 performs the first analysis processing or in a case where the myocardial activity parameter set input to the signal conversion device 2 is obtained by the first analysis processing, and includes processing (substep S250A-1) of obtaining a synthesized waveform in the time section of the R wave of the cycle, processing (substep S250A-2) of obtaining a synthesized waveform in the time section of the T wave of the cycle, and processing (substep S250A-3) of connecting the synthesized waveform in the time section of the R wave of the cycle and the synthesized waveform in the time section of the T wave of the cycle together to obtain a synthesized signal of the cycle.

The processing in substep S250A-1 performed by the waveform synthesis unit 250 is processing of obtaining, as the synthesized waveform in the time section of the R wave, a time waveform represented by a function $k'_aF_a(x) - k'_bF_b(x) + \beta'_R$ obtained by subtracting, from a function $k'_aF_a(x)$ obtained by multiplying a cumulative distribution function $F_a(x)$ specified by the value of the converted myocardial activity parameter of the parameter specifying the first unimodal distribution or the parameter specifying the first cumulative distribution function by the value $k'_a$ of the converted myocardial activity parameter of the first weight parameter, a function $k'_bF_b(x)$ obtained by multiplying a cumulative distribution function $F_b(x)$ specified by the value of the converted myocardial activity parameter of the parameter specifying the second unimodal distribution or the parameter specifying the second cumulative distribution function by the value $k'_b$ of the converted myocardial activity parameter of the second weight parameter, and adding the value $\beta'_R$ of the converted myocardial activity parameter of the first level parameter.

In a case where the first unimodal distribution is a Gaussian distribution, the cumulative distribution function $F_a(x)$ is expressed by Formula (27) using the average value $\mu'_a$ and the standard deviation value $\sigma'_a$. Furthermore, in a case where the second unimodal distribution is a Gaussian distribution, the cumulative distribution function $F_b(x)$ is expressed by Formula (28) using the average value $\mu'_d$ and the standard deviation value $\sigma'_b$. Thus, in substep S250A-1, the waveform synthesis unit 250 only needs to obtain the synthesized waveform in the time section of the R wave by Formula (29).

[Math. 27]

$$F_a(x) = \frac{1}{2}\left(1 + erf\left(\frac{x - \mu'_a}{\sqrt{2\sigma'_a{}^2}}\right)\right) \quad \cdots (27)$$

[Math. 28]

$$F_b(x) = \frac{1}{2}\left(1 + erf\left(\frac{x - \mu'_b}{\sqrt{2\sigma'^2_b}}\right)\right) \quad \cdots (28)$$

[Math. 29]

$$k'_a F_a(x) - k'_b F_b(x) + \beta'_R = k'_a \frac{1}{2}\left(1 + erf\left(\frac{x - \mu'_a}{\sqrt{2\sigma'^2_a}}\right)\right) - k'_b \frac{1}{2}\left(1 + erf\left(\frac{x - \mu'_b}{\sqrt{2\sigma'^2_b}}\right)\right) + \beta'_R \quad \cdots (29)$$

Note that, in the function $k'_aF_a(x) - k'_bF_b(x) + \beta'_R$, an amount of temporal change of the value of the function is smaller than an amount of temporal change of the amplitude due to noise or fluctuation in the actually acquired electrocardiogram in a time range in which the value of the function is close to the minimum value $\beta'_R$ and a time range in which the value of the function is close to the maximum value $k'_a - k'_b + \beta'_R$. That is, for the purpose of obtaining a corrected waveform that makes it easy for the user such as a doctor to grasp the state of the heart, a time range in which the amount of temporal change of the value of the function $k'_aF_a(x) - k'_bF_b(x) + \beta'_R$ is less than the amount of temporal change of the amplitude due to noise or fluctuation does not need to be included in the time section of the R wave. Furthermore, if each unimodal distribution is a Gaussian distribution, for example, information on 99.7% is included in the time range up to the value in which a time difference from the average value is six times the standard deviation value, and thus, for the purpose of obtaining a corrected waveform that makes it easy for the user such as a doctor to grasp the state of the heart, it is not necessary to include other time ranges in the time section of the R wave. Thus, in substep S250A-1 performed by the waveform synthesis unit 250, in the time waveform represented by the function $k'_aF_a(x) - k'_bF_b(x) + \beta'_R$, a waveform in a time section necessary for the user such as the doctor to grasp the state of the heart only needs to be obtained as the synthesized waveform in the time section of the R wave. Specifically, for example, in the time waveform represented by the function $k'_aF_a(x) - k'_bF_b(x) + \beta'_R$, the waveform synthesis unit 250 only needs to obtain, as the synthesized waveform in the time section of the R wave, a time section determined by a predetermined method from the standard deviation value of the Gaussian distribution included in the function, or a time section in which the value of the function falls within a range determined by a predetermined method from the minimum value and the maximum value of the function. More specifically, for example, in the time waveform represented by the function $k'_aF_a(x) - k'_bF_b(x) + \beta'_R$, the waveform synthesis unit 250 only needs to obtain, as the synthesized waveform in the time section of the R wave, a waveform in a time section in which a time difference from the average value of the Gaussian distribution included in the function is up to a value six times the standard deviation value. Since there are two Gaussian distributions included in the function $k'_aF_a(x) - k'_bF_b(x) + B'_r$, there are four values in which the time difference from the average value of the Gaussian distribution is six times the standard deviation value; however, for example, it is sufficient that, a value closest to the start edge of the cycle among the values in which the time difference from the average value of the Gaussian distribution is six times the standard deviation value is set as the time of the start edge of the time section of the R wave, and a value closest to the end edge of the cycle among the values in which the time difference from the average value of the Gaussian distribution is six times the standard deviation value is set as the time of the end edge of the time section of the R wave, whereby a synthesized waveform in the time section of the R wave is obtained.

The processing in substep S250A-2 performed by the waveform synthesis unit 250 is processing of obtaining, as the synthesized waveform in the time section of the T wave, a time waveform obtained by inverting the time axis of a waveform represented by a function $k'_eF_e(x') - k'_gF_g(x') + \beta'_T$ obtained by subtracting, from a function $k'_eF_e(x')$ obtained by multiplying the cumulative distribution function $F_e(x')$ specified by the value of the converted myocardial activity parameter of the parameter specifying the third unimodal distribution or the parameter specifying the third cumulative distribution function by the value $k'_e$ of the converted myocardial activity parameter of the third weight parameter, a function $k'_gF_g(x')$ obtained by multiplying the cumulative distribution function $F_g(x')$ specified by the value of the converted myocardial activity parameter of the parameter specifying the fourth unimodal distribution or the parameter specifying the fourth cumulative distribution function by the value $k'_g$ of the converted myocardial activity parameter of the fourth weight parameter, and adding $\beta'_T$ of the value of the converted myocardial activity parameter of the second level parameter.

In a case where the third unimodal distribution is a Gaussian distribution, the cumulative distribution function $F_e$ (x') is expressed by Formula (30) using the average value $\mu'_e$ and the standard deviation value $\sigma'_e$. Furthermore, in a case where the fourth unimodal distribution is a Gaussian distribution, the cumulative distribution function $F_g(x')$ is expressed by Formula (31) using the average value $\mu'_g$ and the standard deviation value $\sigma'_g$. Thus, in substep S250A-2, the waveform synthesis unit 250 only needs to invert the time axis of the waveform obtained by Formula (32) to obtain the synthesized waveform in the time section of the T wave.

[Math. 30]

$$F_e(x') = \frac{1}{2}\left(1 + erf\left(\frac{x' - \mu'_e}{\sqrt{2\sigma'^2_e}}\right)\right) \quad \cdots (30)$$

[Math. 31]

$$F_g(x') = \frac{1}{2}\left(1 + erf\left(\frac{x' - \mu'_g}{\sqrt{2\sigma'^2_g}}\right)\right) \quad \cdots (31)$$

[Math. 32]

$$k'_e F_e(x') - k'_g F_g(x') + \beta'_T$$
$$= k'_e \frac{1}{2}\left(1 + erf\left(\frac{x' - \mu'_e}{\sqrt{2\sigma'^2_e}}\right)\right) - k'_g \frac{1}{2}\left(1 + erf\left(\frac{x' - \mu'_g}{\sqrt{2\sigma'^2_g}}\right)\right)$$
$$+ \beta'_T$$

・・・(32)

Note that, for a reason similar to that described in substep S250A-2, in substep S250A-2 performed by the waveform synthesis unit 250, it is sufficient to obtain, as the synthesized waveform in the time section of the T wave, a waveform in a time section necessary for the user such as a doctor to grasp the state of the heart, in the time waveform obtained by inverting the time axis of the waveform represented by the function $k'_eF_e(x') - k'_gF_g(x') + \beta'_T$. Specifically, for example, in the time waveform obtained by inverting the time axis of the waveform represented by the function $k'_eF_e(x') - k'_gF_g(x') + \beta'_T$, it is sufficient to obtain, as the synthesized waveform in the time section of the T wave, a time section determined by a predetermined method from the standard deviation value of the Gaussian distribution included in the function, or a time section in which the value of the function falls within a range determined by a predetermined method from the minimum value and the maximum value of the function. More specifically, for example, in the time waveform obtained by inverting the time axis of the waveform represented by the function $k'_eF_e(x') - k'_gF_g(x') + \beta'_T$, it is sufficient to obtain, as the synthesized waveform in the time section of the T wave, a waveform in a time section in which a time difference from the average value of the Gaussian distribution included in the function is up to a value six times the standard deviation value. Since there are two Gaussian distributions included in the time waveform obtained by inverting the time axis of the waveform represented by the function $k'_eF_e(x') - k'_gF_g(x') + \beta'_T$, there are four values in which the time difference from the average value of the Gaussian distribution is six times the standard deviation value; however, for example, it is sufficient that, a value closest to the start edge of the cycle among the values in which the time difference from the average value of the Gaussian distribution is six times the standard deviation value is set as the time of the start edge of the time section of the T wave, and a value closest to the end edge of the cycle among the values in which the time difference from the average value of the Gaussian distribution is six times the standard deviation value is set as the time of the end edge of the time section of the T wave, whereby a synthesized waveform in the time section of the T wave is obtained.

The processing in substep S250A-3 performed by the waveform synthesis unit 250 is processing of obtaining a synthesized signal for one cycle in which the synthesized waveform in the time section of the R wave and the synthesized waveform in the time section of the T wave are connected together, by connecting the start edge of the cycle and the start edge of the synthesized waveform in the time section of the R wave obtained in the processing in substep S250A-1 together by a predetermined method, connecting the end edge of the synthesized waveform in the time section of the R wave obtained in the processing in substep S250A-1 and the start edge of the synthesized waveform in the time section of the T wave obtained in the processing in substep S250A-2 together by the predetermined method, and connecting the end edge of the synthesized waveform in the time section of the T wave obtained in the processing in substep S250A-2 and the end edge of the cycle together by the predetermined method. The predetermined method is, for example, a method of connecting the start edge of the cycle and the start edge of the synthesized waveform in the time section of the R wave together, connecting the end edge of the synthesized waveform in the time section of the R wave and the start edge of the synthesized waveform in the time section of the T wave together, and connecting the end edge of the synthesized waveform in the time section of the T wave and the end edge of the cycle together by a straight line or a predetermined function. The predetermined function is, for example, a function that smoothly connects the waveforms at each of the start edge of the synthesized waveform in the time section of the R wave, the end edge of the synthesized waveform in the time section of the R wave, the start edge of the synthesized waveform in the time section of the T wave, and the end edge of the synthesized waveform in the time section of the T wave. Note that, when connecting the start edge of a certain cycle of the plurality of cycles and the start edge of the synthesized waveform in the time section of the R wave together, the waveform synthesis unit 250 may use a straight line or a predetermined function that connects the end edge of the synthesized waveform in the time section of the T wave of a cycle immediately before the certain cycle and the start edge of the synthesized waveform in the time section of the R wave of the certain cycle together. Similarly, when connecting the end edge of the synthesized waveform in the time section of the T wave of a certain cycle of the plurality of cycles and the end edge of the cycle together, the waveform synthesis unit 250 may use a straight line or a predetermined function that connects the end edge of the synthesized waveform in the time section of the T wave of the certain cycle and the start edge of the synthesized waveform in the time section of the R wave of a cycle immediately after the certain cycle.

The second generation processing (step S250B) performed by the waveform synthesis unit 250 for each cycle is performed in a case where the signal analysis unit 210 performs the second analysis processing or in a case where the myocardial activity parameter set input to the signal conversion device 2 is obtained by the second analysis processing, and includes processing of obtaining a synthesized waveform in the time section of the R wave of the cycle (substep S250B-1), processing of obtaining a synthesized waveform in the time section of the T wave of the cycle (substep S250B-2), and processing of connecting the synthesized waveform in the time section of the R wave of the cycle and the synthesized waveform in the time section of the T wave of the cycle together to obtain a synthesized signal of the cycle (substep S250B-3). Here, the processing in substep S250B-1 is the same as the processing in substep S250A-1, and the processing in substep S250B-3 is the same as the processing in substep S250A-3. Step S250B is different from step S250A in that the following substep S250B-2 is included instead of substep S250A-2.

The processing in substep S250B-2 performed by the waveform synthesis unit 250 is processing of obtaining, as the synthesized waveform in the time section of the T wave, a time waveform represented by a function $k'_c(1 - F_c(x)) - k'_d(1 - F_d(x)) + \beta'_T$ obtained by subtracting, from a function $k'_c(1 - F_c(x))$ obtained by multiplexing a function $1 - F_c(x)$ obtained by subtracting, from 1, a cumulative distribution function $F_c(x)$ specified by the value of the converted myocardial activity parameter of the parameter specifying the parameter of the third unimodal distribution or the parameter specifying the third cumulative distribution function, by the value $k'_c$ of the converted myocardial activity parameter of the third weight parameter, a function $k'_d(1 - F_d(x))$ obtained by multiplying a function $1 - F_d(x)$ obtained by subtracting, from 1, a cumulative distribution function $F_d(x)$ specified by the value of the converted myocardial activity parameter of the parameter specifying the parameter of the fourth unimodal distribution or the parameter specifying the fourth cumulative distribution function, by the value $k'_d$ of the converted myocardial activity parameter of the fourth weight parameter, and adding the value $\beta'_T$ of the converted myocardial activity parameter of the second level parameter.

In a case where the third unimodal distribution is a Gaussian distribution, the cumulative distribution function $F_c(x)$ is expressed by Formula (33) using the average value $\mu'_c$ and the standard deviation value $\sigma'_c$. Furthermore, in a case where the fourth unimodal distribution is a Gaussian distribution, the cumulative distribution function $F_d(x)$ is expressed by Formula (34) using the average value $\mu'_d$ and the standard deviation value $\sigma'_d$. Thus, in substep S250B-2, the waveform synthesis unit 250 only needs to obtain the synthesized waveform in the time section of the T wave by Formula (35).

[Math. 33]

$$F_c(x) = \frac{1}{2}\left(1 + erf\left(\frac{x - \mu'_c}{\sqrt{2\sigma'_c{}^2}}\right)\right) \quad \cdots (33)$$

[Math. 34]

$$F_d(x) = \frac{1}{2}\left(1 + erf\left(\frac{x - \mu'_d}{\sqrt{2\sigma'_d{}^2}}\right)\right) \quad \cdots (34)$$

[Math. 35]

$$k'_c\left(1-F_c(x)\right)-k'_d\left(1-F_d(x)\right)+\beta'_T$$
$$=k'_dF_d(x)-k'_cF_c(x)+k'_c-k'_d+\beta'_T$$
$$=k'_d\frac{1}{2}\left(1+erf\left(\frac{x-\mu'_d}{\sqrt{2\sigma'^2_d}}\right)\right)-k'_c\frac{1}{2}\left(1+erf\left(\frac{x-\mu'_c}{\sqrt{2\sigma'^2_c}}\right)\right)$$
$$+k'_c-k'_d+\beta'_T$$

・・・(35)

Note that, for a reason similar to that described in substep S250A-2, in substep S250B-2 performed by the waveform synthesis unit 250, it is sufficient to obtain, as the synthesized waveform in the time section of the T wave, a waveform in a time section necessary for the user such as a doctor to grasp the state of the heart, in the time waveforms by the function $k'_c(1 - F_c(x)) - k'_d(1 - F_d(x)) + \beta'_T$. Specifically, for example, in the time waveform represented by the function $k'_c(1 - F_c(x)) - k'_d(1 - F_d(x)) + \beta'_T$, it is sufficient to obtain, as the synthesized waveform in the time section of the T wave, a time section determined by a predetermined method from the standard deviation value of the Gaussian distribution included in the function, or a time section in which the value of the function falls within a range determined by a predetermined method from the minimum value and the maximum value of the function. More specifically, for example, in the time waveform represented by the function $k'_c(1 - F_c(x)) - k'_d(1 - F_d(x)) + \beta'_T$, it is sufficient to obtain, as the synthesized waveform in the time section of the T wave, a waveform in a time section in which a time difference from the average value of the Gaussian distribution included in the function is up to a value six times the standard deviation value. Since there are two Gaussian distributions included in the time waveform represented by the function $k'_c(1 - F_c(x)) - k'_d(1 - F_d(x)) + \beta'_T$, there are four values in which the time difference from the average value of the Gaussian distribution is six times the standard deviation value; however, for example, it is sufficient that, a value closest to the start edge of the cycle among the values in which the time difference from the average value of the Gaussian distribution is six times the standard deviation value is set as the time of the start edge of the time section of the T wave, and a value closest to the end edge of the cycle among the values in which the time difference from the average value of the Gaussian distribution is six times the standard deviation value is set as the time of the end edge of the time section of the T wave, whereby a synthesized waveform in the time section of the T wave is obtained.

[Method of Using Signal Conversion Device 2 of Second Embodiment]

For example, in a case where the doctor wants to confirm an abnormality of the myocardial outer layer of repolarization among activities of the myocardium of the target heart, the signal conversion device 2 of the second embodiment only needs to be operated, with the parameter specifying the fourth unimodal distribution or the parameter specifying the fourth cumulative distribution function, which is the myocardial outer layer parameter of repolarization, and the fourth weight parameter, as designated parameters. In this way, the signal conversion device 2 of the second embodiment outputs a synthesized signal in which characteristics other than a characteristic of the activity of the myocardial outer layer in repolarization among the activities of the myocardium of the target heart are greatly reduced, that is, a synthesized signal in which the characteristics of the activity of the myocardial outer layer in repolarization among the activities of the myocardium of the target heart is remarkably left. Thus, by using the signal conversion device 2 of the second embodiment, the doctor can confirm a corrected waveform in which the characteristic of the activity of the myocardial outer layer in repolarization among the activities of the myocardium of the target heart is remarkably left.

Alternatively, for example, in a case where the doctor wants to confirm an abnormality regarding ischemia of the target heart, the signal conversion device 2 of the second embodiment only needs to be operated with a level parameter and a weight parameter related to ischemia as designated parameters. As a result, the signal conversion device 2 of the second embodiment outputs a synthesized signal in which a characteristic related to ischemia of the target heart is remarkably left, and the doctor can confirm a corrected waveform in which the characteristic related to ischemia of the target heart is remarkably left.

That is, according to the second embodiment, the user such as a doctor can confirm a corrected waveform in which characteristics other than a characteristic related to a desired activity or a desired abnormality among the activities of the myocardium of the target heart are greatly reduced, that is, a corrected waveform in which the characteristic related to the desired activity or the desired abnormality among the activities of the myocardium of the target heart is remarkably left. Note that the characteristics reduced by the signal conversion device 2 of the second embodiment also include individual differences of the target heart, and the like.

<Modification 1 of Second Embodiment>

For each myocardial activity parameter, a value between a value of the myocardial activity parameter obtained from the waveform of the electrocardiogram of the target heart and a value obtained by converting the value of the myocardial activity parameter by the converter may be set as the value of the converted myocardial activity parameter. This mode will be described as Modification 1 of the second embodiment, focusing on differences from the second embodiment.

Similarly to the signal conversion device 2 of the second embodiment, the signal conversion device 2 of Modification 1 of the second embodiment includes the signal analysis unit 210, the converter acquisition unit 220, the conversion information setting unit 230, the myocardial activity parameter conversion unit 240, and the waveform synthesis unit 250, but operation of the conversion information setting unit 230 and operation of the myocardial activity parameter conversion unit 240 are different from those of the signal conversion device 2 of the second embodiment. The signal conversion device 2 of Modification 1 of the second embodiment performs steps S210, S220, S230B, S240B, and S250 illustrated in Fig. 61. Step S210 performed by the signal analysis unit 210 of the signal conversion device 2 of Modification 1 of the second embodiment, step S220 performed by the converter acquisition unit 220, and step S250 performed by the waveform synthesis unit 250 are similar to those of the signal conversion device 2 of the second embodiment.

Note that, similarly to the second embodiment, in a case where the myocardial activity parameter set of the target heart has already been obtained, the myocardial activity parameter set of the target heart may be input instead of the waveform of the electrocardiogram of the target heart. In this case, the signal conversion device 2 does not have to include the signal analysis unit 210, does not have to perform step S210, and can be said to be the signal synthesis device 3 that obtains and outputs a synthesized signal from the myocardial activity parameter set.

[Conversion Information Setting Unit 230]

The conversion information setting unit 230 receives designation of a reflection weight value of each myocardial activity parameter by the user, and outputs, to the myocardial activity parameter conversion unit 240, conversion information that is information specifying the designated reflection weight value for each myocardial activity parameter (step S230B). The reflection weight value of each myocardial activity parameter is designated by the user such as a doctor for the purpose of, for example, finding or confirming an abnormality in the activity of the myocardium of the target heart. For example, the conversion information setting unit 230 only needs to display a field or a slide bar for setting the reflection weight value of each myocardial activity parameter on the display unit, and output the conversion information including an input value by the user received by the input unit for each field or each slide bar as the reflection weight value of each myocardial activity parameter. The myocardial activity parameter to be a target of designation of the reflection weight value is, for example, the 14 types of myocardial activity parameters described above.

[Myocardial Activity Parameter Conversion Unit 240]

For each myocardial activity parameter of each cycle, the myocardial activity parameter conversion unit 240 sets, as a value of the converted myocardial activity parameter, a weighted average value using reflection weight values of a value of the myocardial activity parameter included in the input myocardial activity parameter set and a value obtained by converting the value of the myocardial activity parameter included in the input myocardial activity parameter set by the converter, thereby obtaining a converted myocardial activity parameter set of each cycle of the electrocardiogram of the target heart (step S240B). For example, for each myocardial activity parameter of each cycle, the myocardial activity parameter conversion unit 240 only needs to use the reflection weight value as weight given to the value of the myocardial activity parameter included in the input myocardial activity parameter set to obtain the weighted average value described above, and set the obtained weighted average value as the value of the converted myocardial activity parameter.

For example, when the lower limit value of a possible range of the reflection weight value is 0 and the upper limit value is 1, for each myocardial activity parameter of each cycle, the myocardial activity parameter conversion unit 240 obtains, as the value of the converted myocardial activity parameter, a value obtained by adding a value obtained by multiplying the value of the myocardial activity parameter included in the input myocardial activity parameter set by the reflection weight value and a value obtained by multiplying a value obtained by converting the value of the myocardial activity parameter included in the input myocardial activity parameter set by the converter by a value obtained by subtracting the reflection weight value from 1. That is, for each myocardial activity parameter of each cycle, it is set that the value of the myocardial activity parameter included in the input myocardial activity parameter set is a value of a first end of a possible range of the value of the converted myocardial activity parameter, and it is set that the value obtained by converting the value of the myocardial activity parameter included in the input myocardial activity parameter set by the converter is a value of a second end of the possible range of the value of the converted myocardial activity parameter, and the myocardial activity parameter conversion unit 240 obtains the value of the converted myocardial activity parameter so that a value closer to the first end of the possible range of the value of the converted myocardial activity parameter as the reflection weight value specified by the conversion information is larger is the value of the converted myocardial activity parameter, and a value closer to the second end of the possible range of the value of the converted myocardial activity parameter as the reflection weight value specified by the conversion information is smaller is the value of the converted myocardial activity parameter. Note that, the myocardial activity parameter conversion unit 240 only needs to set the value of the myocardial activity parameter included in the input myocardial activity parameter set as it is as the value of the converted myocardial activity parameter, for the myocardial activity parameter of which the reflection weight value is 1, and only needs to set the value obtained by converting the value of the myocardial activity parameter included in the input myocardial activity parameter set by the converter as the value of the converted myocardial activity parameter for the myocardial activity parameter of which the reflection weight value **is 0.**

Note that, for example, the myocardial activity parameter conversion unit 240 may set the lower limit value of the possible range of the reflection weight value to 0, set the upper limit value to a value larger than 1, and for each myocardial activity parameter of each cycle, obtain, as the value of the converted myocardial activity parameter, a value obtained by adding the value obtained by multiplying the value of the myocardial activity parameter included in the input myocardial activity parameter set by the reflection weight value and the value obtained by multiplying the value obtained by converting the value of the myocardial activity parameter included in the input myocardial activity parameter set by the converter by the value obtained by subtracting the reflection weight value from 1. That is, for each myocardial activity parameter of each cycle, it is set that a predetermined value in a direction opposite to a value obtained by converting the value of the myocardial activity parameter by the converter with respect to the value of the myocardial activity parameter included in the input myocardial activity parameter set is a value of the first end of the possible range of the value of the converted myocardial activity parameter, and it is set that a value obtained by converting the value of the myocardial activity parameter included in the input myocardial activity parameter set by the converter is a value of the second end of the possible range of the value of the converted myocardial activity parameter, and the myocardial activity parameter conversion unit 240 may obtain the value of the myocardial activity parameter so that a value closer to the first end of the possible range of the value of the converted myocardial activity parameter as the reflection weight value specified by the conversion information is larger is the value of the converted myocardial activity parameter, and a value closer to the second end of the possible range of the value of the converted myocardial activity parameter as the reflection weight value specified by the conversion information is smaller is the value of the converted myocardial activity parameter. Note that, in this case, in addition to the value between the value of the myocardial activity parameter obtained from the waveform of the electrocardiogram of the target heart and the value obtained by converting the value of the myocardial activity parameter by the converter, a value in the direction opposite to the value obtained by converting the value of the myocardial activity parameter by the converter with respect to the value of the myocardial activity parameter obtained from the waveform of the electrocardiogram of the target heart is also allowed to be the value of the converted myocardial activity parameter.

[Method of Using Signal Conversion Device 2 of Modification 1 of Second Embodiment]

For example, in a case where the doctor wants to confirm an abnormality of the myocardial outer layer of repolarization among activities of the myocardium of the target heart, the signal conversion device 2 of Modification 1 of the second embodiment only needs to be operated by designating reflection weight values such that reflection weight values of the parameter specifying the fourth unimodal distribution or the parameter specifying the fourth cumulative distribution function, which is the myocardial outer layer parameter of repolarization, and the fourth weight parameter are larger than reflection weight values of myocardial activity parameters other than these. In this way, the signal conversion device 2 of Modification 1 of the second embodiment outputs a synthesized signal in which characteristics other than the characteristic of the activity of the myocardial outer layer in repolarization among the activities of the myocardium of the target heart are further reduced, that is, a synthesized signal in which the characteristic of the activity of the myocardial outer layer in repolarization among the activities of the myocardium of the target heart is more greatly reflected. Thus, by using the signal conversion device 2 of the second embodiment, the doctor can confirm a corrected waveform in which the characteristic of the activity of the myocardial outer layer in repolarization among the activities of the myocardium of the target heart is more greatly reflected.

Furthermore, for example, in a case where the doctor wants to confirm an abnormality regarding ischemia of the target heart, the signal conversion device 2 of Modification 1 of the second embodiment only needs be operated by designating reflection weight values such that reflection weight values of the level parameter and the weight parameter related to ischemia are values larger than reflection weight values of the myocardial activity parameter other than these. As a result, the signal conversion device 2 of Modification 1 of the second embodiment outputs a synthesized signal in which the characteristic related to ischemia of the target heart is more greatly reflected, and the doctor can confirm a corrected waveform in which the characteristic related to ischemia of the target heart is more greatly reflected.

That is, according to Modification 1 of the second embodiment, the user such as a doctor can confirm a corrected waveform in which characteristics other than a characteristic related to a desired activity or a desired abnormality among the activities of the myocardium of the target heart are further reduced, that is, a corrected waveform in which the characteristic related to the desired activity or the desired abnormality among the activities of the myocardium of the target heart is more greatly reflected. Note that the characteristics reduced by the signal conversion device 2 of Modification 1 of the second embodiment also include individual differences of the target heart, and the like.

<Modification 2 of Second Embodiment>

Instead of the value obtained by converting the value of the myocardial activity parameter obtained from the waveform of the electrocardiogram of the target heart by the converter in the second embodiment, a value of the myocardial activity parameter obtained from the waveform of the standard electrocardiogram may be used. That is, for each myocardial activity parameter, either the value of the myocardial activity parameter obtained from the waveform of the electrocardiogram of the target heart or the value of the myocardial activity parameter obtained from the waveform of the standard electrocardiogram may be used as the value of the converted myocardial activity parameter. This mode will be described as Modification 2 of the second embodiment, focusing on differences from the second embodiment.

Fig. 62 is a diagram illustrating an example of a functional configuration of the signal conversion device 2 of Modification 2 of the second embodiment. The signal conversion device 2 of Modification 2 of the second embodiment includes the signal analysis unit 210, the conversion information setting unit 230, the myocardial activity parameter conversion unit 240, and the waveform synthesis unit 250. The signal conversion device 2 of Modification 2 of the second embodiment is different from the signal conversion device 2 of the second embodiment in that the converter acquisition unit 220 is not included, and in the operation and stored contents of the myocardial activity parameter conversion unit 240. The signal conversion device 2 of Modification 2 of the second embodiment performs steps S210, S230A, S240C, and S250 illustrated in Fig. 63. Step S210 performed by the signal analysis unit 210 of the signal conversion device 2 of Modification 2 of the second embodiment, step S230A performed by the conversion information setting unit 230, and step S250 performed by the waveform synthesis unit 250 are similar to those of the signal conversion device 2 of the second embodiment. A method of using the signal conversion device 2 of Modification 2 of the second embodiment is similar to the method of using the signal conversion device 2 of the second embodiment.

Similarly to the second embodiment, in a case where the myocardial activity parameter set of the target heart has already been obtained, the myocardial activity parameter set of the target heart may be input instead of the waveform of the electrocardiogram of the target heart. In this case, the signal conversion device 2 does not have to include the signal analysis unit 210, does not have to perform step S210, and can be said to be the signal synthesis device 3 that obtains and outputs a synthesized signal from the myocardial activity parameter set.

[Myocardial Activity Parameter Conversion Unit 240]

The myocardial activity parameter conversion unit 240 inputs the myocardial activity parameter set of each cycle of the electrocardiogram of the target heart output from the signal analysis unit 210, and the conversion information output from the conversion information setting unit 230. The myocardial activity parameter set of the standard electrocardiogram is stored in advance in a storage unit (not illustrated) in the myocardial activity parameter conversion unit 240. The myocardial activity parameter conversion unit 240 sets the value of the myocardial activity parameter included in the input myocardial activity parameter set as it is as a value of the converted myocardial activity parameter, for the designated parameter specified by the conversion information, and sets a value of the myocardial activity parameter included in the myocardial activity parameter set of the standard electrocardiogram as a value of the converted myocardial activity parameter, for the myocardial activity parameter that is not the designated parameter specified by the conversion information (that is, the non-designated parameter), thereby obtaining a converted myocardial activity parameter set of each cycle of the electrocardiogram of the target heart (step S240C).

<Modification 3 of Second Embodiment>

Instead of the value obtained by converting the value of the myocardial activity parameter obtained from the waveform of the electrocardiogram of the target heart by the converter in Modification 1 of the second embodiment, the value of the myocardial activity parameter obtained from the waveform of the standard electrocardiogram may be used. That is, for each myocardial activity parameter, a value between the value of the myocardial activity parameter obtained from the waveform of the electrocardiogram of the target heart and the value of the myocardial activity parameter obtained from the waveform of the standard electrocardiogram may be set as the value of the converted myocardial activity parameter. This mode will be described as Modification 3 of the second embodiment, focusing on differences from Modification 1 of the second embodiment.

An example of a functional configuration of the signal conversion device 2 of Modification 3 of the second embodiment is illustrated in Fig. 62 similarly to the functional configuration of the signal conversion device 2 of Modification 2 of the second embodiment. That is, the signal conversion device 2 of Modification 3 of the second embodiment includes the signal analysis unit 210, the conversion information setting unit 230, the myocardial activity parameter conversion unit 240, and the waveform synthesis unit 250. The signal conversion device 2 of Modification 3 of the second embodiment is different from the signal conversion device 2 of Modification 1 of the second embodiment in that the converter acquisition unit 220 is not included, and in the operation and stored contents of the myocardial activity parameter conversion unit 240. The signal conversion device 2 of Modification 3 of the second embodiment performs steps S210, S230B, S240D, and S250 illustrated in Fig. 63. Step S210 performed by the signal analysis unit 210 of the signal conversion device 2 of Modification 3 of the second embodiment, step S230B performed by the conversion information setting unit 230, and step S250 performed by the waveform synthesis unit 250 are similar to those of the signal conversion device 2 of Modification 1 of the second embodiment. A method of using the signal conversion device 2 of Modification 3 of the second embodiment is similar to the method of using the signal conversion device 2 of Modification 1 of the second embodiment.

Similarly to Modification 1 of the second embodiment, in a case where the myocardial activity parameter set of the target heart has already been obtained, the myocardial activity parameter set of the target heart may be input instead of the waveform of the electrocardiogram of the target heart. In this case, the signal conversion device 2 does not have to include the signal analysis unit 210, does not have to perform step S210, and can be said to be the signal synthesis device 3 that obtains and outputs a synthesized signal from the myocardial activity parameter set.

[Myocardial Activity Parameter Conversion Unit 240]

The myocardial activity parameter conversion unit 240 inputs the myocardial activity parameter set of each cycle of the electrocardiogram of the target heart output from the signal analysis unit 210, and the conversion information output from the conversion information setting unit 230. In a case where the myocardial activity parameter set of each cycle of the electrocardiogram of the target heart is input to the signal conversion device 2, the myocardial activity parameter set of each cycle of the electrocardiogram of the target heart input to the signal conversion device 2 is input to the myocardial activity parameter conversion unit 240 instead of the myocardial activity parameter set of each cycle of the electrocardiogram of the target heart output from the signal analysis unit 210. The myocardial activity parameter set of the standard electrocardiogram is stored in advance in a storage unit (not illustrated) in the myocardial activity parameter conversion unit 240. For each myocardial activity parameter of each cycle, the myocardial activity parameter conversion unit 240 sets, as a value of the converted myocardial activity parameter, a weighted average value using reflection weight values of a value of the myocardial activity parameter included in the input myocardial activity parameter set and the value of the myocardial activity parameter included in the myocardial activity parameter set of the standard electrocardiogram, thereby obtaining a converted myocardial activity parameter set of each cycle of the electrocardiogram of the target heart (step S240D).

For example, when the lower limit value of the possible range of the reflection weight value is 0 and the upper limit value is 1, for each myocardial activity parameter of each cycle, the myocardial activity parameter conversion unit 240 obtains, as the value of the converted myocardial activity parameter, a value obtained by adding the value obtained by multiplying the value of the myocardial activity parameter included in the input myocardial activity parameter set by the reflection weight value and a value obtained by multiplying the value of the myocardial activity parameter included in the myocardial activity parameter set of the standard electrocardiogram by the value obtained by subtracting the reflection weight value from 1. That is, for each myocardial activity parameter of each cycle, it is set that the value of the myocardial activity parameter included in the input myocardial activity parameter set is the value of the first end of the possible range of the value of the converted myocardial activity parameter, and it is set that the value of the myocardial activity parameter included in the myocardial activity parameter set of the standard electrocardiogram is the value of the second end of the possible range of the value of the converted myocardial activity parameter, and the myocardial activity parameter conversion unit 240 obtains the value of the converted myocardial activity parameter so that a value closer to the first end of the possible range of the value of the converted myocardial activity parameter as the reflection weight value specified by the conversion information is larger is the value of the converted myocardial activity parameter, and a value closer to the second end of the possible range of the value of the converted myocardial activity parameter as the reflection weight value specified by the conversion information is smaller is the value of the converted myocardial activity parameter. Note that, the myocardial activity parameter conversion unit 240 only needs to set the value of the myocardial activity parameter included in the input myocardial activity parameter set as it is as the value of the converted myocardial activity parameter, for the myocardial activity parameter of which the reflection weight value is 1, and only needs to set the value of the myocardial activity parameter included in the myocardial activity parameter set of the standard electrocardiogram as it is as the value of the converted myocardial activity parameter, for the myocardial activity parameter of which the reflection weight value is 0.

Note that, for example, the myocardial activity parameter conversion unit 240 may set the lower limit value of the possible range of the reflection weight value to 0, set the upper limit value to a value larger than 1, and for each myocardial activity parameter of each cycle, obtain, as the value of the converted myocardial activity parameter, a value obtained by adding the value obtained by multiplying the value of the myocardial activity parameter included in the input myocardial activity parameter set by the reflection weight value and the value obtained by multiplying the value of the myocardial activity parameter included in the myocardial activity parameter set of the standard electrocardiogram by the value obtained by subtracting the reflection weight value from 1. That is, for each myocardial activity parameter of each cycle, it is set that a predetermined value in a direction opposite to the value of the myocardial activity parameter included in the myocardial activity parameter set of the standard electrocardiogram with respect to the value of the myocardial activity parameter included in the input myocardial activity parameter set is the value of the first end of the possible range of the value of the converted myocardial activity parameter, and it is set that the value of the myocardial activity parameter included in the myocardial activity parameter set of the standard electrocardiogram is the value of the second end of the possible range of the value of the converted myocardial activity parameter, and the myocardial activity parameter conversion unit 240 may obtain the value of the myocardial activity parameter so that a value closer to the first end of the possible range of the value of the converted myocardial activity parameter as the reflection weight value specified by the conversion information is larger is the value of the converted myocardial activity parameter, and a value closer to the second end of the possible range of the value of the converted myocardial activity parameter as the reflection weight value specified by the conversion information is smaller is the value of the converted myocardial activity parameter. Note that, in this case, in addition to a value between the value of the myocardial activity parameter obtained from the waveform of the electrocardiogram of the target heart and the value of the myocardial activity parameter included in the myocardial activity parameter set of the standard electrocardiogram, a value in a direction opposite to the value of the myocardial activity parameter included in the myocardial activity parameter set of the standard electrocardiogram with respect to the value of the myocardial activity parameter obtained from the waveform of the electrocardiogram of the target heart is also allowed to be the value of the converted myocardial activity parameter.

As described above, the myocardial activity parameter conversion unit 240 of the second embodiment and Modifications 1 to 3 thereof obtains a converted myocardial activity parameter value by performing conversion of bringing the myocardial activity parameter value included in the myocardial activity parameter set of the target heart close to or away from a myocardial activity parameter value included in the myocardial activity parameter set of the standard electrocardiogram on one or more types of myocardial activity parameters.

<Modification 4 of Second Embodiment>

For each myocardial activity parameter, a representative value of values of myocardial activity parameters obtained from waveforms of a plurality of cycles of the electrocardiogram of the target heart may be used as a value of the converted myocardial activity parameter. This mode will be described as Modification 4 of the second embodiment, focusing on differences from the second embodiment.

Fig. 64 is a diagram illustrating an example of a functional configuration of the signal conversion device 2 of Modification 4 of the second embodiment. The signal conversion device 2 of Modification 4 of the second embodiment includes the signal analysis unit 210, the myocardial activity parameter conversion unit 240, and the waveform synthesis unit 250. The signal conversion device 2 of Modification 4 of the second embodiment is different from the signal conversion device 2 of the second embodiment in that the converter acquisition unit 220 and the conversion information setting unit 230 are not included, and in the operation of the myocardial activity parameter conversion unit 240. The signal conversion device 2 of Modification 4 of the second embodiment performs steps S210, S240E, and S250 illustrated in Fig. 65. Step S210 performed by the signal analysis unit 210 of the signal conversion device 2 of Modification 4 of the second embodiment and step S250 performed by the waveform synthesis unit 250 are similar to those of the signal conversion device 2 of the second embodiment.

Similarly to the second embodiment, in a case where the myocardial activity parameter set of the target heart has already been obtained, the myocardial activity parameter set of the target heart may be input instead of the waveform of the electrocardiogram of the target heart. In this case, the signal conversion device 2 does not have to include the signal analysis unit 210, does not have to perform step S210, and can be said to be the signal synthesis device 3 that obtains and outputs a synthesized signal from the myocardial activity parameter set.

[Myocardial Activity Parameter Conversion Unit 240]

The myocardial activity parameter set of each cycle of the electrocardiogram of the target heart output from the signal analysis unit 210 or the myocardial activity parameter set of each cycle of the electrocardiogram of the target heart input to the signal conversion device 2 is input to the myocardial activity parameter conversion unit 240. For each myocardial activity parameter of each cycle, the myocardial activity parameter conversion unit 240 sets, as a value of the converted myocardial activity parameter, a representative value of values of the myocardial activity parameters of a plurality of cycles including the cycle (Hereinafter, the cycle is referred to as a "target cycle".), thereby obtaining a converted myocardial activity parameter set of each cycle of the electrocardiogram of the target heart (step S240E). The representative value is, for example, a median value, an average value, a minimum value, a maximum value, or a moving average value.

The plurality of cycles including the target cycle may be a plurality of consecutive cycles including the target cycle, or may be a plurality of non-consecutive cycles including the target cycle. Furthermore, the plurality of cycles including the target cycle may be a plurality of cycles including a past cycle with respect to the target cycle and the target cycle, may be a plurality of cycles including a past cycle with respect to the target cycle, a future cycle with respect to the target cycle, and the target cycle, or may be a plurality of cycles including a future cycle with respect to the target cycle and the target cycle. Types of cycles to be used as the plurality of cycles including the target cycle may be set in advance, but may be designated by the user such as a doctor. In a case where designation is made by the user such as a doctor, it is sufficient that the signal conversion device 2 includes a display unit that is a display screen displaying options for the plurality of cycles including the target cycle, and an input unit that is an input interface such as a keyboard or a mouse that receives selection by the user, and the display unit displays the options, the input unit receives selection by the user for any of the options, and a selection result received by the input unit is input to the myocardial activity parameter conversion unit 240.

[Method of Using Signal Conversion Device 2 of Modification 4 of Second Embodiment]

For example, in a case where the doctor wants to confirm the activity of the myocardium other than the respiratory fluctuation of the target heart, the signal conversion device 2 of Modification 4 of the second embodiment only needs to be operated using the number of cardiac cycles corresponding to the time of one or more cycles of respiration by inspiration and expiration, with consecutive cycles of greater than or equal to the number of cardiac cycles as the "plurality of cycles including the target cycle". In this way, for each myocardial activity parameter of each cycle, the signal conversion device 2 obtains a synthesized signal by using a representative value of values of myocardial activity parameters of the cardiac cycles corresponding to the time of one or more cycles of respiration including the cycle as a value of the converted myocardial activity parameter. As a result, the signal conversion device 2 of Modification 4 of the second embodiment outputs a synthesized signal in which the influence of the respiratory fluctuation is reduced, and the doctor can confirm a corrected waveform in which the influence of the respiratory fluctuation is reduced of the electrocardiogram of the target heart.

Furthermore, for example, in a case where the doctor wants to confirm the activity of the myocardium other than a desired physiological fluctuation of the target heart, the signal conversion device 2 of Modification 4 of the second embodiment only needs to be operated such that both a cycle of the electrocardiogram of the target heart acquired by applying a predetermined load that causes the desired physiological fluctuation and a cycle of the electrocardiogram of the target heart acquired without applying the load are included in the "plurality of cycles including the target cycle". For example, the signal conversion device 2 of Modification 4 of the second embodiment only needs to continuously acquire the electrocardiogram of the target heart in a state where the predetermined load that causes the desired physiological fluctuation is applied and a state where the predetermined load is not applied, and obtain a synthesized signal by using a representative value of the entire cycle of the acquired electrocardiogram as the value of the converted myocardial activity parameter.

The load that causes the desired physiological fluctuation includes a load related to respiration (for example, deep breath or ventilation by a ventilator), a load related to posture (for example, a body motion of a posture change such as lying, standing, sitting, side lying, changes of these, head-up, headdown, leg raising, or standing up), a load related to acceleration or pressure (pressurization or depressurization of the body such as the lower limbs or the abdomen, which provides acceleration to the body), a load due to exercise, a load due to drug administration, a load due to a change in circulatory dynamics (for example, infusion, diuretic, dialysis), and the like.

In a case where the doctor wants to confirm the activity of the myocardium other than the desired physiological fluctuation of the target heart, the signal conversion device 2 of Modification 4 of the second embodiment may be operated such that both a cycle of the electrocardiogram of the target heart acquired by greatly applying the predetermined load that causes the desired physiological fluctuation and a cycle of the electrocardiogram of the target heart acquired by applying the load in a small amount are included in the "plurality of cycles including the target cycle". For example, the signal conversion device 2 of Modification 4 of the second embodiment may continuously acquire the electrocardiogram of the target heart in a state where the predetermined load is applied greatly and a state where the predetermined load is applied in a small amount, and obtain a synthesized signal by using a representative value of the entire cycle of the acquired electrocardiogram as the value of the converted myocardial activity parameter.

In a case where the doctor wants to confirm the activity of the myocardium other than the desired physiological fluctuation of the target heart, the signal conversion device 2 of Modification 4 of the second embodiment may be operated such that both a cycle of the electrocardiogram of the target heart acquired by applying a predetermined first load that causes the desired physiological fluctuation and a cycle of the electrocardiogram of the target heart acquired by applying a predetermined second load that causes the physiological fluctuation are included in the "plurality of cycles including the target cycle". For example, the signal conversion device 2 of Modification 4 of the second embodiment may continuously acquire the electrocardiogram of the target heart in a state where the predetermined first load is applied and a state where the predetermined second load is applied, and obtain a synthesized signal by using a representative value of the entire cycle of the acquired electrocardiogram as the value of the converted myocardial activity parameter.

As described above, for each myocardial activity parameter, the signal conversion device 2 obtains the synthesized signal by using the representative value of the values of the myocardial activity parameters of the cardiac cycles when the loads are different as the value of the converted myocardial activity parameter. As a result, the signal conversion device 2 of Modification 4 of the second embodiment outputs a synthesized signal in which the influence of the desired physiological fluctuation is reduced, and the doctor can confirm a corrected waveform in which the influence of the desired physiological fluctuation is reduced of the electrocardiogram of the target heart.

<Modification 5 of Second Embodiment>

In the myocardial activity parameter conversion unit 240 of the second embodiment and Modifications 1 to 3 thereof, instead of the value of the myocardial activity parameter obtained from the waveform of the electrocardiogram of the target heart, a representative value of values of myocardial activity parameters obtained from waveforms of a plurality of cycles of the electrocardiogram of the target heart may be used. This mode will be described as Modification 5 of the second embodiment, and points different from the second embodiment and Modifications 1 to 3 will be described. Modification 5 of the second embodiment is different from the second embodiment and Modifications 1 to 3 thereof in the operation of the myocardial activity parameter conversion unit 240.

[Myocardial Activity Parameter Conversion Unit 240]

As indicated by a two-dot chain line in Figs. 61 and 63, for each myocardial activity parameter of each cycle, the myocardial activity parameter conversion unit 240 first obtains a representative value of values of myocardial activity parameters of a plurality of cycles including the cycle (step S240$\alpha$). A method by which the myocardial activity parameter conversion unit 240 obtains the representative value is similar to that in the second embodiment and Modification 4 thereof. Then, for each myocardial activity parameter of each cycle, the myocardial activity parameter conversion unit 240 performs step S240A of the second embodiment, step S240B of Modification 1 of the second embodiment, step S240C of Modification 2 of the second embodiment, or step S240D of Modification 3 of the second embodiment using the representative value obtained in step S240$\alpha$ instead of the value of the myocardial activity parameter included in the input myocardial activity parameter set.

By using the signal conversion device 2 of Modification 5 of the second embodiment, it is possible to obtain a synthesized signal in which a characteristic related to a desired activity or abnormality is more greatly left in a state where the influence of a desired physiological fluctuation is reduced. That is, by using the signal conversion device 2 of Modification 5 of the second embodiment, it is possible for the user such as a doctor to confirm a corrected waveform in which the influence of the desired physiological fluctuation among the activities of the myocardium of the target heart is reduced and the characteristic related to the desired activity or abnormality among the activities of the myocardium of the target heart is more greatly left.

<Modification 6 of Second Embodiment>

For any of the myocardial activity parameters, the value of the myocardial activity parameter obtained from the waveform of the electrocardiogram of the target heart may be used as it is to obtain the synthesized signal without performing the conversion performed by the myocardial activity parameter conversion unit 240 of the second embodiment and Modifications 1 to 5 thereof. This mode will be described as Modification 6 of the second embodiment, focusing on differences from the second embodiment.

Fig. 66 is a diagram illustrating an example of a functional configuration of the signal conversion device 2 of Modification 6 of the second embodiment. The signal conversion device 2 of Modification 6 of the second embodiment includes the signal analysis unit 210 and the waveform synthesis unit 250. The signal conversion device 2 of Modification 6 of the second embodiment is different from the signal conversion device 2 in that the converter acquisition unit 220, the conversion information setting unit 230, and the myocardial activity parameter conversion unit 240 are not included. The signal conversion device 2 of Modification 6 of the second embodiment performs steps S210 and S250 illustrated in Fig. 67. Step S210 performed by the signal analysis unit 210 of the signal conversion device 2 of Modification 6 of the second embodiment is similar to that of the signal conversion device 2 of the second embodiment.

Note that, similarly to the second embodiment, in a case where the myocardial activity parameter set of the target heart has already been obtained, the myocardial activity parameter set of the target heart may be input instead of the waveform of the electrocardiogram of the target heart. In this case, the signal conversion device 2 does not have to include the signal analysis unit 210, does not have to perform step S210, and can be said to be the signal synthesis device 3 that obtains and outputs a synthesized signal from the myocardial activity parameter set.

[Waveform Synthesis Unit 250]

The myocardial activity parameter set of each cycle of the electrocardiogram of the target heart output from the signal analysis unit 210 is input to the waveform synthesis unit 250. For each cycle of the electrocardiogram of the target heart, the waveform synthesis unit 250 performs the first generation processing or the second generation processing described in the second embodiment by using the value of the myocardial activity parameter included in the input myocardial activity parameter set instead of the value of the converted myocardial activity parameter described in the second embodiment and Modifications 1 to 5 thereof, thereby obtaining a synthesized signal of each cycle (step S250).

The synthesized signal output from the signal conversion device 2 of Modification 6 of the second embodiment is neither a waveform in which characteristics other than a characteristic related to a desired activity or a desired abnormality are greatly reduced nor a waveform in which the influence of a desired physiological fluctuation is reduced, but is a waveform generated by a myocardial activity parameter representing a main characteristic of the activity of the myocardium of the target heart. That is, the synthesized signal output from the signal conversion device 2 of Modification 6 of the second embodiment is a waveform in which noise is reduced that is not a main characteristic of the activity of the myocardium included in the waveform of the electrocardiogram of the target heart. That is, according to the signal conversion device 2 of Modification 6 of the second embodiment, the user such as a doctor can confirm a corrected waveform in which the noise included in the waveform of the electrocardiogram of the target heart is reduced.

Note that, all or some of the functions of the signal analysis device 1 and/or the signal conversion device 2 and/or the signal synthesis device 3 may be implemented by using hardware such as an application specific integrated circuit (ASIC), a programmable logic device (PLD), or a field programmable gate array (FPGA). The program may be recorded on a computer-readable recording medium. The computer-readable recording medium is, for example, a portable medium such as a flexible disk, a magnetooptical disk, a ROM, or a CD-ROM or a storage device such as a hard disk built in a computer system. The program may be transmitted via an electrical communication line.

Although the embodiments of the present invention have been described in detail with reference to the drawings, specific configurations are not limited to the embodiments and include design and the like within the gist of the present invention.

Reference Signs List

| | |
|---|---|
| 1 | Signal analysis device |
| 2 | Signal conversion device |
| 3 | Signal synthesis device |
| 11 | Control unit |
| 12 | Input unit |
| 13 | Communication unit |
| 14 | Storage unit |
| 15 | Output unit |
| 91 | Processor |
| 92 | Memory |
| 110 | Electrocardiogram acquisition unit |
| 120 | Fitting information acquisition unit |
| 130 | Analysis unit |
| 131 | Fitting unit |
| 132 | Myocardial activity information parameter acquisition unit |
| 140 | Recording unit |

210 Signal analysis unit
220 Converter acquisition unit
230 Conversion information setting unit
240 Myocardial activity parameter conversion unit
250 Waveform synthesis unit

## Claims

**1.** A signal synthesis device comprising
a waveform synthesis unit that:

sets a waveform in a time section of an R wave included in a waveform for one cycle indicating a cardiac cycle of a heart as a first target time waveform;
sets a waveform in a time section of a T wave included in the waveform as a second target time waveform;
sets a waveform obtained by inverting a time axis of the second target time waveform as a second target inverse time waveform;
sets a set as a myocardial activity parameter set, the set including
a value of a parameter specifying a first unimodal distribution or a value of a parameter specifying a first cumulative distribution function, a value of a parameter specifying a second unimodal distribution or a value of a parameter specifying a second cumulative distribution function, a value of a first weight parameter, a value of a second weight parameter, and a value of a first level parameter when the first target time waveform is approximated by a first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplying the second cumulative distribution function that is a cumulative distribution function of the second unimodal distribution by the value of the second weight parameter, and adding the value of the first level parameter; and
a value of a parameter specifying a third unimodal distribution or a value of a parameter specifying a third cumulative distribution function, a value of a parameter specifying a fourth unimodal distribution or a value of a parameter specifying a fourth cumulative distribution function, a value of a third weight parameter, a value of a fourth weight parameter, and a value of a second level parameter when the second target inverse time waveform is approximated by a second approximate inverse time waveform that is a waveform represented by a function obtained by subtracting, from a function obtained by multiplying the third cumulative distribution function that is a cumulative distribution function of the third unimodal distribution by the value of the third weight parameter, a function obtained by multiplying the fourth cumulative distribution function that is a cumulative distribution function of the fourth unimodal distribution by the value of the fourth weight parameter, and adding the value of the second level parameter;
sets each of element values included in the myocardial activity parameter set as a myocardial activity parameter value;
uses each of myocardial activity parameter values included in the myocardial activity parameter set of a heart to be a target (Hereinafter, the heart is referred to as a "target heart".);
obtains, as a synthesized waveform in the time section of the R wave, a time waveform represented by a function obtained by subtracting, from a function obtained by multiplying a cumulative distribution function specified by the myocardial activity parameter value of the parameter specifying the first unimodal distribution or the first cumulative distribution function by the myocardial activity parameter value of the first weight parameter, a function obtained by multiplying a cumulative distribution function specified by the myocardial activity parameter value of the parameter specifying the second unimodal distribution or the second cumulative distribution function by the myocardial activity parameter value of the second weight parameter, and adding the myocardial activity parameter value of the first level parameter;
obtains, as a synthesized waveform in the time section of the T wave, a waveform obtained by inverting a time axis of a waveform represented by a function obtained by subtracting, from a function obtained by multiplying a cumulative distribution function specified by the myocardial activity parameter value of the parameter specifying the third unimodal distribution or the third cumulative distribution function by the myocardial activity parameter value of the third weight parameter, a function obtained by multiplying a cumulative distribution function specified by the myocardial activity parameter value of the parameter specifying the fourth unimodal distribution or the fourth cumulative distribution function by the myocardial activity parameter value of the fourth weight parameter, and adding the myocardial activity parameter value of the second level parameter; and
connects the synthesized waveform in the time section of the R wave and the synthesized waveform in the time section of the T wave together to obtain a synthesized signal for a waveform indicating the cardiac cycle of the target heart.

**2.** A signal synthesis device comprising
a waveform synthesis unit that:

sets a waveform in a time section of an R wave included in a waveform for one cycle indicating a cardiac cycle of a heart as a first target time waveform;
sets a waveform in a time section of a T wave included in the waveform as a second target time waveform;
sets a set as a myocardial activity parameter set, the set including
a value of a parameter specifying a first unimodal distribution or a value of a parameter specifying a first cumulative distribution function, a value of a parameter specifying a second unimodal distribution or a value of a parameter specifying a second cumulative distribution function, a value of a first weight parameter, a value of a second weight parameter, and a value of a first level parameter when the first target time waveform is approximated by a first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplying the second cumulative distribution function that is a cumulative distribution function of the second unimodal distribution by the value of the second weight parameter, and adding the value of the first level parameter, and
a value of a parameter specifying a third unimodal distribution or a value of a parameter specifying a third cumulative distribution function, a value of a parameter specifying a fourth unimodal distribution or a value of a parameter specifying a fourth cumulative distribution function, a value of a third weight parameter, a value of a fourth weight parameter, and a value of a second level parameter when the second target time waveform is approximated by a second approximate time waveform that is a time waveform represented by a function obtained by subtracting, from a function obtained by subtracting, from 1, a function obtained by multiplying the third cumulative distribution function that is a cumulative distribution function of the third unimodal distribution by the value of the third weight parameter, a function obtained by subtracting, from 1, a function obtained by multiplying the fourth cumulative distribution function that is a cumulative distribution function of the fourth unimodal distribution by the value of the fourth weight parameter, and adding the value of the second level parameter;
sets each of element values included in the myocardial activity parameter set as a myocardial activity parameter value;
uses each of myocardial activity parameter values included in the myocardial activity parameter set of a heart to be a target (Hereinafter, the heart is referred to as a "target heart".);
obtains, as a synthesized waveform in the time section of the R wave, a time waveform represented by a function obtained by subtracting, from a function obtained by multiplying a cumulative distribution function specified by the myocardial activity parameter value of the parameter specifying the first unimodal distribution or the first cumulative distribution function by the myocardial activity parameter value of the first weight parameter, a function obtained by multiplying a cumulative distribution function specified by the myocardial activity parameter value of the parameter specifying the second unimodal distribution or the second cumulative distribution function by the myocardial activity parameter value of the second weight parameter, and adding the myocardial activity parameter value of the first level parameter;
obtains, as a synthesized waveform in the time section of the T wave, a time waveform represented by a function obtained by subtracting, from a function obtained by subtracting, from 1, a function obtained by multiplying a cumulative distribution function specified by the myocardial activity parameter value of the parameter specifying the third unimodal distribution or the third cumulative distribution function by the myocardial activity parameter value of the third weight parameter, a function obtained by subtracting, from 1, a function obtained by multiplying a cumulative distribution function specified by the myocardial activity parameter value of the parameter specifying the fourth unimodal distribution or the fourth cumulative distribution function by the myocardial activity parameter value of the fourth weight parameter, and adding the myocardial activity parameter value of the second level parameter; and
connects the synthesized waveform in the time section of the R wave and the synthesized waveform in the time section of the T wave together to obtain a synthesized signal for a waveform indicating the cardiac cycle of the target heart.

**3.** The signal synthesis device according to claim 1 or 2, further comprising

a myocardial activity parameter conversion unit that performs conversion of bringing the myocardial activity parameter value included in the myocardial activity parameter set of the target heart close to or away from a myocardial activity parameter value included in a myocardial activity parameter set of a standard electrocardiogram on one or more types of myocardial activity parameters to obtain a converted myocardial activity parameter value,
wherein
the waveform synthesis unit obtains a synthesized signal by using the converted myocardial activity parameter value instead of the myocardial activity parameter value included in the myocardial activity parameter set of the target heart.

**4.** The signal synthesis device according to claim 3, further comprising:

a converter acquisition unit that obtains a converter that converts the myocardial activity parameter set of the target heart into bijective to cause the myocardial activity parameter set of the target heart to approach the myocardial activity parameter set of the standard electrocardiogram; and
a conversion information setting unit that obtains conversion information specifying one or more types of myocardial activity parameters,
wherein
the myocardial activity parameter conversion unit
obtains the myocardial activity parameter value included in the myocardial activity parameter set of the target heart as it is as the converted myocardial activity parameter value, regarding a myocardial activity parameter specified by the conversion information, and
converts the myocardial activity parameter value included in the myocardial activity parameter set of the target heart by the converter to obtain the converted myocardial activity parameter value, regarding a myocardial activity parameter that is not the myocardial activity parameter specified by the conversion information.

**5.** The signal synthesis device according to claim **3,** further comprising

a converter acquisition unit that obtains a converter that converts the myocardial activity parameter set of the target heart into bijective to cause the myocardial activity parameter set of the target heart to approach the myocardial activity parameter set of the standard electrocardiogram; and
a conversion information setting unit that obtains conversion information specifying a reflection weight value of each of myocardial activity parameters,
wherein
the myocardial activity parameter conversion unit
obtains, for each of myocardial activity parameters, as the converted myocardial activity parameter value, a weighted average value of, the myocardial activity parameter value included in the myocardial activity parameter set of the target heart, and a value obtained by converting the myocardial activity parameter value included in the myocardial activity parameter set of the target heart by the converter, in which the reflection weight value is used as weight given to the myocardial activity parameter value included in the myocardial activity parameter set of the target heart.

**6.** The signal synthesis device according to claim 1 or 2, further comprising

a myocardial activity parameter conversion unit that obtains, as a converted myocardial activity parameter value, a representative value of myocardial activity parameter values included in myocardial activity parameter sets of a plurality of cycles of the target heart for one or more types of myocardial activity parameters,
wherein
the waveform synthesis unit
obtains a synthesized signal by using the converted myocardial activity parameter value instead of the myocardial activity parameter value included in the myocardial activity parameter set of the target heart.

**7.** A signal synthesis method executed by a signal synthesis device,

the signal synthesis method comprising
a waveform synthesis step of:

setting a waveform in a time section of an R wave included in a waveform for one cycle indicating a cardiac cycle of a heart as a first target time waveform;

setting a waveform in a time section of a T wave included in the waveform as a second target time waveform;

setting a waveform obtained by inverting a time axis of the second target time waveform as a second target inverse time waveform;

setting a set as a myocardial activity parameter set, the set including

a value of a parameter specifying a first unimodal distribution or a value of a parameter specifying a first cumulative distribution function, a value of a parameter specifying a second unimodal distribution or a value of a parameter specifying a second cumulative distribution function, a value of a first weight parameter, a value of a second weight parameter, and a value of a first level parameter when the first target time waveform is approximated by a first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplying the second cumulative distribution function that is a cumulative distribution function of the second unimodal distribution by the value of the second weight parameter, and adding the value of the first level parameter, and

a value of a parameter specifying a third unimodal distribution or a value of a parameter specifying a third cumulative distribution function, a value of a parameter specifying a fourth unimodal distribution or a value of a parameter specifying a fourth cumulative distribution function, a value of a third weight parameter, a value of a fourth weight parameter, and a value of a second level parameter when the second target inverse time waveform is approximated by a second approximate inverse time waveform that is a waveform represented by a function obtained by subtracting, from a function obtained by multiplying the third cumulative distribution function that is a cumulative distribution function of the third unimodal distribution by the value of the third weight parameter, a function obtained by multiplying the fourth cumulative distribution function that is a cumulative distribution function of the fourth unimodal distribution by the value of the fourth weight parameter, and adding the value of the second level parameter;

setting each of element values included in the myocardial activity parameter set as a myocardial activity parameter value;

using each of myocardial activity parameter values included in the myocardial activity parameter set of a heart to be a target (Hereinafter, the heart is referred to as a "target heart".);

obtaining, as a synthesized waveform in the time section of the R wave, a time waveform represented by a function obtained by subtracting, from a function obtained by multiplying a cumulative distribution function specified by the myocardial activity parameter value of the parameter specifying the first unimodal distribution or the first cumulative distribution function by the myocardial activity parameter value of the first weight parameter, a function obtained by multiplying a cumulative distribution function specified by the myocardial activity parameter value of the parameter specifying the second unimodal distribution or the second cumulative distribution function by the myocardial activity parameter value of the second weight parameter, and adding the myocardial activity parameter value of the first level parameter;

obtaining, as a synthesized waveform in the time section of the T wave, a waveform obtained by inverting a time axis of a waveform represented by a function obtained by subtracting, from a function obtained by multiplying a cumulative distribution function specified by the myocardial activity parameter value of the parameter specifying the third unimodal distribution or the third cumulative distribution function by the myocardial activity parameter value of the third weight parameter, a function obtained by multiplying a cumulative distribution function specified by the myocardial activity parameter value of the parameter specifying the fourth unimodal distribution or the fourth cumulative distribution function by the myocardial activity parameter value of the fourth weight parameter, and adding the myocardial activity parameter value of the second level parameter; and

connecting the synthesized waveform in the time section of the R wave and the synthesized waveform in the time section of the T wave together to obtain a synthesized signal for a waveform indicating the cardiac cycle of the target heart.

**8.** A signal synthesis method executed by a signal synthesis device,

the signal synthesis method comprising
a waveform synthesis step of:

setting a waveform in a time section of an R wave included in a waveform for one cycle indicating a cardiac cycle of a heart as a first target time waveform;

setting a waveform in a time section of a T wave included in the waveform as a second target time waveform;

setting a set as a myocardial activity parameter set, the set including

a value of a parameter specifying a first unimodal distribution or a value of a parameter specifying a first cumulative distribution function, a value of a parameter specifying a second unimodal distribution or a value of a parameter specifying a second cumulative distribution function, a value of a first weight parameter, a value of a second weight parameter, and a value of a first level parameter when the first target time waveform is approximated by a first approximate time waveform that is a time waveform represented by a function obtained by subtracting, from a function obtained by multiplying the first cumulative distribution function that is a cumulative distribution function of the first unimodal distribution by the value of the first weight parameter, a function obtained by multiplying the second cumulative distribution function that is a cumulative distribution function of the second unimodal distribution by the value of the second weight parameter, and adding the value of the first level parameter, and

a value of a parameter specifying a third unimodal distribution or a value of a parameter specifying a third cumulative distribution function, a value of a parameter specifying a fourth unimodal distribution or a value of a parameter specifying a fourth cumulative distribution function, a value of a third weight parameter, a value of a fourth weight parameter, and a value of a second level parameter when the second target time waveform is approximated by a second approximate time waveform that is a time waveform represented by a function obtained by subtracting, from a function obtained by subtracting, from 1, a function obtained by multiplying the third cumulative distribution function that is a cumulative distribution function of the third unimodal distribution by the value of the third weight parameter, a function obtained by subtracting, from 1, a function obtained by multiplying the fourth cumulative distribution function that is a cumulative distribution function of the fourth unimodal distribution by the value of the fourth weight parameter, and adding the value of the second level parameter;

setting each of element values included in the myocardial activity parameter set as a myocardial activity parameter value;

using each of myocardial activity parameter values included in the myocardial activity parameter set of a heart to be a target (Hereinafter, the heart is referred to as a "target heart".);

obtaining, as a synthesized waveform in the time section of the R wave, a time waveform represented by a function obtained by subtracting, from a function obtained by multiplying a cumulative distribution function specified by the myocardial activity parameter value of the parameter specifying the first unimodal distribution or the first cumulative distribution function by the myocardial activity parameter value of the first weight parameter, a function obtained by multiplying a cumulative distribution function specified by the myocardial activity parameter value of the parameter specifying the second unimodal distribution or the second cumulative distribution function by the myocardial activity parameter value of the second weight parameter, and adding the myocardial activity parameter value of the first level parameter;

obtaining, as a synthesized waveform in the time section of the T wave, a time waveform represented by a function obtained by subtracting, from a function obtained by subtracting, from 1, a function obtained by multiplying a cumulative distribution function specified by the myocardial activity parameter value of the parameter specifying the third unimodal distribution or the third cumulative distribution function by the myocardial activity parameter value of the third weight parameter, a function obtained by subtracting, from 1, a function obtained by multiplying a cumulative distribution function specified by the myocardial activity parameter value of the parameter specifying the fourth unimodal distribution or the fourth cumulative distribution function by the myocardial activity parameter value of the fourth weight parameter, and adding the myocardial activity parameter value of the second level parameter; and

connecting the synthesized waveform in the time section of the R wave and the synthesized waveform in the time section of the T wave together to obtain a synthesized signal for a waveform indicating the cardiac cycle of the target heart.

**9.** A program for causing a computer to function as the signal synthesis device according to claim 1 or 2.

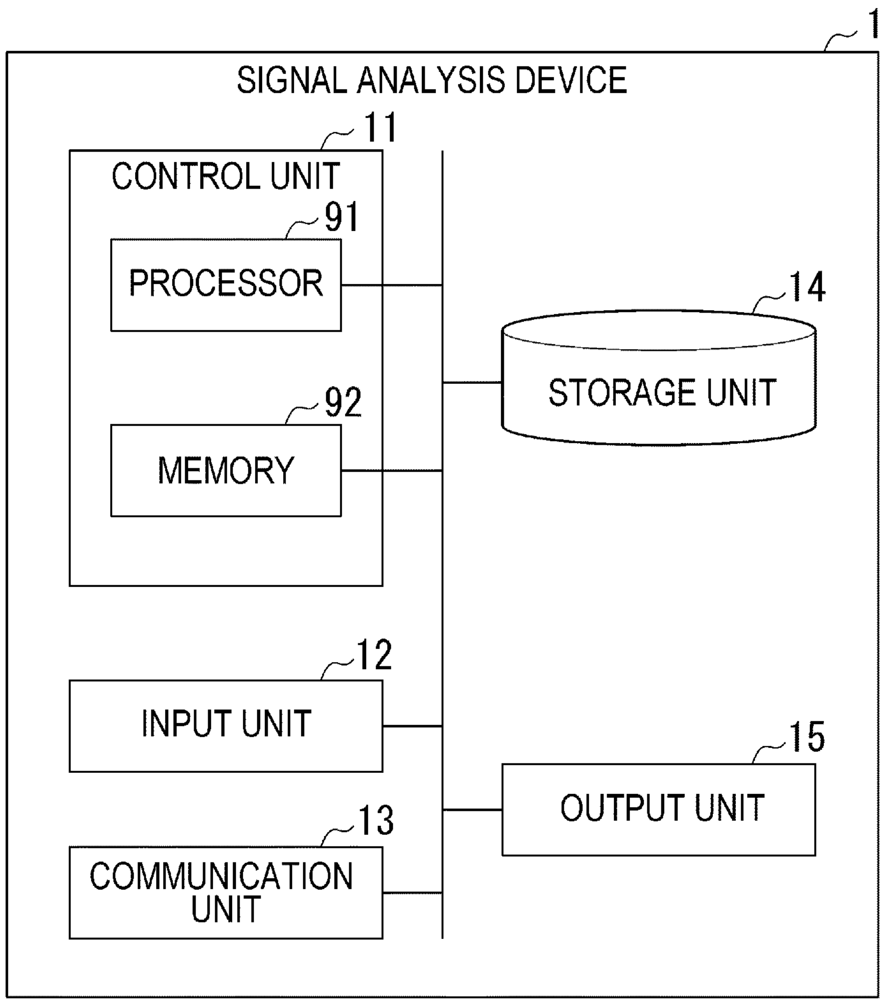
1
SIGNAL ANALYSIS DEVICE
11
CONTROL UNIT
91
PROCESSOR
92
MEMORY
14
STORAGE UNIT
12
INPUT UNIT
13
COMMUNICATION UNIT
15
OUTPUT UNIT

FIG. 1

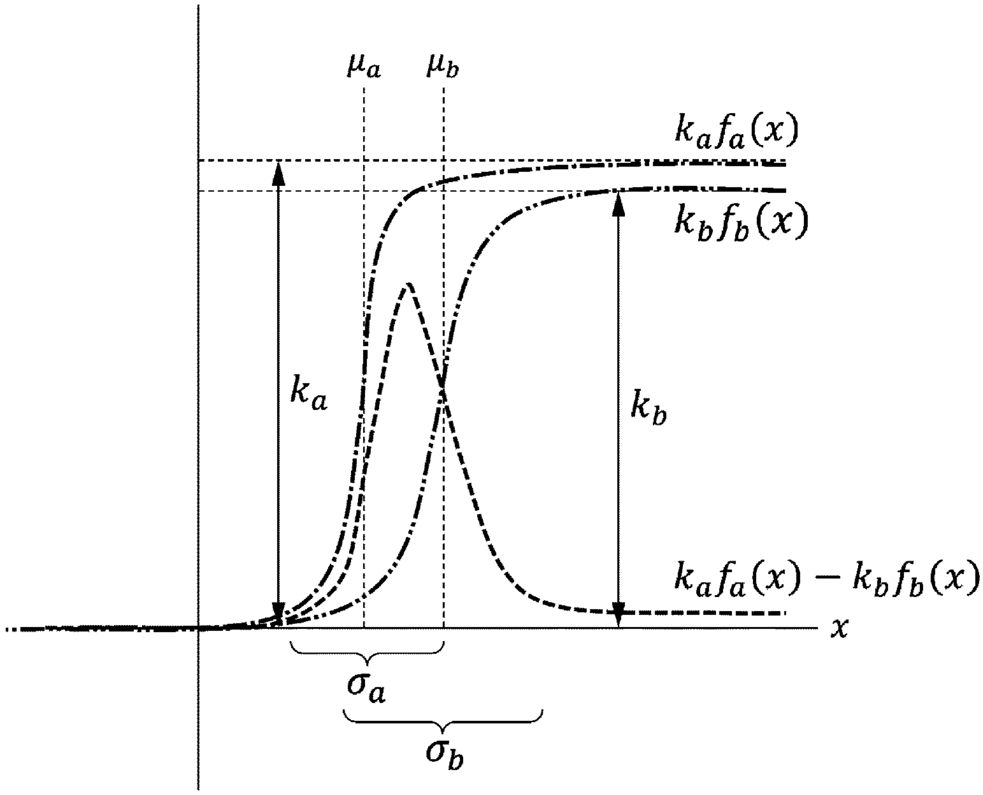
$\mu_a$
$\mu_b$
$k_a f_a(x)$
$k_b f_b(x)$
$k_a$
$k_b$
$k_a f_a(x) - k_b f_b(x)$
$x$
$\sigma_a$
$\sigma_b$

FIG. 2

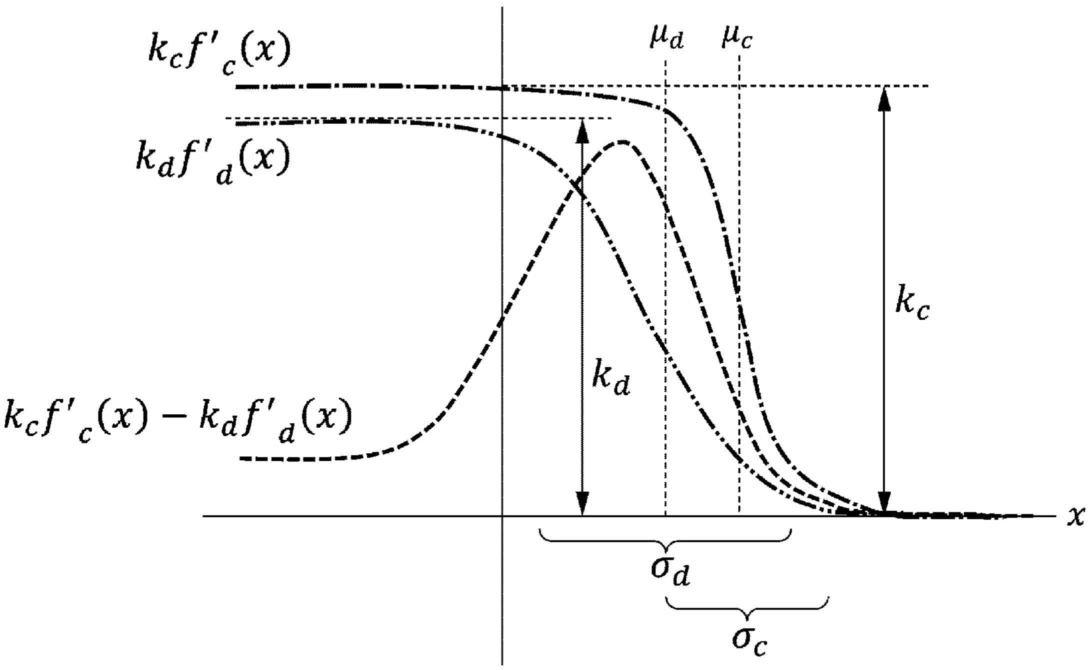
$k_c f'_c(x)$
$k_d f'_d(x)$
$\mu_d$
$\mu_c$
$k_c$
$k_d$
$k_c f'_c(x) - k_d f'_d(x)$
$x$
$\sigma_d$
$\sigma_c$

FIG. 3

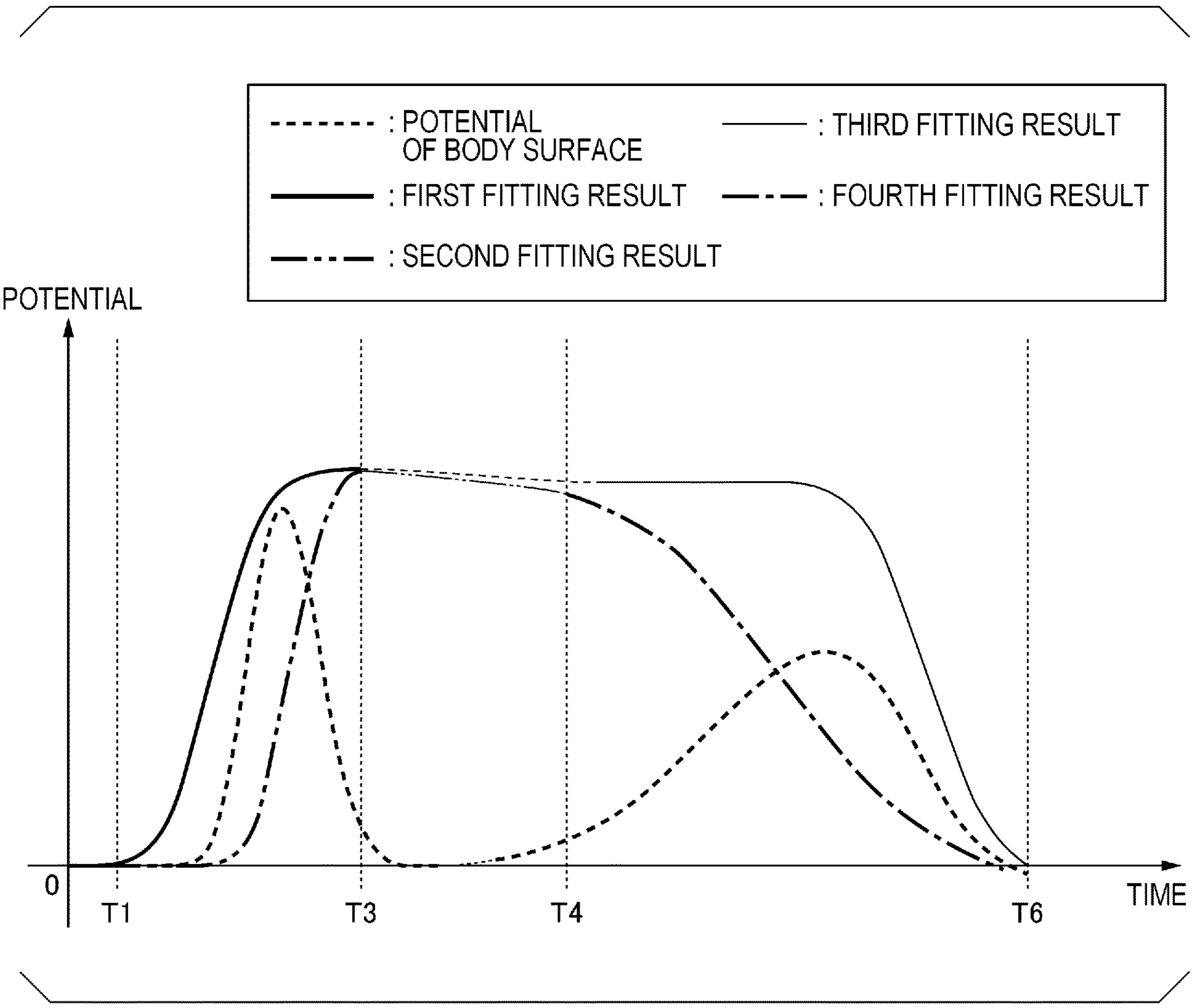
: POTENTIAL OF BODY SURFACE
: THIRD FITTING RESULT
: FIRST FITTING RESULT
: FOURTH FITTING RESULT
: SECOND FITTING RESULT
POTENTIAL
0
T1
T3
T4
T6
TIME

FIG. 4

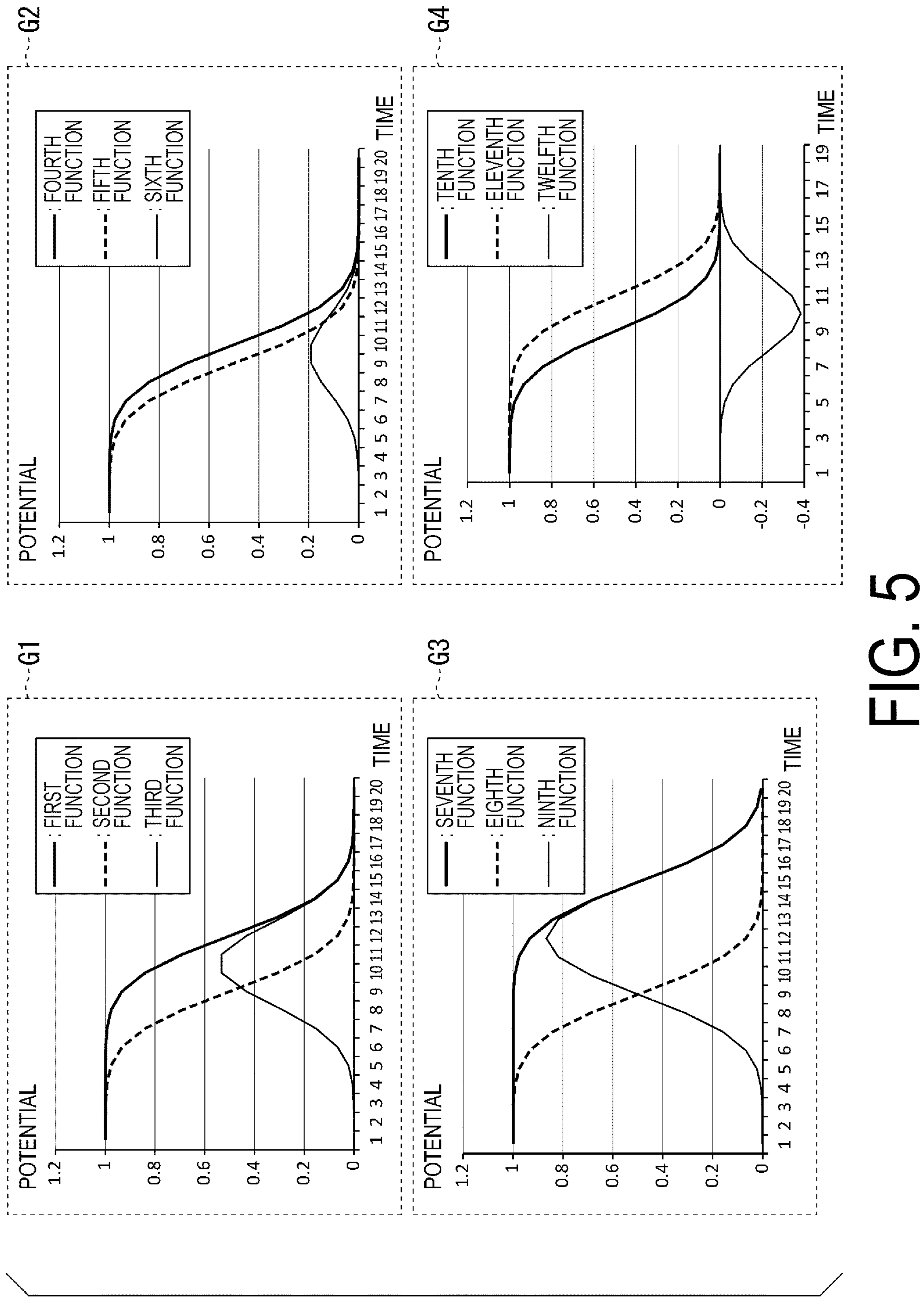
G1
POTENTIAL
TIME
FIRST FUNCTION
SECOND FUNCTION
THIRD FUNCTION
G2
POTENTIAL
TIME
FOURTH FUNCTION
FIFTH FUNCTION
SIXTH FUNCTION
G3
POTENTIAL
TIME
SEVENTH FUNCTION
EIGHTH FUNCTION
NINTH FUNCTION
G4
POTENTIAL
TIME
TENTH FUNCTION
ELEVENTH FUNCTION
TWELFTH FUNCTION

FIG. 5

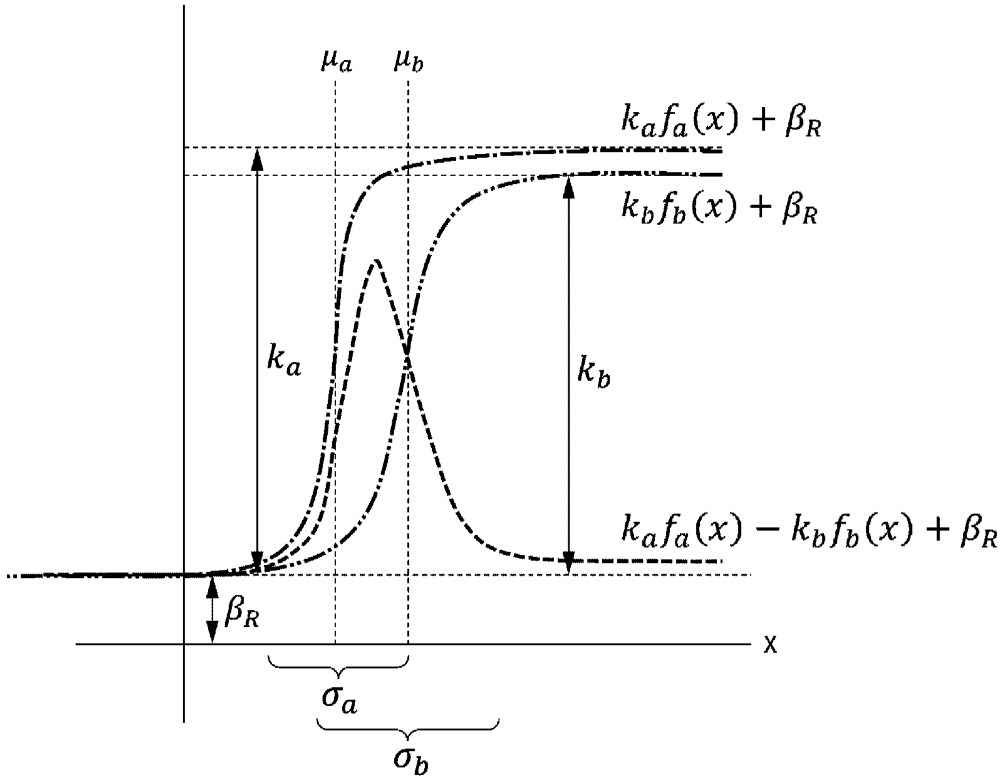
$\mu_a$
$\mu_b$
$k_a f_a(x) + \beta_R$
$k_b f_b(x) + \beta_R$
$k_a$
$k_b$
$k_a f_a(x) - k_b f_b(x) + \beta_R$
$\beta_R$
X
$\sigma_a$
$\sigma_b$

FIG. 6

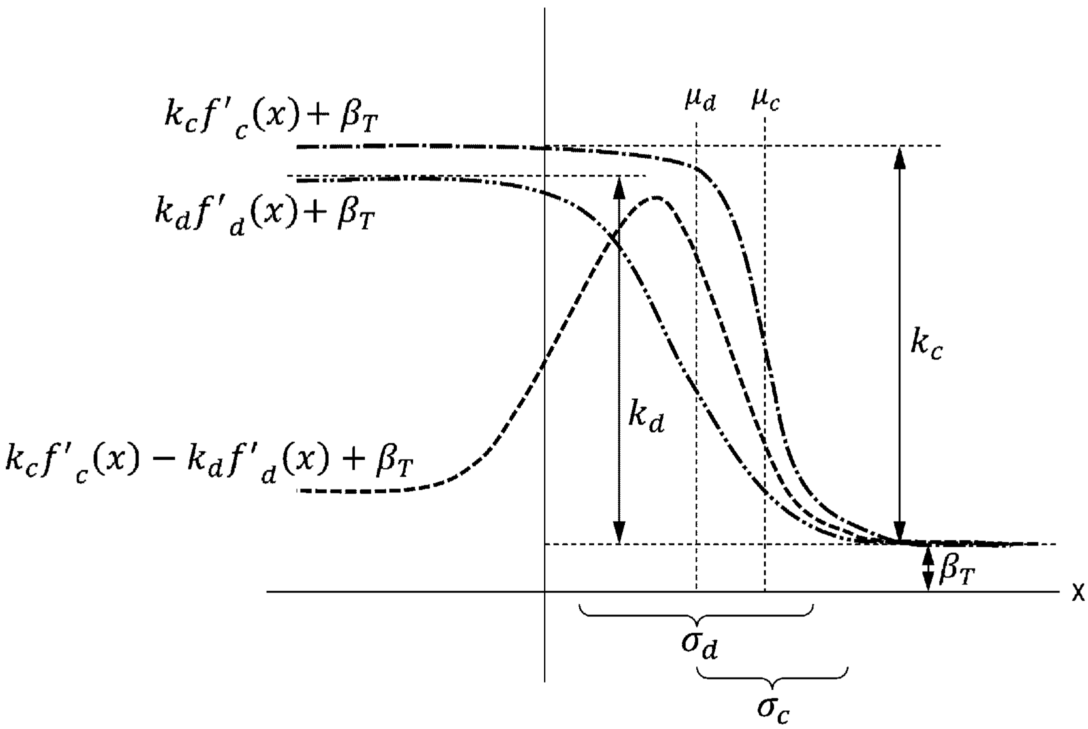
$k_c f'_c(x) + \beta_T$
$\mu_d$
$\mu_c$
$k_d f'_d(x) + \beta_T$
$k_c$
$k_d$
$k_c f'_c(x) - k_d f'_d(x) + \beta_T$
$\beta_T$
X
$\sigma_d$
$\sigma_c$

FIG. 7

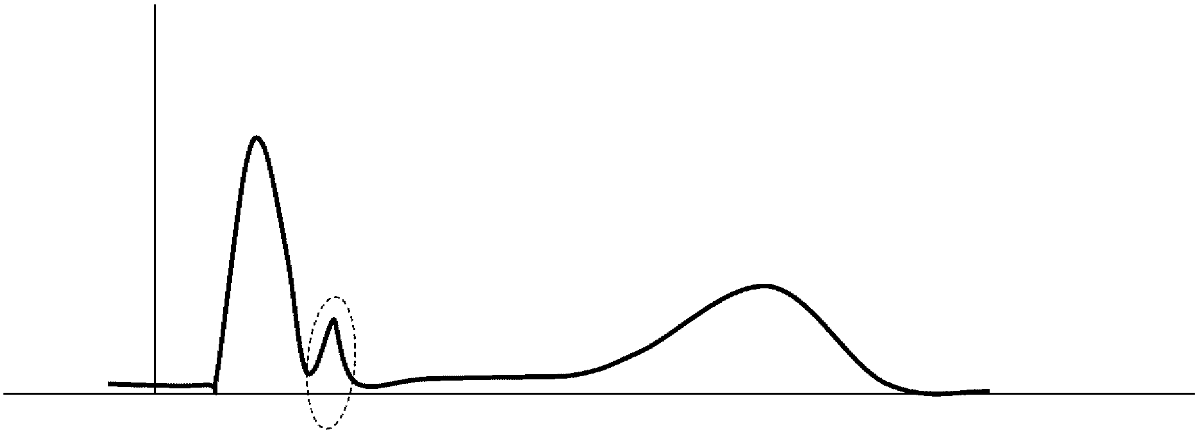

FIG. 8

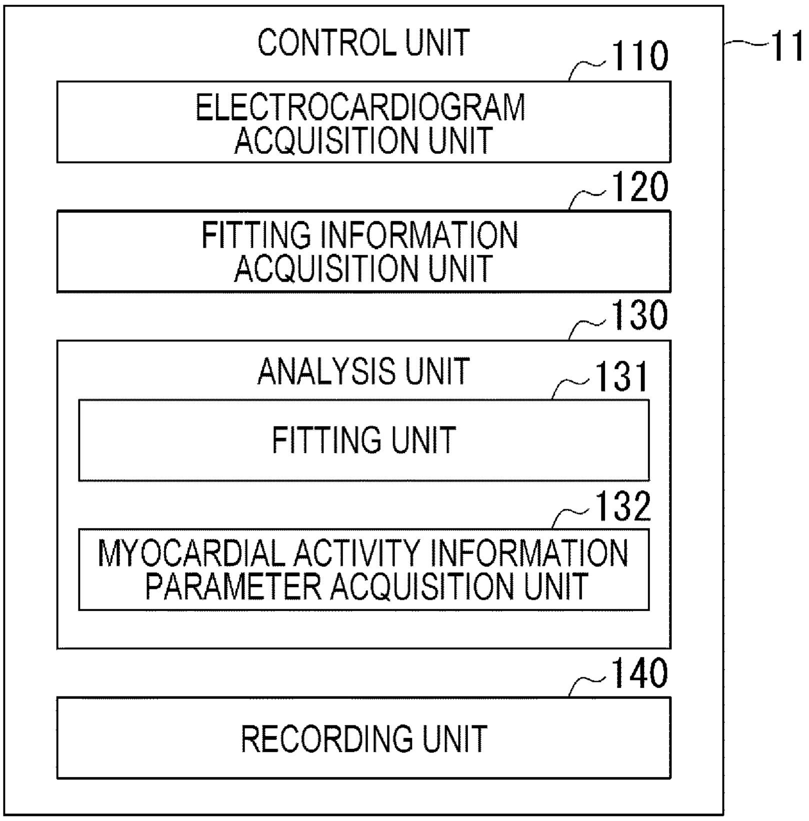
CONTROL UNIT
110
ELECTROCARDIOGRAM ACQUISITION UNIT
120
FITTING INFORMATION ACQUISITION UNIT
130
ANALYSIS UNIT
131
FITTING UNIT
132
MYOCARDIAL ACTIVITY INFORMATION PARAMETER ACQUISITION UNIT
140
RECORDING UNIT
11

FIG. 9

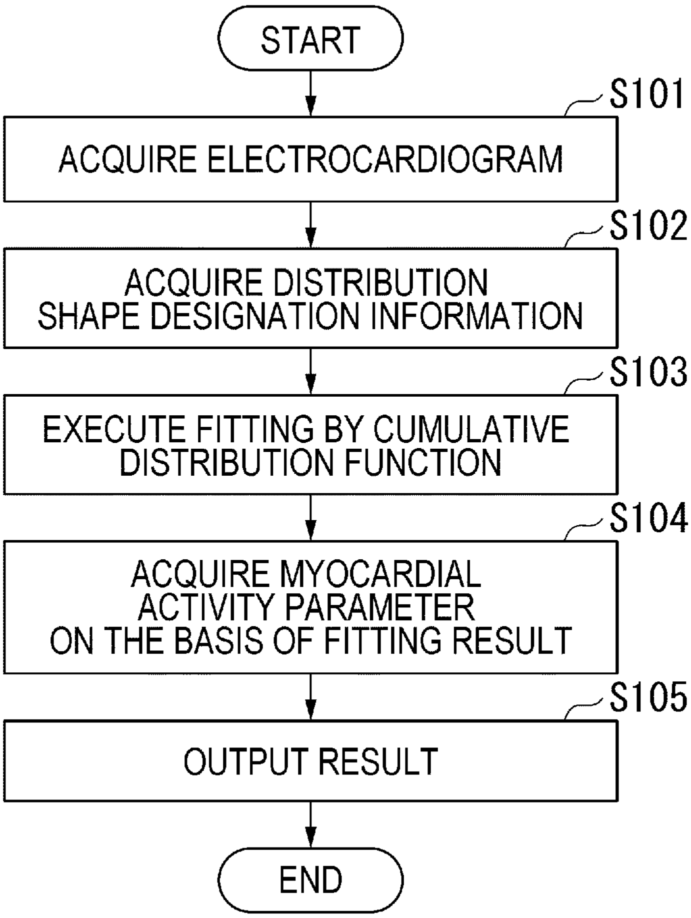
START
S101
ACQUIRE ELECTROCARDIOGRAM
S102
ACQUIRE DISTRIBUTION SHAPE DESIGNATION INFORMATION
S103
EXECUTE FITTING BY CUMULATIVE DISTRIBUTION FUNCTION
S104
ACQUIRE MYOCARDIAL ACTIVITY PARAMETER ON THE BASIS OF FITTING RESULT
S105
OUTPUT RESULT
END

FIG. 10

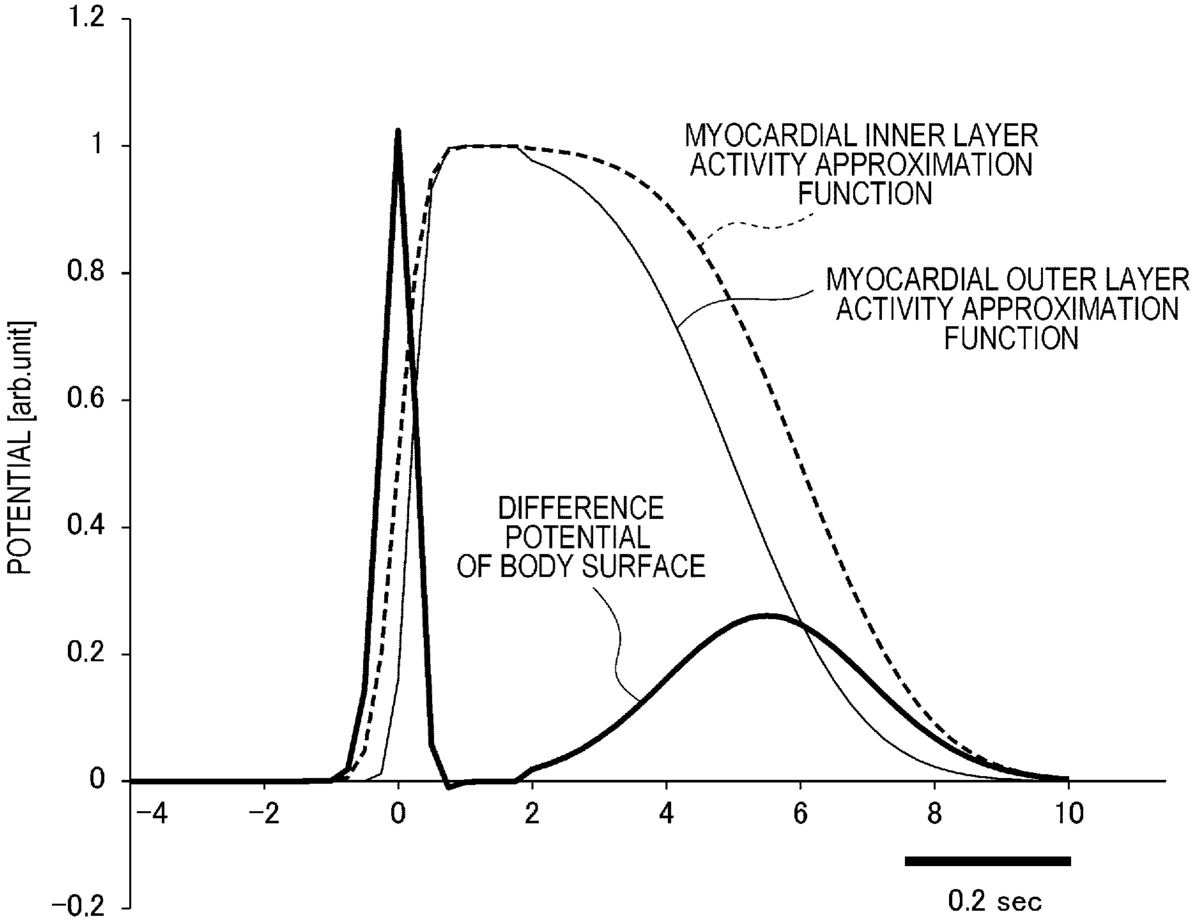
1.2
1
0.8
0.6
0.4
0.2
0
−0.2
POTENTIAL [arb.unit]
−4
−2
0
2
4
6
8
10
MYOCARDIAL INNER LAYER ACTIVITY APPROXIMATION FUNCTION
MYOCARDIAL OUTER LAYER ACTIVITY APPROXIMATION FUNCTION
DIFFERENCE POTENTIAL OF BODY SURFACE
0.2 sec

FIG. 11

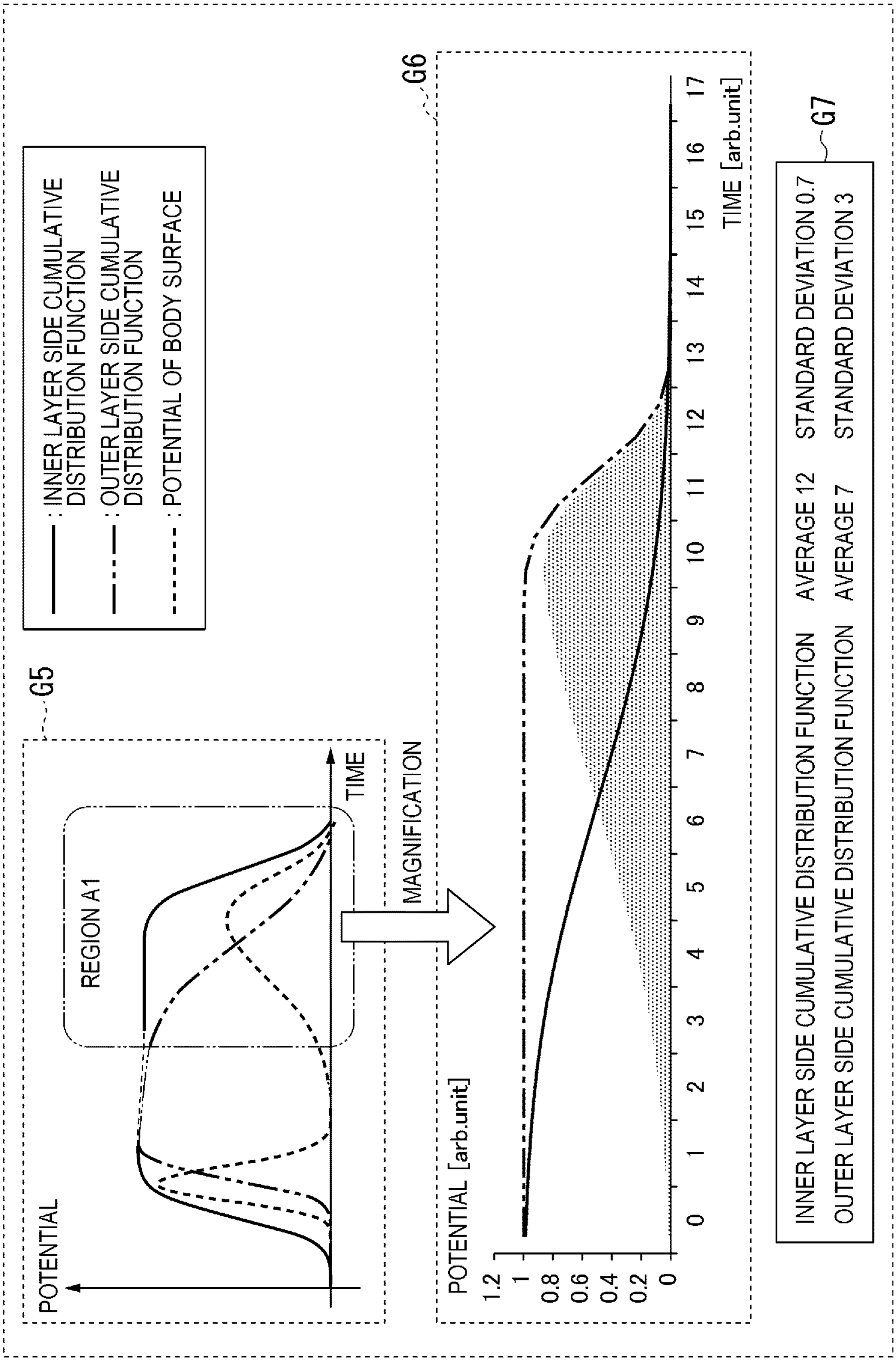
: INNER LAYER SIDE CUMULATIVE DISTRIBUTION FUNCTION
: OUTER LAYER SIDE CUMULATIVE DISTRIBUTION FUNCTION
: POTENTIAL OF BODY SURFACE
G5
POTENTIAL
TIME
REGION A1
MAGNIFICATION
G6
POTENTIAL [arb.unit]
1.2
1
0.8
0.6
0.4
0.2
0
0 1 2 3 4 5 6 7 8 9 10 11 12 13 14 15 16 17
TIME [arb.unit]
G7
INNER LAYER SIDE CUMULATIVE DISTRIBUTION FUNCTION AVERAGE 12 STANDARD DEVIATION 0.7
OUTER LAYER SIDE CUMULATIVE DISTRIBUTION FUNCTION AVERAGE 7 STANDARD DEVIATION 3

FIG. 12

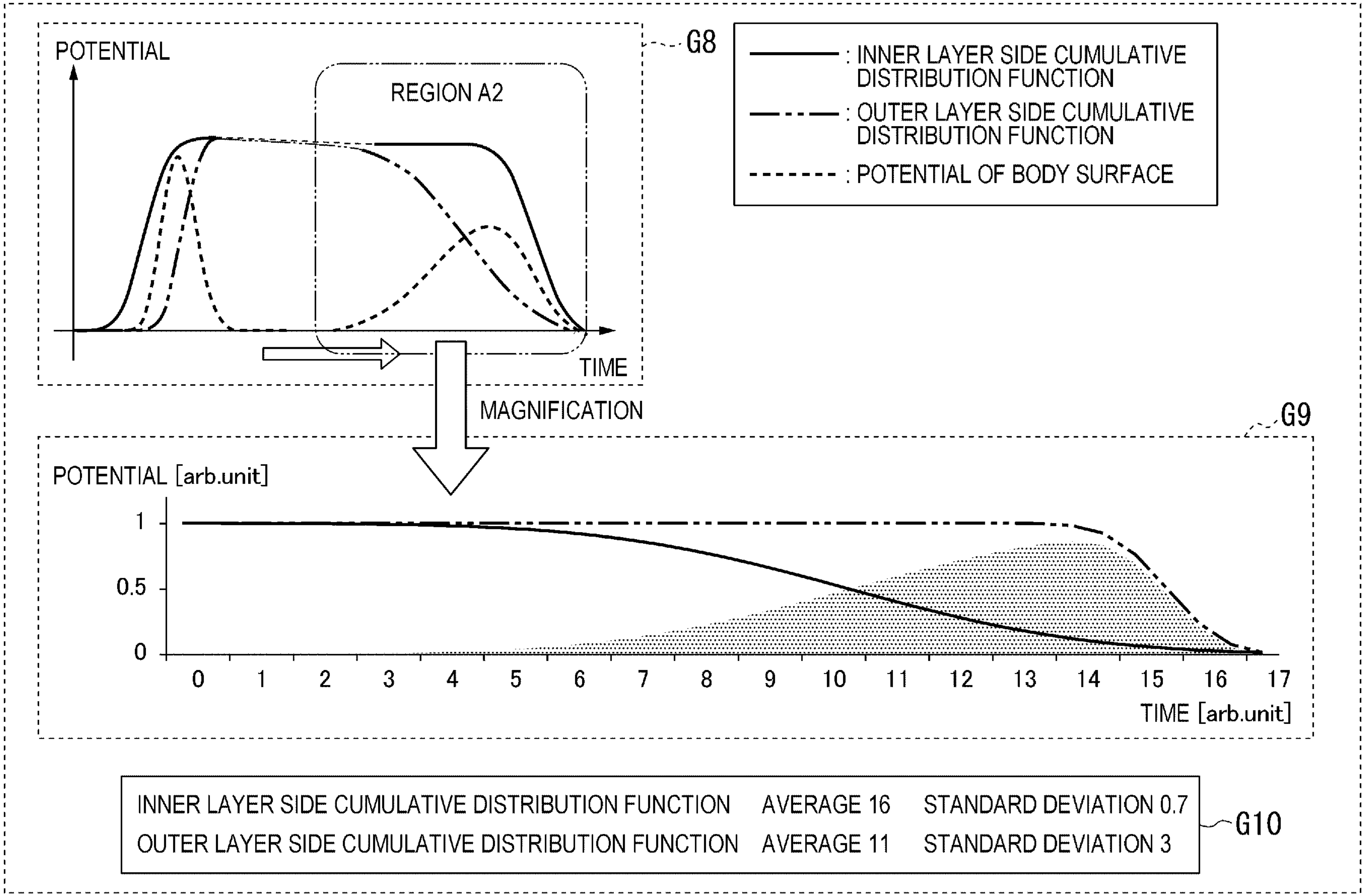
G8
POTENTIAL
REGION A2
TIME
: INNER LAYER SIDE CUMULATIVE DISTRIBUTION FUNCTION
: OUTER LAYER SIDE CUMULATIVE DISTRIBUTION FUNCTION
: POTENTIAL OF BODY SURFACE
MAGNIFICATION
G9
POTENTIAL [arb.unit]
1
0.5
0
0 1 2 3 4 5 6 7 8 9 10 11 12 13 14 15 16 17
TIME [arb.unit]
INNER LAYER SIDE CUMULATIVE DISTRIBUTION FUNCTION AVERAGE 16 STANDARD DEVIATION 0.7
OUTER LAYER SIDE CUMULATIVE DISTRIBUTION FUNCTION AVERAGE 11 STANDARD DEVIATION 3
G10

FIG. 13

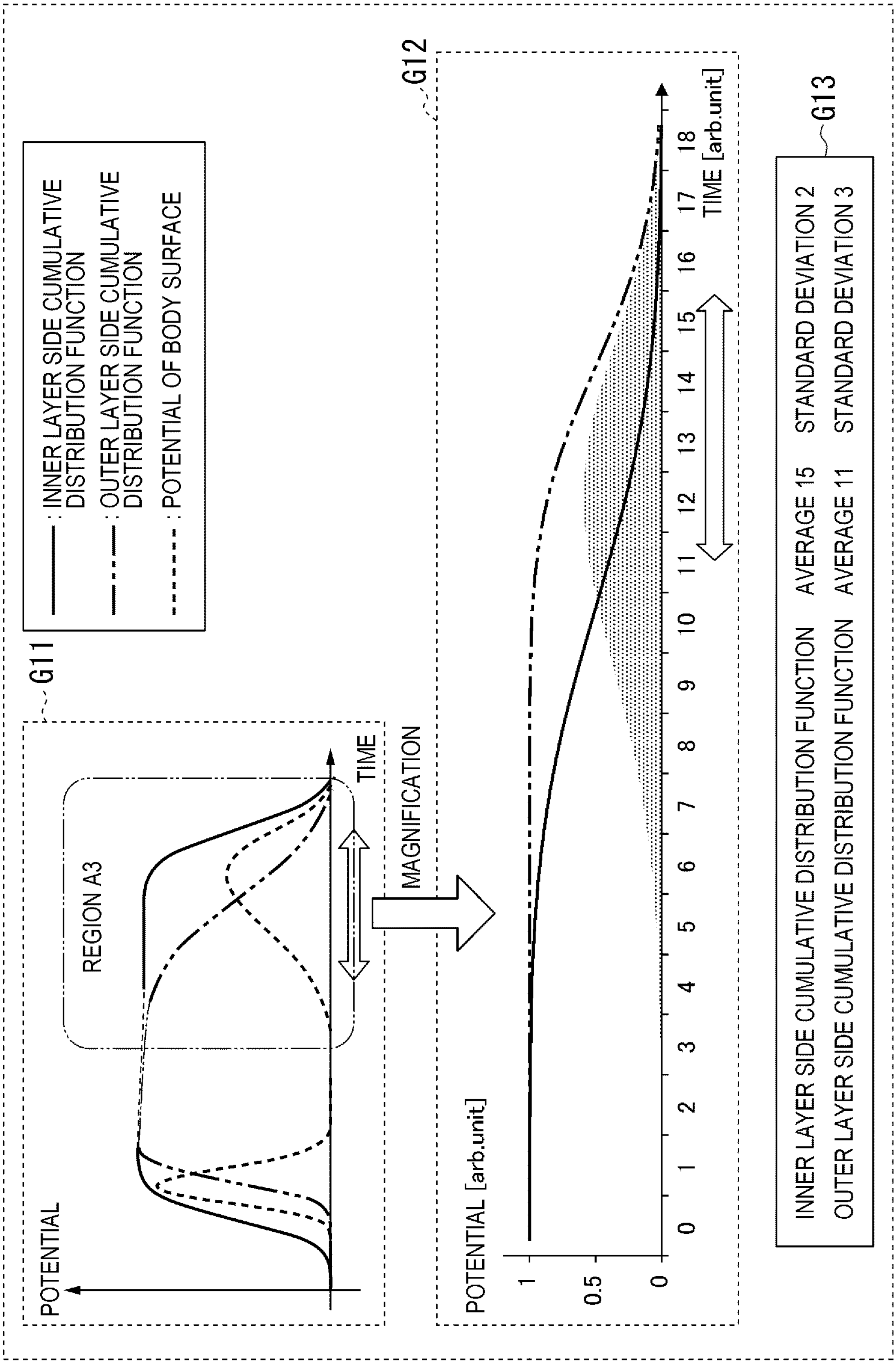
INNER LAYER SIDE CUMULATIVE DISTRIBUTION FUNCTION
OUTER LAYER SIDE CUMULATIVE DISTRIBUTION FUNCTION
POTENTIAL OF BODY SURFACE
G11
G12
G13
POTENTIAL
TIME
REGION A3
MAGNIFICATION
POTENTIAL [arb.unit]
TIME [arb.unit]
1
0.5
0
0 1 2 3 4 5 6 7 8 9 10 11 12 13 14 15 16 17 18
INNER LAYER SIDE CUMULATIVE DISTRIBUTION FUNCTION AVERAGE 15 STANDARD DEVIATION 2
OUTER LAYER SIDE CUMULATIVE DISTRIBUTION FUNCTION AVERAGE 11 STANDARD DEVIATION 3

FIG. 14

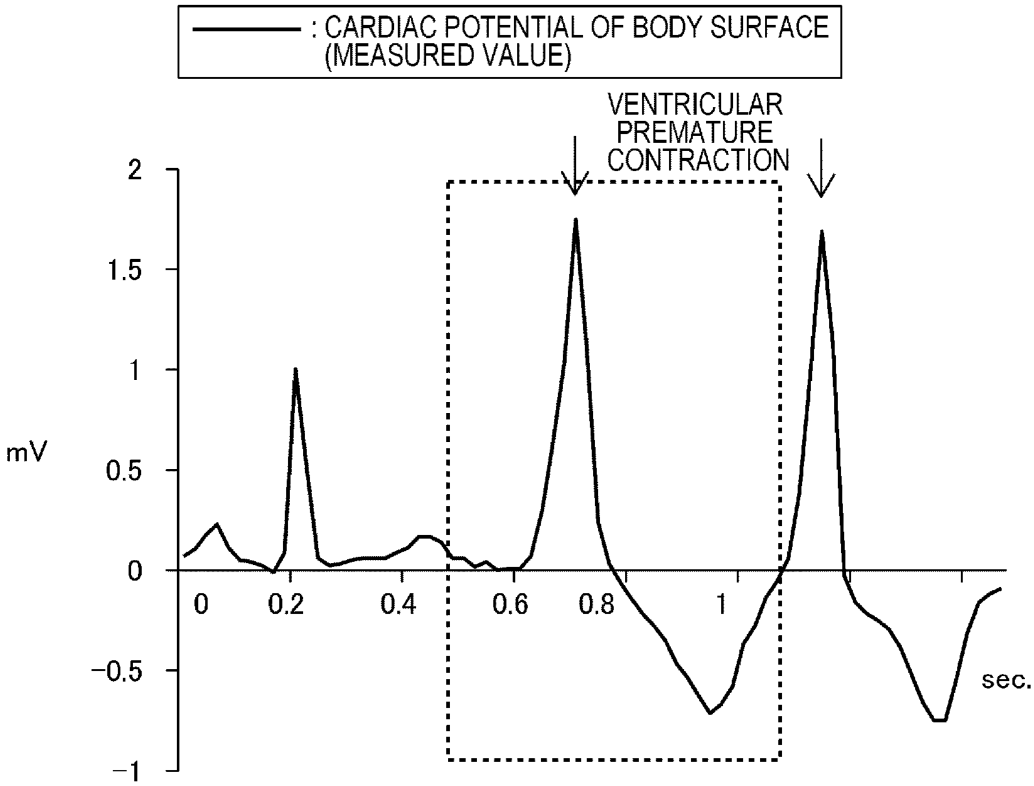
: CARDIAC POTENTIAL OF BODY SURFACE (MEASURED VALUE)
VENTRICULAR PREMATURE CONTRACTION
2
1.5
1
mV
0.5
0
−0.5
−1
0
0.2
0.4
0.6
0.8
1
sec.

FIG. 15

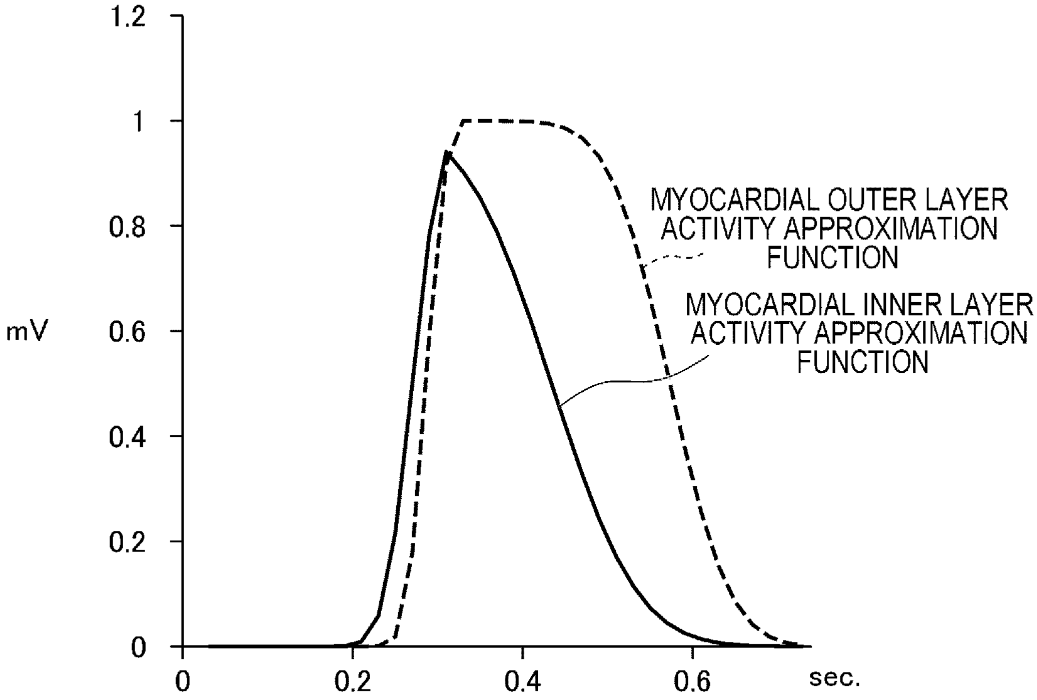
1.2
1
0.8
mV
0.6
0.4
0.2
0
0
0.2
0.4
0.6
sec.
MYOCARDIAL OUTER LAYER ACTIVITY APPROXIMATION FUNCTION
MYOCARDIAL INNER LAYER ACTIVITY APPROXIMATION FUNCTION

FIG. 16

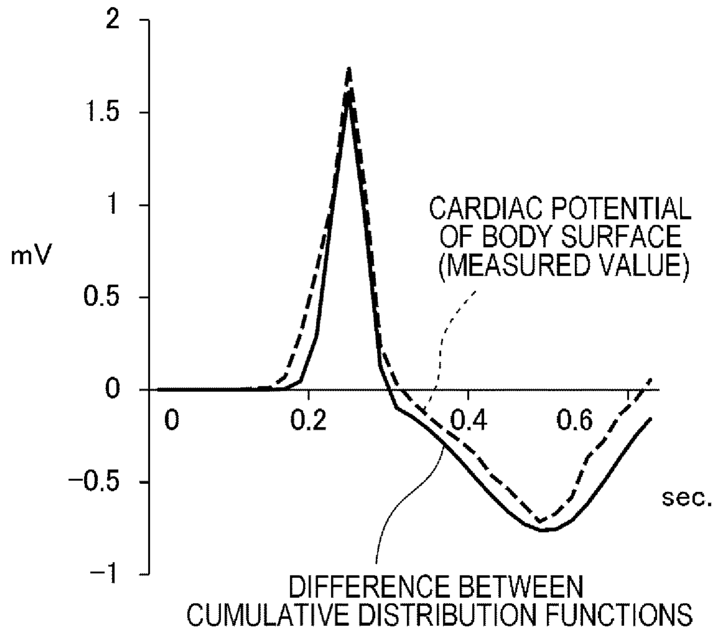
2
1.5
1
mV
0.5
0
−0.5
−1
0
0.2
0.4
0.6
sec.
CARDIAC POTENTIAL
OF BODY SURFACE
(MEASURED VALUE)
DIFFERENCE BETWEEN
CUMULATIVE DISTRIBUTION FUNCTIONS

FIG. 17

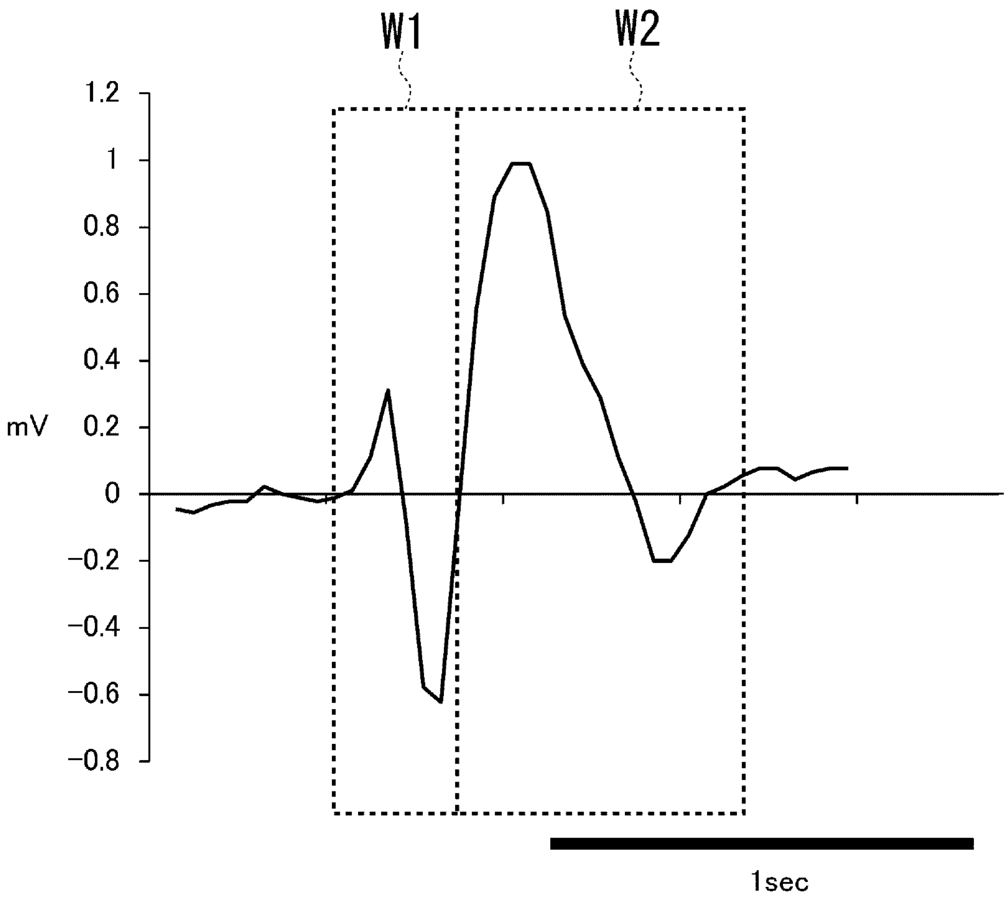
W1
W2
1.2
1
0.8
0.6
0.4
mV
0.2
0
−0.2
−0.4
−0.6
−0.8
1sec

FIG. 18

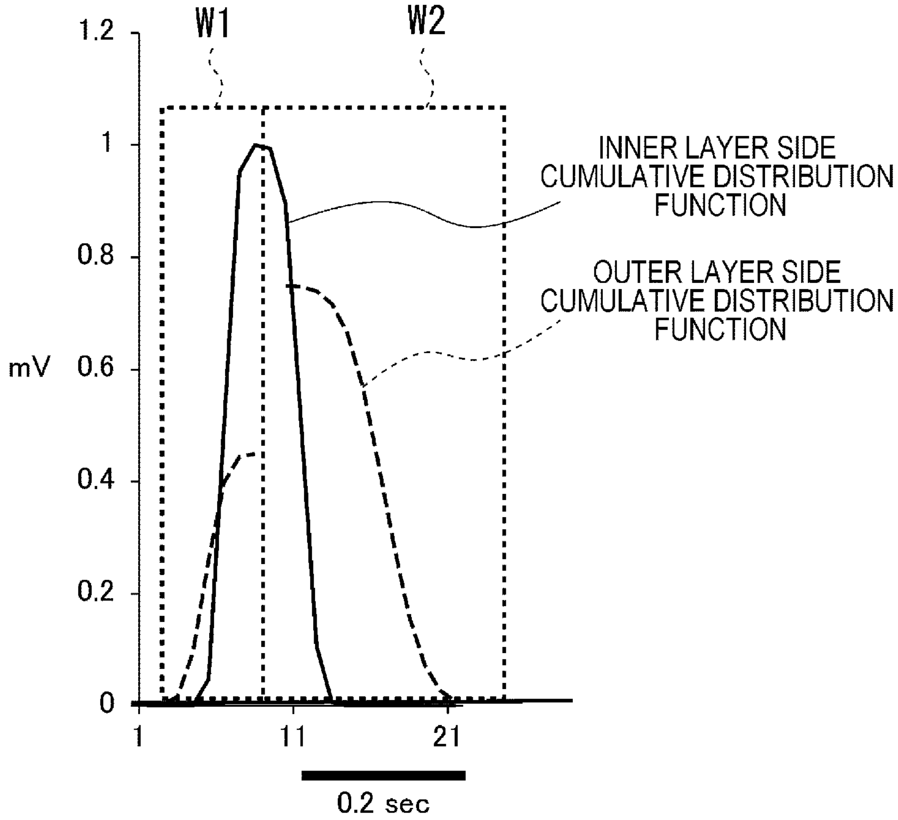
W1
W2
1.2
1
0.8
0.6
0.4
0.2
0
mV
1
11
21
INNER LAYER SIDE CUMULATIVE DISTRIBUTION FUNCTION
OUTER LAYER SIDE CUMULATIVE DISTRIBUTION FUNCTION
0.2 sec

FIG. 19

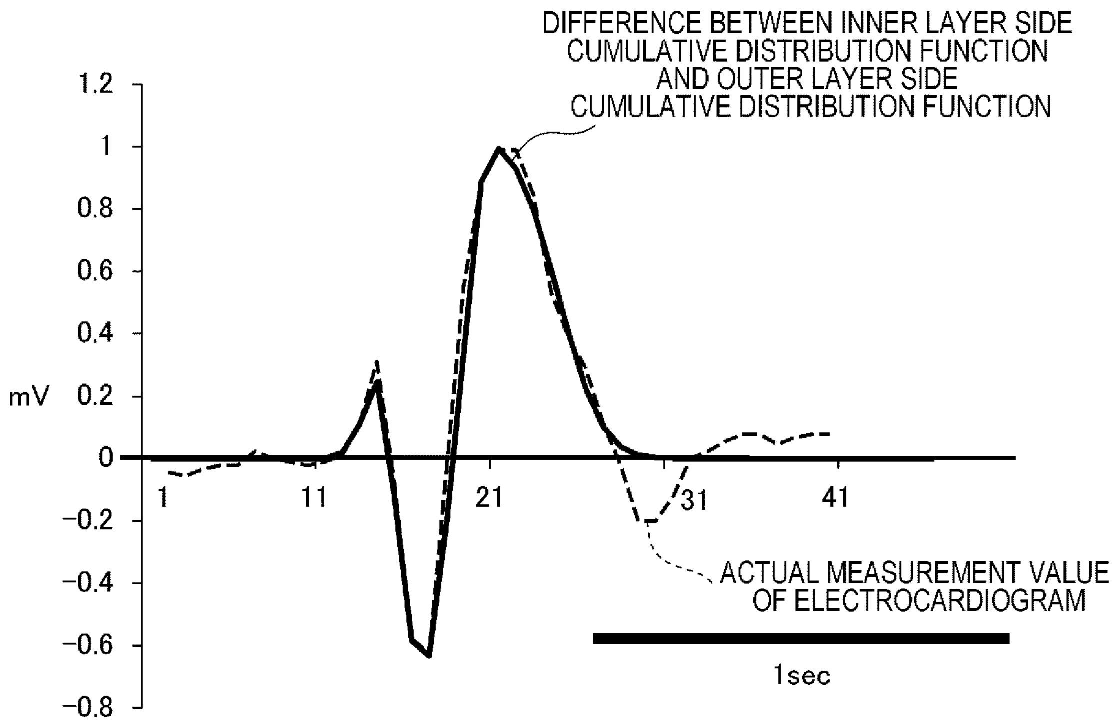
DIFFERENCE BETWEEN INNER LAYER SIDE CUMULATIVE DISTRIBUTION FUNCTION AND OUTER LAYER SIDE CUMULATIVE DISTRIBUTION FUNCTION
1.2
1
0.8
0.6
0.4
0.2
0
−0.2
−0.4
−0.6
−0.8
mV
1
11
21
31
41
ACTUAL MEASUREMENT VALUE OF ELECTROCARDIOGRAM
1sec

FIG. 20

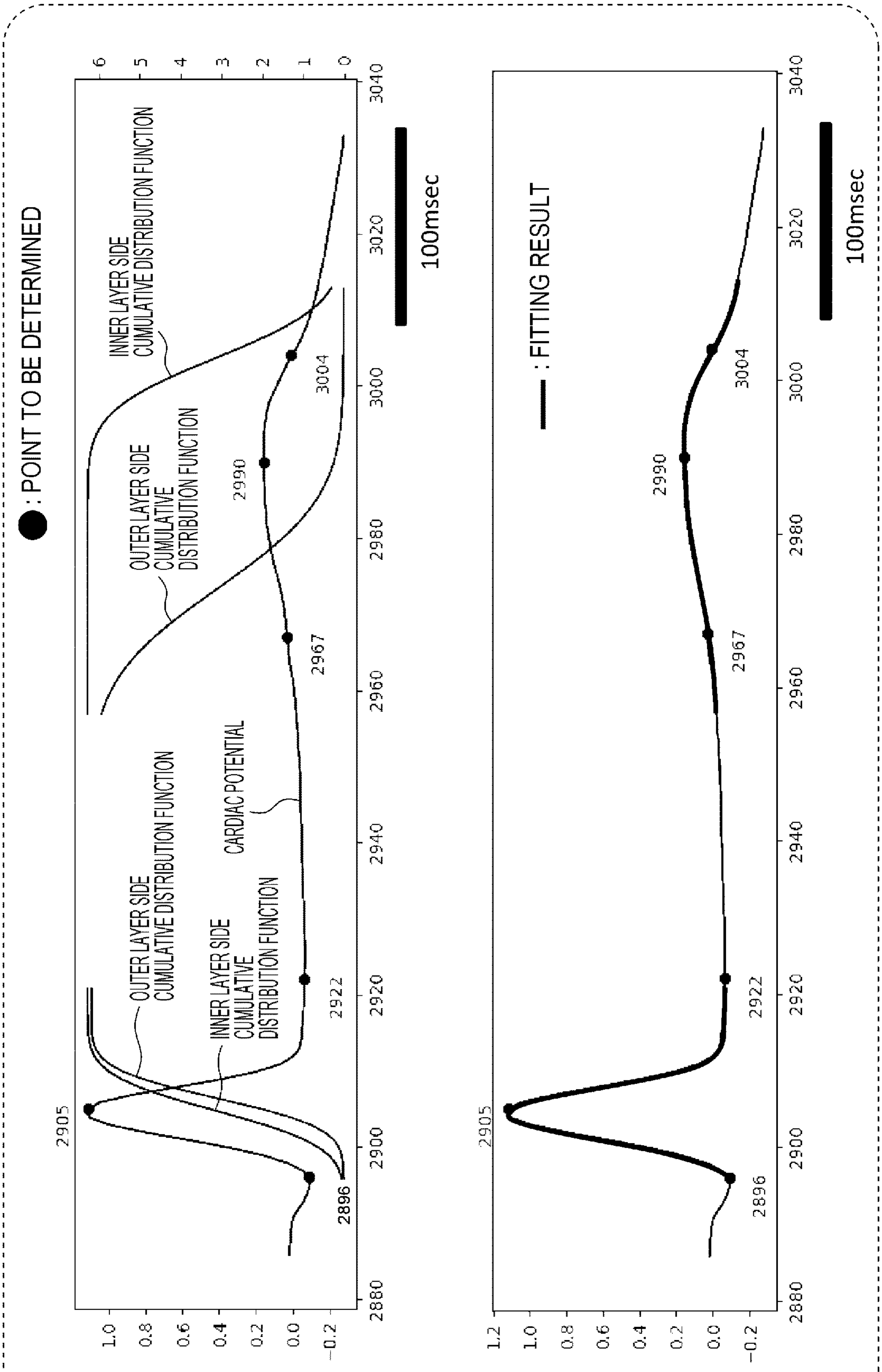
● : POINT TO BE DETERMINED
mV
1.0
0.8
0.6
0.4
0.2
0.0
−0.2
6
5
4
3
2
1
0
2880
2900
2920
2940
2960
2980
3000
3020
3040
2896
2905
2922
2967
2990
3004
OUTER LAYER SIDE CUMULATIVE DISTRIBUTION FUNCTION
INNER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
100msec
mV
1.2
1.0
0.8
0.6
0.4
0.2
0.0
−0.2
2880
2900
2920
2940
2960
2980
3000
3020
3040
2896
2905
2922
2967
2990
3004
: FITTING RESULT
100msec

FIG. 21

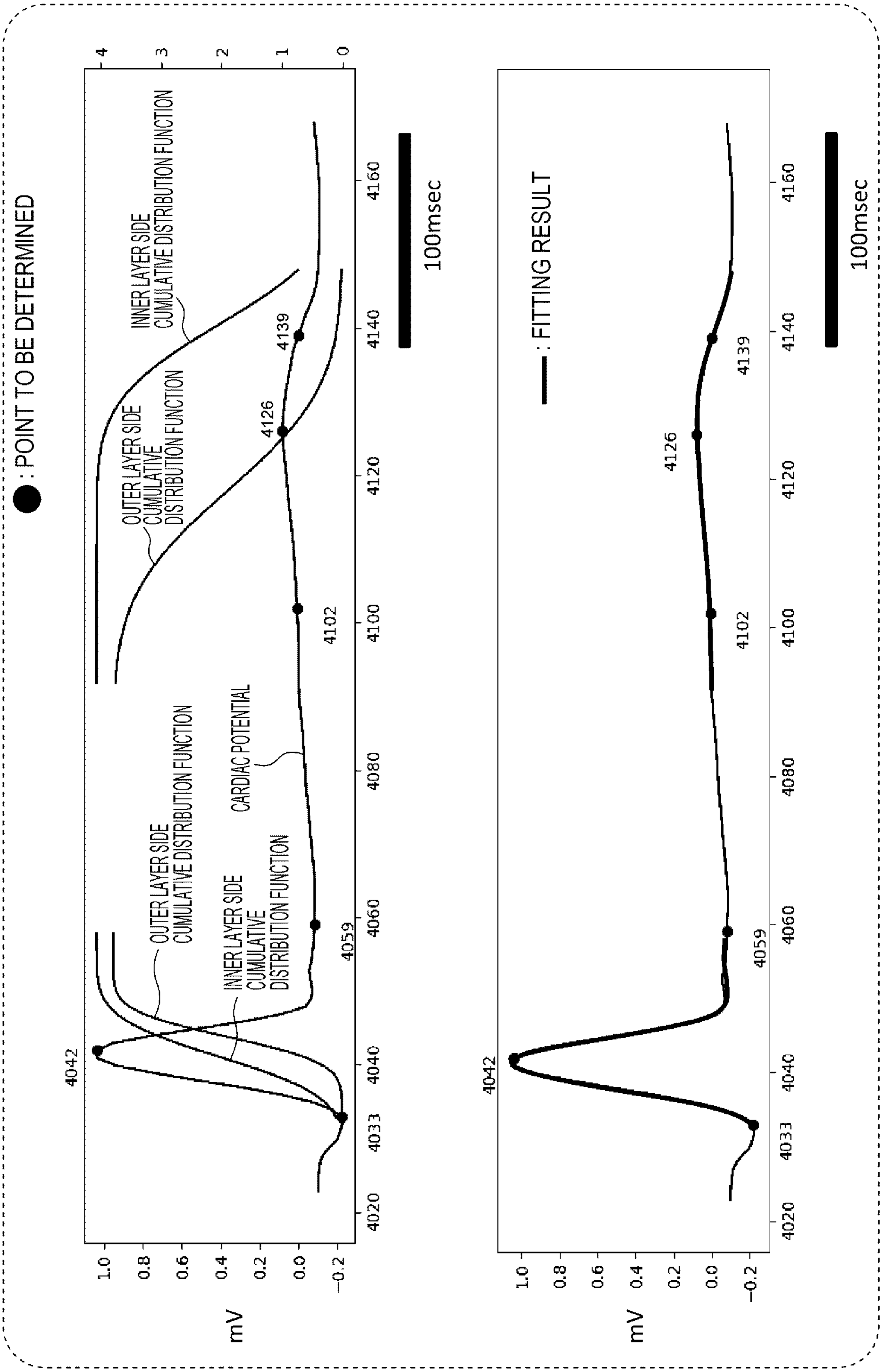
: POINT TO BE DETERMINED
OUTER LAYER SIDE CUMULATIVE DISTRIBUTION FUNCTION
INNER LAYER SIDE CUMULATIVE DISTRIBUTION FUNCTION
CARDIAC POTENTIAL
INNER LAYER SIDE CUMULATIVE DISTRIBUTION FUNCTION
OUTER LAYER SIDE CUMULATIVE DISTRIBUTION FUNCTION
4042
4033
4059
4102
4126
4139
mV
100msec
: FITTING RESULT

FIG. 22

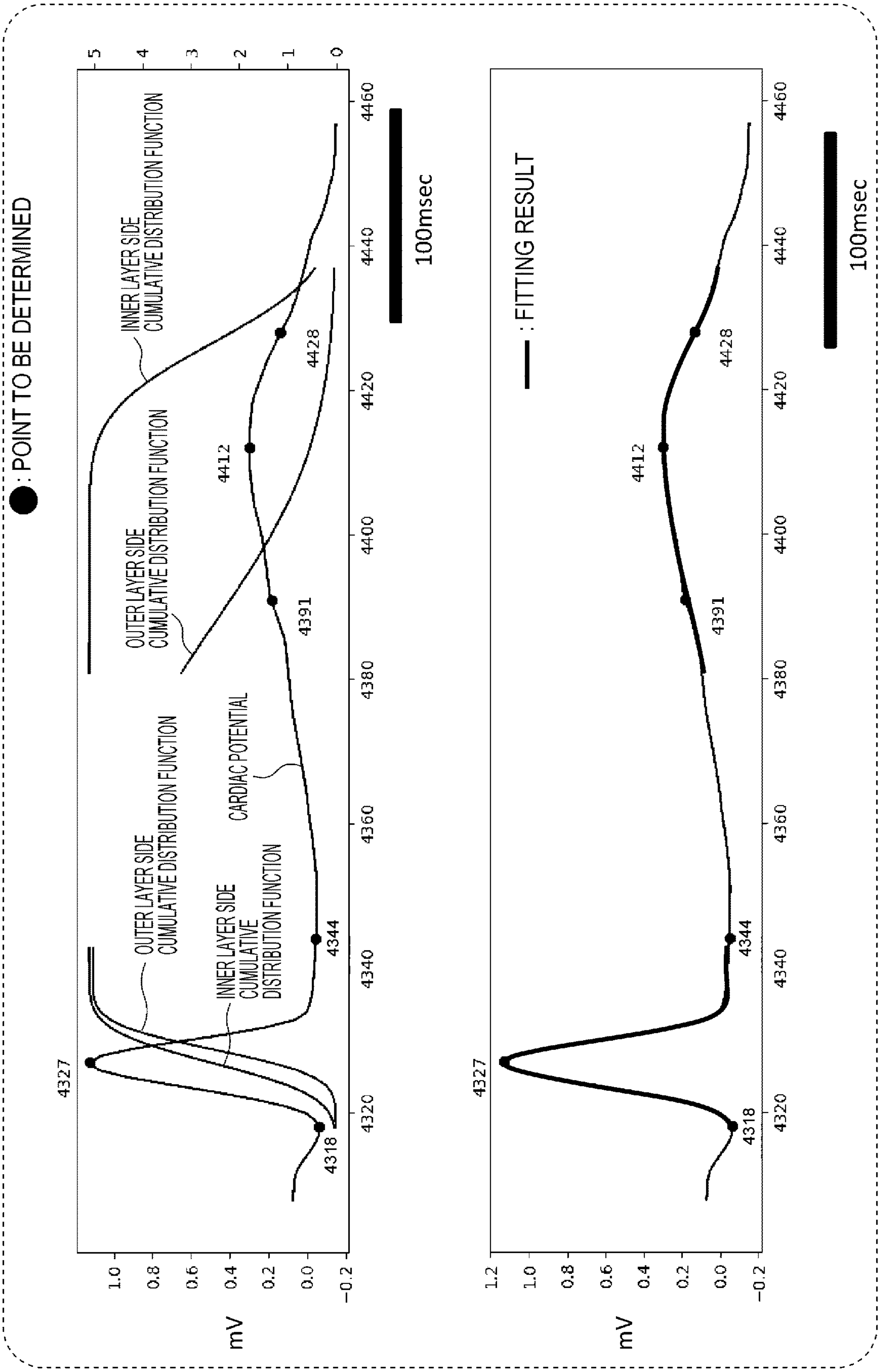
: POINT TO BE DETERMINED
mV
1.0
0.8
0.6
0.4
0.2
0.0
−0.2
5
4
3
2
1
0
4320
4340
4360
4380
4400
4420
4440
4460
4318
4327
4344
4391
4412
4428
OUTER LAYER SIDE CUMULATIVE DISTRIBUTION FUNCTION
INNER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
100msec
: FITTING RESULT
mV
1.2
1.0
0.8
0.6
0.4
0.2
0.0
−0.2
4320
4340
4360
4380
4400
4420
4440
4460
4318
4327
4344
4391
4412
4428
100msec

FIG. 23

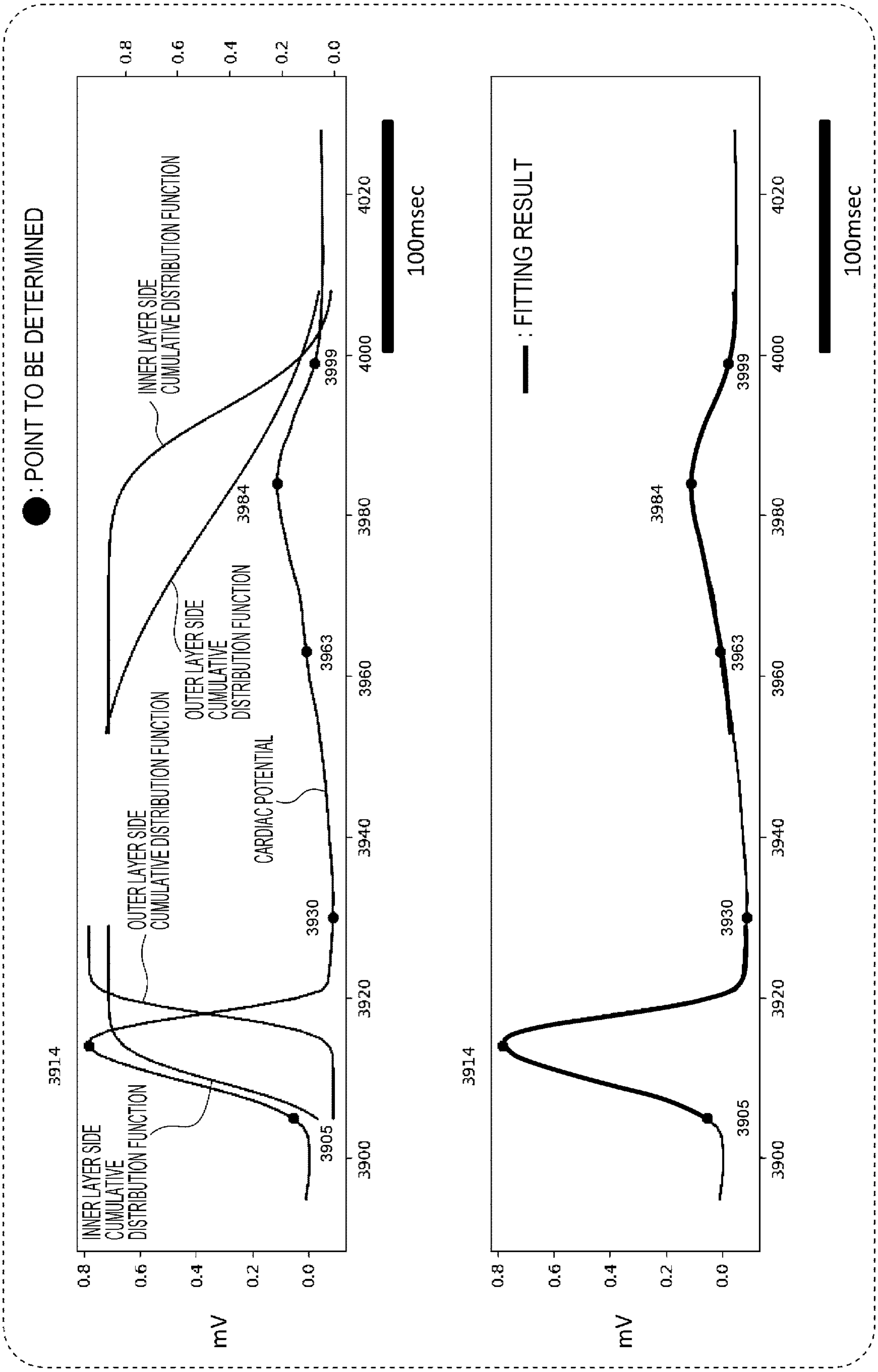

: POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
3905
3914
3930
3963
3984
3999
mV
0.8
0.6
0.4
0.2
0.0
3900
3920
3940
3960
3980
4000
4020
100msec
: FITTING RESULT


FIG. 24

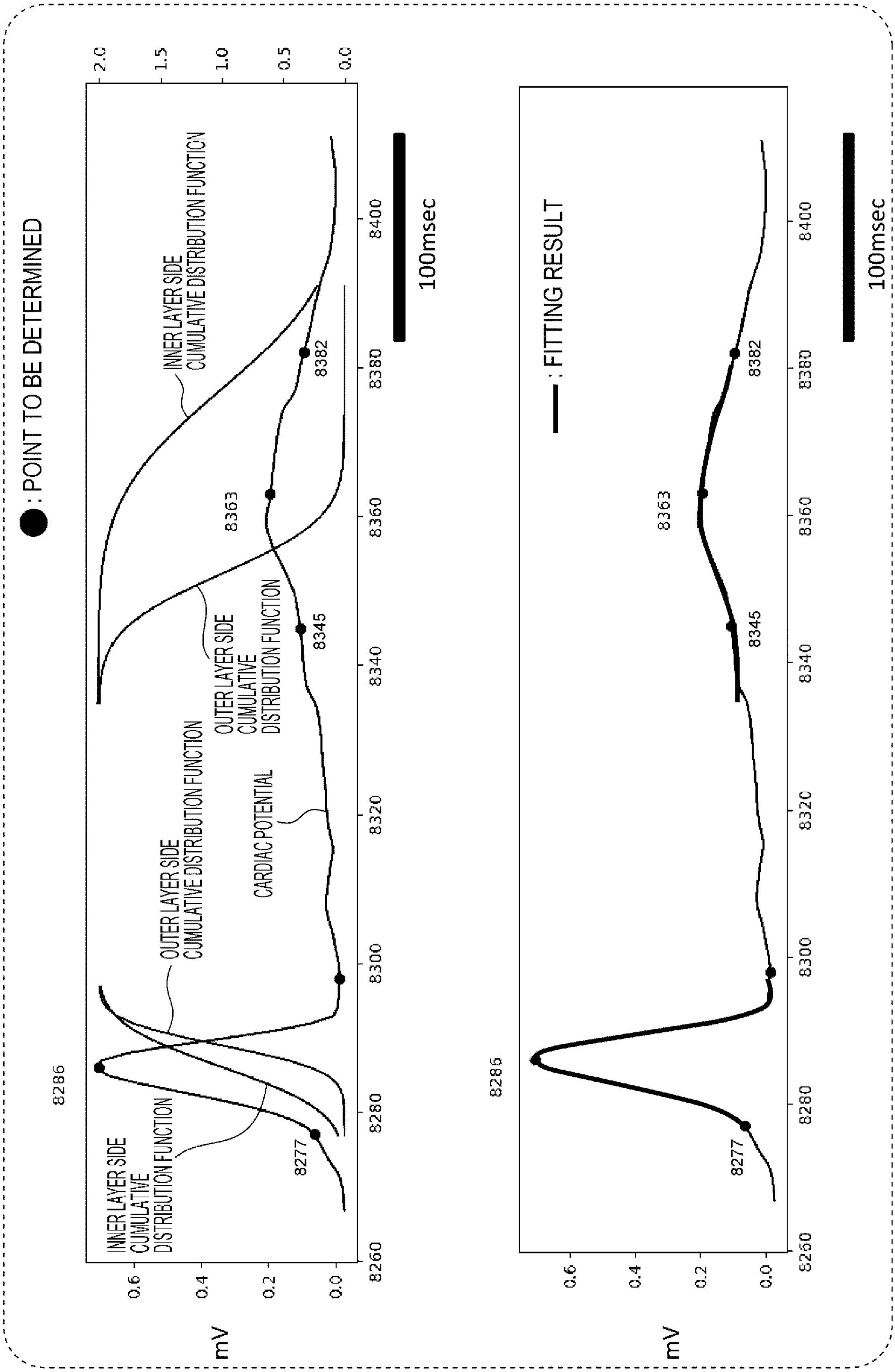
: POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
8277
8286
8345
8363
8382
mV
100msec
: FITTING RESULT

FIG. 25

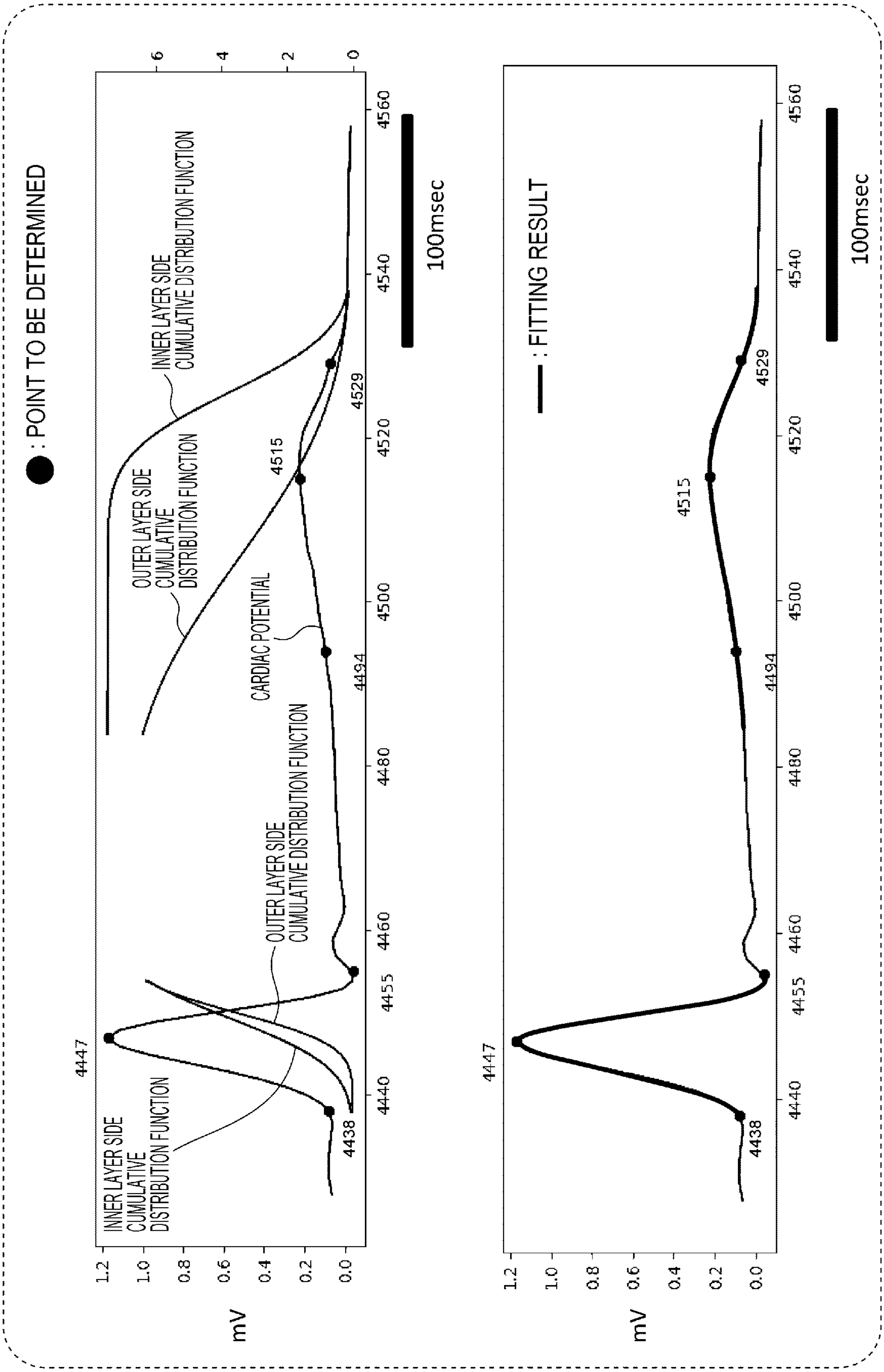
● : POINT TO BE DETERMINED
mV
INNER LAYER SIDE CUMULATIVE DISTRIBUTION FUNCTION
OUTER LAYER SIDE CUMULATIVE DISTRIBUTION FUNCTION
OUTER LAYER SIDE CUMULATIVE DISTRIBUTION FUNCTION
INNER LAYER SIDE CUMULATIVE DISTRIBUTION FUNCTION
CARDIAC POTENTIAL
4438
4447
4455
4494
4515
4529
100msec
: FITTING RESULT

FIG. 26

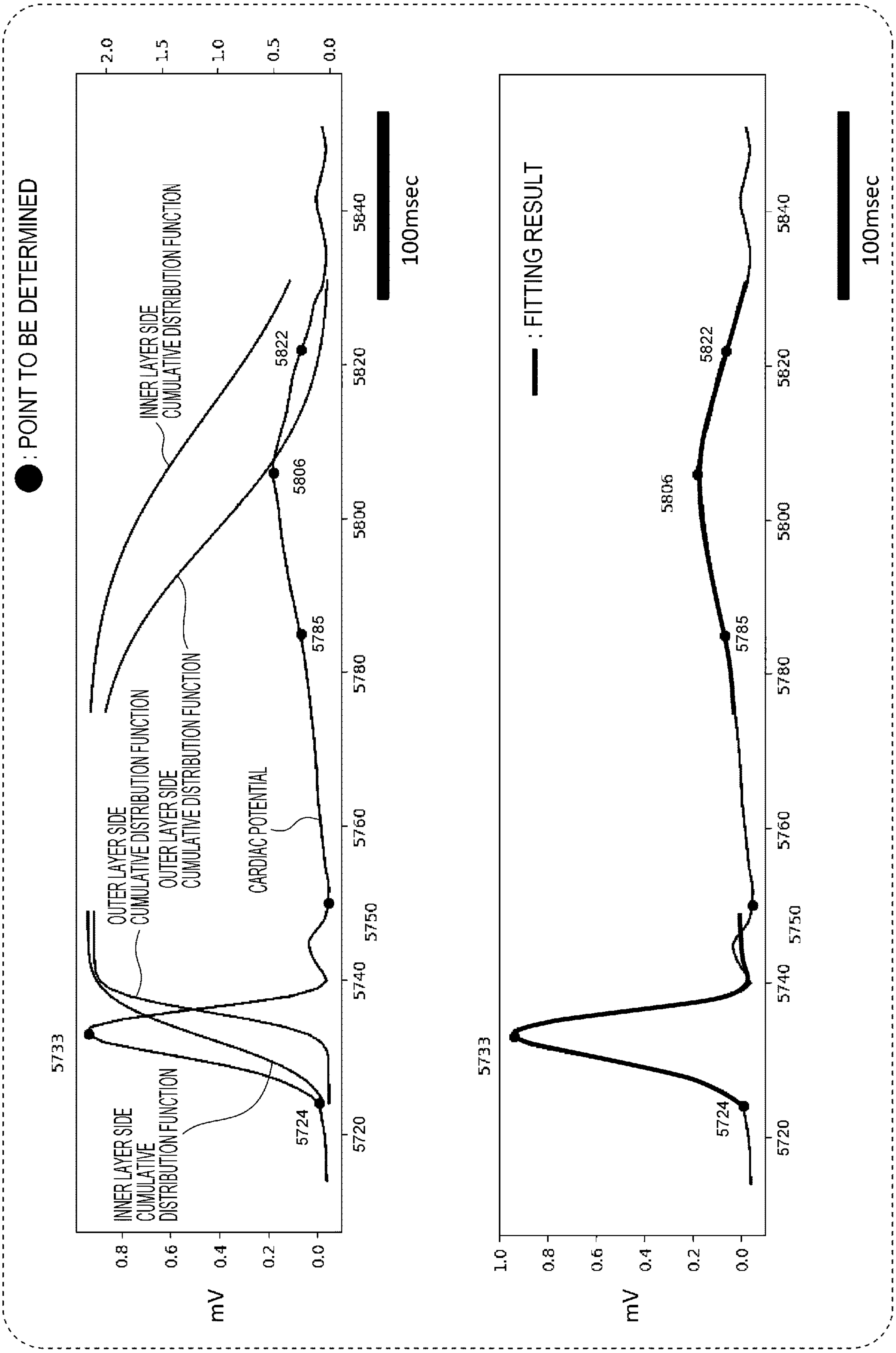
: POINT TO BE DETERMINED
mV
0.8
0.6
0.4
0.2
0.0
2.0
1.5
1.0
0.5
0.0
INNER LAYER SIDE CUMULATIVE DISTRIBUTION FUNCTION
OUTER LAYER SIDE CUMULATIVE DISTRIBUTION FUNCTION
OUTER LAYER SIDE CUMULATIVE DISTRIBUTION FUNCTION
CARDIAC POTENTIAL
INNER LAYER SIDE CUMULATIVE DISTRIBUTION FUNCTION
5724
5733
5750
5785
5806
5822
5720
5740
5750
5760
5780
5800
5820
5840
100msec
: FITTING RESULT
mV
1.0
0.8
0.6
0.4
0.2
0.0
5724
5733
5785
5806
5822
5720
5740
5750
5760
5780
5800
5820
5840
100msec

FIG. 27

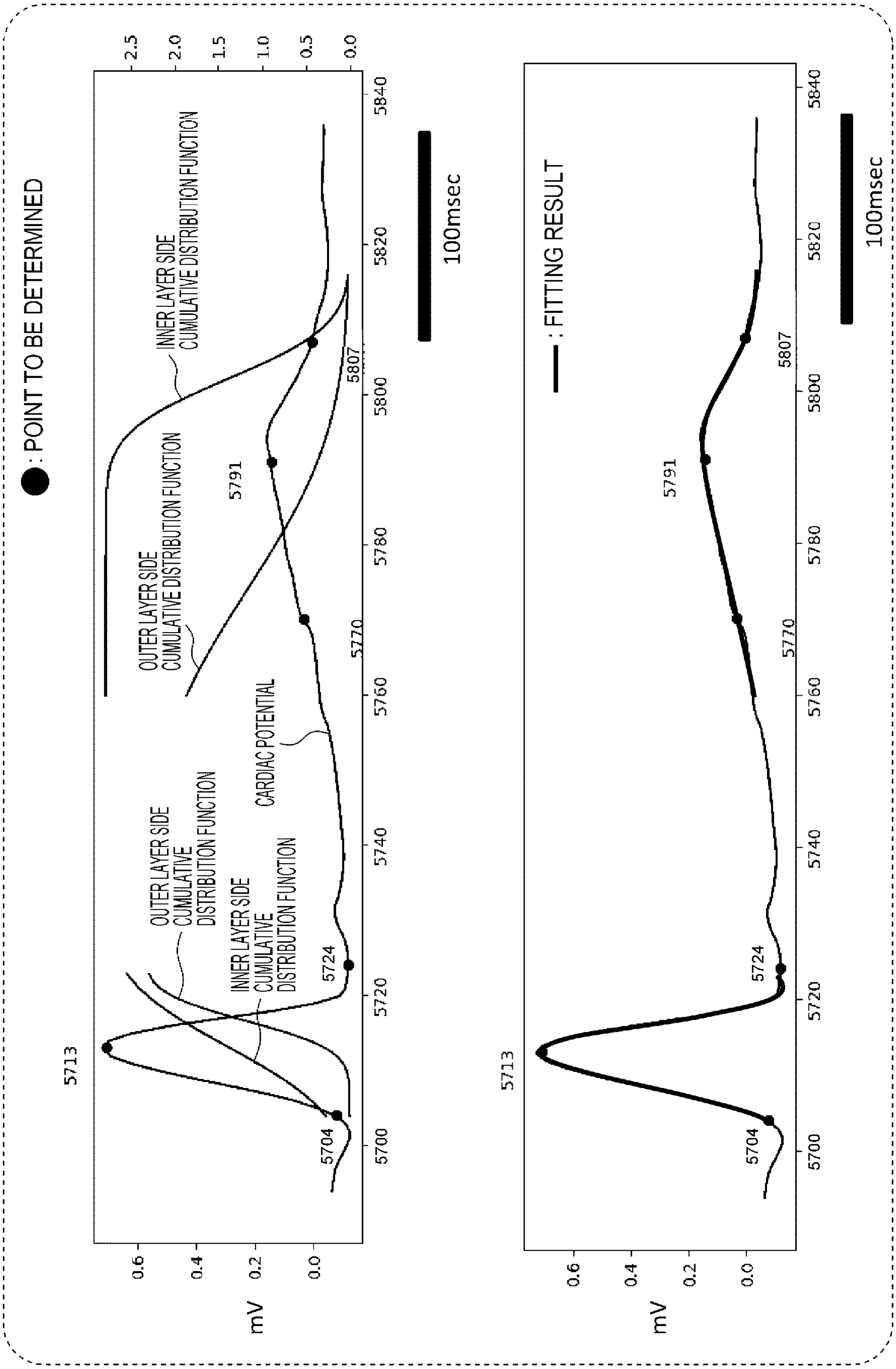
●: POINT TO BE DETERMINED
mV
0.6
0.4
0.2
0.0
2.5
2.0
1.5
1.0
0.5
0.0
5700
5720
5740
5760
5780
5800
5820
5840
5704
5713
5724
5770
5791
5807
OUTER LAYER SIDE CUMULATIVE DISTRIBUTION FUNCTION
INNER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
100msec
: FITTING RESULT
100msec

FIG. 28

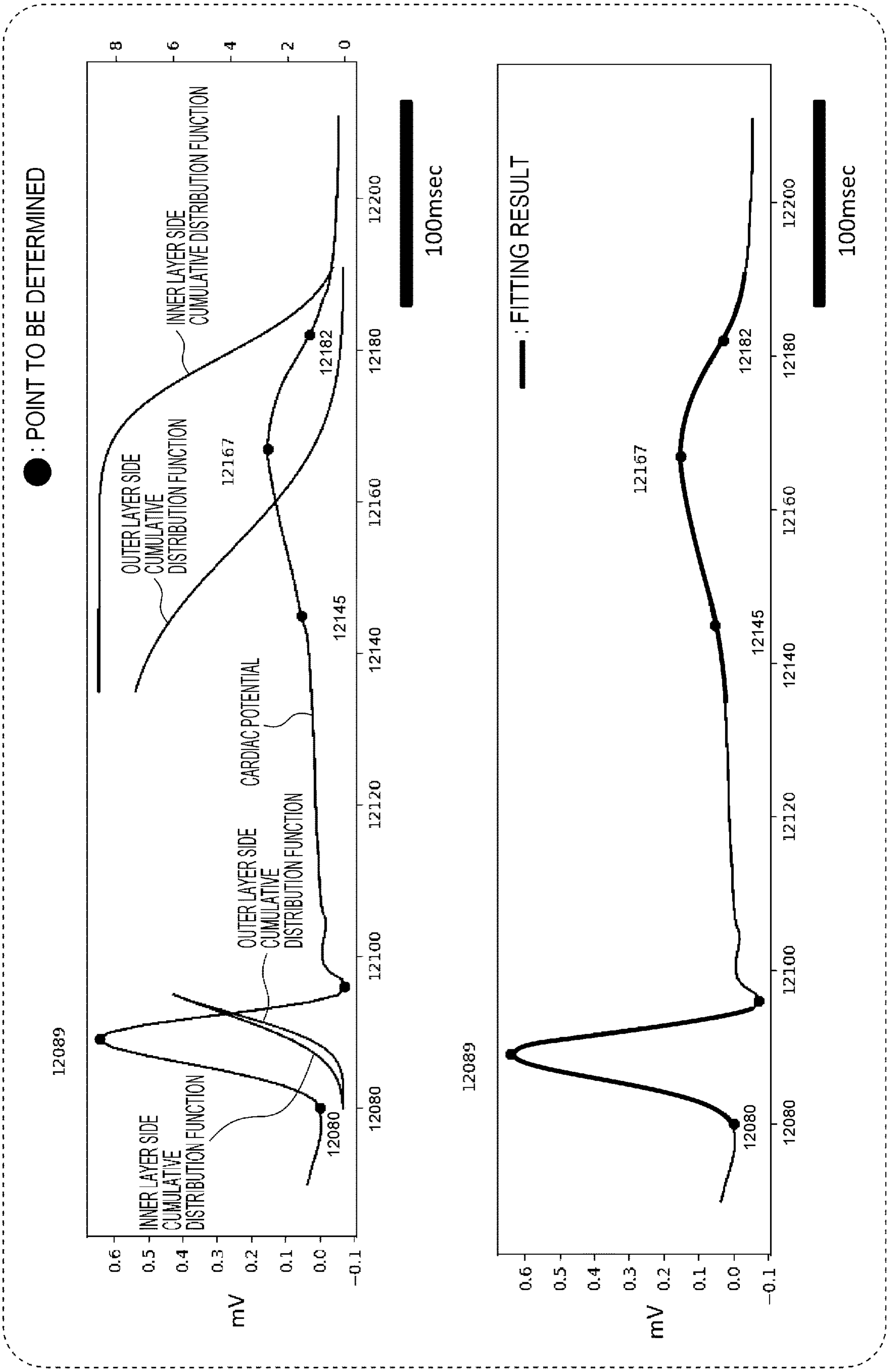
: POINT TO BE DETERMINED
INNER LAYER SIDE CUMULATIVE DISTRIBUTION FUNCTION
OUTER LAYER SIDE CUMULATIVE DISTRIBUTION FUNCTION
OUTER LAYER SIDE CUMULATIVE DISTRIBUTION FUNCTION
CARDIAC POTENTIAL
INNER LAYER SIDE CUMULATIVE DISTRIBUTION FUNCTION
12080
12089
12145
12167
12182
mV
0.6
0.5
0.4
0.3
0.2
0.1
0.0
−0.1
12080
12100
12120
12140
12160
12180
12200
8
6
4
2
0
100msec
: FITTING RESULT
12080
12089
12145
12167
12182
mV
100msec

FIG. 29

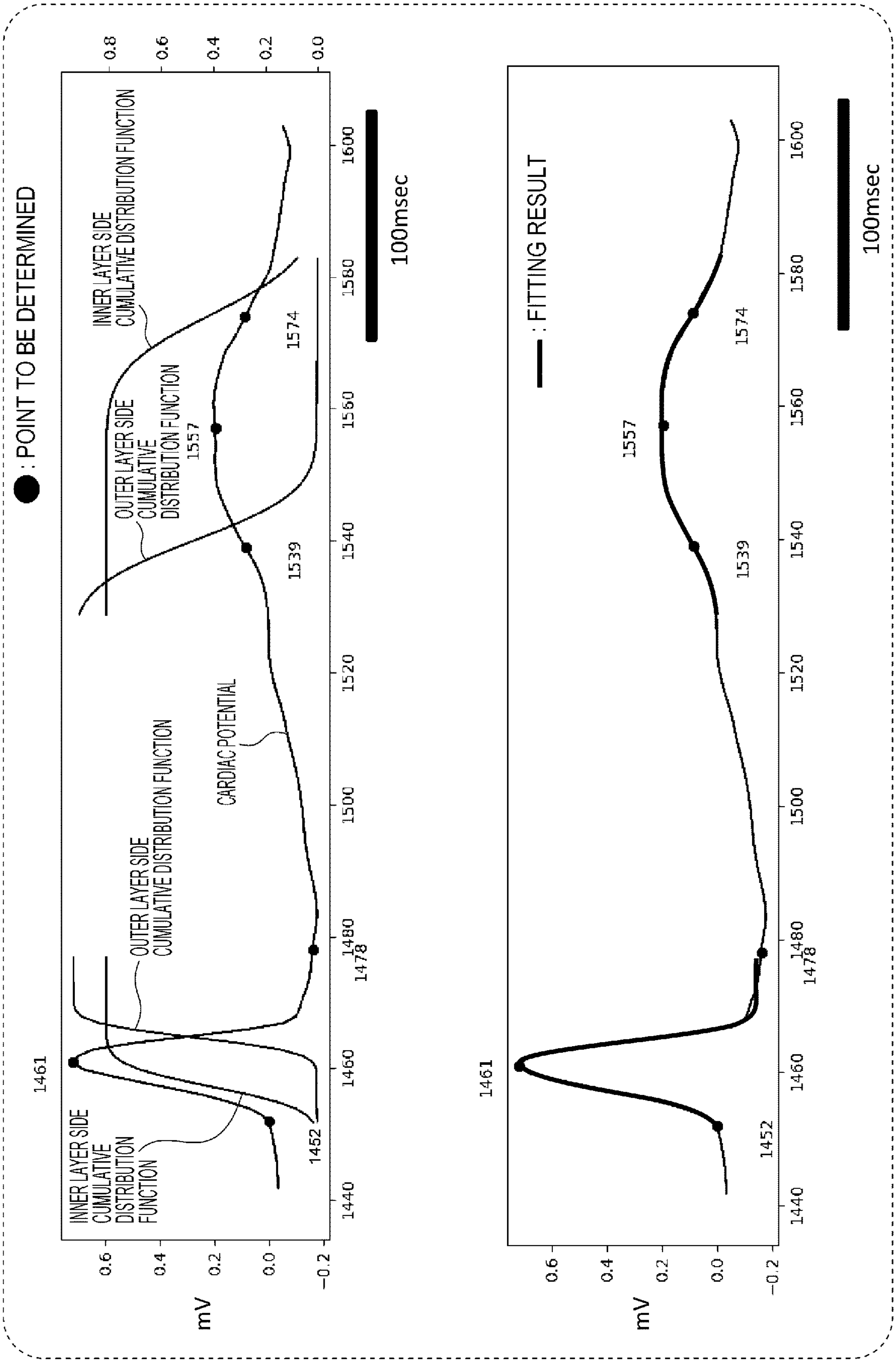
●: POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
1452
1461
1478
1539
1557
1574
mV
100msec
: FITTING RESULT
1452
1461
1478
1539
1557
1574
mV
100msec

FIG. 30

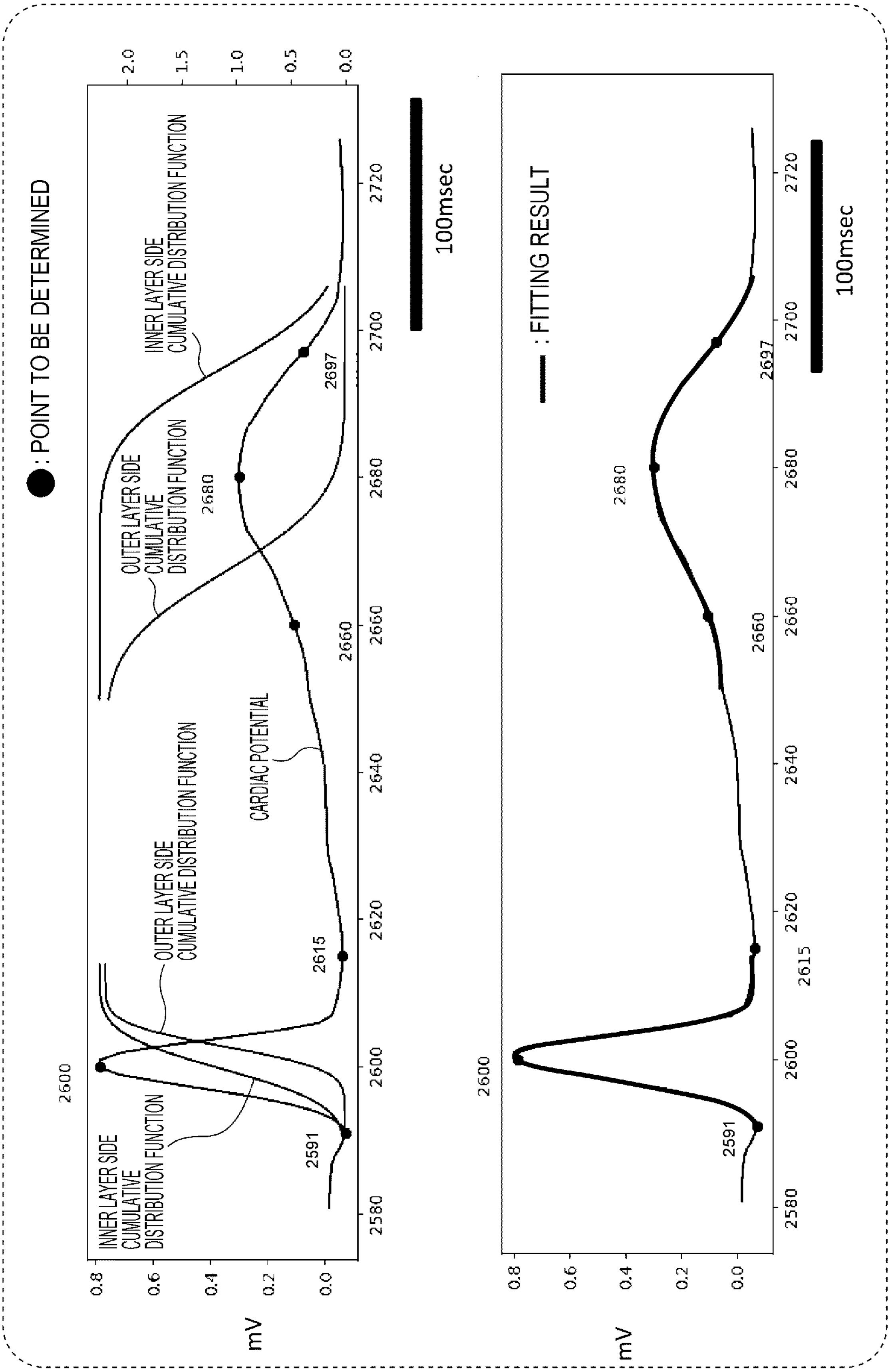

: POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
2591
2600
2615
2660
2680
2697
mV
0.8
0.6
0.4
0.2
0.0
2.0
1.5
1.0
0.5
0.0
2580
2600
2620
2640
2660
2680
2700
2720
100msec
: FITTING RESULT


FIG. 31

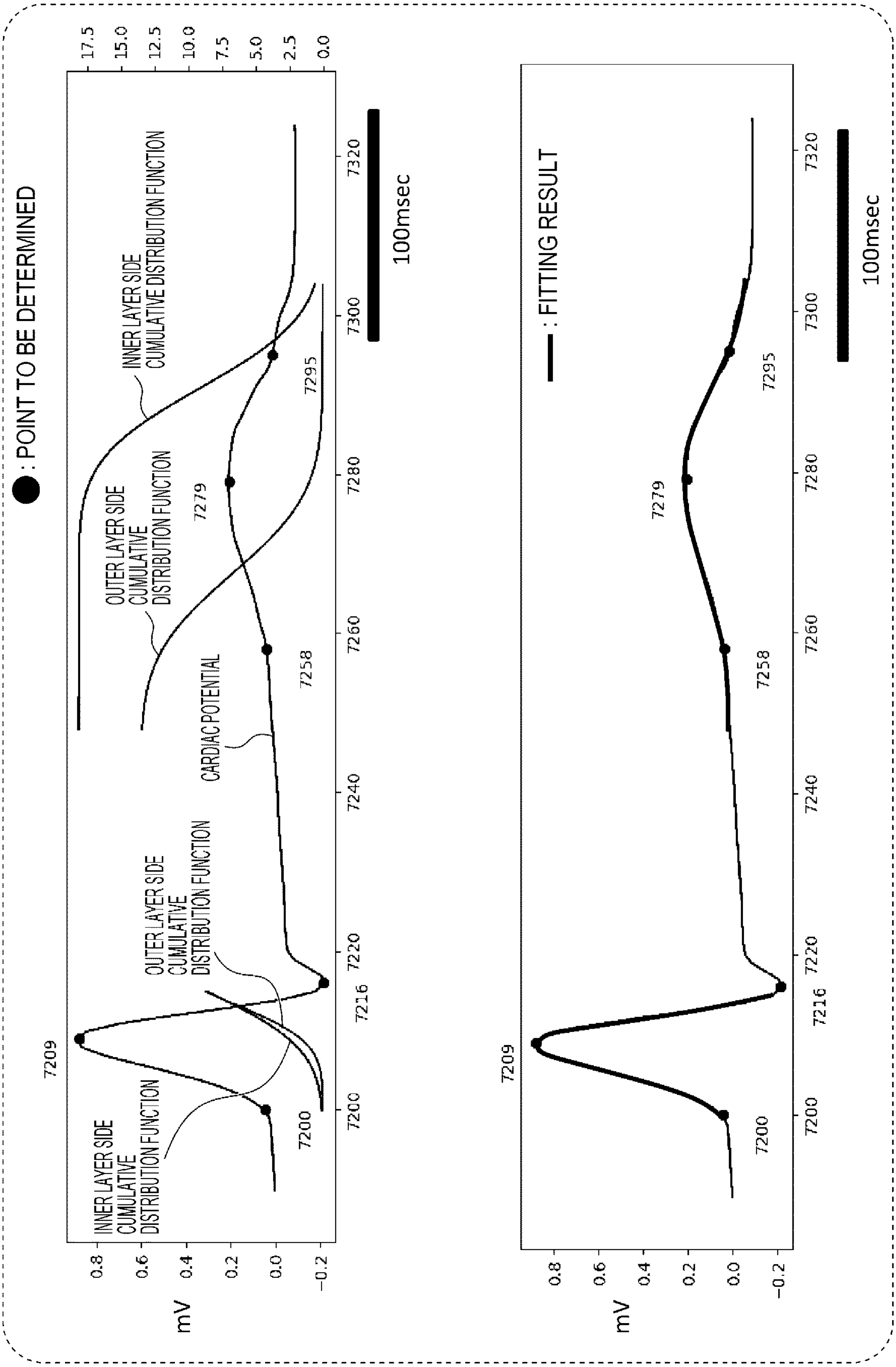

: POINT TO BE DETERMINED
INNER LAYER SIDE CUMULATIVE DISTRIBUTION FUNCTION
OUTER LAYER SIDE CUMULATIVE DISTRIBUTION FUNCTION
OUTER LAYER SIDE CUMULATIVE DISTRIBUTION FUNCTION
INNER LAYER SIDE CUMULATIVE DISTRIBUTION FUNCTION
CARDIAC POTENTIAL
7200
7209
7216
7258
7279
7295
mV
0.8
0.6
0.4
0.2
0.0
−0.2
17.5
15.0
12.5
10.0
7.5
5.0
2.5
0.0
7200
7220
7240
7260
7280
7300
7320
100msec
: FITTING RESULT


FIG. 32

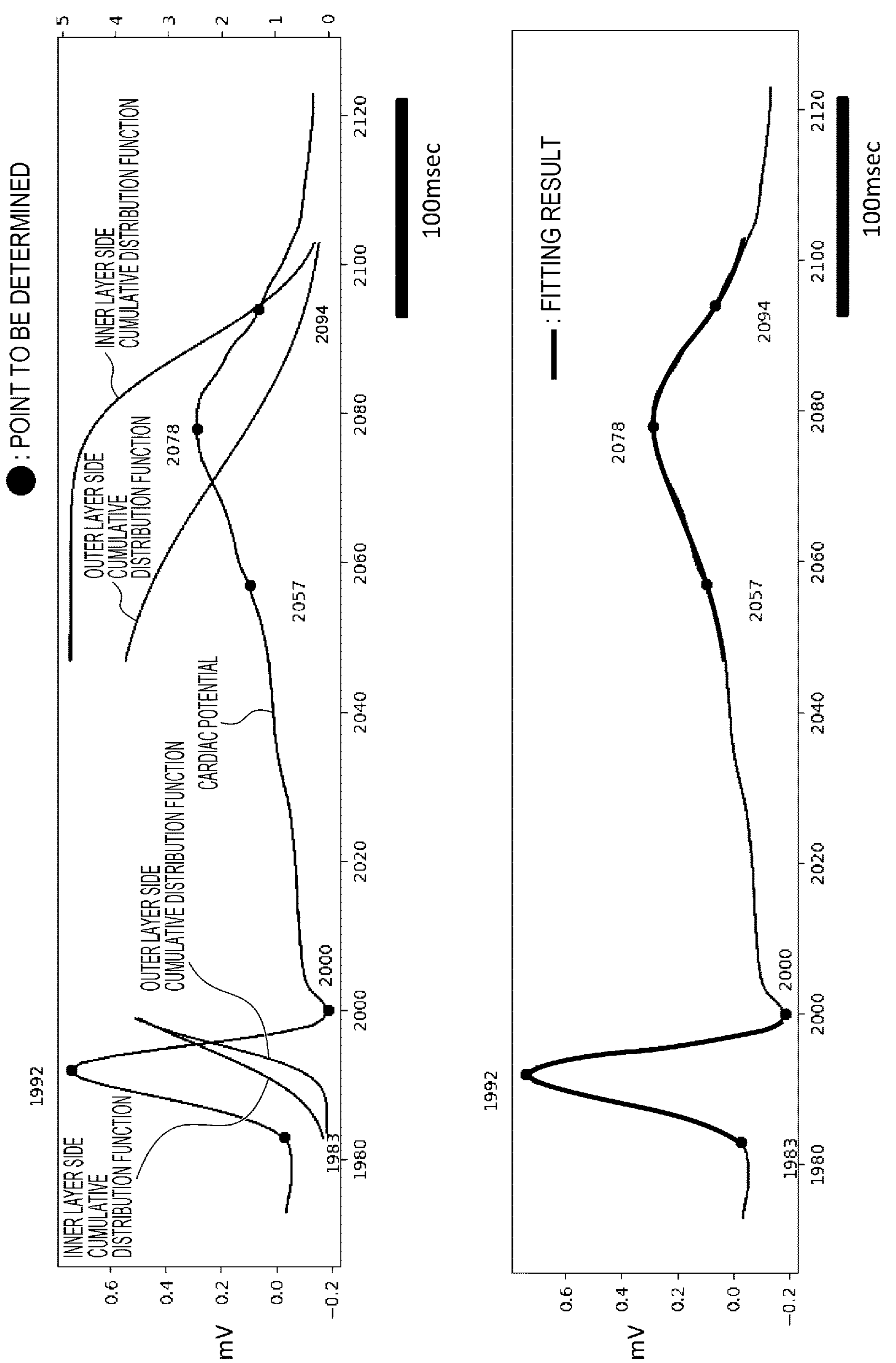
● : POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
1983
1992
2000
2057
2078
2094
mV
0.6
0.4
0.2
0.0
−0.2
1980
2000
2020
2040
2060
2080
2100
2120
0
1
2
3
4
5
100msec
— : FITTING RESULT

FIG. 33

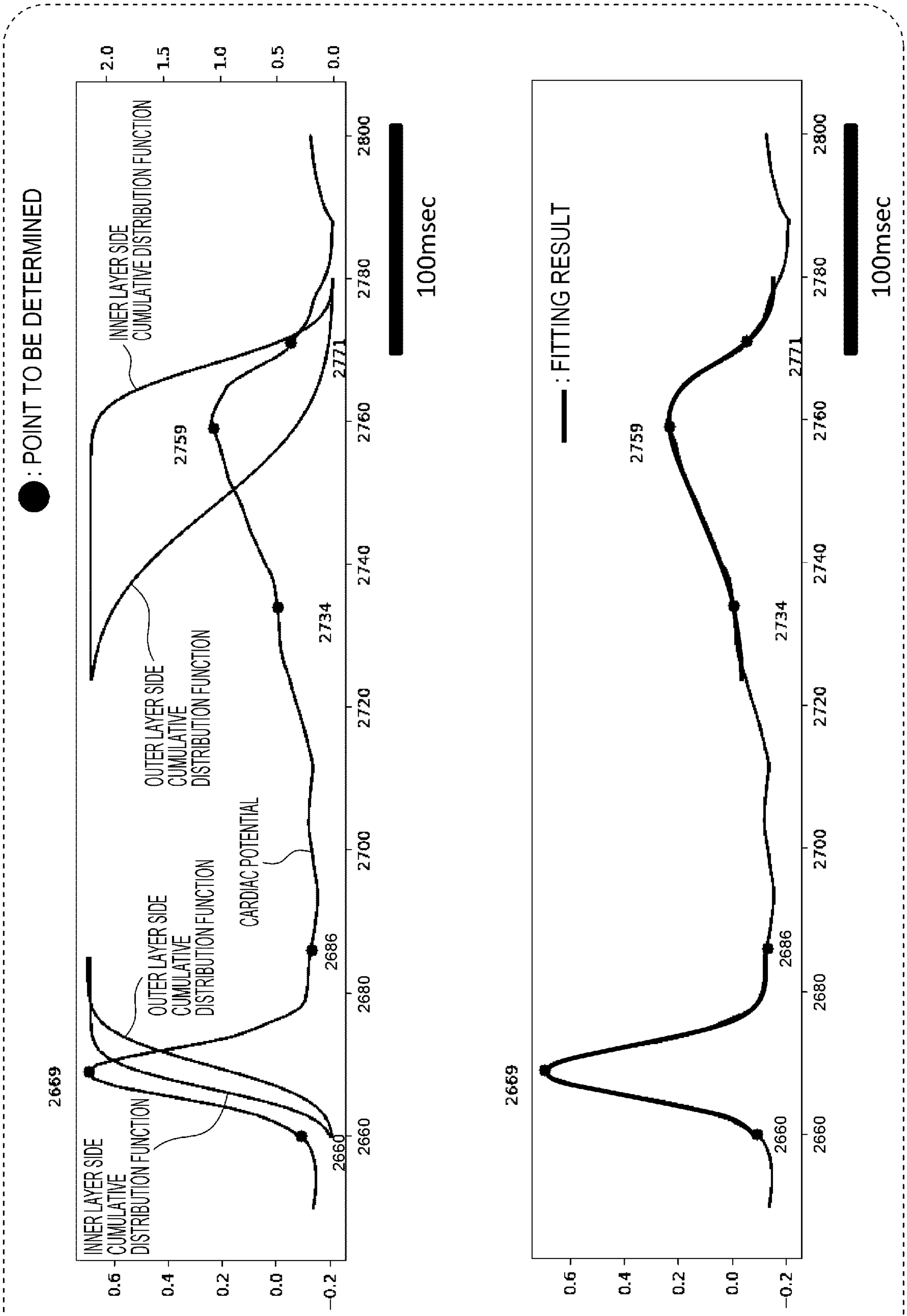
: POINT TO BE DETERMINED
INNER LAYER SIDE CUMULATIVE DISTRIBUTION FUNCTION
OUTER LAYER SIDE CUMULATIVE DISTRIBUTION FUNCTION
INNER LAYER SIDE CUMULATIVE DISTRIBUTION FUNCTION
OUTER LAYER SIDE CUMULATIVE DISTRIBUTION FUNCTION
CARDIAC POTENTIAL
2669
2660
2686
2734
2759
2771
mV
0.6
0.4
0.2
0.0
−0.2
2.0
1.5
1.0
0.5
0.0
2660
2680
2700
2720
2740
2760
2780
2800
100msec
: FITTING RESULT
2669
2660
2686
2734
2759
2771
mV
0.6
0.4
0.2
0.0
−0.2
2660
2680
2700
2720
2740
2760
2780
2800
100msec

FIG. 34

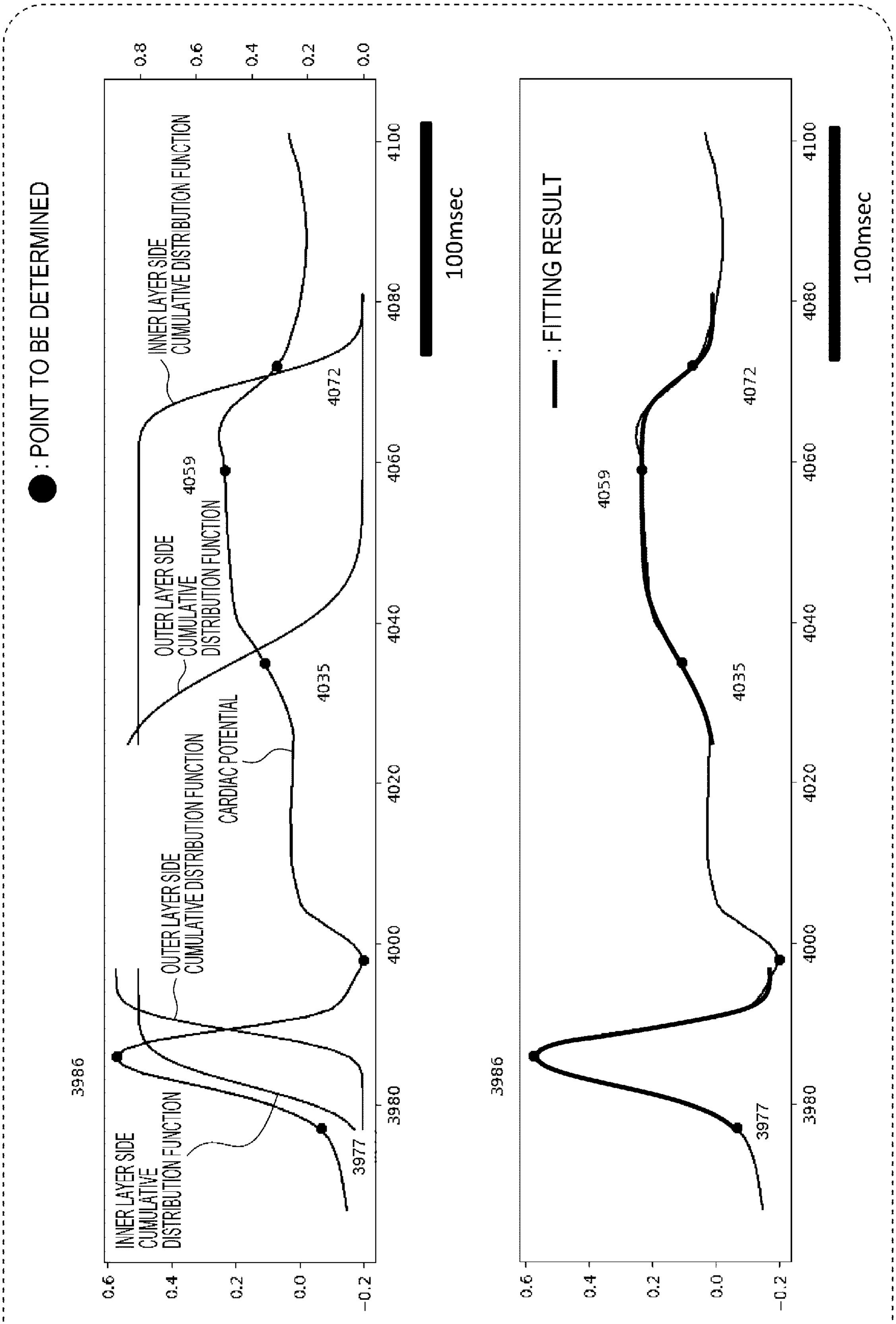
● : POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
3977
3986
4035
4059
4072
mV
0.6
0.4
0.2
0.0
−0.2
0.8
3980
4000
4020
4040
4060
4080
4100
100msec
: FITTING RESULT

FIG. 35

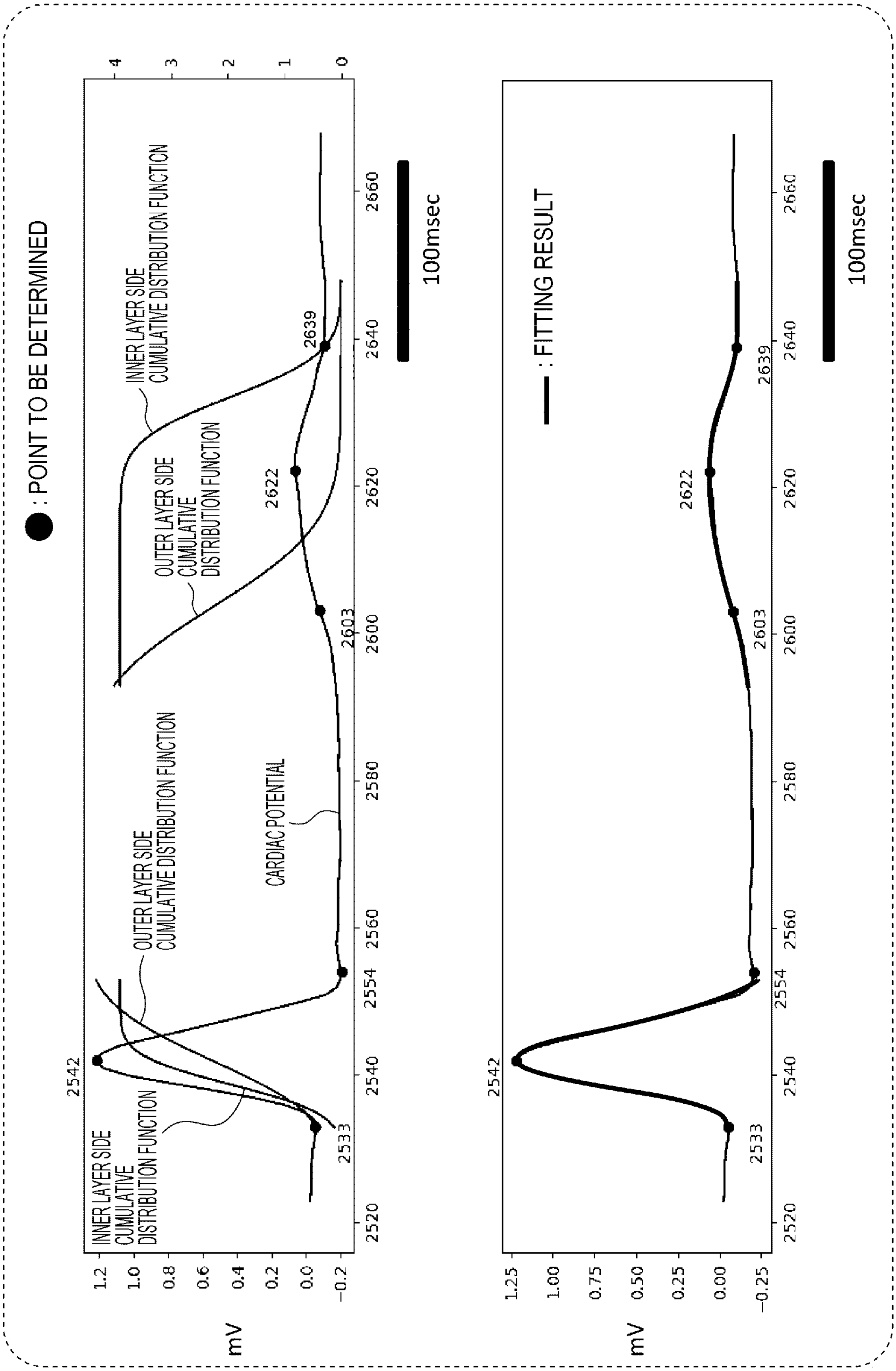
: POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
2533
2542
2554
2603
2622
2639
mV
1.2
1.0
0.8
0.6
0.4
0.2
0.0
−0.2
4
3
2
1
0
2520
2540
2560
2580
2600
2620
2640
2660
100msec
: FITTING RESULT
1.25
1.00
0.75
0.50
0.25
0.00
−0.25

FIG. 36

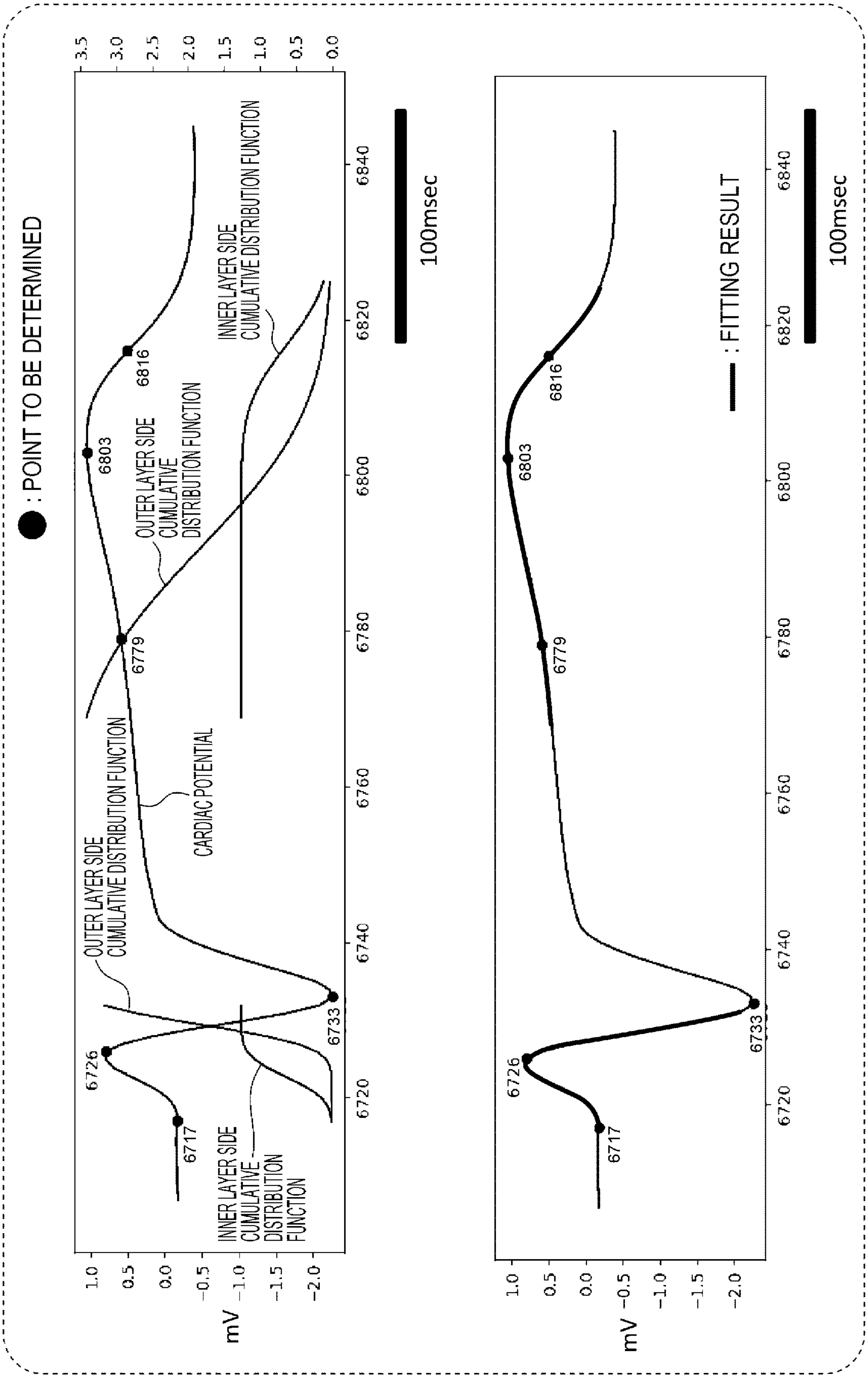
: POINT TO BE DETERMINED
6717
6726
6733
6779
6803
6816
OUTER LAYER SIDE CUMULATIVE DISTRIBUTION FUNCTION
INNER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
mV
1.0
0.5
0.0
−0.5
−1.0
−1.5
−2.0
3.5
3.0
2.5
2.0
1.5
1.0
0.5
0.0
6720
6740
6760
6780
6800
6820
6840
100msec
: FITTING RESULT
6717
6726
6733
6779
6803
6816
mV
1.0
0.5
0.0
−0.5
−1.0
−1.5
−2.0
6720
6740
6760
6780
6800
6820
6840
100msec

FIG. 37

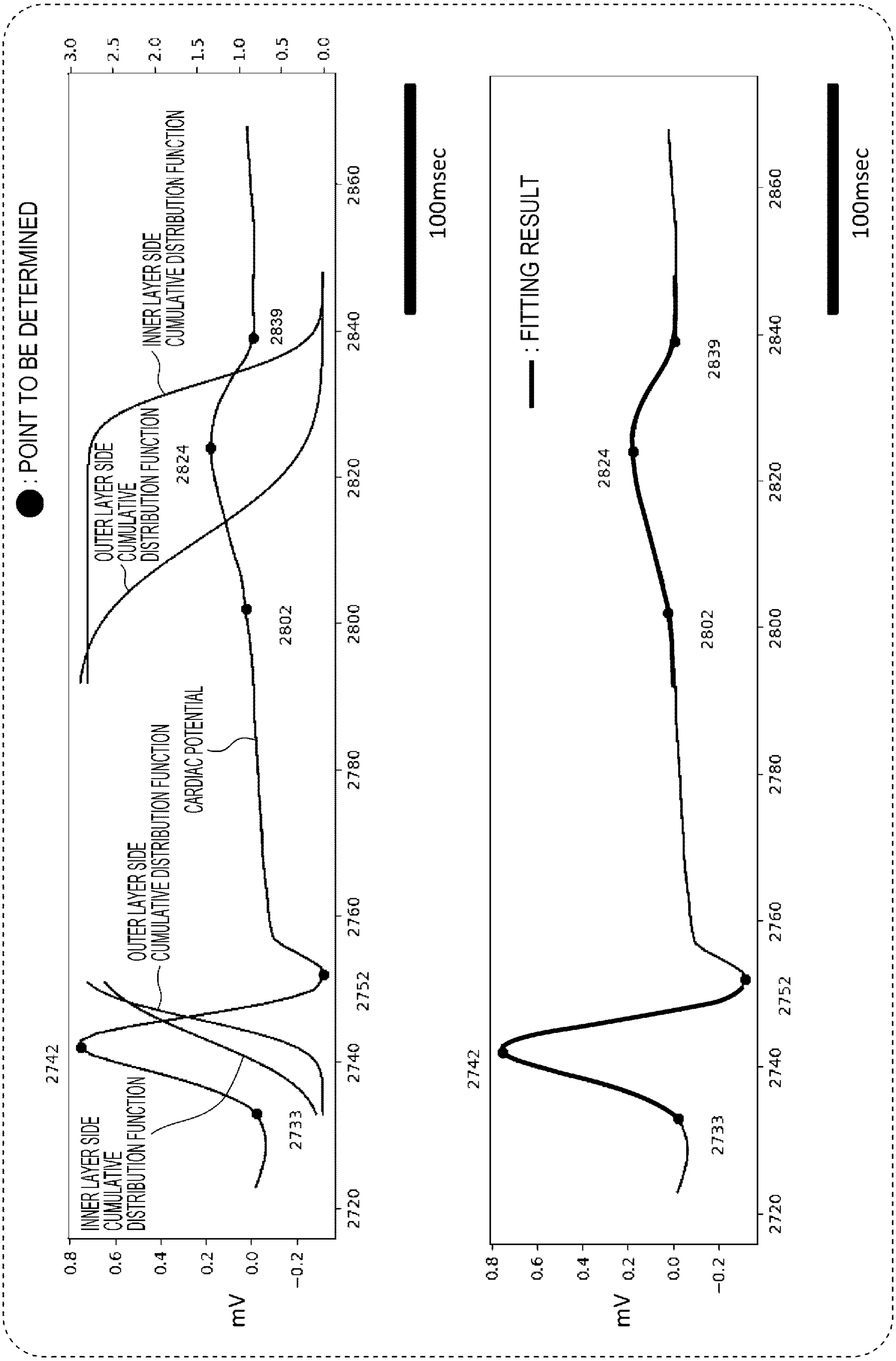
● : POINT TO BE DETERMINED
mV
0.8
0.6
0.4
0.2
0.0
−0.2
3.0
2.5
2.0
1.5
1.0
0.5
0.0
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
2733
2742
2752
2802
2824
2839
2720
2740
2760
2780
2800
2820
2840
2860
100msec
: FITTING RESULT

FIG. 38

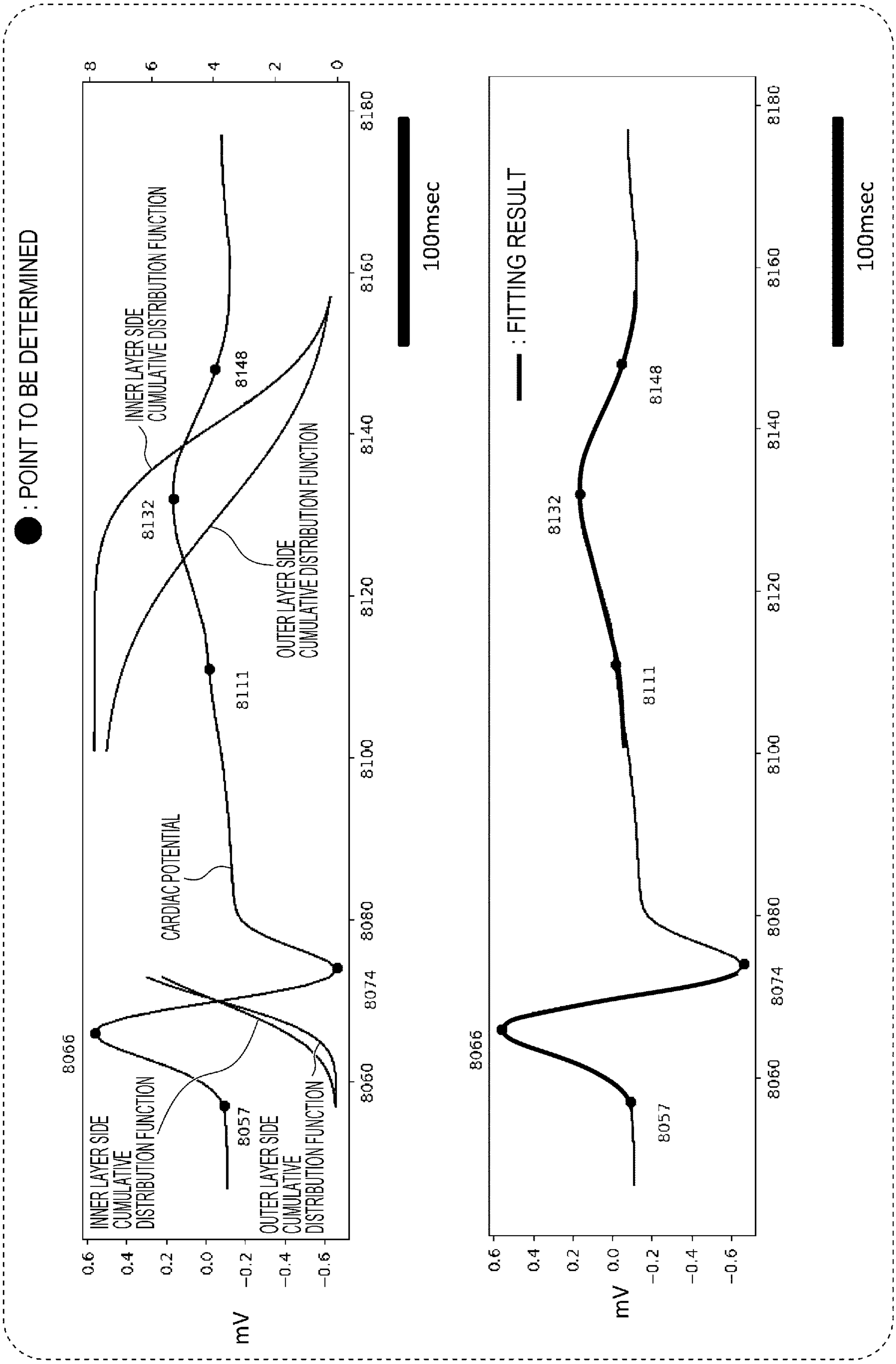
: POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
INNER LAYER SIDE CUMULATIVE DISTRIBUTION FUNCTION
OUTER LAYER SIDE CUMULATIVE DISTRIBUTION FUNCTION
8057
8066
8074
8111
8132
8148
mV
0.6
0.4
0.2
0.0
−0.2
−0.4
−0.6
8060
8080
8100
8120
8140
8160
8180
8
6
4
2
0
100msec
: FITTING RESULT
8057
8066
8074
8111
8132
8148
mV
0.6
0.4
0.2
0.0
−0.2
−0.4
−0.6
8060
8080
8100
8120
8140
8160
8180
100msec

FIG. 39

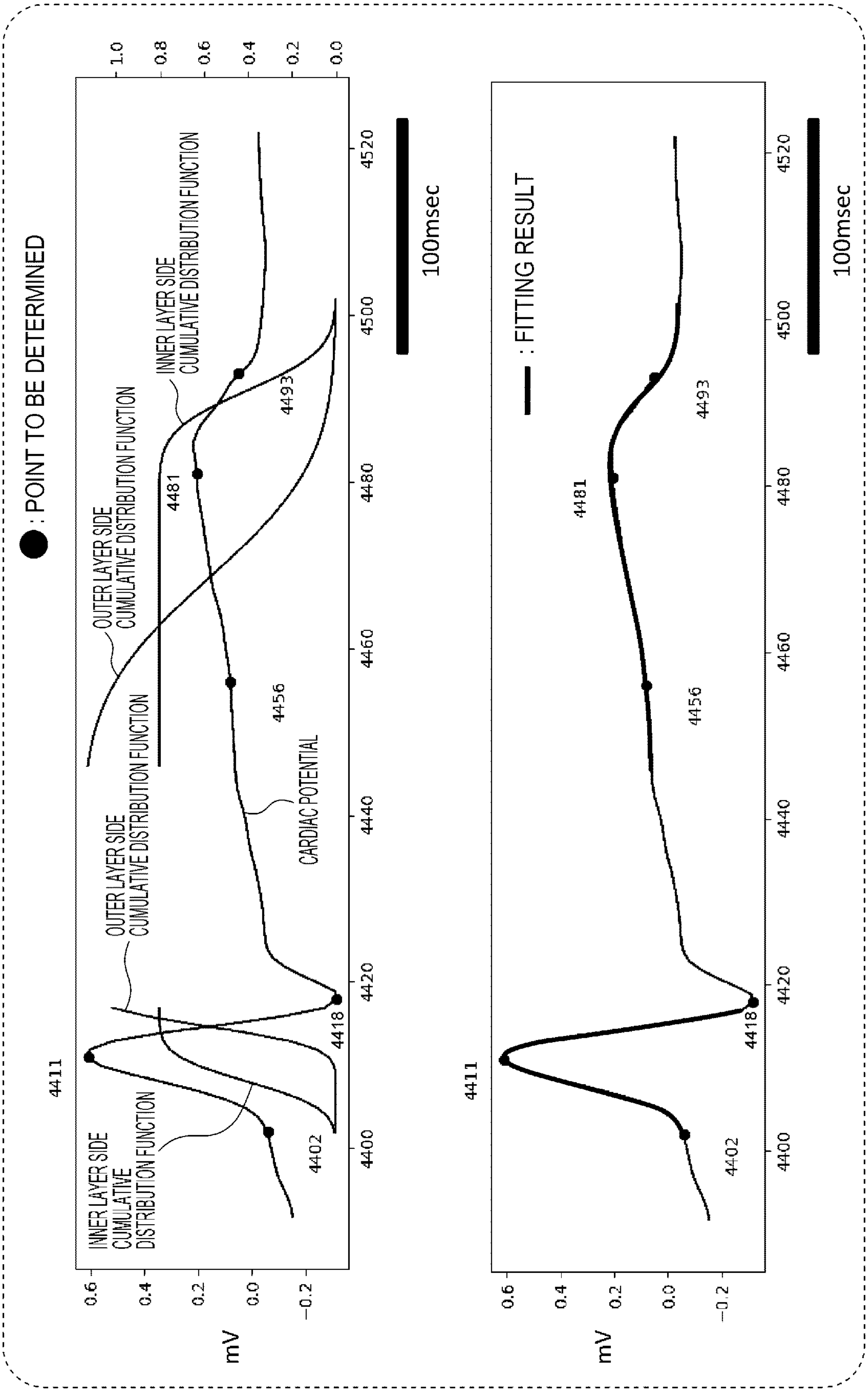
: POINT TO BE DETERMINED
OUTER LAYER SIDE CUMULATIVE DISTRIBUTION FUNCTION
INNER LAYER SIDE CUMULATIVE DISTRIBUTION FUNCTION
INNER LAYER SIDE CUMULATIVE DISTRIBUTION FUNCTION
OUTER LAYER SIDE CUMULATIVE DISTRIBUTION FUNCTION
CARDIAC POTENTIAL
4402
4411
4418
4456
4481
4493
mV
0.6
0.4
0.2
0.0
−0.2
1.0
0.8
0.6
0.4
0.2
0.0
4400
4420
4440
4460
4480
4500
4520
100msec
: FITTING RESULT
4402
4411
4418
4456
4481
4493
mV
0.6
0.4
0.2
0.0
−0.2
4400
4420
4440
4460
4480
4500
4520
100msec

FIG. 40

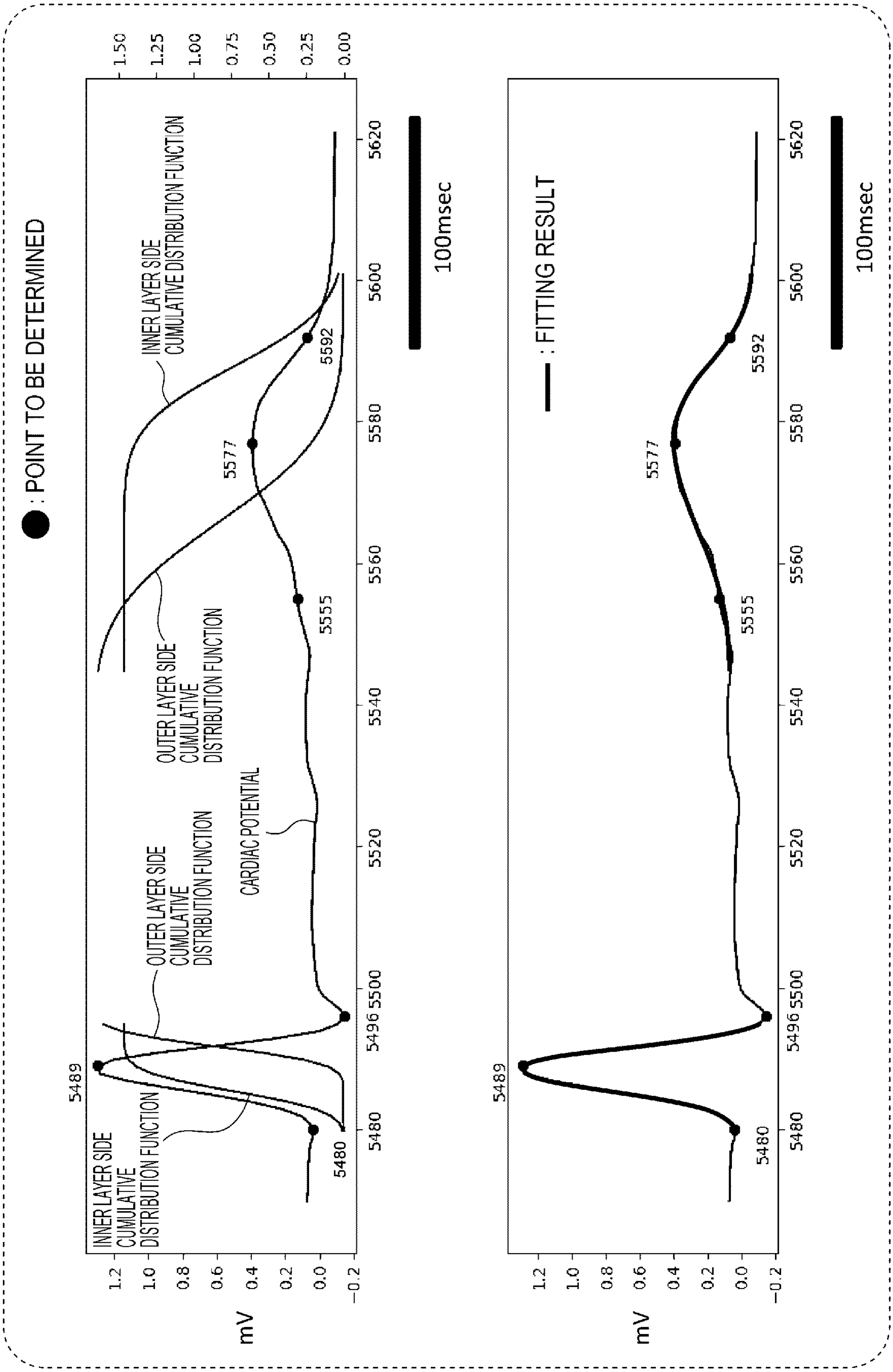
● : POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
5480
5489
5496
5555
5577
5592
mV
100msec
: FITTING RESULT
5480
5489
5496
5555
5577
5592
mV
100msec

FIG. 41

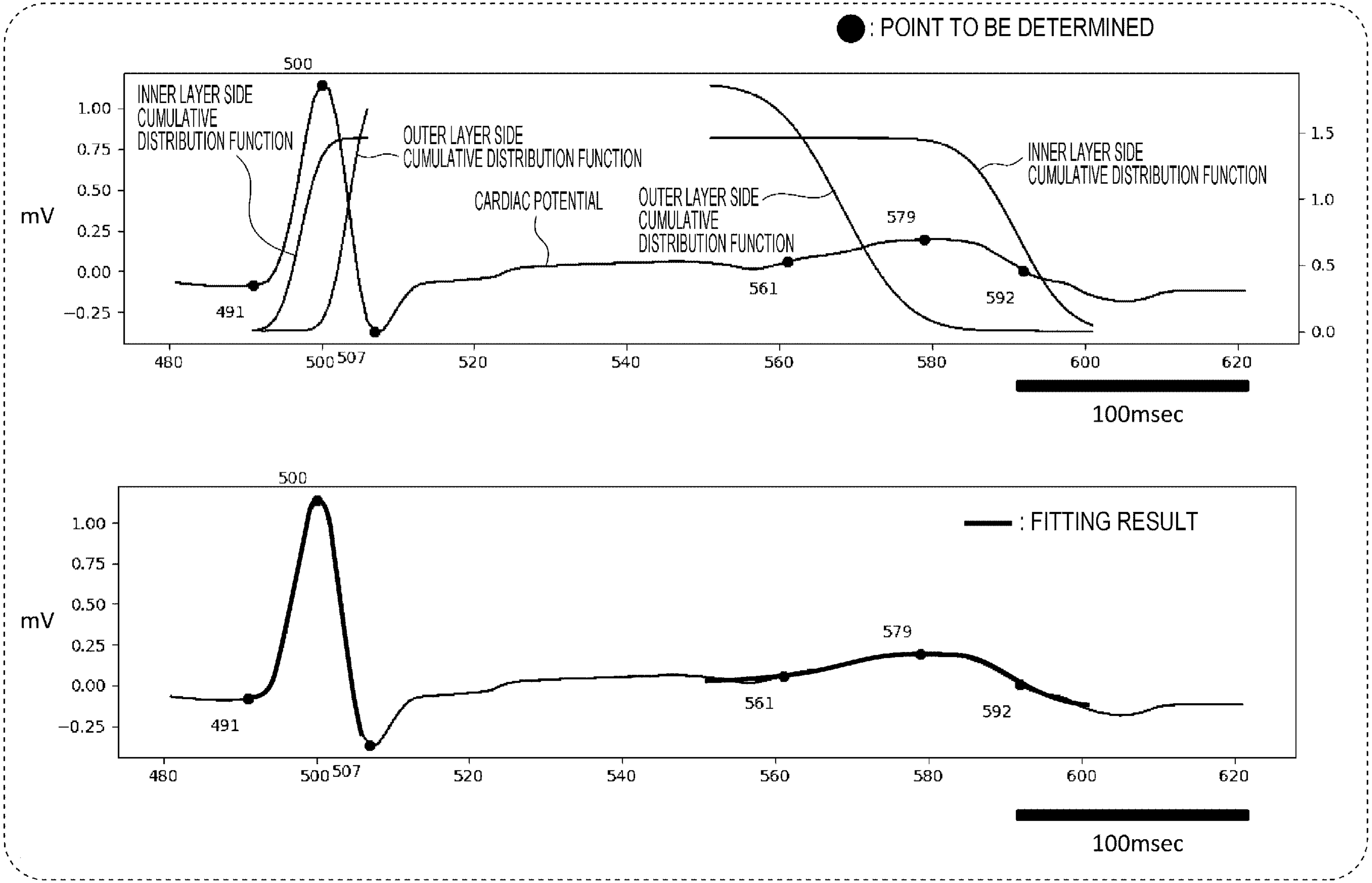
: POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
mV
1.00
0.75
0.50
0.25
0.00
−0.25
1.5
1.0
0.5
0.0
500
491
507
561
579
592
480
500
520
540
560
580
600
620
100msec
: FITTING RESULT
mV
1.00
0.75
0.50
0.25
0.00
−0.25
500
491
507
561
579
592
480
500
520
540
560
580
600
620
100msec

FIG. 42

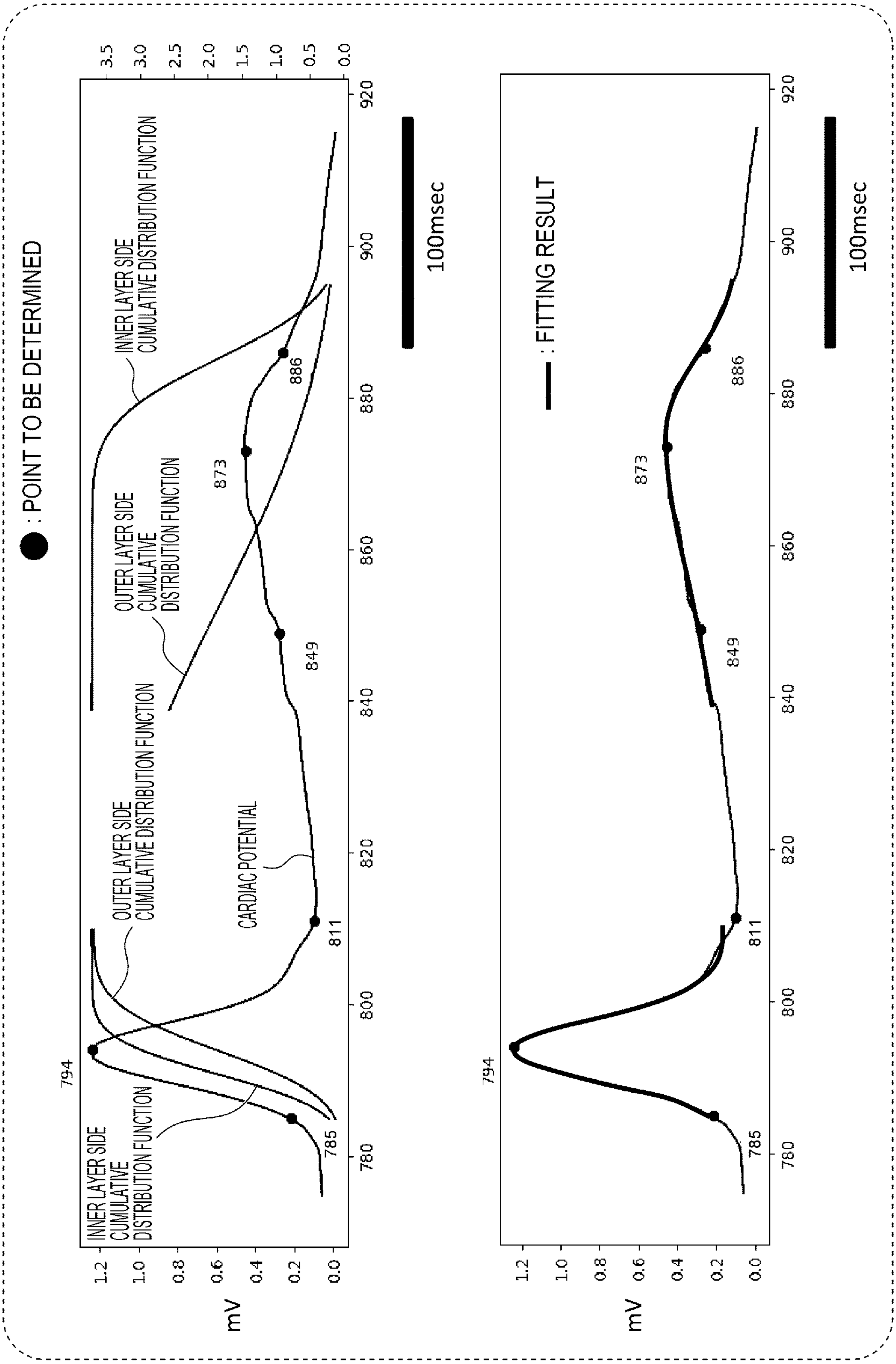
: POINT TO BE DETERMINED
INNER LAYER SIDE CUMULATIVE DISTRIBUTION FUNCTION
OUTER LAYER SIDE CUMULATIVE DISTRIBUTION FUNCTION
OUTER LAYER SIDE CUMULATIVE DISTRIBUTION FUNCTION
INNER LAYER SIDE CUMULATIVE DISTRIBUTION FUNCTION
CARDIAC POTENTIAL
785
794
811
849
873
886
mV
100msec
: FITTING RESULT
785
794
811
849
873
886
mV
100msec

FIG. 43

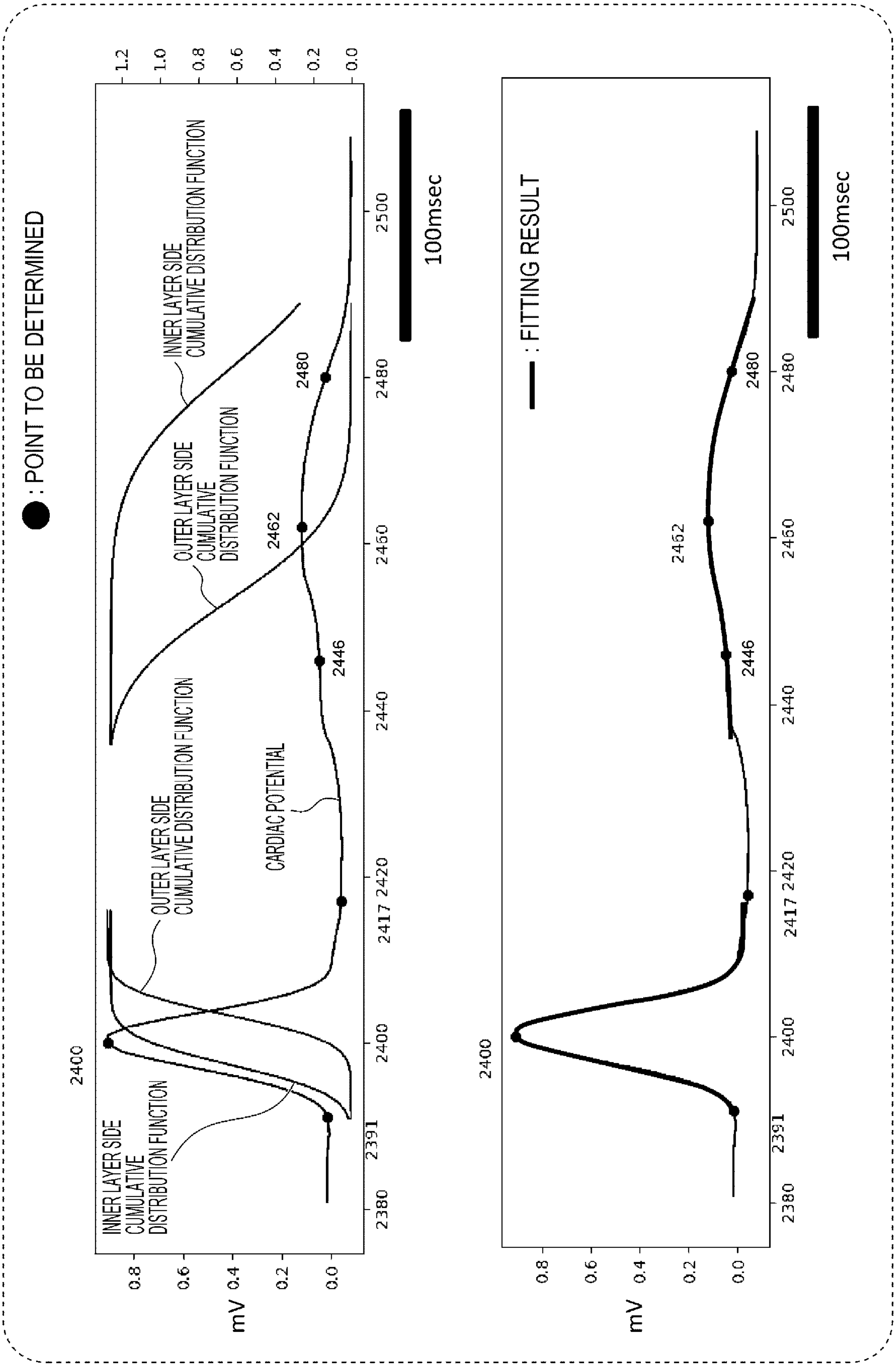
: POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
2400
2391
2417
2446
2462
2480
mV
0.0
0.2
0.4
0.6
0.8
1.0
1.2
2380
2420
2440
2460
2500
100msec
: FITTING RESULT
2452

FIG. 44

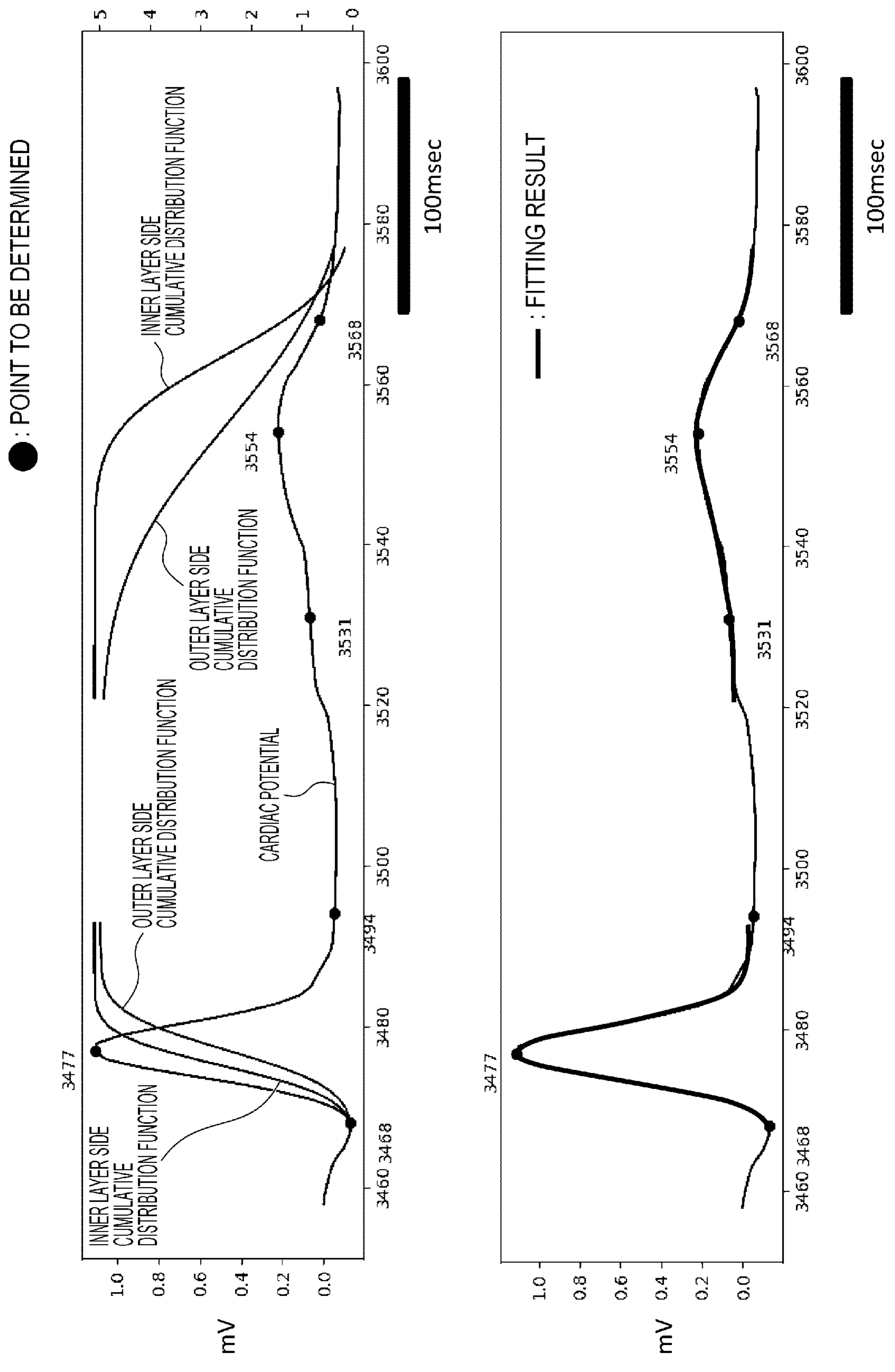
: POINT TO BE DETERMINED
INNER LAYER SIDE CUMULATIVE DISTRIBUTION FUNCTION
OUTER LAYER SIDE CUMULATIVE DISTRIBUTION FUNCTION
OUTER LAYER SIDE CUMULATIVE DISTRIBUTION FUNCTION
INNER LAYER SIDE CUMULATIVE DISTRIBUTION FUNCTION
CARDIAC POTENTIAL
mV
1.0
0.8
0.6
0.4
0.2
0.0
5
4
3
2
1
0
3460
3468
3477
3480
3494
3500
3520
3531
3540
3554
3560
3568
3580
3600
100msec
: FITTING RESULT
mV
1.0
0.8
0.6
0.4
0.2
0.0
3460
3468
3477
3480
3494
3500
3520
3531
3540
3554
3560
3568
3580
3600
100msec

FIG. 45

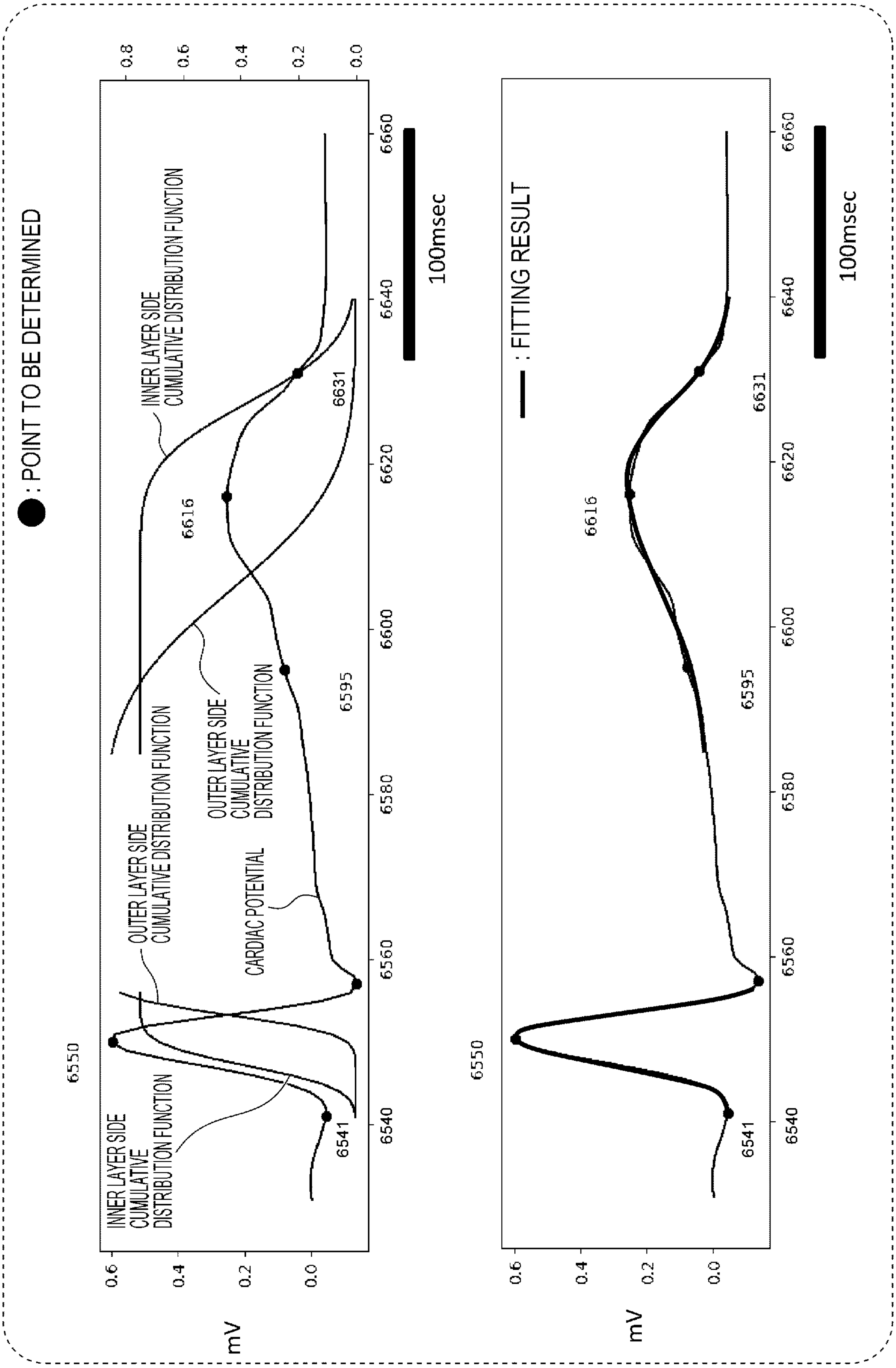

●: POINT TO BE DETERMINED
mV
0.6
0.4
0.2
0.0
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
6541
6550
6595
6616
6631
6540
6560
6580
6600
6620
6640
6660
0.8
0.6
0.4
0.2
0.0
100msec
mV
0.6
0.4
0.2
0.0
: FITTING RESULT
6541
6550
6595
6616
6631
6540
6560
6580
6600
6620
6640
6660
100msec


FIG. 46

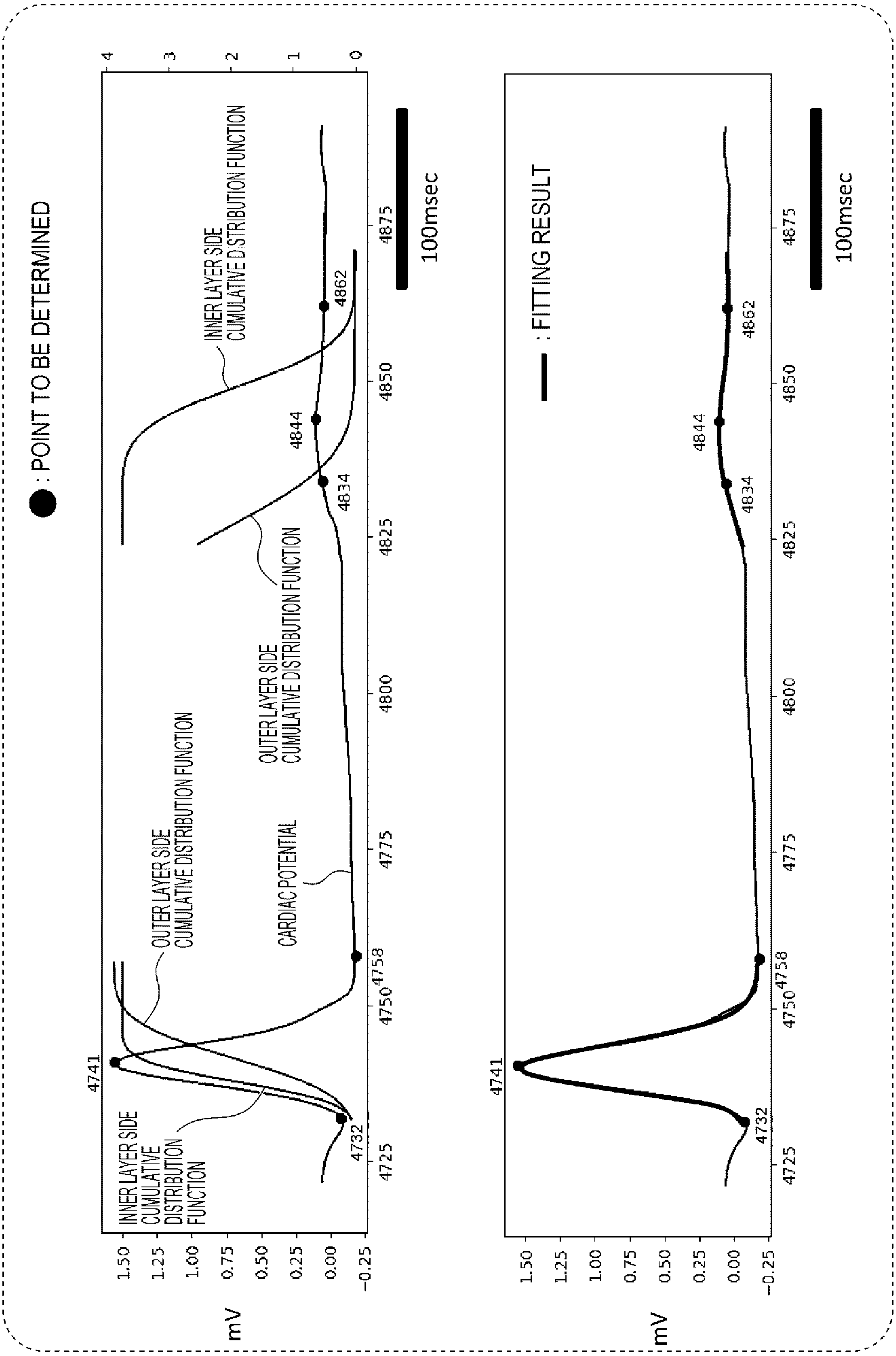
: POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
4732
4741
4758
4834
4844
4862
4725
4750
4775
4800
4825
4850
4875
100msec
mV
1.50
1.25
1.00
0.75
0.50
0.25
0.00
−0.25
0
1
2
3
4
: FITTING RESULT
4732
4741
4758
4834
4844
4862
4725
4750
4775
4800
4825
4850
4875
100msec
mV
1.50
1.25
1.00
0.75
0.50
0.25
0.00
−0.25

FIG. 47

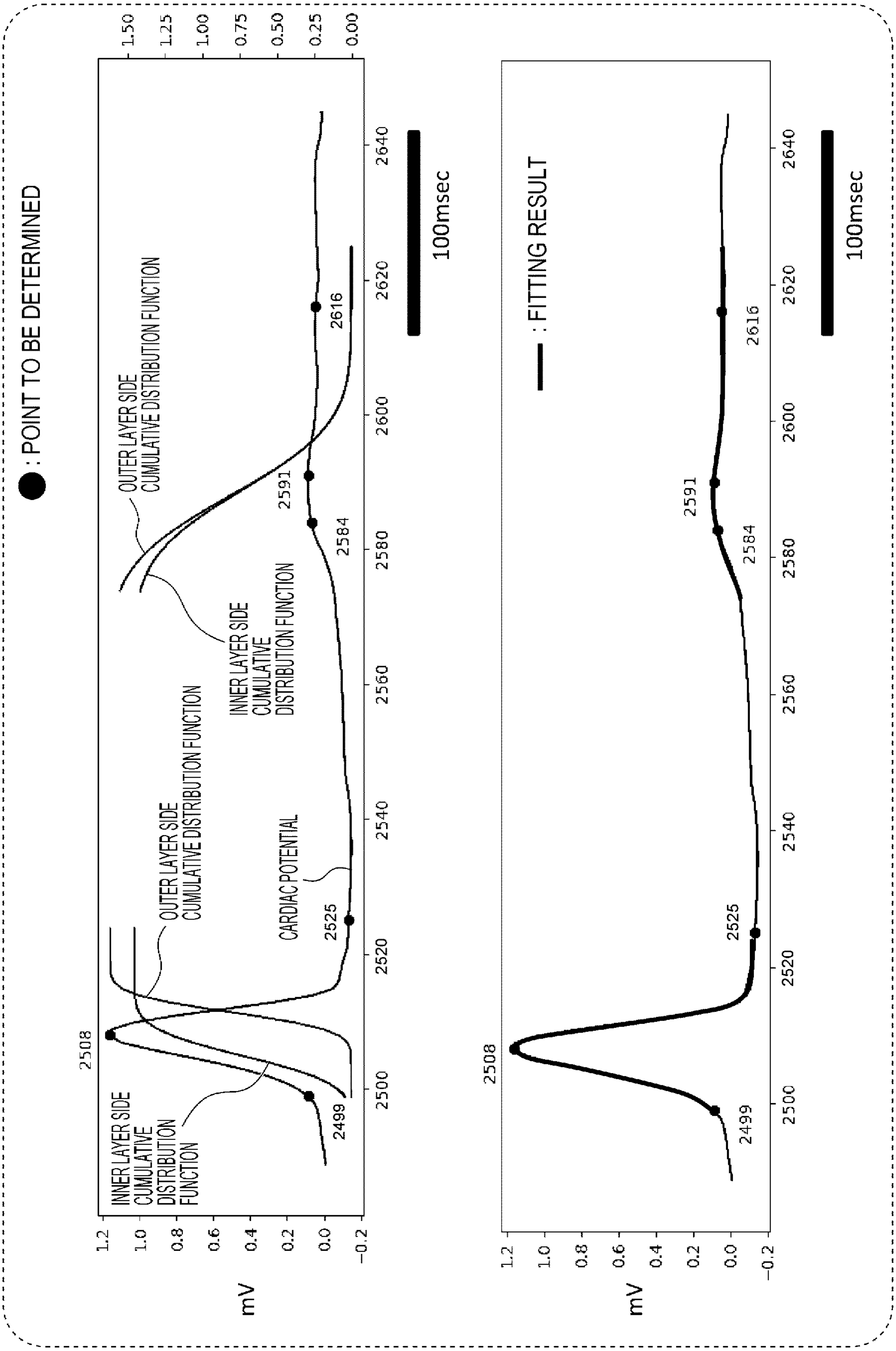
● : POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
2499
2508
2525
2584
2591
2616
mV
1.2
1.0
0.8
0.6
0.4
0.2
0.0
−0.2
1.50
1.25
1.00
0.75
0.50
0.25
0.00
2500
2520
2540
2560
2580
2600
2620
2640
100msec
: FITTING RESULT

FIG. 48

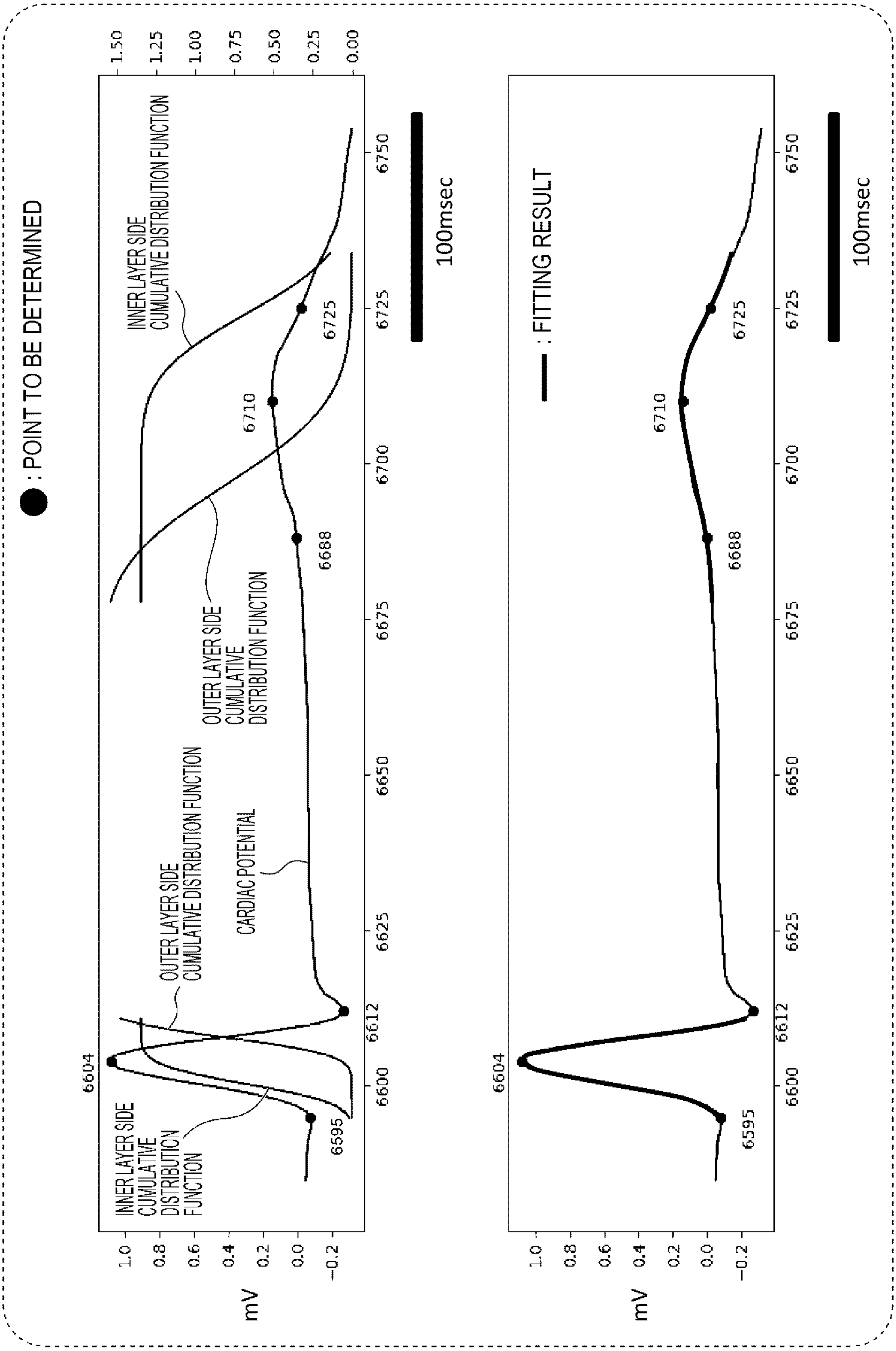
: POINT TO BE DETERMINED
mV
1.0
0.8
0.6
0.4
0.2
0.0
−0.2
1.50
1.25
1.00
0.75
0.50
0.25
0.00
6600
6625
6650
6675
6700
6725
6750
6595
6604
6612
6688
6710
6725
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
100msec
: FITTING RESULT
100msec

FIG. 49

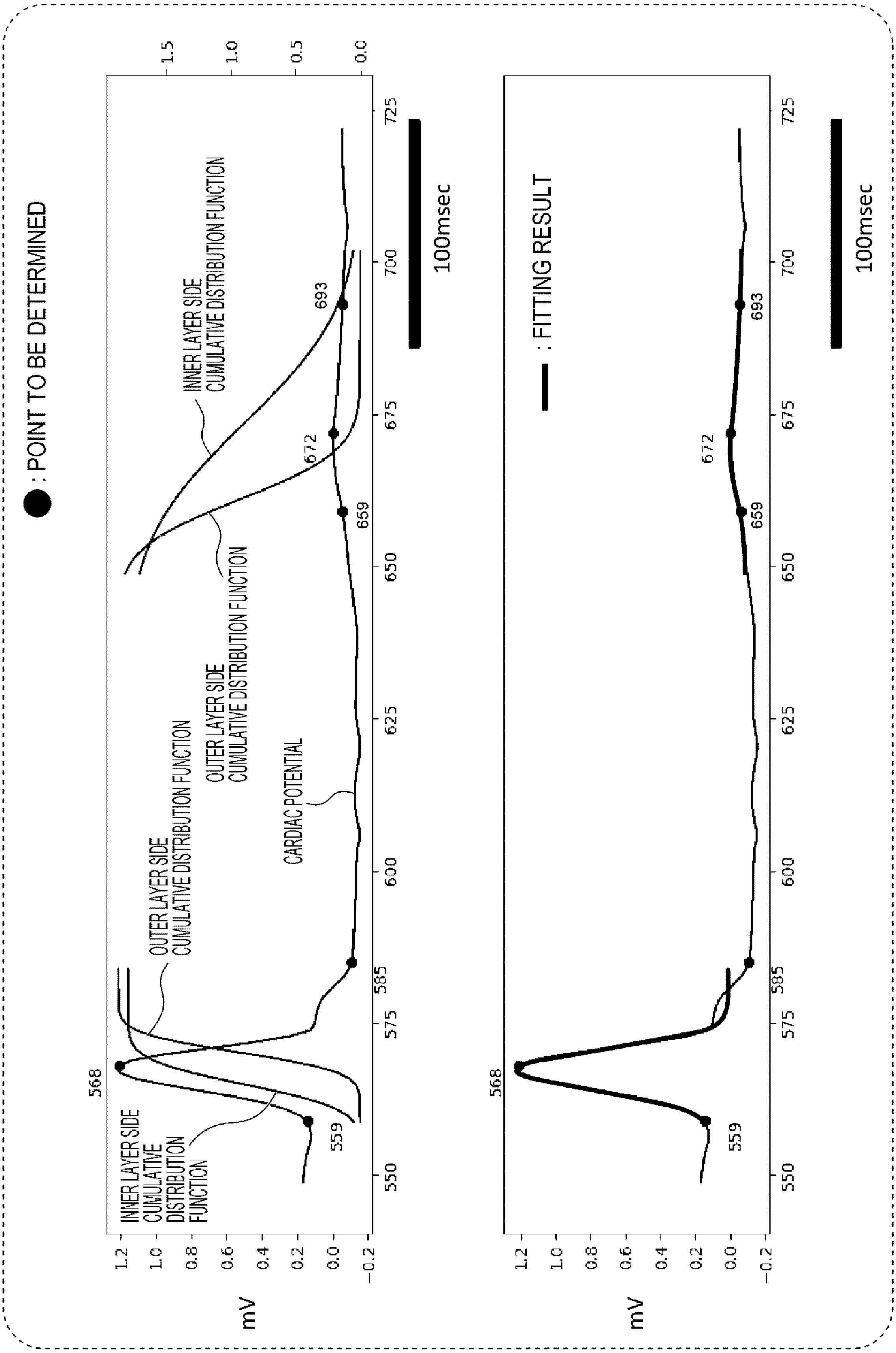
: POINT TO BE DETERMINED
INNER LAYER SIDE CUMULATIVE DISTRIBUTION FUNCTION
OUTER LAYER SIDE CUMULATIVE DISTRIBUTION FUNCTION
OUTER LAYER SIDE CUMULATIVE DISTRIBUTION FUNCTION
CARDIAC POTENTIAL
INNER LAYER SIDE CUMULATIVE DISTRIBUTION FUNCTION
INNER LAYER SIDE CUMULATIVE DISTRIBUTION FUNCTION
OUTER LAYER SIDE CUMULATIVE DISTRIBUTION FUNCTION
559
568
585
659
672
693
mV
1.2
1.0
0.8
0.6
0.4
0.2
0.0
−0.2
1.5
1.0
0.5
0.0
550
575
600
625
650
675
700
725
100msec
: FITTING RESULT
559
568
585
659
672
693
mV
100msec

FIG. 50

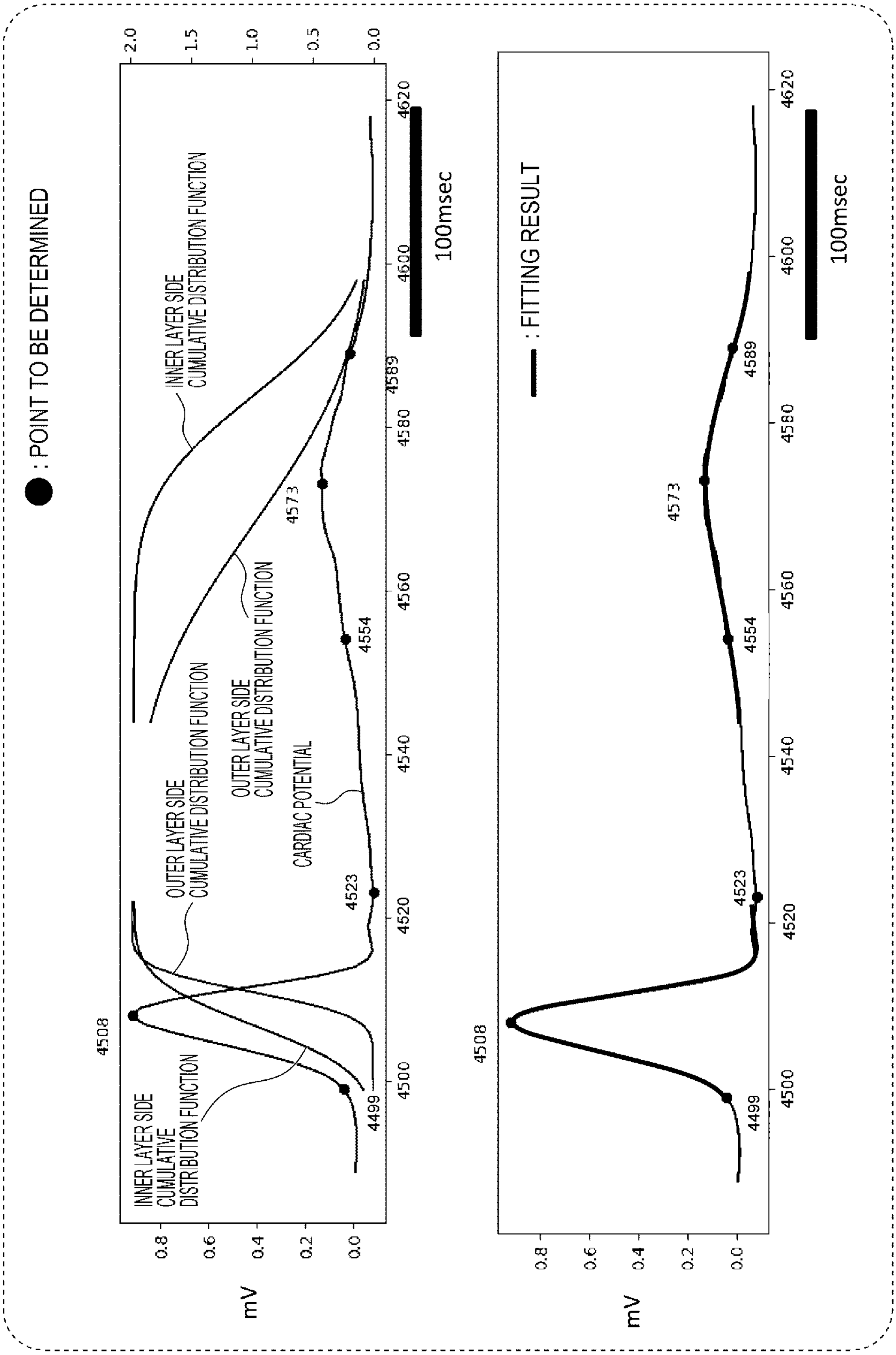
: POINT TO BE DETERMINED
INNER LAYER SIDE CUMULATIVE DISTRIBUTION FUNCTION
OUTER LAYER SIDE CUMULATIVE DISTRIBUTION FUNCTION
OUTER LAYER SIDE CUMULATIVE DISTRIBUTION FUNCTION
INNER LAYER SIDE CUMULATIVE DISTRIBUTION FUNCTION
CARDIAC POTENTIAL
mV
100msec
4499
4508
4523
4554
4573
4589
: FITTING RESULT
mV
100msec


FIG. 51

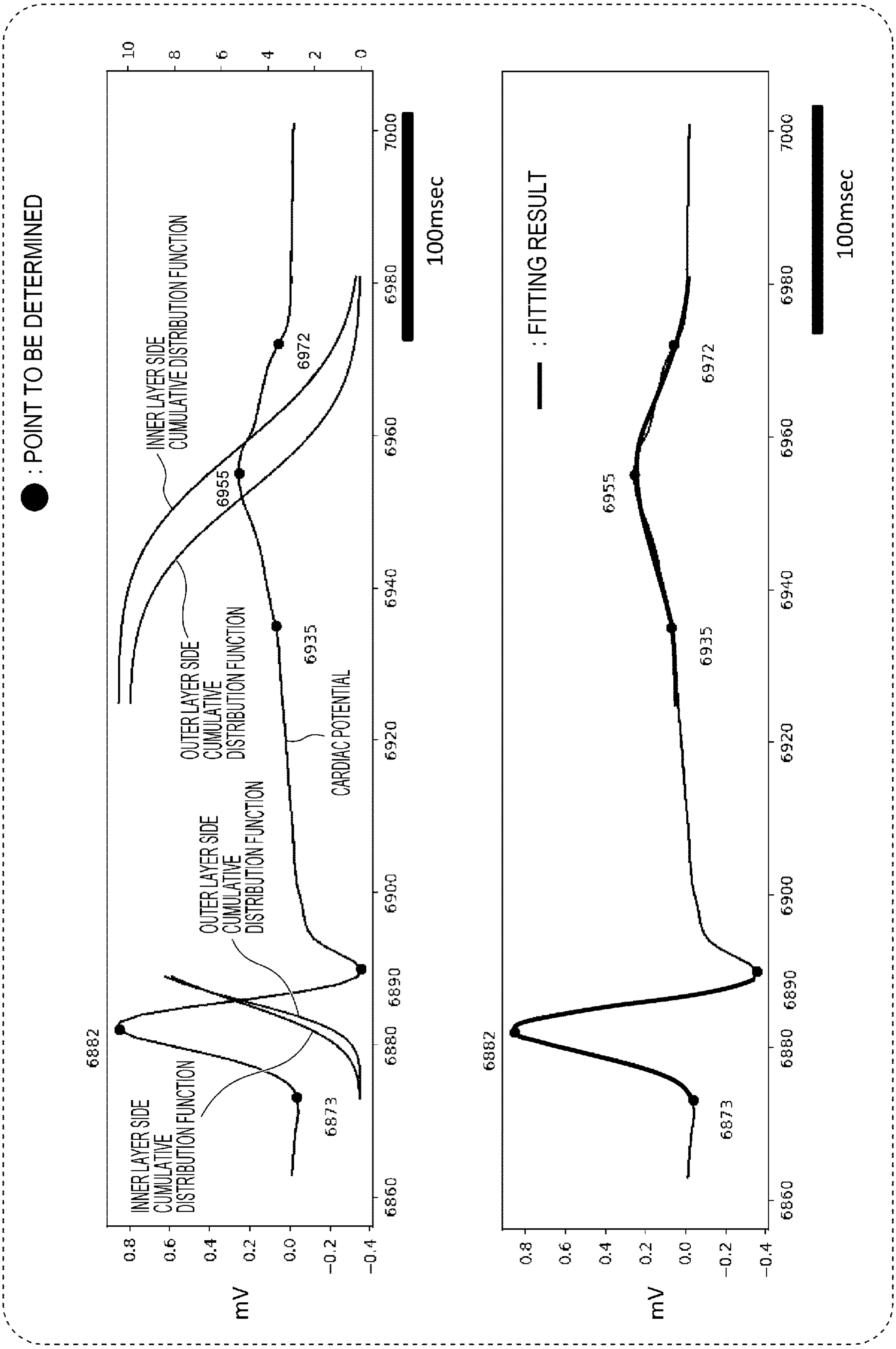
● : POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
6873
6882
6935
6955
6972
mV
100msec
— : FITTING RESULT

FIG. 52

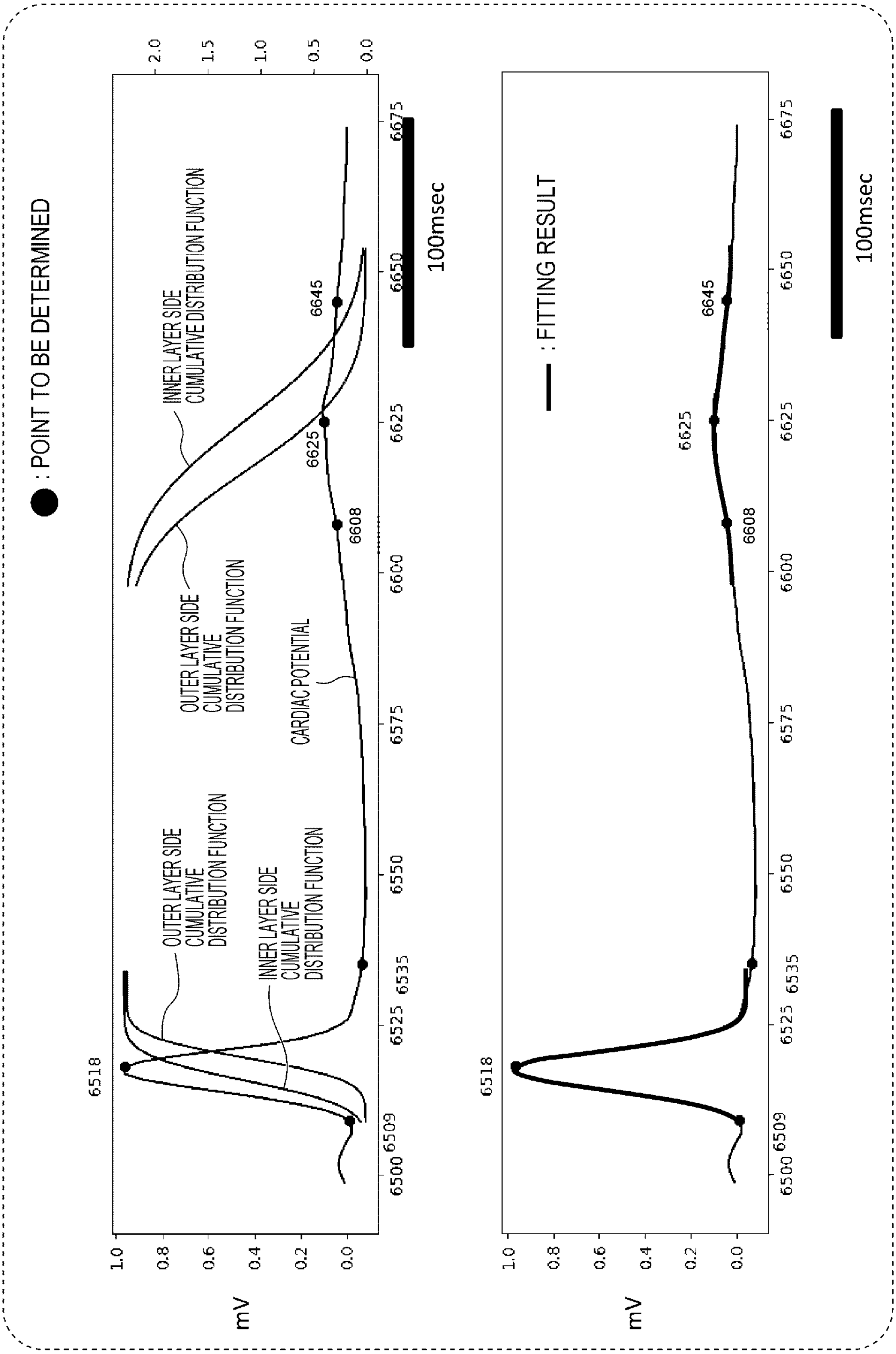
● : POINT TO BE DETERMINED
mV
1.0
0.8
0.6
0.4
0.2
0.0
2.0
1.5
1.0
0.5
0.0
6500
6509
6518
6525
6535
6550
6575
6600
6608
6625
6645
6650
6675
OUTER LAYER SIDE CUMULATIVE DISTRIBUTION FUNCTION
INNER LAYER SIDE CUMULATIVE DISTRIBUTION FUNCTION
CARDIAC POTENTIAL
100msec
: FITTING RESULT

FIG. 53

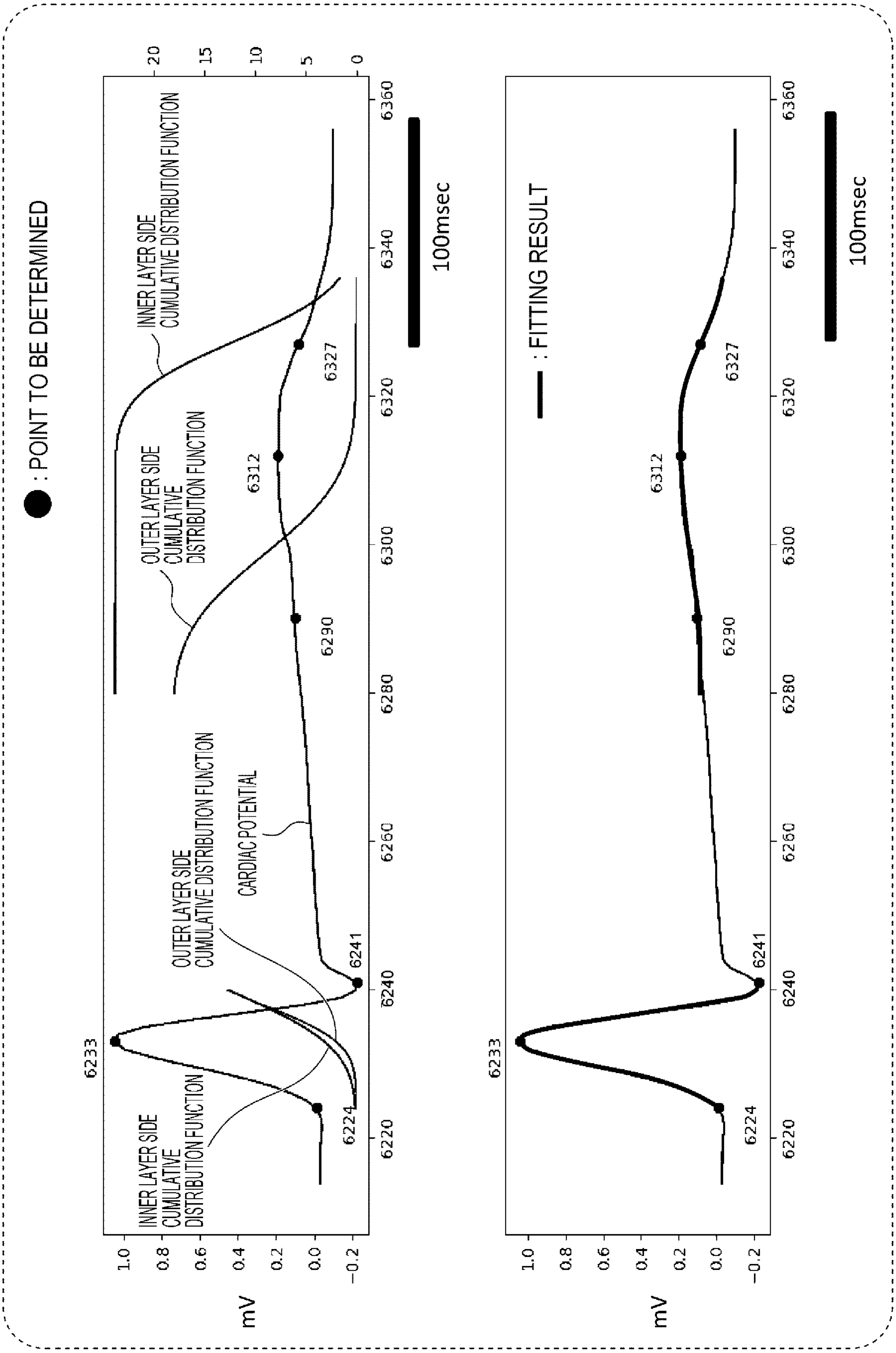

● : POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
6224
6233
6240
6241
6290
6312
6327
mV
1.0
0.8
0.6
0.4
0.2
0.0
−0.2
6220
6240
6260
6280
6300
6320
6340
6360
20
15
10
5
0
100msec
— : FITTING RESULT


FIG. 54

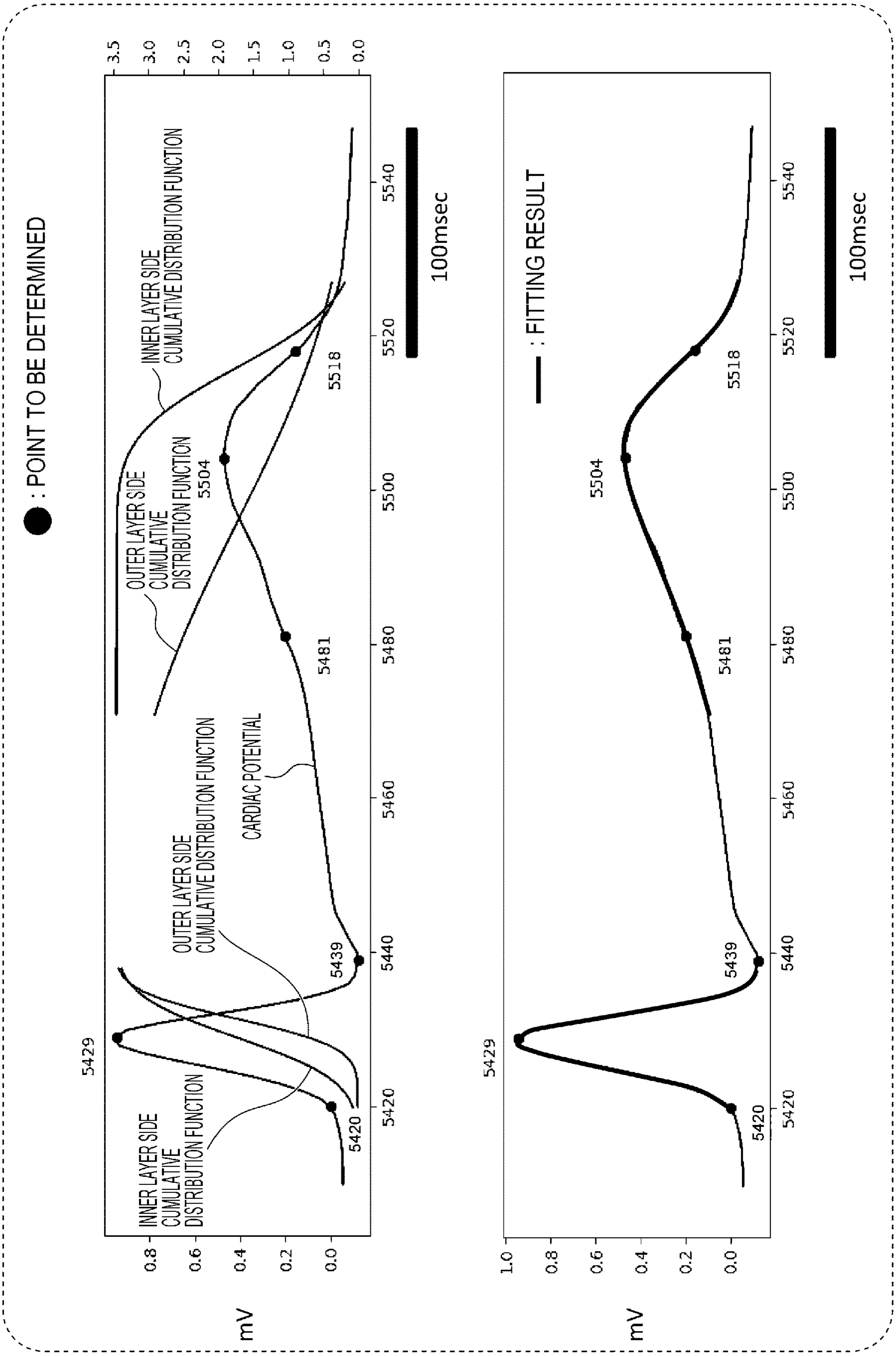
● : POINT TO BE DETERMINED
mV
0.8
0.6
0.4
0.2
0.0
3.5
3.0
2.5
2.0
1.5
1.0
0.5
0.0
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
5420
5429
5439
5481
5504
5518
5420
5440
5460
5480
5500
5520
5540
100msec
mV
1.0
0.8
0.6
0.4
0.2
0.0
: FITTING RESULT
5420
5429
5439
5481
5504
5518
5420
5440
5460
5480
5500
5520
5540
100msec

FIG. 55

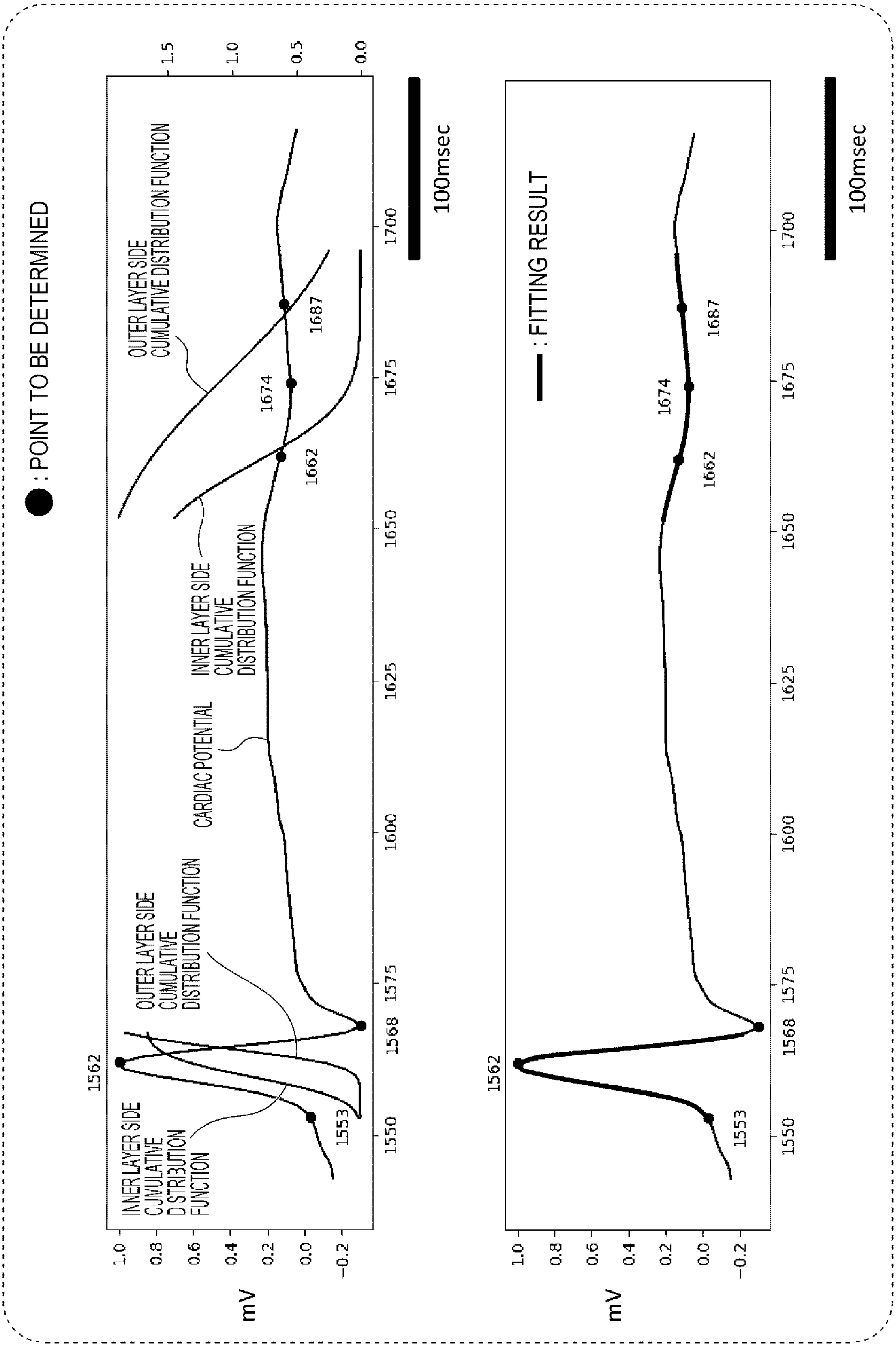
: POINT TO BE DETERMINED
OUTER LAYER SIDE CUMULATIVE DISTRIBUTION FUNCTION
INNER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
1553
1562
1568
1662
1674
1687
mV
1.0
0.8
0.6
0.4
0.2
0.0
−0.2
1.5
1.0
0.5
0.0
1550
1575
1600
1625
1650
1675
1700
100msec
: FITTING RESULT

FIG. 56

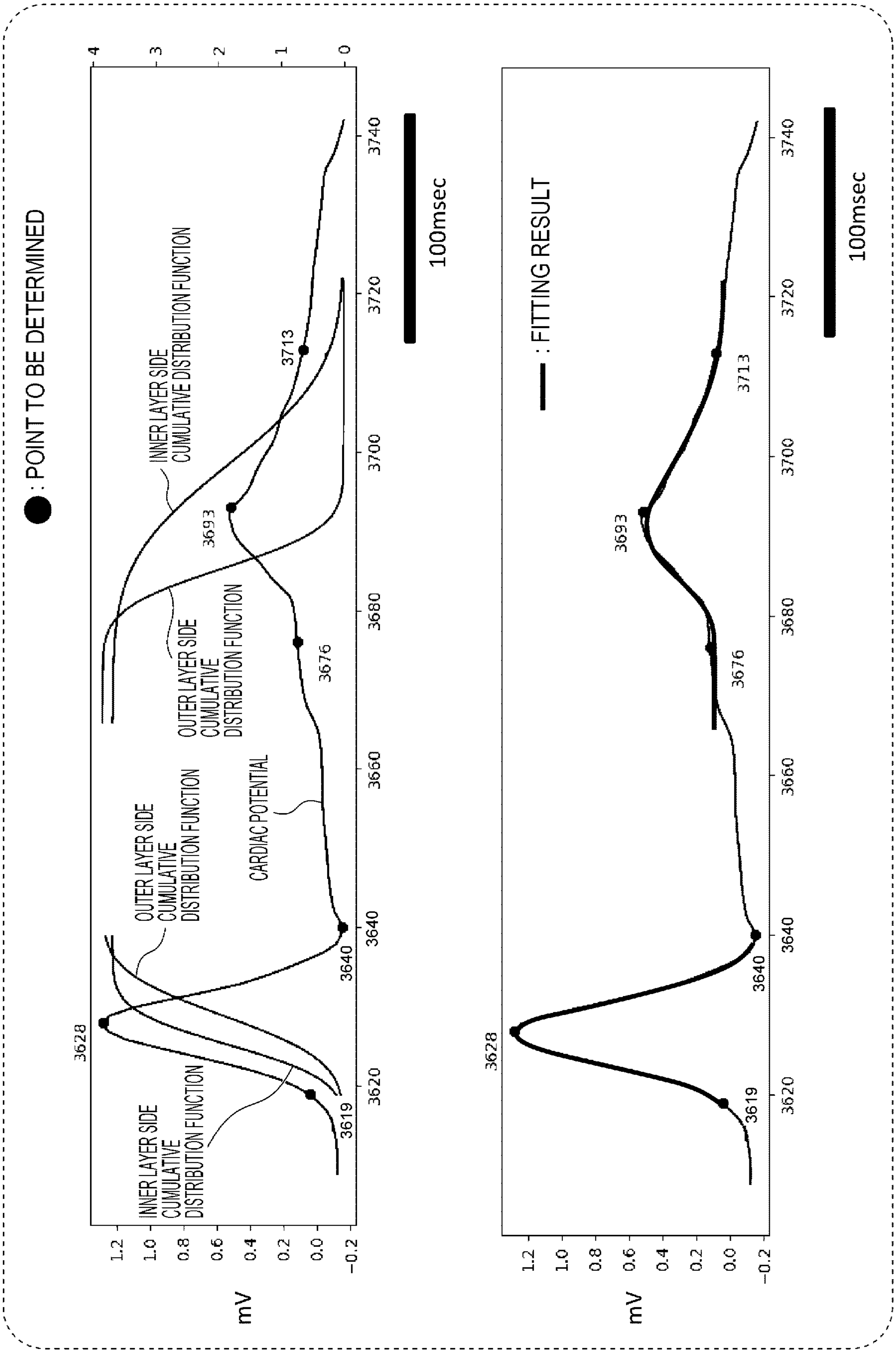
● : POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
3619
3628
3640
3676
3693
3713
mV
100msec
— : FITTING RESULT

FIG. 57

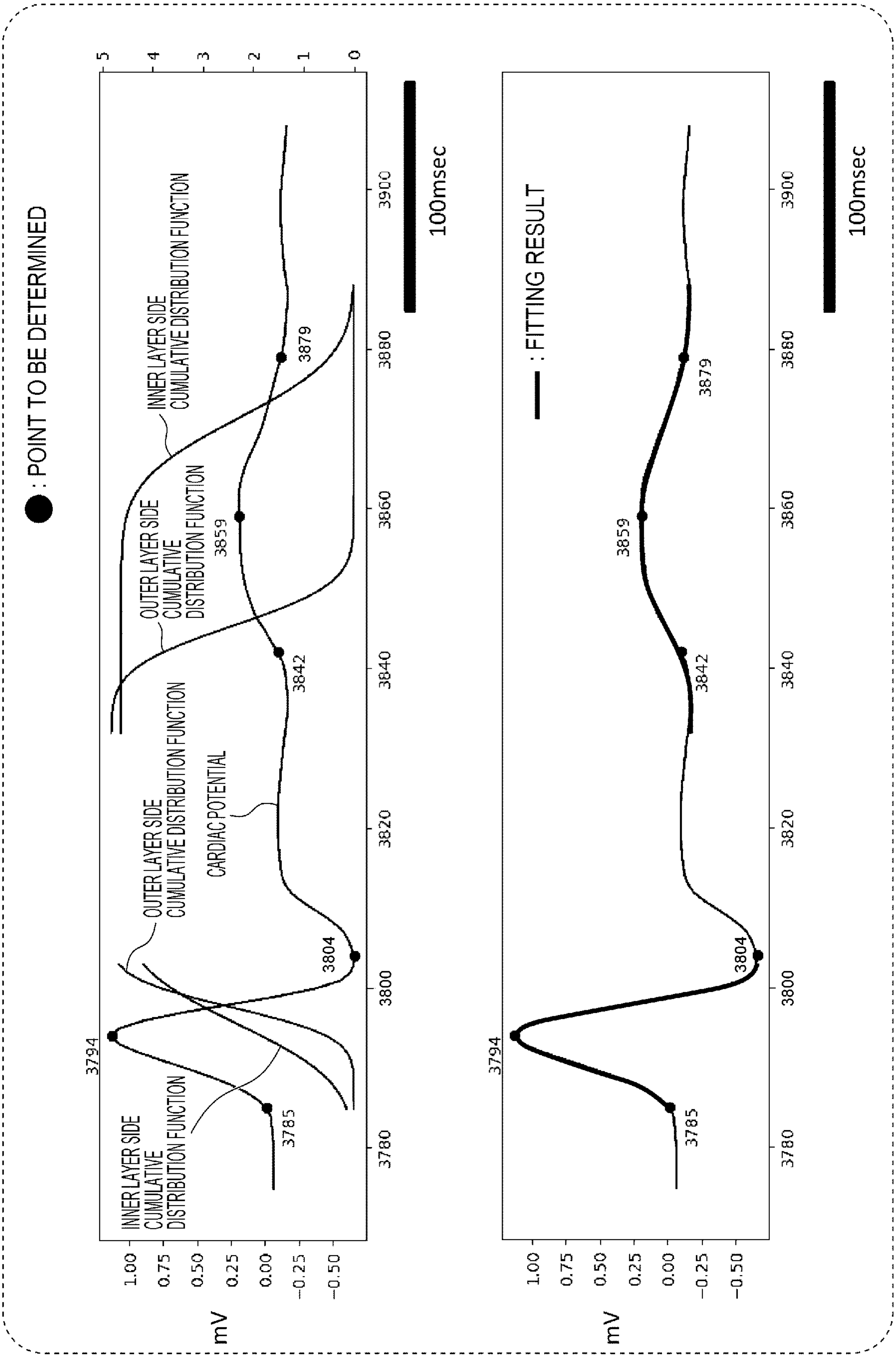

● : POINT TO BE DETERMINED
mV
1.00
0.75
0.50
0.25
0.00
−0.25
−0.50
5
4
3
2
1
0
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
3785
3794
3804
3842
3859
3879
3780
3800
3820
3840
3860
3880
3900
100msec
: FITTING RESULT
mV
1.00
0.75
0.50
0.25
0.00
−0.25
−0.50
3785
3794
3804
3842
3859
3879
3780
3800
3820
3840
3860
3880
3900
100msec


FIG. 58

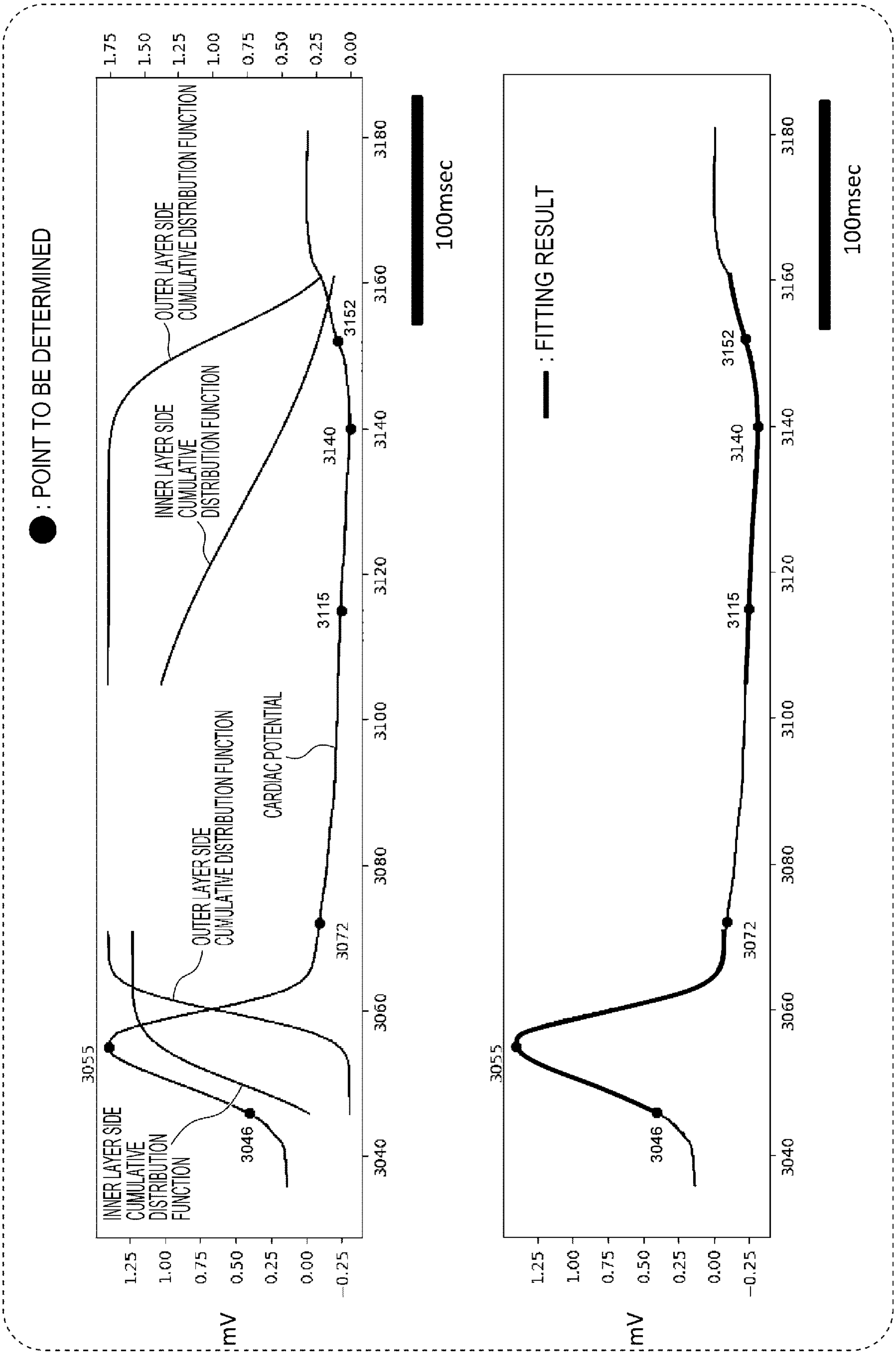
: POINT TO BE DETERMINED
INNER LAYER SIDE CUMULATIVE DISTRIBUTION FUNCTION
OUTER LAYER SIDE CUMULATIVE DISTRIBUTION FUNCTION
CARDIAC POTENTIAL
OUTER LAYER SIDE CUMULATIVE DISTRIBUTION FUNCTION
INNER LAYER SIDE CUMULATIVE DISTRIBUTION FUNCTION
3046
3055
3072
3115
3140
3152
mV
1.25
1.00
0.75
0.50
0.25
0.00
−0.25
1.75
1.50
3040
3060
3080
3100
3120
3140
3160
3180
100msec
: FITTING RESULT

FIG. 59

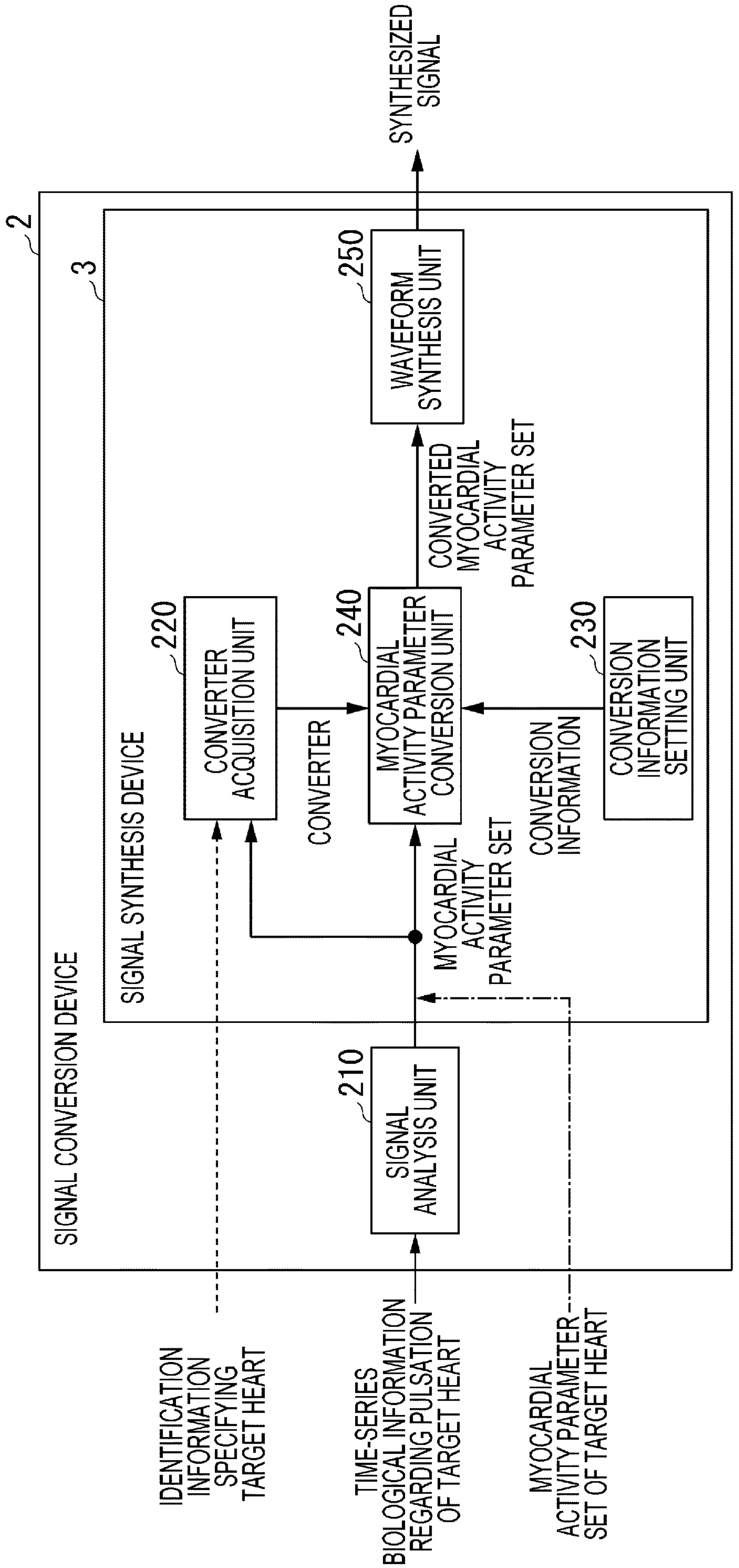
SIGNAL CONVERSION DEVICE
2
SIGNAL SYNTHESIS DEVICE
3
210
SIGNAL ANALYSIS UNIT
220
CONVERTER ACQUISITION UNIT
230
CONVERSION INFORMATION SETTING UNIT
240
MYOCARDIAL ACTIVITY PARAMETER CONVERSION UNIT
250
WAVEFORM SYNTHESIS UNIT
CONVERTER
CONVERSION INFORMATION
MYOCARDIAL ACTIVITY PARAMETER SET
CONVERTED MYOCARDIAL ACTIVITY PARAMETER SET
SYNTHESIZED SIGNAL
IDENTIFICATION INFORMATION SPECIFYING TARGET HEART
TIME-SERIES BIOLOGICAL INFORMATION REGARDING PULSATION OF TARGET HEART
MYOCARDIAL ACTIVITY PARAMETER SET OF TARGET HEART

FIG. 60

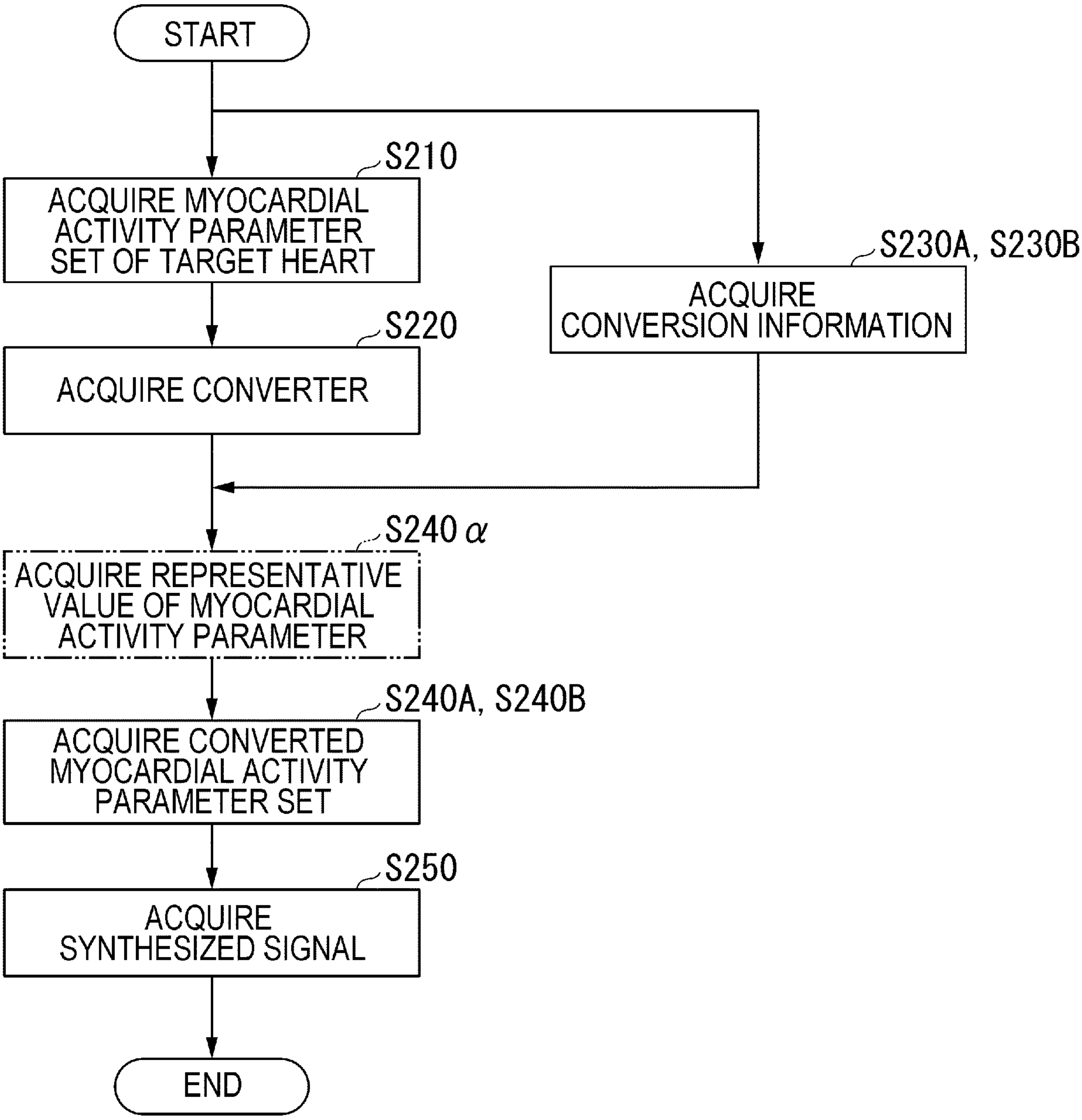
START
S210
ACQUIRE MYOCARDIAL ACTIVITY PARAMETER SET OF TARGET HEART
S220
ACQUIRE CONVERTER
S230A, S230B
ACQUIRE CONVERSION INFORMATION
S240α
ACQUIRE REPRESENTATIVE VALUE OF MYOCARDIAL ACTIVITY PARAMETER
S240A, S240B
ACQUIRE CONVERTED MYOCARDIAL ACTIVITY PARAMETER SET
S250
ACQUIRE SYNTHESIZED SIGNAL
END

FIG. 61

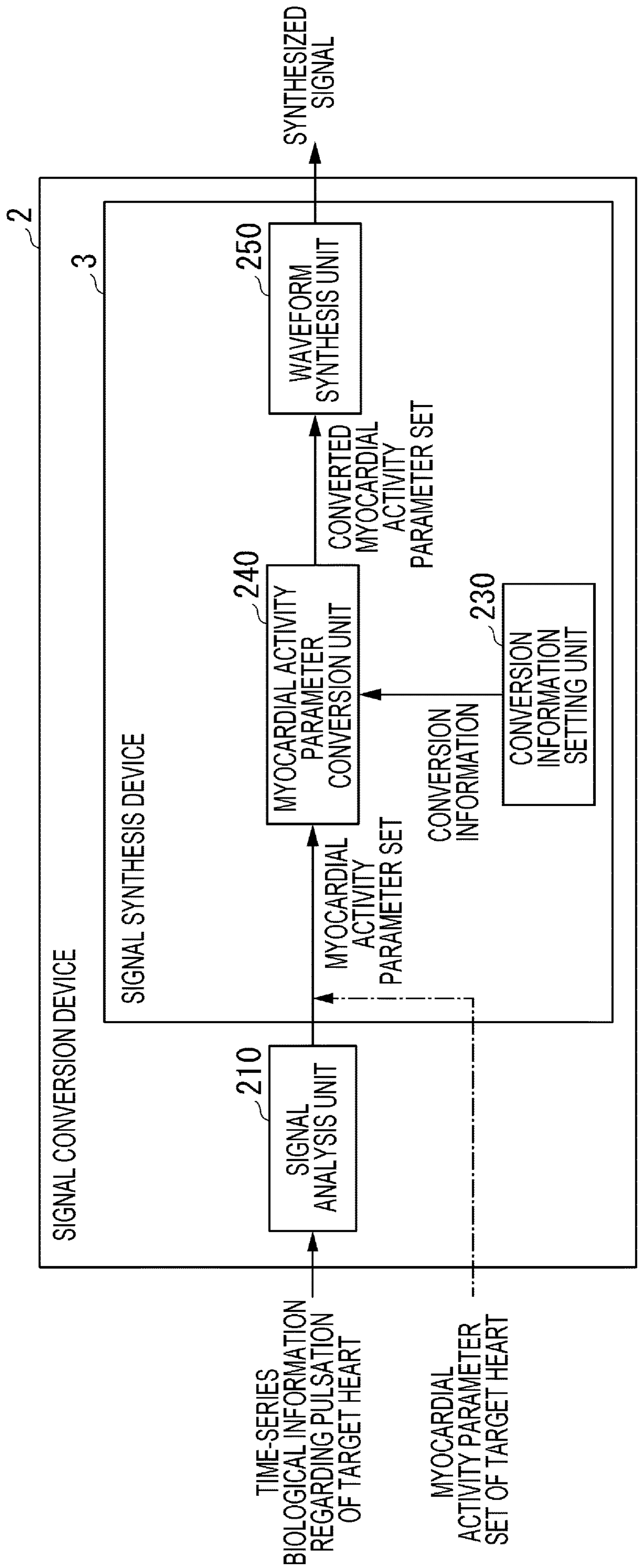

2
SIGNAL CONVERSION DEVICE
3
SIGNAL SYNTHESIS DEVICE
TIME-SERIES BIOLOGICAL INFORMATION REGARDING PULSATION OF TARGET HEART
MYOCARDIAL ACTIVITY PARAMETER SET OF TARGET HEART
210
SIGNAL ANALYSIS UNIT
MYOCARDIAL ACTIVITY PARAMETER SET
240
MYOCARDIAL ACTIVITY PARAMETER CONVERSION UNIT
CONVERSION INFORMATION
230
CONVERSION INFORMATION SETTING UNIT
CONVERTED MYOCARDIAL ACTIVITY PARAMETER SET
250
WAVEFORM SYNTHESIS UNIT
SYNTHESIZED SIGNAL


FIG. 62

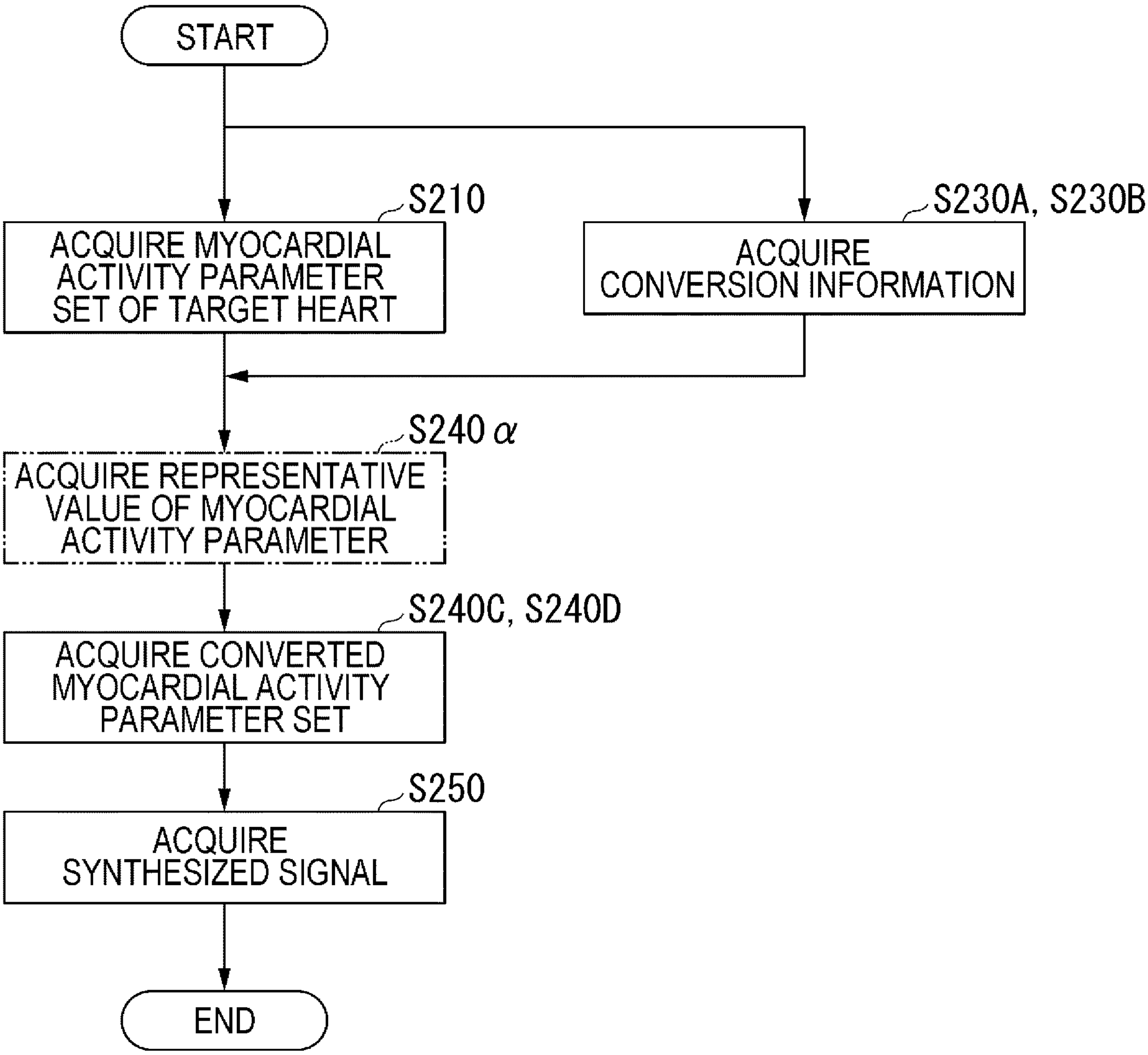
START
S210
ACQUIRE MYOCARDIAL ACTIVITY PARAMETER SET OF TARGET HEART
S230A, S230B
ACQUIRE CONVERSION INFORMATION
S240α
ACQUIRE REPRESENTATIVE VALUE OF MYOCARDIAL ACTIVITY PARAMETER
S240C, S240D
ACQUIRE CONVERTED MYOCARDIAL ACTIVITY PARAMETER SET
S250
ACQUIRE SYNTHESIZED SIGNAL
END

FIG. 63

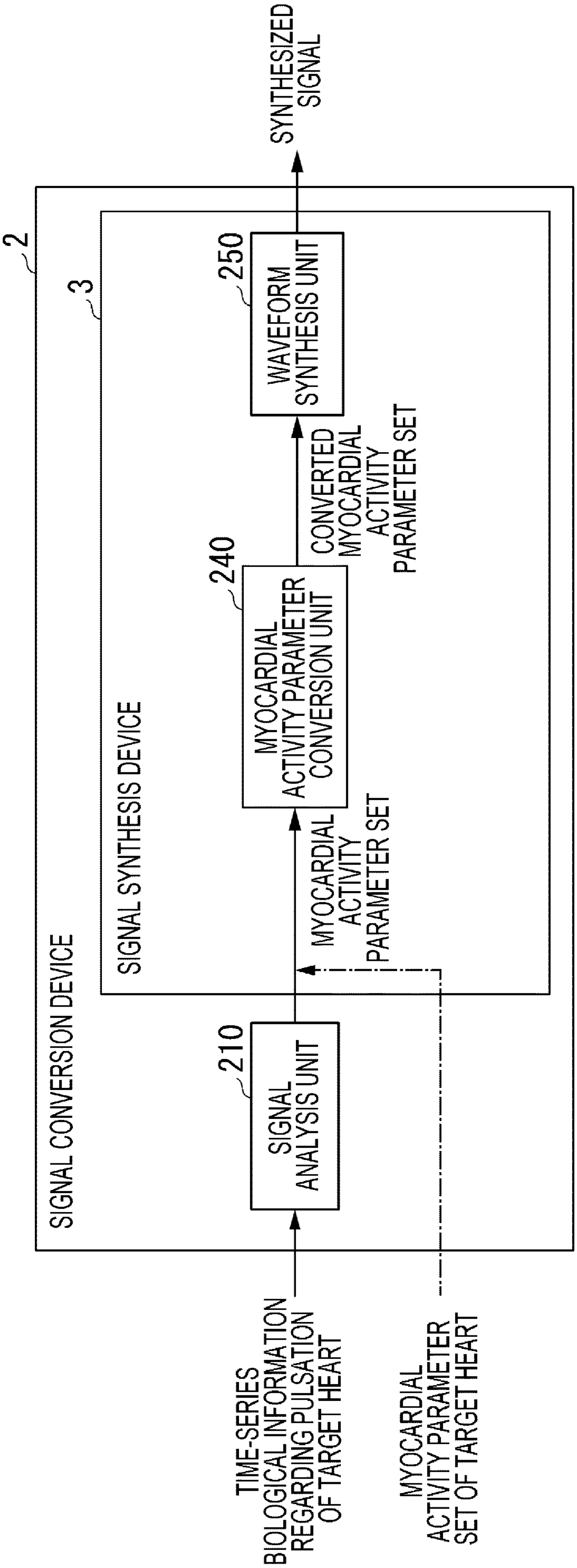
2
SIGNAL CONVERSION DEVICE
3
SIGNAL SYNTHESIS DEVICE
210
SIGNAL ANALYSIS UNIT
240
MYOCARDIAL ACTIVITY PARAMETER CONVERSION UNIT
250
WAVEFORM SYNTHESIS UNIT
TIME-SERIES BIOLOGICAL INFORMATION REGARDING PULSATION OF TARGET HEART
MYOCARDIAL ACTIVITY PARAMETER SET OF TARGET HEART
MYOCARDIAL ACTIVITY PARAMETER SET
CONVERTED MYOCARDIAL ACTIVITY PARAMETER SET
SYNTHESIZED SIGNAL

FIG. 64

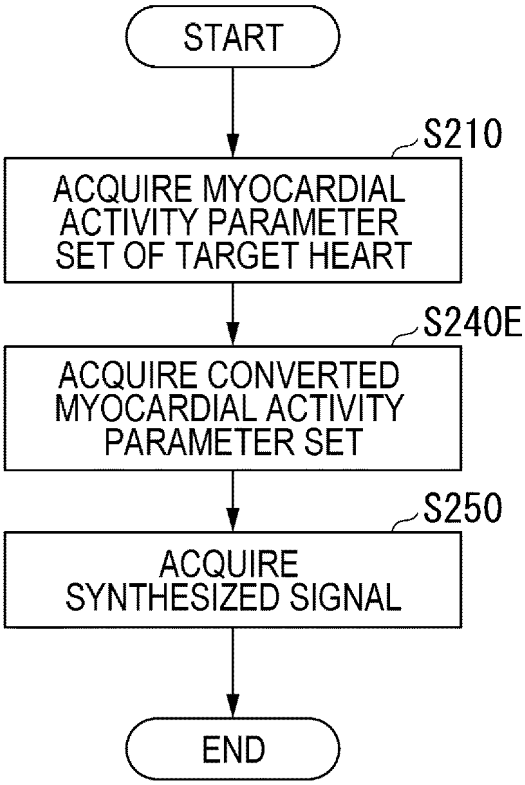
START
S210
ACQUIRE MYOCARDIAL ACTIVITY PARAMETER SET OF TARGET HEART
S240E
ACQUIRE CONVERTED MYOCARDIAL ACTIVITY PARAMETER SET
S250
ACQUIRE SYNTHESIZED SIGNAL
END

FIG. 65

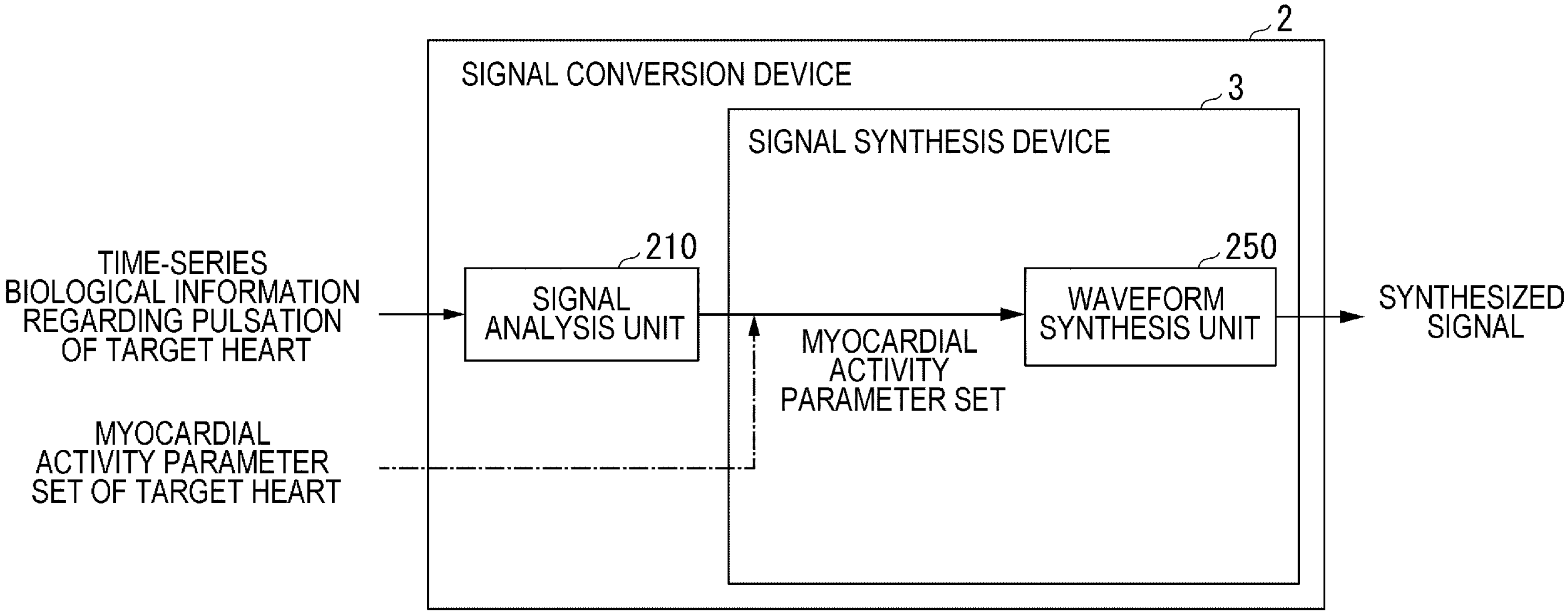
TIME-SERIES
BIOLOGICAL INFORMATION
REGARDING PULSATION
OF TARGET HEART
MYOCARDIAL
ACTIVITY PARAMETER
SET OF TARGET HEART
SIGNAL CONVERSION DEVICE
2
SIGNAL SYNTHESIS DEVICE
3
210
SIGNAL
ANALYSIS UNIT
MYOCARDIAL
ACTIVITY
PARAMETER SET
250
WAVEFORM
SYNTHESIS UNIT
SYNTHESIZED
SIGNAL

FIG. 66

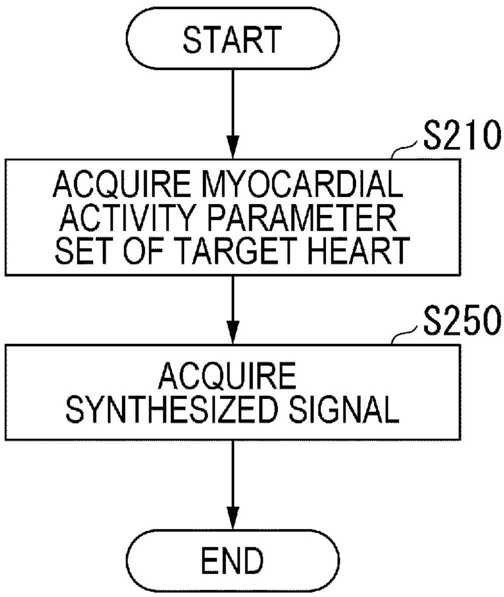
START
S210
ACQUIRE MYOCARDIAL ACTIVITY PARAMETER SET OF TARGET HEART
S250
ACQUIRE SYNTHESIZED SIGNAL
END

FIG. 67

**INTERNATIONAL SEARCH REPORT**

International application No. **PCT/JP2022/024370**

**A. CLASSIFICATION OF SUBJECT MATTER**

***A61B 5/346***(2021.01)i
FI: A61B5/346

According to International Patent Classification (IPC) or to both national classification and IPC

**B. FIELDS SEARCHED**

Minimum documentation searched (classification system followed by classification symbols)

A61B5/24-5/398; A61B5/02-5/03

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2004-160237 A (ELA MEDICAL SOCIETE ANONYME) 10 June 2004 (2004-06-10) paragraphs [0019]-[0029], fig. 1-4 | 1-9 |
| A | US 2012/0302903 A1 (SIEMENS MEDICAL SOLUTIONS USA, INC.) 29 November 2012 (2012-11-29) paragraphs [0017]-[0048], fig. 1-9 | 1-9 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 August 2022** | **30 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/024370**

| Patent document cited in search report | Publication date (day/month/year) | Patent family member(s) | Publication date (day/month/year) |
|---|---|---|---|
| JP 2004-160237 A | 10 June 2004 | US 2004/0162495 A1<br>paragraphs [0033]-[0057], fig. 1-4<br>EP 1419733 A1<br>FR 2847361 A1 | |
| US 2012/0302903 A1 | 29 November 2012 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HIROSHI TANAKA**. On the Inverse Solution of Electrocardiology. *Medical Electronics and Biological Engineering*, 1985, vol. 23 (3), 147-158
- **YOSHIFUMI TANAKA**. Understanding electrocardiogram waveforms from their origins, Interpreting myocardial action potentials. Gakken Medical Shujunsha Co., Ltd., 2012